(19) Europäisches Patentamt
European Patent Office
Office européen des brevets

(11) EP 4 556 027 A1

(12) **EUROPEAN PATENT APPLICATION**
published in accordance with Art. 153(4) EPC

(43) Date of publication:
**21.05.2025 Bulletin 2025/21**

(21) Application number: **23839031.4**

(22) Date of filing: **13.07.2023**

(51) International Patent Classification (IPC):
***A61K 47/68*** *(2017.01)* ***A61K 47/65*** *(2017.01)*
***C07K 16/28*** *(2006.01)* ***A61P 35/00*** *(2006.01)*

(52) Cooperative Patent Classification (CPC):
**A61K 47/65; A61K 47/68; A61P 35/00; C07K 16/28**

(86) International application number:
**PCT/CN2023/107301**

(87) International publication number:
**WO 2024/012541 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.07.2022 CN 202210827914**

(71) Applicant: **Bio-Thera Solutions, Ltd.**
**Guangzhou, Guangdong 510530 (CN)**

(72) Inventors:
- **MEI, Xingxing**
  **Guangzhou, Guangdong 510530 (CN)**
- **FENG, Cuiying**
  **Guangzhou, Guangdong 510530 (CN)**
- **MAI, Siqi**
  **Guangzhou, Guangdong 510530 (CN)**
- **TANG, Weijia**
  **Guangzhou, Guangdong 510530 (CN)**
- **QI, Yao**
  **Guangzhou, Guangdong 510530 (CN)**
- **ZHOU, Jiaqi**
  **Guangzhou, Guangdong 510530 (CN)**
- **YU, Jin-Chen**
  **Guangzhou, Guangdong 510530 (CN)**
- **LI, Shengfeng**
  **Guangzhou, Guangdong 510530 (CN)**

(74) Representative: **Altmann Stößel Dick Patentanwälte PartG mbB**
**Theodor-Heuss-Anlage 2**
**68165 Mannheim (DE)**

Remarks:
The complete document including Reference Table(s) and the Sequence Listing(s) can be downloaded from the EPO website

# (54) ANTI-NECTIN-4 ANTIBODY DRUG CONJUGATE AND USE THEREOF

(57) An anti-Nectin-4 antibody-drug conjugate and use thereof. The anti-Nectin-4 antibody and the antibody-drug conjugate thereof can be used for preventing and/or treating diseases.

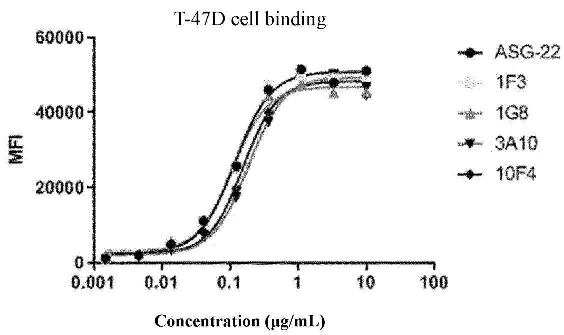


FIG. 2

EP 4 556 027 A1

## Description

TECHNICAL FIELD

The present disclosure relates to the field of biopharmaceuticals, and particularly, to an antibody-drug conjugate and use thereof.

BACKGROUND

Nectin-4 is one of the cell adhesion molecules (CAMs) in the immunoglobulin superfamily (IgSF). It is a calcium-independent cell adhesion molecule, and is also known as poliovirus receptor-like protein 4 (PVRL4) or poliovirus receptor-related 4 (PRR4). Nectin-4 is a single-pass transmembrane protein comprising an extracellular region, a transmembrane region, and an intracellular region, where the extracellular region contains three highly glycosylated domains, namely two C2 domains proximal to the cell membrane and one V domain distal to the cell membrane. The Nectin-4 molecule binds to H protein through the V domain at the N-terminal of the Nectin-4 molecule, thereby achieving the infection of a virus to cells. Nectin-4 is involved in the establishment and maintenance of adhering junctions by interacting with cadherin. Nectin-4 mediates Ca2+-independent adhesion, and promotes anchorage-independent growth by driving intercellular adhesion and activating matrix-independent integrin β4/SHP-2/c-Src.

Nectin-4 is expressed during fetal development, but unlike the widespread expression of other Nectins in adult tissues, its expression in adult tissues is significantly reduced. Several institutions have confirmed that Nectin-4 is overexpressed in many tumors, including breast cancer, bladder cancer, and the like. Nectin-4 has low or moderate expression in the stratum corneum of skin, skin appendages (sweat glands and hair follicles), transitional epithelium of bladder, salivary glands, esophagus, mammary glands, and stomach, and low expression in the larynx, pituitary, placenta, testis, ureter, and uterus among normal adult tissues. The target has good specificity and can mediate the endocytosis of antibodies, thus becoming a potential therapeutic target for ADC drugs.

SUMMARY

The present application provides drug conjugate containing the antibody, antigen-binding unit or polypeptide that specifically recognizes and binds to Nectin-4. In one or more embodiments, the antibody, antigen-binding unit or polypeptide of the present disclosure specifically recognizes and binds to human Nectin-4.

In one or more embodiments, the anti-Nectin-4 antibody or antigen-binding unit comprises one or more of the following HCDR1, HCDR2 and HCDR3, or variants thereof: according to the Kabat numbering system, the HCDR1 sequence comprising or consisting of the sequence set forth in any one of SEQ ID NO: 8, SEQ ID NOs: 181-195, SEQ ID NOs: 234-248, and SEQ ID NOs: 304-317, the HCDR2 sequence comprising or consisting of the sequence set forth in any one of SEQ ID NO: 9, SEQ ID NOs: 196-231, SEQ ID NOs: 251-303, and SEQ ID NOs: 318-358, and the HCDR3 sequence comprising or consisting of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24.

In one or more embodiments, the anti-Nectin-4 antibody or antigen-binding unit comprises one or more of the HCDR1, HCDR2 and HCDR3, or variants thereof contained in the heavy chain variable region set forth in any one of SEQ ID NO:7, SEQ ID NO: 11, SEQ ID NO: 13, SEQ ID NO:15, SEQ ID NO: 17, SEQ ID NO: 19, SEQ ID NO:21 and SEQ ID NO:23.

In one or more embodiments, the anti-Nectin-4 antibody or antigen-binding unit comprises one or more of the following HCDR1, HCDR2 and HCDR3, or variants thereof : according to the Kabat numbering system, the HCDR1 sequence comprising or consisting of the sequence set forth in SEQ ID NO: 8, the HCDR2 sequence comprising or consisting of the sequence set forth in SEQ ID NO: 9, and the HCDR3 sequence comprising or consisting of the sequence set forth in SEQ ID NO: 10.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO: 11. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 12.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO: 13. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 14.

In one or more embodiments, the anti-Nectin-4 antibody or antigen-binding unit comprises one or more of the

HCDR1, HCDR2 and HCDR3, or variants thereof contained in the heavy chain variable region set forth in SEQ ID NO: 15. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprising or consisting of the sequence set forth in SEQ ID NO: 16.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO: 17. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO: 19. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO:21. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22.

In one or more embodiments, the anti-Nectin-4 antibody or antigen-binding unit comprises one or more of the HCDR1, HCDR2 and HCDR3, or variants thereof contained in the heavy chain variable region set forth in SEQ ID NO:23. In one or more embodiments, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24.

In one or more embodiments, the anti-Nectin-4 antibody or antigen-binding unit comprises one or more of the following HCDR1, HCDR2 and HCDR3, or variants thereof: the HCDR1 sequence comprising or consisting of the sequence set forth in X1X2X3MS, the HCDR2 sequence comprising or consisting of the sequence set forth in X1'IX2'X3'X4'X5'X6'X7'X8'X9'YADSVKG (SEQ ID NO: 368), and the HCDR3 sequence comprising or consisting of the sequence set forth in SEQ ID NO: 12, 14, or 16, wherein X1, X2, X3, X1', X2', X3', X4', X5', X6', X7', X8', X9', and X10' denote amino acids independently selected from any one of G, A, V, L, I, S, T, C, M, N, Q, K, R, D, E, F, Y, H, P and W.

In one or more embodiments, X1 denotes S, N, T, D, or G, X2 denotes Y, F, or S, X3 denotes A, G, S, or Y, X1' denotes A, R, G, I, or W, X2' denotes S, K, Y, or D, X3' denotes G, P, S, A, Q, or W, X4' denotes S, T, G, Y, H, D, W, or I, X5' denotes G, D, T, S, A, or K, X6' denotes G, S, D, A, or W, X7' denotes S, Y, T, N, D, V, E, or G, X8' denotes T, A, N, K, I, R, S, or P, and X9' denotes Y, S, H, R, N, G, F, or D.

In one or more embodiments, X1 denotes S, N, T, D, or G, X2 denotes Y, N, F, or S, X3 denotes A, G, S, or absence, X1' denotes A, R, G, W, or S, X2' denotes S, K, Y, or D, X3' denotes G, P, S, T, A, Q, or Y, X4' denotes S, T, G, Y, H, D, W, or I, X5' denotes G, D, T, S, F, or K, X6' denotes G, S, D, A, W, or absence, X7' denotes S, Y, T, N, D, V, E, or G, X8' denotes T, A, N, K, I, R, S, or P, and X9' denotes Y, S, H, R, N, F, or D.

In one or more embodiments, X1 denotes S, N, D, G, or T, X2 denotes Y, F, or S, X3 denotes A, D, W, S, or Y, X1' denotes A, S, G, or V, X2' denotes S, K, I, Y, or D, X3' denotes G, P, S, A, Q, Y, T, or D, X4' denotes S, T, G, Y, H, D, or W, X5' denotes G, D, T, S, or K, X6' denotes G, S, D, A, or Y, X7' denotes S, Y, T, N, D, V, or G, X8' denotes T, A, N, K, I, R, or S, and X9' denotes Y, S, H, R, N, G, F, or D.

In one or more embodiments, the anti-Nectin-4 antibody or antigen-binding unit comprises one or more of the following HCDR1, HCDR2 and HCDR3, or variants thereof: the HCDR1 sequence comprising or consisting of the sequence set forth in any one of SEQ ID NO: 8 and SEQ ID NOs: 181-195, the HCDR2 sequence comprising or consisting of the sequence set forth in any one of SEQ ID NO: 9 and SEQ ID NOs: 196-231, and the HCDR3 sequence comprising or consisting of the sequence set forth in SEQ ID NO: 14.

In one or more embodiments, the anti-Nectin-4 antibody or antigen-binding unit comprises one or more of the following HCDR1, HCDR2 and HCDR3, or variants thereof: the HCDR1 sequence comprising or consisting of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprising or consisting of the sequence set forth in any one of SEQ ID NO: 9, SEQ ID NO: 202 and SEQ ID NOs: 251-303, and the HCDR3 sequence comprising or consisting of the sequence set forth in SEQ ID NO: 12.

In one or more embodiments, the anti-Nectin-4 antibody or antigen-binding unit comprises one or more of the following HCDR1, HCDR2 and HCDR3, or variants thereof: the HCDR1 sequence comprising or consisting of the sequence set forth in any one of SEQ ID NO: 235, SEQ ID NO: 238, SEQ ID NO: 241, and SEQ ID NOs: 304-317, the HCDR2 sequence comprising or consisting of the sequence set forth in any one of SEQ ID NOs: 318-358, and the HCDR3 sequence comprising or consisting of the sequence set forth in SEQ ID NO: 16.

In one or more embodiments, the anti-Nectin-4 antibody or antigen-binding unit further comprises the LCDR1, LCDR2 and LCDR3 contained in the light chain variable region set forth in SEQ ID NO:25. In one or more embodiments, according to the Kabat numbering system, the LCDR1 sequence comprises or consists of the sequence set forth in SEQ ID NO: 26, the LCDR2 sequence comprises or consists of the sequence set forth in SEQ ID NO: 27, and the LCDR3 sequence comprises or consists of the sequence set forth in SEQ ID NO: 28.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 10.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 14.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 16.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 18.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 20.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 22.

In one or more embodiments, the anti-Nectin-4 antibody or antigen-binding unit comprises the HCDR1 set forth in SEQ ID NO: 8, the HCDR2 set forth in SEQ ID NO: 9, and the HCDR3 set forth in SEQ ID NO: 24.

In one or more embodiments, the anti-Nectin-4 antibody or antigen-binding unit comprises the HCDR1 set forth in SEQ ID NO: 181, the HCDR2 set forth in SEQ ID NO: 196, and the HCDR3 set forth in SEQ ID NO: 14.

In one or more embodiments, the anti-Nectin-4 antibody or antigen-binding unit comprises the HCDR1 set forth in SEQ ID NO: 182, the HCDR2 set forth in SEQ ID NO: 197, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 9, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 199, and the HCDR3 set forth in SEQ ID NO: 14.

In one or more embodiments, the anti-Nectin-4 antibody or antigen-binding unit comprises the HCDR1 set forth in SEQ ID NO: 185, the HCDR2 set forth in SEQ ID NO: 200, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 201, and the HCDR3 set forth in SEQ ID NO: 14.

In one or more embodiments, the anti-Nectin-4 antibody or antigen-binding unit comprises the HCDR1 set forth in SEQ ID NO: 186, the HCDR2 set forth in SEQ ID NO: 202, and the HCDR3 set forth in SEQ ID NO: 14.

In one or more embodiments, the anti-Nectin-4 antibody or antigen-binding unit comprises the HCDR1 set forth in SEQ ID NO: 187, the HCDR2 set forth in SEQ ID NO: 203, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 204, and the HCDR3 set forth in SEQ ID NO: 14.

In one or more embodiments, the anti-Nectin-4 antibody or antigen-binding unit comprises the HCDR1 set forth in SEQ ID NO: 182, the HCDR2 set forth in SEQ ID NO: 205, and the HCDR3 set forth in SEQ ID NO: 14.

In one or more embodiments, the anti-Nectin-4 antibody or antigen-binding unit comprises the HCDR1 set forth in SEQ ID NO: 187, the HCDR2 set forth in SEQ ID NO: 206, and the HCDR3 set forth in SEQ ID NO: 14.

In one or more embodiments, the anti-Nectin-4 antibody or antigen-binding unit comprises the HCDR1 set forth in SEQ ID NO: 188, the HCDR2 set forth in SEQ ID NO: 207, and the HCDR3 set forth in SEQ ID NO: 14.

In one or more embodiments, the anti-Nectin-4 antibody or antigen-binding unit comprises the HCDR1 set forth in SEQ ID NO: 189, the HCDR2 set forth in SEQ ID NO: 208, and the HCDR3 set forth in SEQ ID NO: 14.

In one or more embodiments, the anti-Nectin-4 antibody or antigen-binding unit comprises the HCDR1 set forth in SEQ ID NO: 190, the HCDR2 set forth in SEQ ID NO: 209, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 210, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 211, and the HCDR3 set forth in SEQ ID NO: 14.

In one or more embodiments, the anti-Nectin-4 antibody or antigen-binding unit comprises the HCDR1 set forth in SEQ ID NO: 185, the HCDR2 set forth in SEQ ID NO: 212, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 213, and the HCDR3 set forth in SEQ ID NO: 14.

In one or more embodiments, the anti-Nectin-4 antibody or antigen-binding unit comprises the HCDR1 set forth in

SEQ ID NO: 189, the HCDR2 set forth in SEQ ID NO: 214, and the HCDR3 set forth in SEQ ID NO: 14.

In one or more embodiments, the anti-Nectin-4 antibody or antigen-binding unit comprises the HCDR1 set forth in SEQ ID NO: 191, the HCDR2 set forth in SEQ ID NO: 215, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 216, and the HCDR3 set forth in SEQ ID NO: 14.

In one or more embodiments, the anti-Nectin-4 antibody or antigen-binding unit comprises the HCDR1 set forth in SEQ ID NO: 181, the HCDR2 set forth in SEQ ID NO: 217, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 218, and the HCDR3 set forth in SEQ ID NO: 14.

In one or more embodiments, the anti-Nectin-4 antibody or antigen-binding unit comprises the HCDR1 set forth in SEQ ID NO: 192, the HCDR2 set forth in SEQ ID NO: 219, and the HCDR3 set forth in SEQ ID NO: 14.

In one or more embodiments, the anti-Nectin-4 antibody or antigen-binding unit comprises the HCDR1 set forth in SEQ ID NO: 8, the HCDR2 set forth in SEQ ID NO: 220, and the HCDR3 set forth in SEQ ID NO: 14.

In one or more embodiments, the anti-Nectin-4 antibody or antigen-binding unit comprises the HCDR1 set forth in SEQ ID NO: 185, the HCDR2 set forth in SEQ ID NO: 207, and the HCDR3 set forth in SEQ ID NO: 14.

In one or more embodiments, the anti-Nectin-4 antibody or antigen-binding unit comprises the HCDR1 set forth in SEQ ID NO: 190, the HCDR2 set forth in SEQ ID NO: 222, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 223, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 224, and the HCDR3 set forth in SEQ ID NO: 14.

In one or more embodiments, the anti-Nectin-4 antibody or antigen-binding unit comprises the HCDR1 set forth in SEQ ID NO: 193, the HCDR2 set forth in SEQ ID NO: 225, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 226, and the HCDR3 set forth in SEQ ID NO: 14.

In one or more embodiments, the anti-Nectin-4 antibody or antigen-binding unit comprises the HCDR1 set forth in SEQ ID NO: 194, the HCDR2 set forth in SEQ ID NO: 227, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 228, and the HCDR3 set forth in SEQ ID NO: 14.

In one or more embodiments, the anti-Nectin-4 antibody or antigen-binding unit comprises the HCDR1 set forth in SEQ ID NO: 184, the HCDR2 set forth in SEQ ID NO: 229, and the HCDR3 set forth in SEQ ID NO: 14.

In one or more embodiments, the anti-Nectin-4 antibody or antigen-binding unit comprises the HCDR1 set forth in SEQ ID NO: 189, the HCDR2 set forth in SEQ ID NO: 230, and the HCDR3 set forth in SEQ ID NO: 14.

In one or more embodiments, the anti-Nectin-4 antibody or antigen-binding unit comprises the HCDR1 set forth in SEQ ID NO: 190, the HCDR2 set forth in SEQ ID NO: 231, and the HCDR3 set forth in SEQ ID NO: 14.

In one or more embodiments, the anti-Nectin-4 antibody or antigen-binding unit comprises the HCDR1 set forth in SEQ ID NO: 195, the HCDR2 set forth in SEQ ID NO: 198, and the HCDR3 set forth in SEQ ID NO: 14.

In one or more embodiments, the anti-Nectin-4 antibody or antigen-binding unit comprises the HCDR1 set forth in SEQ ID NO: 183, the HCDR2 set forth in SEQ ID NO: 221, and the HCDR3 set forth in SEQ ID NO: 14.

In one or more embodiments, the anti-Nectin-4 antibody or antigen-binding unit comprises the HCDR1 set forth in SEQ ID NO: 234, the HCDR2 set forth in SEQ ID NO: 251, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 252, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 253, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 254, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 255, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 256, and the HCDR3 set forth in SEQ ID NO: 12.

In one or more embodiments, the anti-Nectin-4 antibody or antigen-binding unit comprises the HCDR1 set forth in SEQ ID NO: 235, the HCDR2 set forth in SEQ ID NO: 257, and the HCDR3 set forth in SEQ ID NO: 12.

In one or more embodiments, the anti-Nectin-4 antibody or antigen-binding unit comprises the HCDR1 set forth in SEQ ID NO: 186, the HCDR2 set forth in SEQ ID NO: 202, and the HCDR3 set forth in SEQ ID NO: 12.

In one or more embodiments, the anti-Nectin-4 antibody or antigen-binding unit comprises the HCDR1 set forth in SEQ ID NO: 236, the HCDR2 set forth in SEQ ID NO: 258, and the HCDR3 set forth in SEQ ID NO: 12.

In one or more embodiments, the anti-Nectin-4 antibody or antigen-binding unit comprises the HCDR1 set forth in

SEQ ID NO: 237, the HCDR2 set forth in SEQ ID NO: 259, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 260, and the HCDR3 set forth in SEQ ID NO: 12.

In one or more embodiments, the anti-Nectin-4 antibody or antigen-binding unit comprises the HCDR1 set forth in SEQ ID NO: 238, the HCDR2 set forth in SEQ ID NO: 261, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 262, and the HCDR3 set forth in SEQ ID NO: 12.

In one or more embodiments, the anti-Nectin-4 antibody or antigen-binding unit comprises the HCDR1 set forth in SEQ ID NO: 235, the HCDR2 set forth in SEQ ID NO: 263, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 264, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 265, and the HCDR3 set forth in SEQ ID NO: 12.

In one or more embodiments, the anti-Nectin-4 antibody or antigen-binding unit comprises the HCDR1 set forth in SEQ ID NO: 239, the HCDR2 set forth in SEQ ID NO: 266, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 267, and the HCDR3 set forth in SEQ ID NO: 12.

In one or more embodiments, the anti-Nectin-4 antibody or antigen-binding unit comprises the HCDR1 set forth in SEQ ID NO: 236, the HCDR2 set forth in SEQ ID NO: 268, and the HCDR3 set forth in SEQ ID NO: 12.

In one or more embodiments, the anti-Nectin-4 antibody or antigen-binding unit comprises the HCDR1 set forth in SEQ ID NO: 238, the HCDR2 set forth in SEQ ID NO: 269, and the HCDR3 set forth in SEQ ID NO: 12.

In one or more embodiments, the anti-Nectin-4 antibody or antigen-binding unit comprises the HCDR1 set forth in SEQ ID NO: 240, the HCDR2 set forth in SEQ ID NO: 270, and the HCDR3 set forth in SEQ ID NO: 12.

In one or more embodiments, the anti-Nectin-4 antibody or antigen-binding unit comprises the HCDR1 set forth in SEQ ID NO: 241, the HCDR2 set forth in SEQ ID NO: 271, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 272, and the HCDR3 set forth in SEQ ID NO: 12.

In one or more embodiments, the anti-Nectin-4 antibody or antigen-binding unit comprises the HCDR1 set forth in SEQ ID NO: 242, the HCDR2 set forth in SEQ ID NO: 273, and the HCDR3 set forth in SEQ ID NO: 12.

In one or more embodiments, the anti-Nectin-4 antibody or antigen-binding unit comprises the HCDR1 set forth in SEQ ID NO: 242, the HCDR2 set forth in SEQ ID NO: 274, and the HCDR3 set forth in SEQ ID NO: 12.

In one or more embodiments, the anti-Nectin-4 antibody or antigen-binding unit comprises the HCDR1 set forth in SEQ ID NO: 185, the HCDR2 set forth in SEQ ID NO: 275, and the HCDR3 set forth in SEQ ID NO: 12.

In one or more embodiments, the anti-Nectin-4 antibody or antigen-binding unit comprises the HCDR1 set forth in SEQ ID NO: 243, the HCDR2 set forth in SEQ ID NO: 276, and the HCDR3 set forth in SEQ ID NO: 12.

In one or more embodiments, the anti-Nectin-4 antibody or antigen-binding unit comprises the HCDR1 set forth in SEQ ID NO: 187, the HCDR2 set forth in SEQ ID NO: 277, and the HCDR3 set forth in SEQ ID NO: 12.

In one or more embodiments, the anti-Nectin-4 antibody or antigen-binding unit comprises the HCDR1 set forth in SEQ ID NO: 244, the HCDR2 set forth in SEQ ID NO: 278, and the HCDR3 set forth in SEQ ID NO: 12.

In one or more embodiments, the anti-Nectin-4 antibody or antigen-binding unit comprises the HCDR1 set forth in SEQ ID NO: 245, the HCDR2 set forth in SEQ ID NO: 279, and the HCDR3 set forth in SEQ ID NO: 12.

In one or more embodiments, the anti-Nectin-4 antibody or antigen-binding unit comprises the HCDR1 set forth in SEQ ID NO: 240, the HCDR2 set forth in SEQ ID NO: 280, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 281, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 282, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 283, and the HCDR3 set forth in SEQ ID NO: 12.

In one or more embodiments, the anti-Nectin-4 antibody or antigen-binding unit comprises the HCDR1 set forth in SEQ ID NO: 246, the HCDR2 set forth in SEQ ID NO: 284, and the HCDR3 set forth in SEQ ID NO: 12.

In one or more embodiments, the anti-Nectin-4 antibody or antigen-binding unit comprises the HCDR1 set forth in SEQ ID NO: 247, the HCDR2 set forth in SEQ ID NO: 285, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 286, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 287, and the HCDR3 set forth in SEQ ID NO: 12.

In one or more embodiments, the anti-Nectin-4 antibody or antigen-binding unit comprises the HCDR1 set forth in

SEQ ID NO: 8, the HCDR2 set forth in SEQ ID NO: 288, and the HCDR3 set forth in SEQ ID NO: 12.

In one or more embodiments, the anti-Nectin-4 antibody or antigen-binding unit comprises the HCDR1 set forth in SEQ ID NO: 194, the HCDR2 set forth in SEQ ID NO: 289, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 290, and the HCDR3 set forth in SEQ ID NO: 12.

In one or more embodiments, the anti-Nectin-4 antibody or antigen-binding unit comprises the HCDR1 set forth in SEQ ID NO: 243, the HCDR2 set forth in SEQ ID NO: 291, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 292, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 255, and the HCDR3 set forth in SEQ ID NO: 12

In one or more embodiments, the anti-Nectin-4 antibody or antigen-binding unit comprises the HCDR1 set forth in SEQ ID NO: 244, the HCDR2 set forth in SEQ ID NO: 293, and the HCDR3 set forth in SEQ ID NO: 12

In one or more embodiments, the anti-Nectin-4 antibody or antigen-binding unit comprises the HCDR1 set forth in SEQ ID NO: 247, the HCDR2 set forth in SEQ ID NO: 294, and the HCDR3 set forth in SEQ ID NO: 12.

In one or more embodiments, the anti-Nectin-4 antibody or antigen-binding unit comprises the HCDR1 set forth in SEQ ID NO: 194, the HCDR2 set forth in SEQ ID NO: 295, and the HCDR3 set forth in SEQ ID NO: 12.

In one or more embodiments, the anti-Nectin-4 antibody or antigen-binding unit comprises the HCDR1 set forth in SEQ ID NO: 238, the HCDR2 set forth in SEQ ID NO: 296, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 297, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 298, and the HCDR3 set forth in SEQ ID NO: 12.

In one or more embodiments, the anti-Nectin-4 antibody or antigen-binding unit comprises the HCDR1 set forth in SEQ ID NO: 8, the HCDR2 set forth in SEQ ID NO: 299, and the HCDR3 set forth in SEQ ID NO: 12.

In one or more embodiments, the anti-Nectin-4 antibody or antigen-binding unit comprises the HCDR1 set forth in SEQ ID NO: 248, the HCDR2 set forth in SEQ ID NO: 300, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 301, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 302, and the HCDR3 set forth in SEQ ID NO: 12.

In one or more embodiments, the anti-Nectin-4 antibody or antigen-binding unit comprises the HCDR1 set forth in SEQ ID NO: 237, the HCDR2 set forth in SEQ ID NO: 9, and the HCDR3 set forth in SEQ ID NO: 12.

In one or more embodiments, the anti-Nectin-4 antibody or antigen-binding unit comprises the HCDR1 set forth in SEQ ID NO: 181, the HCDR2 set forth in SEQ ID NO: 303, and the HCDR3 set forth in SEQ ID NO: 12.

In one or more embodiments, the anti-Nectin-4 antibody or antigen-binding unit comprises the HCDR1 set forth in SEQ ID NO: 304, the HCDR2 set forth in SEQ ID NO: 318, and the HCDR3 set forth in SEQ ID NO: 16.

In one or more embodiments, the anti-Nectin-4 antibody or antigen-binding unit comprises the HCDR1 set forth in SEQ ID NO: 304, the HCDR2 set forth in SEQ ID NO: 319, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 320, and the HCDR3 set forth in SEQ ID NO: 16.

In one or more embodiments, the anti-Nectin-4 antibody or antigen-binding unit comprises the HCDR1 set forth in SEQ ID NO: 306, the HCDR2 set forth in SEQ ID NO: 321, and the HCDR3 set forth in SEQ ID NO: 16.

In one or more embodiments, the anti-Nectin-4 antibody or antigen-binding unit comprises the HCDR1 set forth in SEQ ID NO: 307, the HCDR2 set forth in SEQ ID NO: 322, and the HCDR3 set forth in SEQ ID NO: 16.

In one or more embodiments, the anti-Nectin-4 antibody or antigen-binding unit comprises the HCDR1 set forth in SEQ ID NO: 308, the HCDR2 set forth in SEQ ID NO: 323, and the HCDR3 set forth in SEQ ID NO: 16.

In one or more embodiments, the anti-Nectin-4 antibody or antigen-binding unit comprises the HCDR1 set forth in SEQ ID NO: 306, the HCDR2 set forth in SEQ ID NO: 324, and the HCDR3 set forth in SEQ ID NO: 16.

In one or more embodiments, the anti-Nectin-4 antibody or antigen-binding unit comprises the HCDR1 set forth in SEQ ID NO: 306, the HCDR2 set forth in SEQ ID NO: 321, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 325, and the HCDR3 set forth in SEQ ID NO: 16.

In one or more embodiments, the anti-Nectin-4 antibody or antigen-binding unit comprises the HCDR1 set forth in SEQ ID NO: 309, the HCDR2 set forth in SEQ ID NO: 326, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 327, and the HCDR3 set forth in SEQ ID NO: 16.

In one or more embodiments, the anti-Nectin-4 antibody or antigen-binding unit comprises the HCDR1 set forth in

SEQ ID NO: 311, the HCDR2 set forth in SEQ ID NO: 328, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 329, and the HCDR3 set forth in SEQ ID NO: 16.

In one or more embodiments, the anti-Nectin-4 antibody or antigen-binding unit comprises the HCDR1 set forth in SEQ ID NO: 238, the HCDR2 set forth in SEQ ID NO: 330, and the HCDR3 set forth in SEQ ID NO: 16.

In one or more embodiments, the anti-Nectin-4 antibody or antigen-binding unit comprises the HCDR1 set forth in SEQ ID NO: 309, the HCDR2 set forth in SEQ ID NO: 331, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 332, and the HCDR3 set forth in SEQ ID NO: 16.

In one or more embodiments, the anti-Nectin-4 antibody or antigen-binding unit comprises the HCDR1 set forth in SEQ ID NO: 308, the HCDR2 set forth in SEQ ID NO: 333, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 334, and the HCDR3 set forth in SEQ ID NO: 16.

In one or more embodiments, the anti-Nectin-4 antibody or antigen-binding unit comprises the HCDR1 set forth in SEQ ID NO: 307, the HCDR2 set forth in SEQ ID NO: 335, and the HCDR3 set forth in SEQ ID NO: 16.

In one or more embodiments, the anti-Nectin-4 antibody or antigen-binding unit comprises the HCDR1 set forth in SEQ ID NO: 312, the HCDR2 set forth in SEQ ID NO: 336, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 337, and the HCDR3 set forth in SEQ ID NO: 16.

In one or more embodiments, the anti-Nectin-4 antibody or antigen-binding unit comprises the HCDR1 set forth in SEQ ID NO: 311, the HCDR2 set forth in SEQ ID NO: 338, and the HCDR3 set forth in SEQ ID NO: 16.

In one or more embodiments, the anti-Nectin-4 antibody or antigen-binding unit comprises the HCDR1 set forth in SEQ ID NO: 313, the HCDR2 set forth in SEQ ID NO: 339, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 340, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 341, and the HCDR3 set forth in SEQ ID NO: 16.

In one or more embodiments, the anti-Nectin-4 antibody or antigen-binding unit comprises the HCDR1 set forth in SEQ ID NO: 314, the HCDR2 set forth in SEQ ID NO: 339, and the HCDR3 set forth in SEQ ID NO: 16.

In one or more embodiments, the anti-Nectin-4 antibody or antigen-binding unit comprises the HCDR1 set forth in SEQ ID NO: 315, the HCDR2 set forth in SEQ ID NO: 342, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 343, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 344, and the HCDR3 set forth in SEQ ID NO: 16.

In one or more embodiments, the anti-Nectin-4 antibody or antigen-binding unit comprises the HCDR1 set forth in SEQ ID NO: 310, the HCDR2 set forth in SEQ ID NO: 339, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 345, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 346, and the HCDR3 set forth in SEQ ID NO: 16.

In one or more embodiments, the anti-Nectin-4 antibody or antigen-binding unit comprises the HCDR1 set forth in SEQ ID NO: 306, the HCDR2 set forth in SEQ ID NO: 347, and the HCDR3 set forth in SEQ ID NO: 16.

In one or more embodiments, the anti-Nectin-4 antibody or antigen-binding unit comprises the HCDR1 set forth in SEQ ID NO: 307, the HCDR2 set forth in SEQ ID NO: 339, and the HCDR3 set forth in SEQ ID NO: 16.

In one or more embodiments, the anti-Nectin-4 antibody or antigen-binding unit comprises the HCDR1 set forth in SEQ ID NO: 235, the HCDR2 set forth in SEQ ID NO: 348, and the HCDR3 set forth in SEQ ID NO: 16.

In one or more embodiments, the anti-Nectin-4 antibody or antigen-binding unit comprises the HCDR1 set forth in SEQ ID NO: 314, the HCDR2 set forth in SEQ ID NO: 349, and the HCDR3 set forth in SEQ ID NO: 16.

In one or more embodiments, the anti-Nectin-4 antibody or antigen-binding unit comprises the HCDR1 set forth in SEQ ID NO: 316, the HCDR2 set forth in SEQ ID NO: 350, and the HCDR3 set forth in SEQ ID NO: 16.

In one or more embodiments, the anti-Nectin-4 antibody or antigen-binding unit comprises the HCDR1 set forth in SEQ ID NO: 306, the HCDR2 set forth in SEQ ID NO: 351, and the HCDR3 set forth in SEQ ID NO: 16.

In one or more embodiments, the anti-Nectin-4 antibody or antigen-binding unit comprises the HCDR1 set forth in SEQ ID NO: 304, the HCDR2 set forth in SEQ ID NO: 352, and the HCDR3 set forth in SEQ ID NO: 16.

In one or more embodiments, the anti-Nectin-4 antibody or antigen-binding unit comprises the HCDR1 set forth in SEQ ID NO: 309, the HCDR2 set forth in SEQ ID NO: 353, and the HCDR3 set forth in SEQ ID NO: 16.

In one or more embodiments, the anti-Nectin-4 antibody or antigen-binding unit comprises the HCDR1 set forth in

SEQ ID NO: 310, the HCDR2 set forth in SEQ ID NO: 354, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 355, and the HCDR3 set forth in SEQ ID NO: 16.

In one or more embodiments, the anti-Nectin-4 antibody or antigen-binding unit comprises the HCDR1 set forth in SEQ ID NO: 305, the HCDR2 set forth in SEQ ID NO: 356, the HCDR3 set forth in SEQ ID NO: 16, the LCDR1 set forth in SEQ ID NO: 26, the LCDR2 set forth in SEQ ID NO: 27, and the LCDR3 set forth in SEQ ID NO: 28.

In one or more embodiments, the anti-Nectin-4 antibody or antigen-binding unit comprises the HCDR1 set forth in SEQ ID NO: 241, the HCDR2 set forth in SEQ ID NO: 357, and the HCDR3 set forth in SEQ ID NO: 16.

In one or more embodiments, the anti-Nectin-4 antibody or antigen-binding unit comprises the HCDR1 set forth in SEQ ID NO: 317, the HCDR2 set forth in SEQ ID NO: 358, and the HCDR3 set forth in SEQ ID NO: 16.

In one or more embodiments, the anti-Nectin-4 antibody or antigen-binding unit further comprises the LCDR1 set forth in SEQ ID NO: 26, the LCDR2 set forth in SEQ ID NO: 27, and the LCDR3 set forth in SEQ ID NO: 28.

In one or more embodiments, the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding CDR sequences, and the variants retain the binding affinity for Nectin-4.

In one or more embodiments, the anti-Nectin-4 antibody or antigen-binding unit is derived from a bird or a mammal. In one or more embodiments, the anti-Nectin-4 antibody or antigen-binding unit is derived from a human, a mouse, a donkey, a rabbit, a goat, a camel, a llama, a horse, or a chicken origin.

In one or more embodiments, the sequences of the framework regions HFR1-HFR4 in the heavy chain variable region of the anti-Nectin-4 antibody or antigen-binding unit are set forth in SEQ ID NOs: 29-32, respectively. In one or more embodiments, the sequence of the framework region HFR1 in the heavy chain variable region of the anti-Nectin-4 antibody or antigen-binding unit is set forth in the amino acid sequence of positions 1-30 of the sequence set forth in any one of SEQ ID NOs: 41-180, and the sequences of HFR2-HFR4 are set forth in SEQ ID NOs: 30-32, respectively. In one or more embodiments, the sequences of the framework regions LFR1-LFR4 in the light chain variable region of the anti-Nectin-4 antibody or antigen-binding unit are set forth in SEQ ID NOs: 33-36, respectively.

In one or more embodiments, the anti-Nectin-4 antibody or antigen-binding unit comprises a heavy chain variable region selected from the group consisting of the following or a variant thereof:

(1) the heavy chain variable region comprising or consisting of the sequence set forth in SEQ ID NO: 7;

(2) the heavy chain variable region comprising or consisting of the sequence set forth in SEQ ID NO: 11;

(3) the heavy chain variable region comprising or consisting of the sequence set forth in SEQ ID NO: 13;

(4) the heavy chain variable region comprising or consisting of the sequence set forth in SEQ ID NO: 15;

(5) the heavy chain variable region comprising or consisting of the sequence set forth in SEQ ID NO: 17;

(6) the heavy chain variable region comprising or consisting of the sequence set forth in SEQ ID NO: 19;

(7) the heavy chain variable region comprising or consisting of the sequence set forth in SEQ ID NO: 21;

(8) the heavy chain variable region comprising or consisting of the sequence set forth in SEQ ID NO: 23;

(9) the heavy chain variable region comprising or consisting of the sequence set forth in any one of SEQ ID NOs: 41-180;

wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding variable region sequences, and the variants retain the binding affinity for Nectin-4.

In one or more embodiments, the antibody or antigen-binding unit further comprises the light chain variable region set forth in SEQ ID NO: 25.

In one or more embodiments, the antibody or antigen-binding fragment further comprises a heavy chain constant region and/or a light chain constant region. In one or more embodiments, the heavy chain constant region is selected from IgG1, IgG2, IgG3 or IgG4 type. In one or more embodiments, the light chain constant region is λ chain or κ chain constant region.

In one or more embodiments, the heavy chain constant region comprises the amino acid sequence set forth in SEQ ID NO: 37, or an amino acid sequence having at least 80%, at least 90%, at least 95%, at least 96%, at least 97%, at least 98% or at least 99% identity to the amino acid sequence set forth in SEQ ID NO: 37, or an amino acid sequence having one or more conservative amino acid substitutions compared with the amino acid sequence set forth in SEQ ID NO: 37. In one or more embodiments, the light chain constant region comprises the amino acid sequence set forth in SEQ ID NO: 38, or an amino acid sequence having at least 80%, at least 90%, at least 95%, at least 96%, at least 97%, at least 98% or at least 99% identity to the amino acid sequence set forth in SEQ ID NO: 38, or an amino acid sequence having one or more conservative amino acid substitutions compared with the amino acid sequence set forth in SEQ ID NO: 38. In one or more embodiments, the heavy chain constant region comprises the amino acid sequence set forth in SEQ ID NO: 37, the light chain constant region comprises the amino acid sequence set forth in SEQ ID NO: 38.

In one or more embodiments, the anti-Nectin-4 antibody comprises a heavy chain and a light chain. In one or more embodiments, the anti-Nectin-4 antibody comprises two heavy chains with identical sequences and two light chains with identical sequences.

In one or more embodiments, the heavy chain of the anti-Nectin-4 antibody comprises the heavy chain variable region set forth in any one of SEQ ID NO: 7, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NOs: 41-180, and the heavy chain constant region set forth in SEQ ID NO: 37. In one or more embodiments, the light chain of the anti-Nectin-4 antibody comprises the light chain variable region set forth in SEQ ID NO: 25 and the light chain constant region set forth in SEQ ID NO: 38.

In one or more embodiments, the amino acid sequence of the heavy chain of the anti-Nectin-4 antibody is set forth in any one of SEQ ID NO: 232, SEQ ID NO: 233, SEQ ID NO: 249, and SEQ ID NOs: 360-363. In one or more embodiments, the amino acid sequence of the light chain is set forth in SEQ ID NO: 250. In one or more embodiments, the amino acid sequence of the heavy chain of the anti-Nectin-4 antibody is set forth in any one of SEQ ID NO: 232, SEQ ID NO: 233, SEQ ID NO: 249, and SEQ ID NOs: 360-363, the amino acid sequence of the light chain is set forth in SEQ ID NO: 250.

In one or more embodiments, the anti-Nectin-4 antibody or antigen-binding unit, wherein the antigen-binding fragment is selected from Fab, Fab', $F(ab')_2$, $F(ab)_2$, Fd, Fv, dAb, Fab/c, a complementarity determining region fragment, scFv, scFv multimers, disulfide-stabilized Fv (dsFv), $(dsFv)_2$, bispecific dsFv (dsFv-dsFv'), a diabody, a disulfide-stabilized diabody (ds-diabody), a multispecific antibody formed from a portion of an antibody comprising one or more CDRs, a single domain antibody (sdAb), a nanobody, a domain antibody, or a bivalent domain antibody.

In one or more embodiments, provided is a polypeptide specifically binding to Nectin-4, selected from the group consisting of:

(1) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NOs: 181-195, SEQ ID NOs: 234-248, and SEQ ID NOs: 304-317, the HCDR2 sequence comprises or consists of the sequence set forth in any one of SEQ ID NO: 9, SEQ ID NOs: 196-231, SEQ ID NOs: 251-303, and SEQ ID NOs: 318-358, and the HCDR3 sequence comprises or consists of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;

(2) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;

(3) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202, and SEQ ID NOs: 251-303, and the HCDR3 sequence comprises or consists of the sequence set forth in SEQ ID NO: 12;

(4) a polypeptide, comprising sequences of an HCDR1, an HCDR2 and an HCDR3, or variants thereof, wherein the polypeptide serves as a portion of the anti-Nectin-4 antibody specifically binding to Nectin-4, wherein the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;

(5) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 235, SEQ ID NO: 238, SEQ ID NO: 241, and SEQ ID NOs: 304-317, the HCDR2 sequence comprises or consists of the sequence set forth in any one of SEQ ID NO: 9 and SEQ ID NOs: 318-358, and the HCDR3 sequence comprises or consists of the sequence set forth in SEQ ID NO: 16;

(6) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;

(7) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;

(8) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;

(9) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;

(10) a polypeptide, comprising the sequence set forth in SEQ ID NO: 7 or a variant thereof, wherein the polypeptide serves as a portion of an anti-Nectin-4 antibody specifically binding to Nectin-4;

(11) a polypeptide, comprising the sequence set forth in SEQ ID NO: 11 or a variant thereof, wherein the polypeptide serves as a portion of an anti-Nectin-4 antibody specifically binding to Nectin-4;

(12) a polypeptide, comprising the sequence set forth in SEQ ID NO: 13 or a variant thereof, wherein the polypeptide serves as a portion of an anti-Nectin-4 antibody specifically binding to Nectin-4;

(13) a polypeptide, comprising the sequence set forth in SEQ ID NO: 15 or a variant thereof, wherein the polypeptide serves as a portion of an anti-Nectin-4 antibody specifically binding to Nectin-4;

(14) a polypeptide, comprising the sequence set forth in SEQ ID NO: 17 or a variant thereof, wherein the polypeptide serves as a portion of an anti-Nectin-4 antibody specifically binding to Nectin-4;

(15) a polypeptide, comprising the sequence set forth in SEQ ID NO: 19 or a variant thereof, wherein the polypeptide serves as a portion of an anti-Nectin-4 antibody specifically binding to Nectin-4;

(16) a polypeptide, comprising the sequence set forth in SEQ ID NO: 21 or a variant thereof, wherein the polypeptide serves as a portion of an anti-Nectin-4 antibody specifically binding to Nectin-4;

(17) a polypeptide, comprising the sequence set forth in SEQ ID NO: 23 or a variant thereof, wherein the polypeptide serves as a portion of an anti-Nectin-4 antibody specifically binding to Nectin-4; and

(18) a polypeptide, comprising the sequence set forth in any one of SEQ ID NOs: 41-180 or a variant thereof, wherein the polypeptide serves as a portion of an anti-Nectin-4 antibody specifically binding to Nectin-4.

In one or more embodiments, the polypeptide further comprises the sequences set forth in SEQ ID NO: 26, SEQ ID NO: 27, and SEQ ID NO: 28. In one or more embodiments, the polypeptide further comprises the sequence set forth in SEQ ID NO: 25 or a variant thereof.

In one or more embodiments, provided is a biomaterial, wherein the biomaterial is

(1) a nucleic acid molecule, encoding the anti-Nectin-4 antibody or antigen-binding unit, or the polypeptide specifically binding to Nectin-4, or a portion thereof;

(2) a vector, comprising the nucleic acid molecule encoding the anti-Nectin-4 antibody or antigen-binding unit, or the polypeptide specifically binding to Nectin-4; or

(3) a host cell, comprising the nucleic acid molecule encoding the anti-Nectin-4 antibody or antigen-binding unit, or the polypeptide specifically binding to Nectin-4.

In one or more embodiments, provided is a method for preparing the anti-Nectin-4 antibody or antigen-binding unit, or the polypeptide specifically binding to Nectin-4 described herein, comprising: culturing a host cell containing a nucleic acid molecule encoding anti-Nectin-4 antibody or antigen-binding unit or the polypeptide, and optionally, isolating the anti-Nectin-4 antibody or antigen-binding fragment or the polypeptide.

In one or more embodiments, provided is an antibody-drug conjugate or a pharmaceutically acceptable salt or solvate thereof, comprising the anti-Nectin-4 antibody or antigen-binding unit conjugated to a drug (the drug described herein) via a linker.

In one or more embodiments, the linker is a cleavable linker.

In one or more embodiments, provided is an antibody-drug conjugate having a structure shown in Formula I-A, Formula I-B, or Formula I-C or a stereoisomer thereof, or a pharmaceutically acceptable salt or solvate thereof:

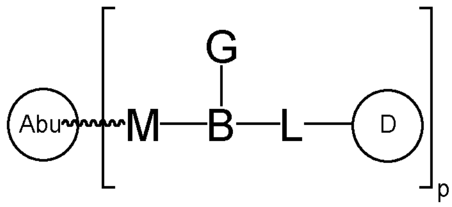

Formula I-A

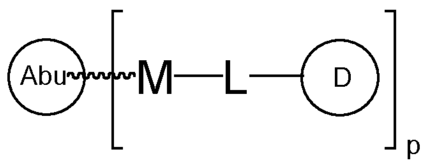

Formula I-B

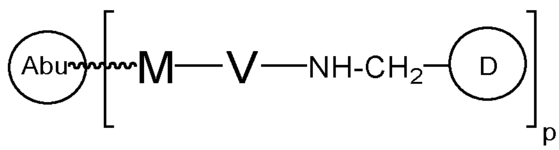

Formula I-C

Abu is the anti-Nectin-4 antibody or antigen-binding unit, or the polypeptide specifically binding to Nectin-4 as described in the application;

D is a drug (such as the drug described herein).

In one or more embodiments, provided is an antibody-drug conjugate having a structure shown in Formula I-A or a stereoisomer thereof, or a pharmaceutically acceptable or solvate thereof:

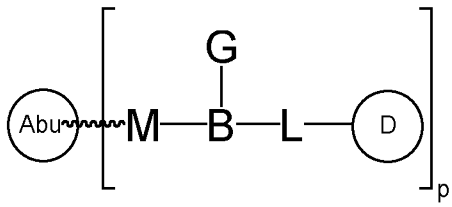

Formula I-A

wherein

Abu is the anti-Nectin-4 antibody or antigen-binding unit, or the polypeptide specifically binding to Nectin-4 as described in the application;

D is a drug (such as the drug described herein);

M is

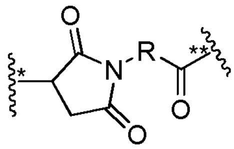

, wherein * links to Abu, ** links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

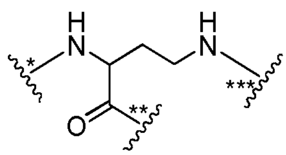

, or is,

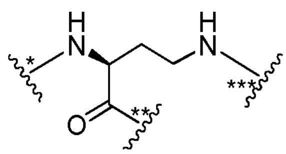

;

wherein * links to M, ** links to L, and *** links to G;

L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19 or 20; or each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly;

each FF is independently

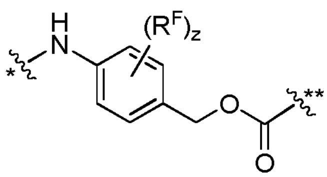

,

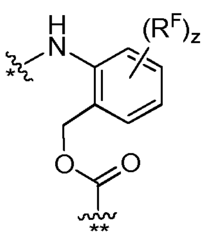

or

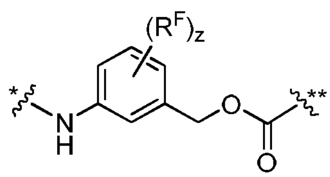

,

wherein each $R^F$ is independently C1-C6 alkyl, C1-C6 alkoxy, $-NO_2$ or halogen, wherein * links to AA, and ** links to D, z is 0, 1, 2, 3 or 4; f is 1, 2, 3, 4, 5, 6, 7, 8, 9 or 10;

G is

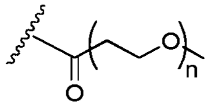

,

wherein n is 1-24, e.g., 1, 2, 3, 4, 5, 6, 7, 8, 9, 10, 11, 12, 13, 14, 15, 16, 17, 18, 19, 20, 21, 22, 23 or 24; e.g., 4, 8, 10, 12, 16, 18, or 24 ;

p is 1-10.

In one or more embodiments, R is $-(CH_2)_r-$, wherein r is 1 or 5.

In one or more embodiments, AA is Val-Cit, and i is 1.

In one or more embodiments, each FF is independently

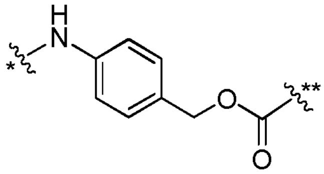

,

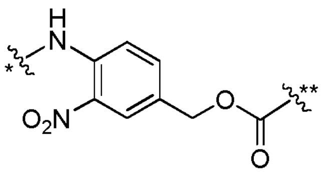

,

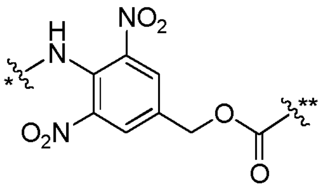

,

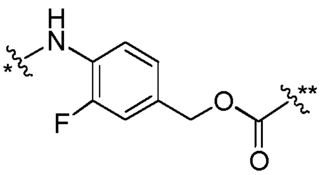

,

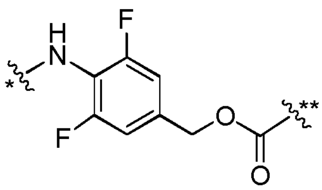

,

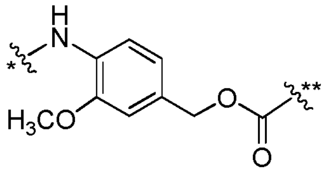

,

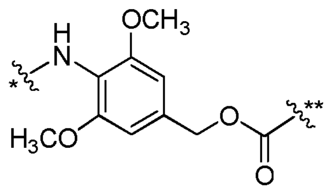

,

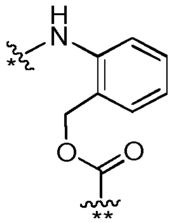

,

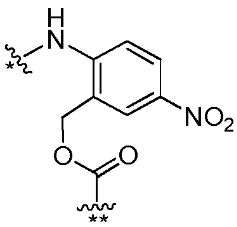

,

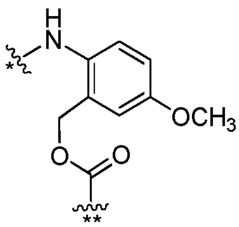

,

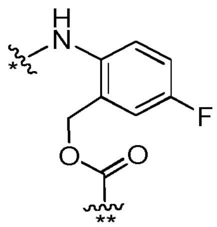

,

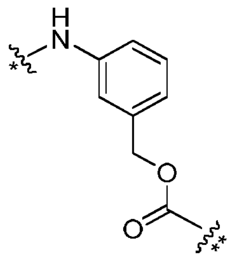

,

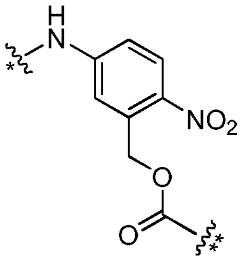,
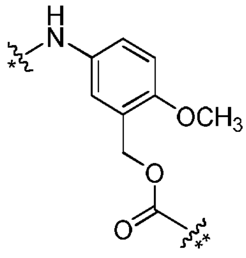, or
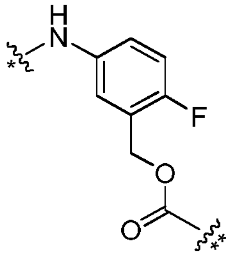,

wherein * links to AA, and ** links to D.

In one or more embodiments, FF is

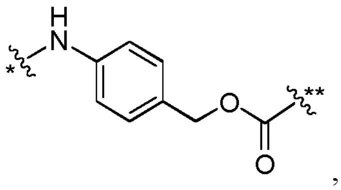,

f is 1, wherein * links to AA, and ** links to D.

In one or more embodiments, L is

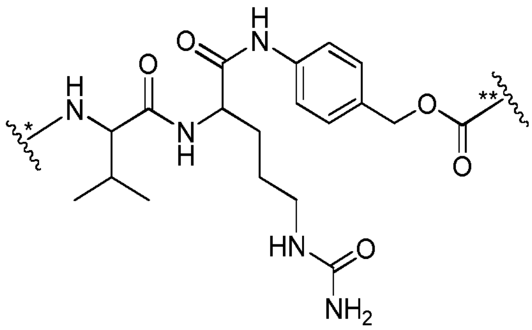,

wherein * links to B and ** links to D.

In one or more embodiments, L is

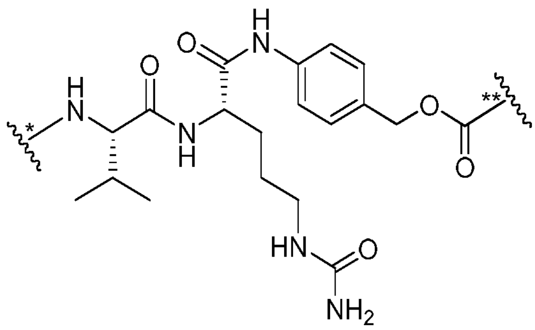,

wherein * links to B, and ** links to D.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-B or a stereoisomer thereof, or a pharmaceutically acceptable salt or solvate thereof:

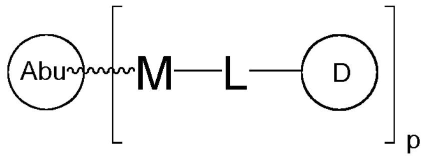

Formula I-B

wherein

Abu is the anti-Nectin-4 antibody or antigen-binding unit, or the polypeptide specifically binding to Nectin-4 as described in the application;

D is a drug (such as the drug described herein);

M is

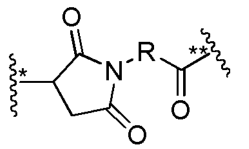

,

wherein * links to Abu, ** links to L, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, - $(CH_2)_rC(O)NR^m(CH_2CH_2O)_r-CH_2-$, $-(CH_2CH_2O)_rC(O)NR^m(CH_2CH_2O)_r-$, - $(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

L is $-(AA)_i-(FF)_f-$, , wherein AA is an amino acid or polypeptide, and i is 1, 2, 3, 4, 5, 6, 7, 8, 9, 10, 11, 12, 13, 14, 15, 16, 17, 18, 19 or 20; each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly; each FF is independently

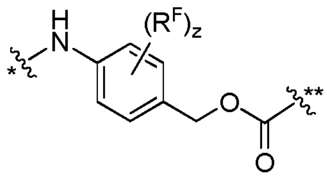

,

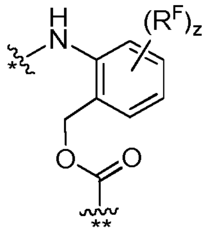

or

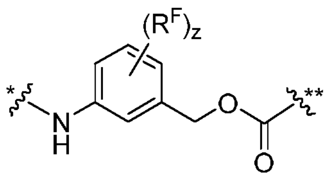

,

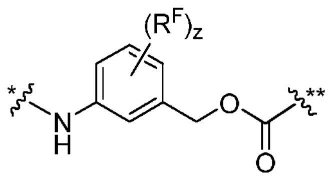

,

wherein each $R^F$ is independently C1-C6 alkyl, C1-C6 alkoxy, $-NO_2$ or halogen, wherein * links to AA, and ** links to D,

z is 0, 1, 2, 3 or 4; f is 1, 2, 3, 4, 5, 6, 7, 8, 9 or 10;

p is 1-10.

In one or more embodiments, R is $-(CH_2)_r-$, wherein r is 1 or 5.

In one or more embodiments, AA is Val-Cit, and i is 1.

In one or more embodiments, each FF is independently

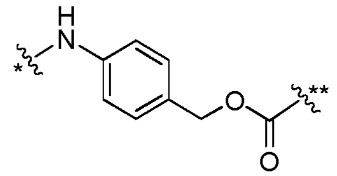,

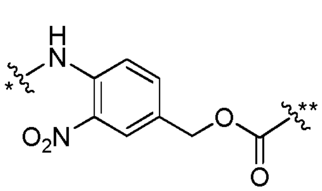, 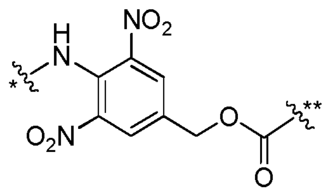, 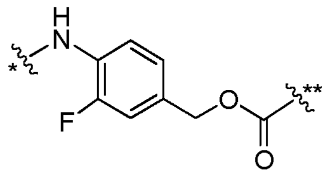,

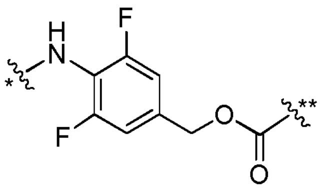, 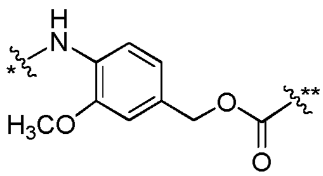, 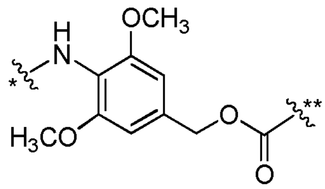, 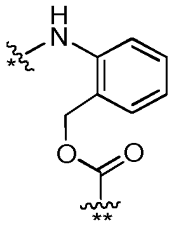,

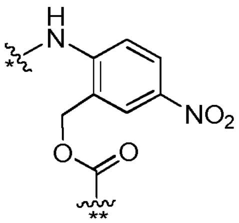, 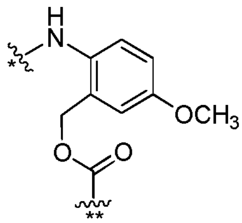, 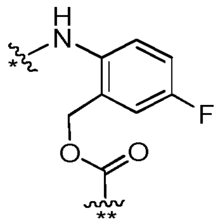, 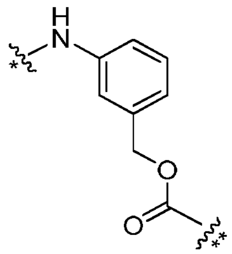,

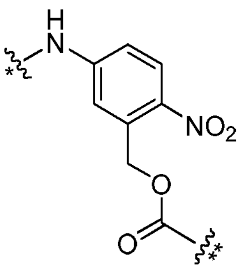, 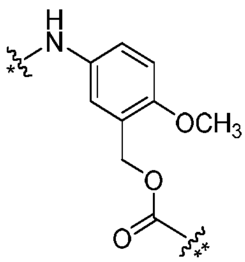, or 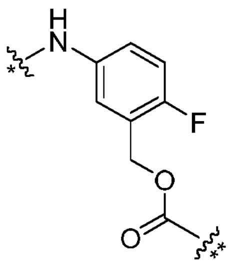,

wherein * links to AA, and ** links to D.

In one or more embodiments, FF is

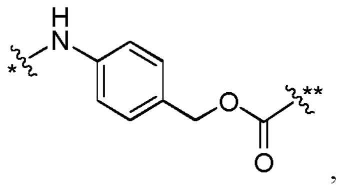,

f is 1, wherein * links to AA, and ** links to D.

In one or more embodiments, L is

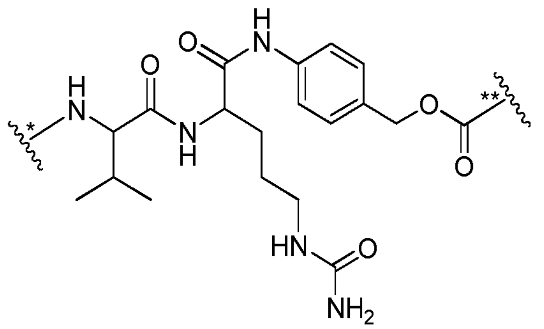,

wherein * links to M, and ** links to D.

In one or more embodiments, L is

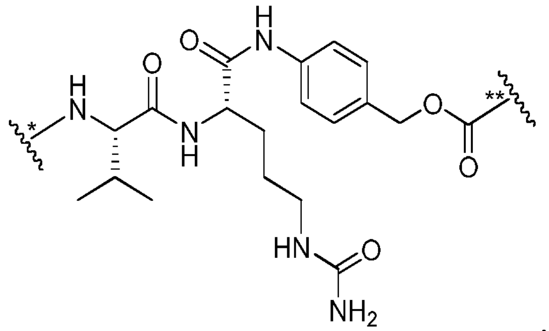,

wherein * links to M, and ** links to D.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-C or a stereoisomer thereof, or a pharmaceutically acceptable salt or solvate whereof:

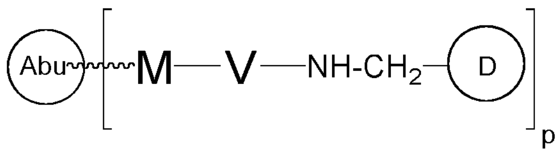

Formula I-C

wherein

Abu is the anti-Nectin-4 antibody or antigen-binding unit, or the polypeptide specifically binding to Nectin-4 as described in the application;

D is a drug (such as the drug described herein);

M is

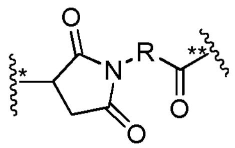

,

wherein * links to Abu, ** links to V, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, - $(CH_2)_rC(O)NR^m(CH_2CH_2O)_r-CH_2-$, $-(CH_2CH_2O)_rC(O)NR^m(CH_2CH_2O)_r-$, - $(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

V is

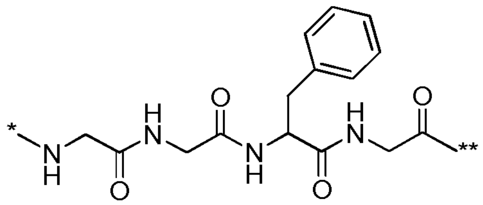

,

wherein * links to M, ** links to $-NH-CH_2-$;

p is 1-10.

In one or more embodiments, R is $-(CH_2)_r-$, wherein r is 1 or 5.

In one or more embodiments, V is

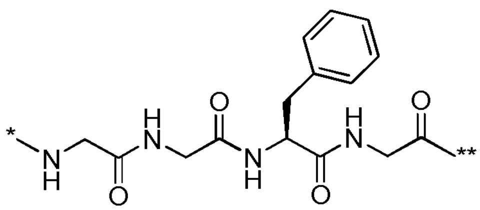

,

wherein * links to M, ** links to $-NH-CH_2-$.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-A-1 or Formula I-A-2 or a stereoisomer thereof, or a pharmaceutically acceptable salt or solvate thereof, wherein the Formula I-A-1 and Formula I-A-2 are:

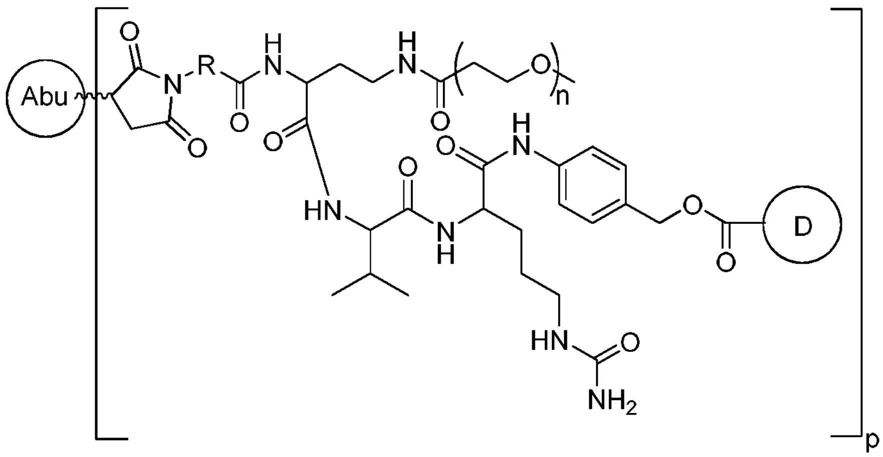

Formula I-A-1

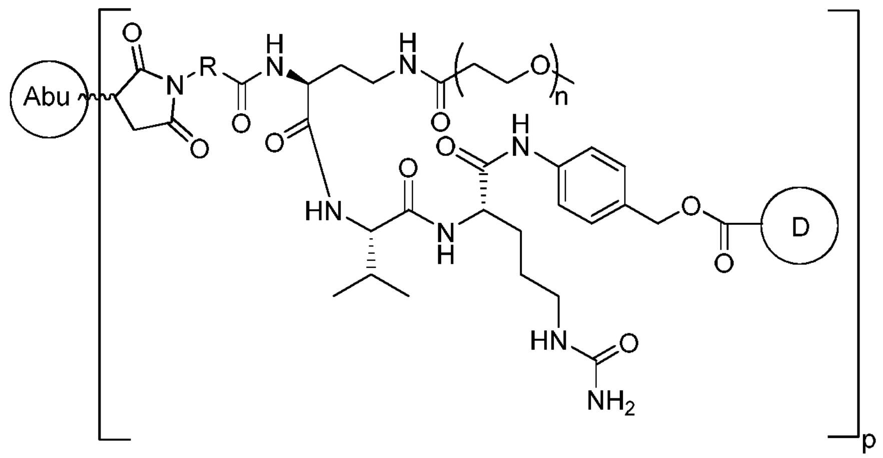

Formula I-A-2

wherein

Abu is the anti-Nectin-4 antibody or antigen-binding unit, or the polypeptide specifically binding to Nectin-4 as described in the application;

R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

D is a drug (such as the drug described herein);

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24; e.g., 4, 8, 10, 12, 16, 18 or 24;

p is 1-10.

In one or more embodiments, R is $-(CH_2)_r-$, wherein r is 1 or 5.
In one or more embodiments, n is 4-12.
In one or more embodiments, n is 4-8.
In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-A-3 or Formula I-A-4 or a stereoisomer thereof, or a pharmaceutically acceptable salt or solvate thereof, wherein the Formula I-A-3 and Formula I-A-4 are:

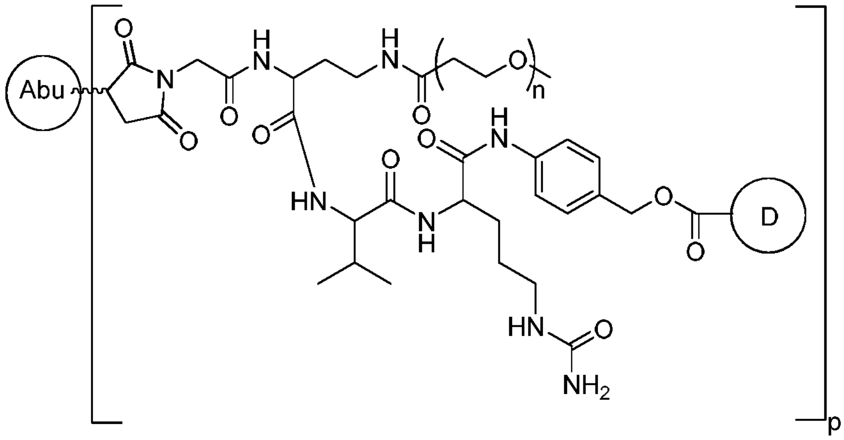

Formula I-A-3

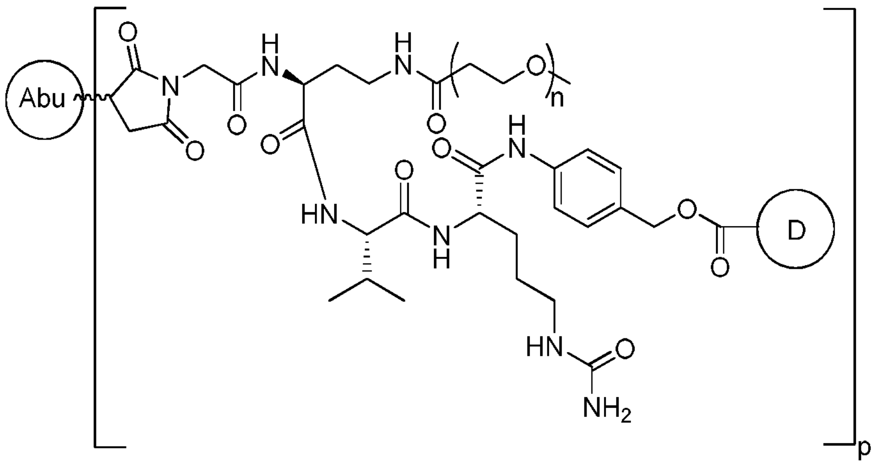

Formula I-A-4

wherein

Abu is the anti-Nectin-4 antibody or antigen-binding unit, or the polypeptide specifically binding to Nectin-4 as described in the application;

D is a drug (such as the drug described herein);

n is an integer from 1 to 24, e.g., 1, 2, 3, 4, 5, 6, 7, 8, 9, 10, 11, 12, 13, 14, 15, 16, 17, 18, 19, 20, 21, 22, 23 or 24; e.g., 4, 8, 10, 12, 16, 18 or 24;

p is 1-10.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-A-5, Formula I-A-6, Formula I-A-7, Formula I-A-8, Formula I-A-9, Formula I-A-10 or Formula I-A-11 or a stereoisomer thereof, or a pharmaceutically acceptable salt or solvate thereof, wherein the Formula I-A-5, Formula I-A-6, Formula I-A-7, Formula I-A-8, Formula I-A-9, Formula I-A-10, and Formula I-A-11 are:

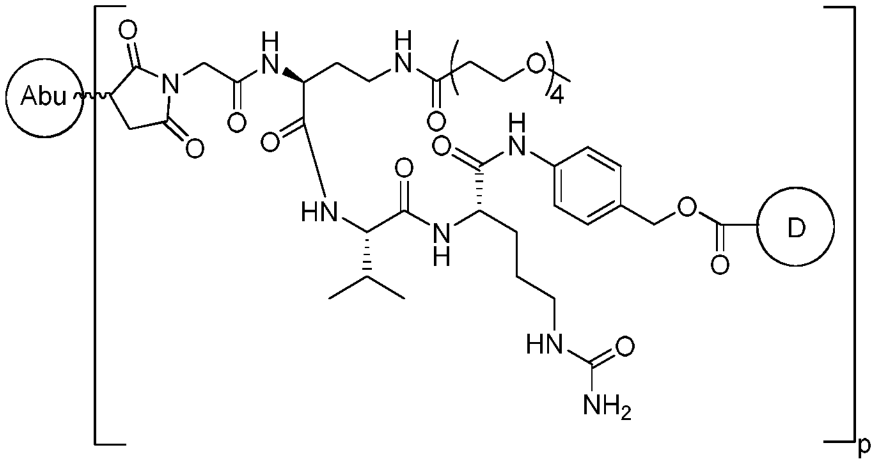

Formula I-A-5

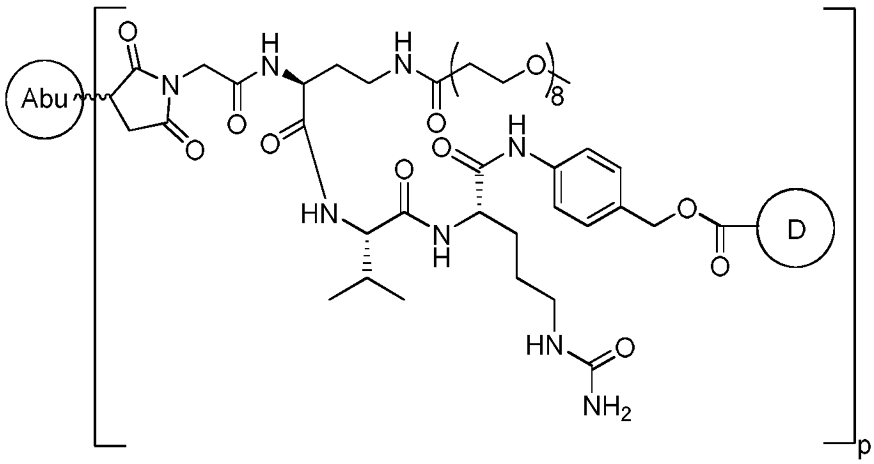

Formula I-A-6

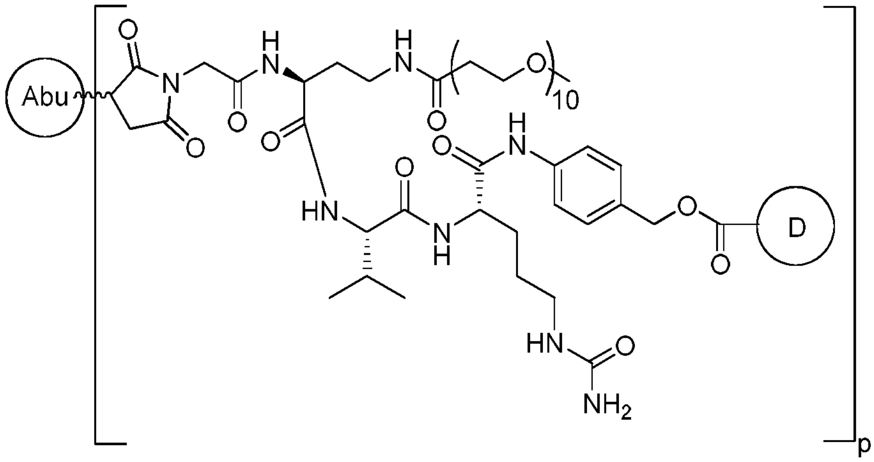

Formula I-A-7

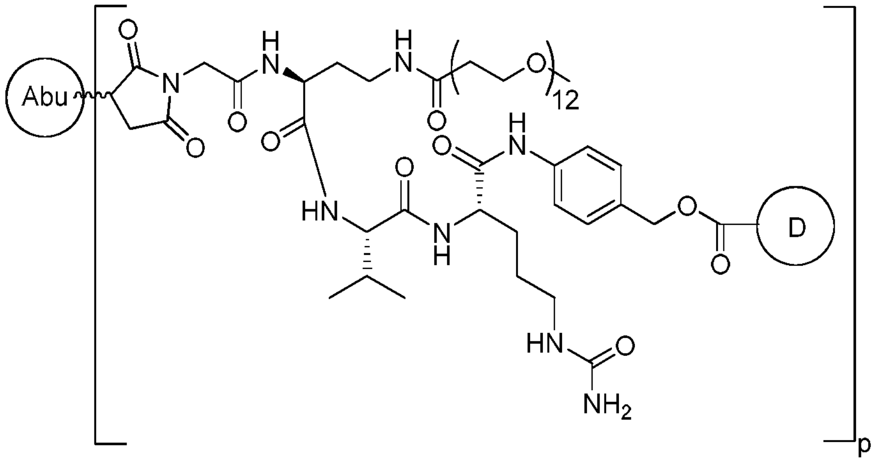

Formula I-A-8

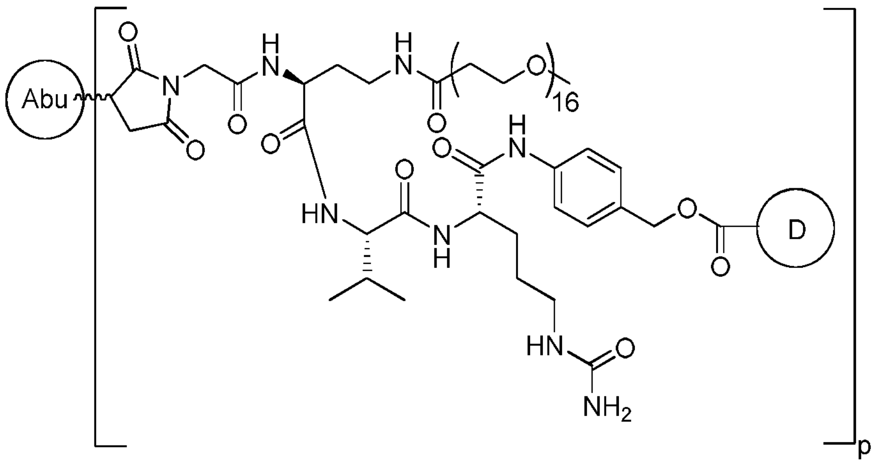

Formula I-A-9

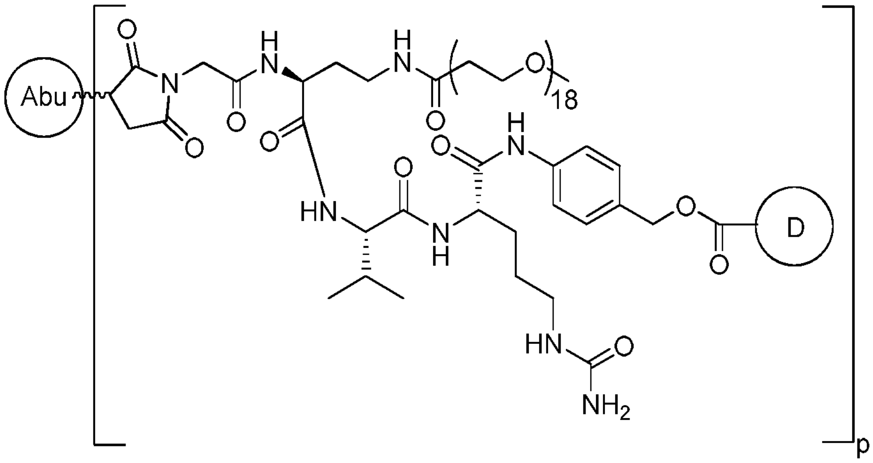

or

Formula I-A-10

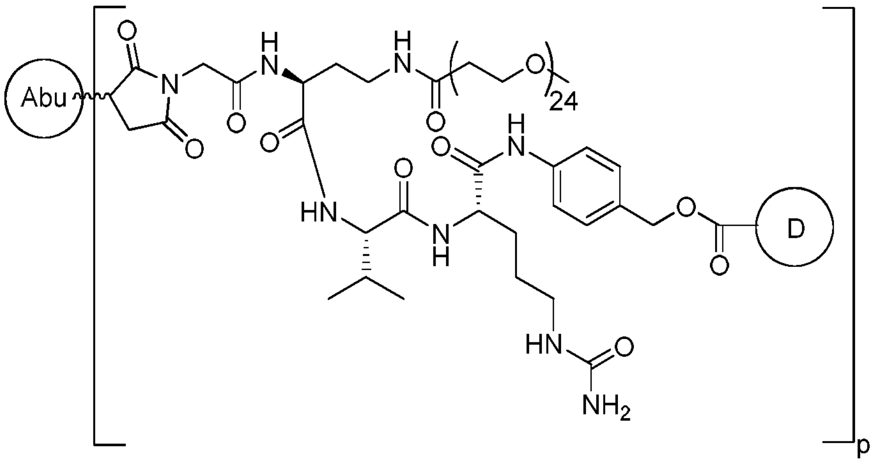

Formula I-A-11

wherein

Abu is the anti-Nectin-4 antibody or antigen-binding unit, or the polypeptide specifically binding to Nectin-4 as described in the application

D is a drug (such as the drug described herein);

p is 1-10.

In one or more embodiments, p is 2-8.

In one or more embodiments, p is 4-8.

In one or more embodiments, p is 6-8.

In one or more embodiments, p is 7-8.

In one or more embodiments, the drug in the antibody-drug conjugate described herein is a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is an anti-cancer drug.

In one or more embodiments, the drug is a tubulin inhibitor, a DNA damaging agent, or a DNA topoisomerase inhibitor.

In one or more embodiments, the drug is a tubulin inhibitor, the tubulin inhibitor is selected from dolastatin, auristatins and maytansines.

In one or more embodiments, the drug is an auristatin, selected from monomethyl auristatin E (MMAE), monomethyl auristatin F (MMAF), and auristatin F (AF).

In one or more embodiments, the drug is a DNA damaging agent, selected from calicheamicin, duocarmycin, and anthramycin derivative PBD (pyrrolobenzodiazepine).

In one or more embodiments, the drug is a DNA topoisomerase inhibitor or a salt thereof, selected from irinotecan, irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, and N-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan.

In one or more embodiments, the drug is

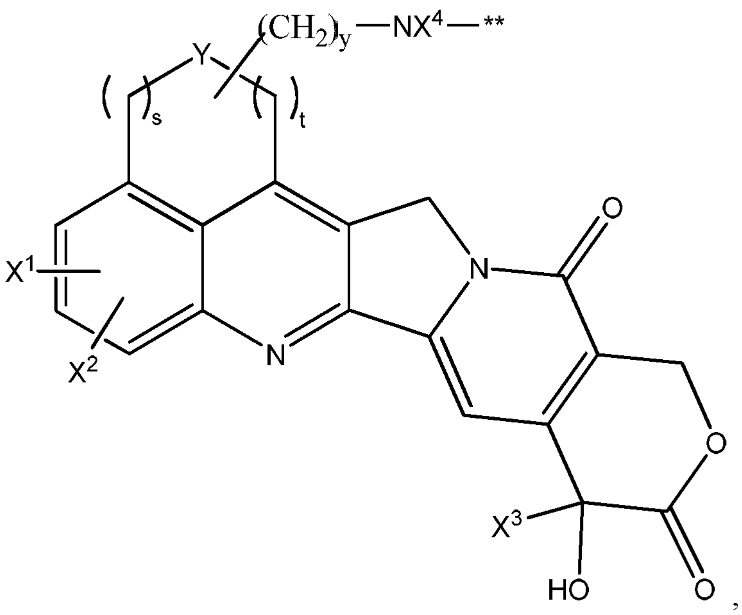

wherein $X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

sulfhydryl,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or **C1-**C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl optionally substituted at a nitrogen atom of the heterocyclyl moiety or at the amino moiety with a protecting group or one or more C1-C6 alkyl groups,

heterocyclylamino optionally substituted at a nitrogen atom of the heterocyclyl moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NRn(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NRn(CH_2CH_2O)_q-CH_2-CH_3$, $-(CH_2CH_2O)_qC(O)NR^n(CH_2CH_2O)_q-CH_3$, - $(CH_2CH_2O)_qC(O)NR^n(CH_2CH_2O)_q-CH_2-CH_3$, or $-(CH_2CH_2O)_qC(O)NR^n(CH_2)_q-CH_3$; wherein each $R^n$ is independently H, C1-C6 alkyl, C3-C8 carbocyclyl, phenyl, or benzyl, and each q is independently 1, 2, 3, 4, 5, 6, 7, 8, 9, or 10;

** is the linking point;

y is 0, 1 or 2;

Y is O, S or $CR^{1D}R^{2D}$, wherein $R^{1D}$ and $R^{2D}$ are each independently H or C1-C6 alkyl;

s and t are each independently 0, 1 or 2, but not both 0.

In one or more embodiments, $X^4$ is H or C1-C6 alkyl.

In one or more embodiments, the drug is

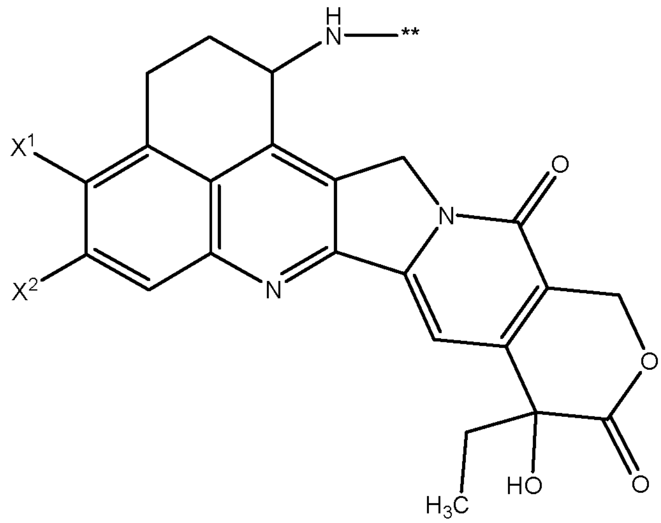

,

wherein $X^1$ and $X^2$ are each independently C1-C6 alkyl, halogen or -OH, ** is the linking point.

In one or more embodiments, the drug is

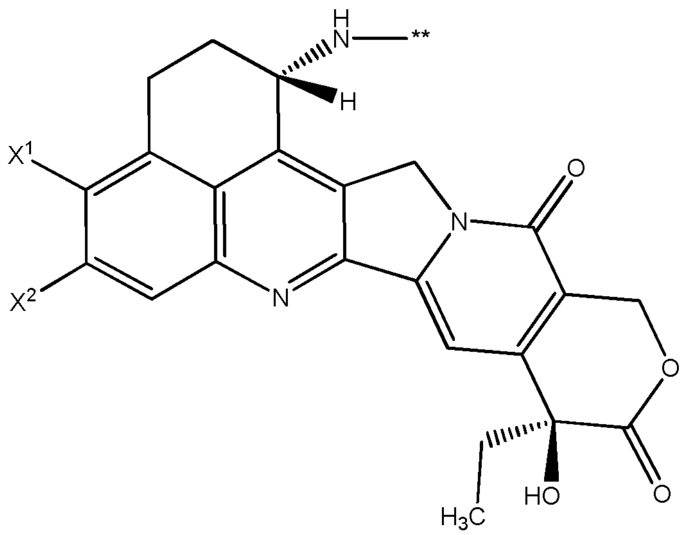

,

wherein $X^1$ and $X^2$ are each independently C1-C6 alkyl, halogen or -OH, ** is the linking point.

In one or more embodiments, the drug is

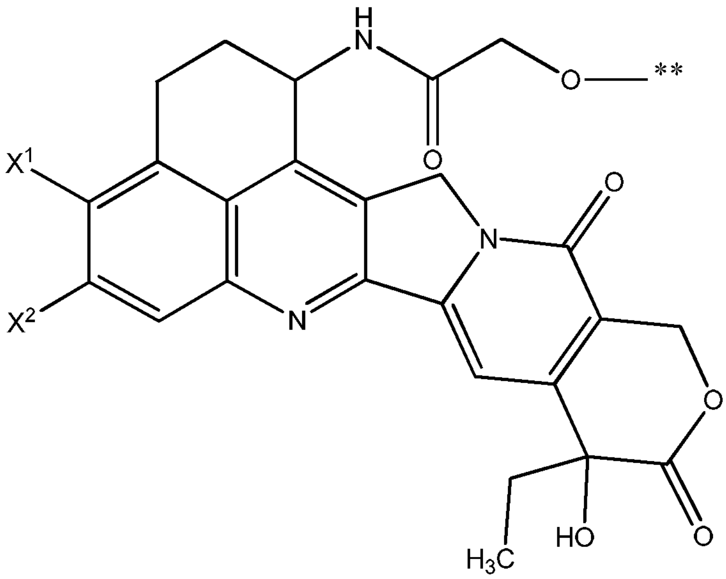

,

wherein $X^1$ and $X^2$ are each independently C1-C6 alkyl, halogen or -OH, ** is the linking point.

In one or more embodiments, the drug is

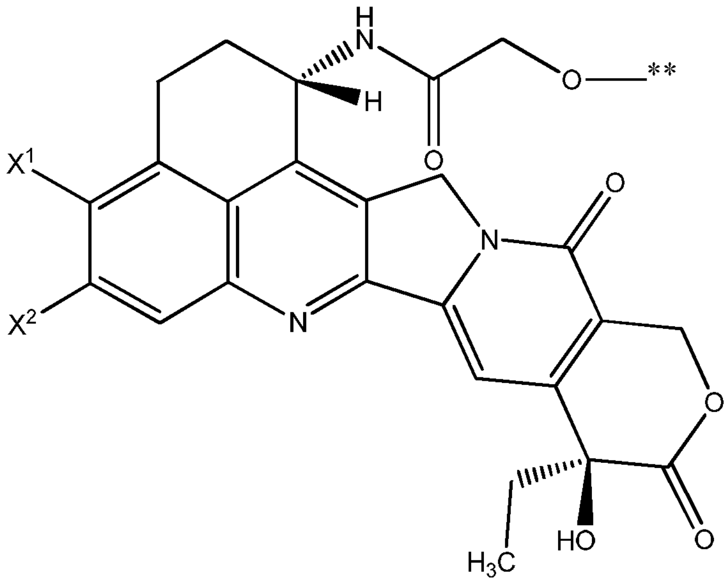

,

wherein $X^1$ and $X^2$ are each independently C1-C6 alkyl, halogen or -OH; ** is the linking point.

In one or more embodiments, C1-C6 alkyl is $-CH_3$.

In one or more embodiments, the halogen is F.

In one or more embodiments, $X^1$ and $X^2$ are each independently $-CH_3$

In one or more embodiments, $X^1$ and $X^2$ are each independently F, Cl, Br, or I.

In one or more embodiments, $X^1$ and $X^2$ are each F.

In one or more embodiments, X1 and X2 are each independently $-CH_3$, F or -OH.

In one or more embodiments, $X^1$ and $X^2$ are each independently F or $-CH_3$.

In one or more embodiments, $X^1$ is $-CH_3$ and $X^2$ is F.

In one or more embodiments, the drug is

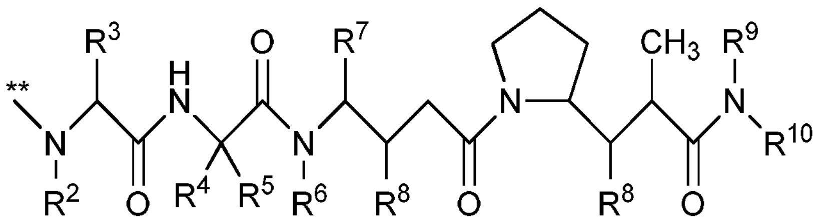

wherein

** is the linking point;

$R^2$ is H or C1-C8 alkyl;

$R^3$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);

$R^4$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);

$R^5$ is H or methyl;

or $R^4$ and $R^5$ are linked to form a carbocyclyl having a formula of $-(CR^aR^b)_j-$, wherein $R^a$ and $R^b$ are each independently H, C1-C8 alkyl, or C3-C8 carbocyclyl, and j is 2, 3, 4, 5, or 6;

$R^6$ is H or C1-C8 alkyl;

$R^7$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);

each $R^8$ is independently H, OH, C1-C8 alkyl, C3-C8 carbocyclyl, or O-(C1-C8 alkyl);

$R^9$ is H or C1-C8 alkyl; and

$R^{10}$ is $-C(R^8)_2-C(R^8)_2$-aryl, $-C(R^8)_2-C(R^8)_2$-(C3-C8 heterocyclyl), or $-C(R^8)_2-C(R^8)_2$-(C3-C8 carbocyclyl).

In one or more embodiments, the drug is

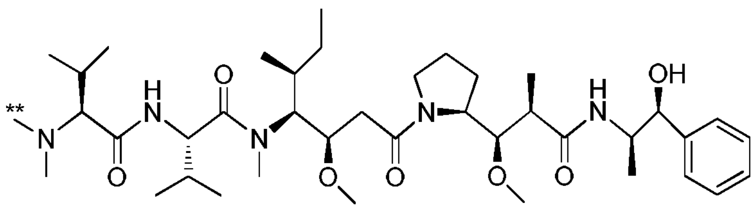

wherein ** is the linking point.

In one or more embodiments, provided is an antibody-drug conjugate having a structure shown in Formula I-A-12, I-A-13, I-A-14, I-A-15, I-A-16, I-A-17, I-A-18, I-A-19, I-A-20, I-A-21, I-A-22, I-A-23, I-A-24, or I-A-25 or a stereoisomer thereof, or a pharmaceutically acceptable salt or solvate thereof, wherein the Formulas I-A-12, I-A-13, I-A-14, I-A-15, I-A-16, I-A-17, I-A-18, I-A-19, I-A-20, I-A-21, I-A-22, I-A-23, I-A-24, and I-A-25 are:

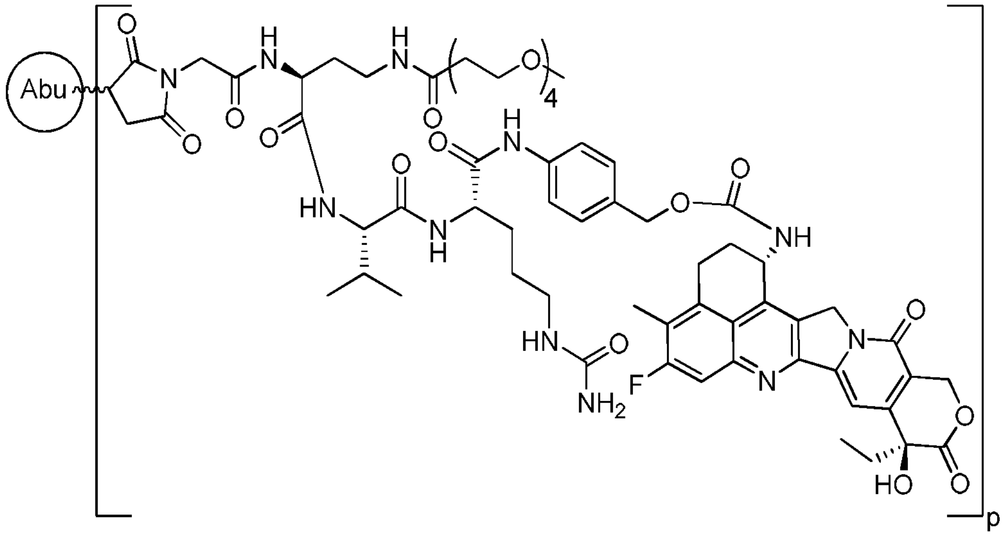

Formula I-A-12

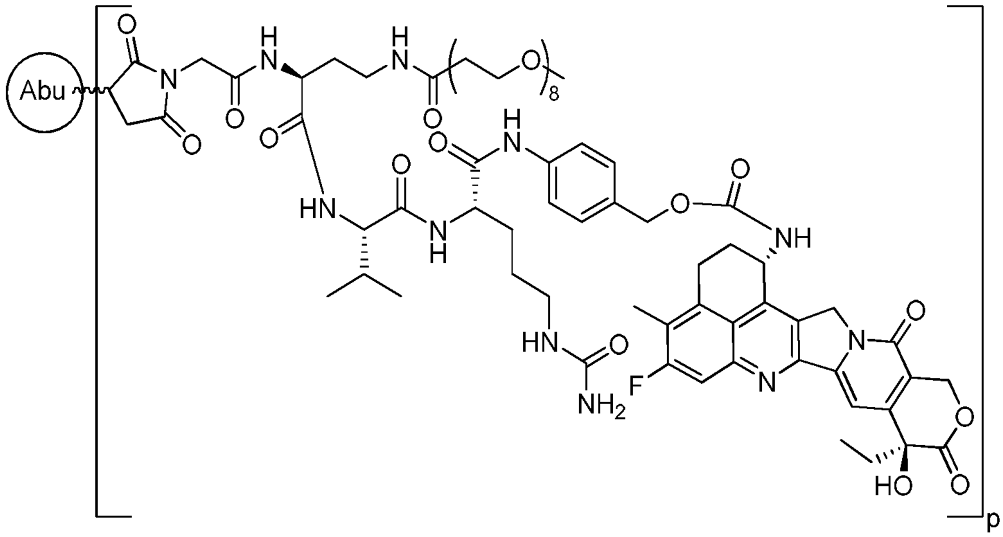

Formula I-A-13

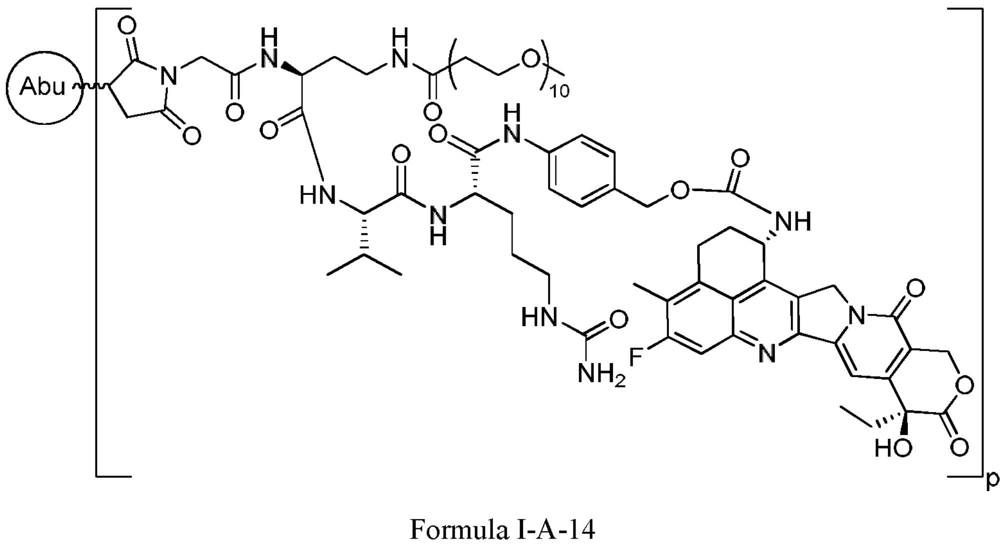

Formula I-A-14

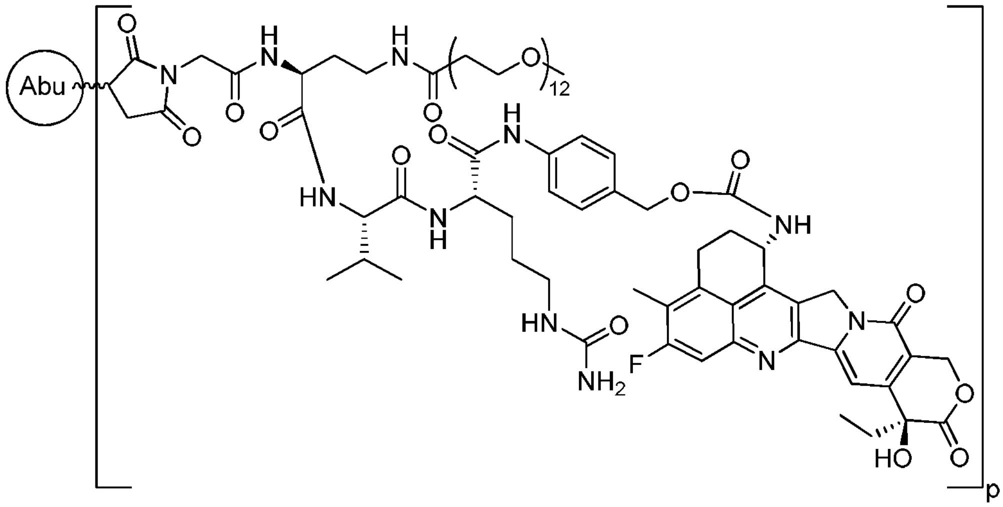

Formula I-A-15

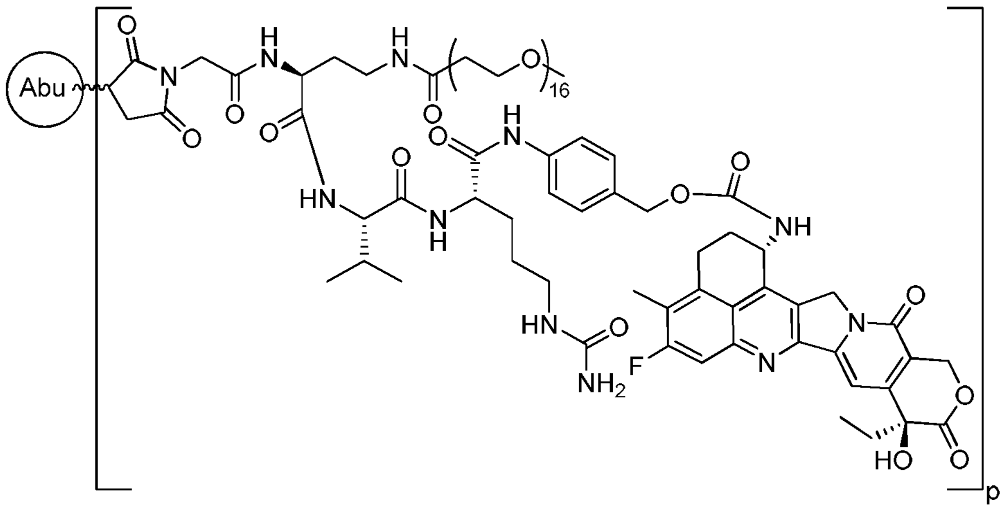

Formula I-A-16

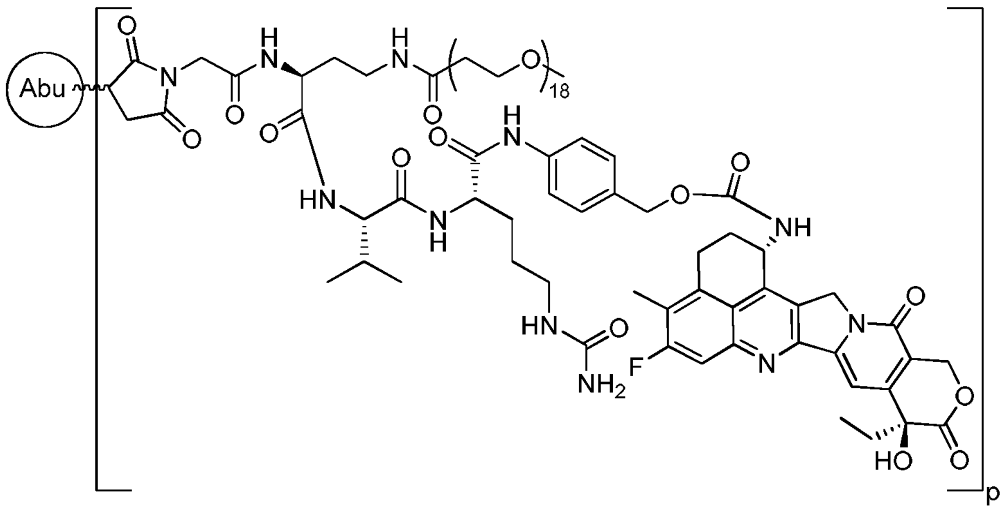

Formula I-A-17

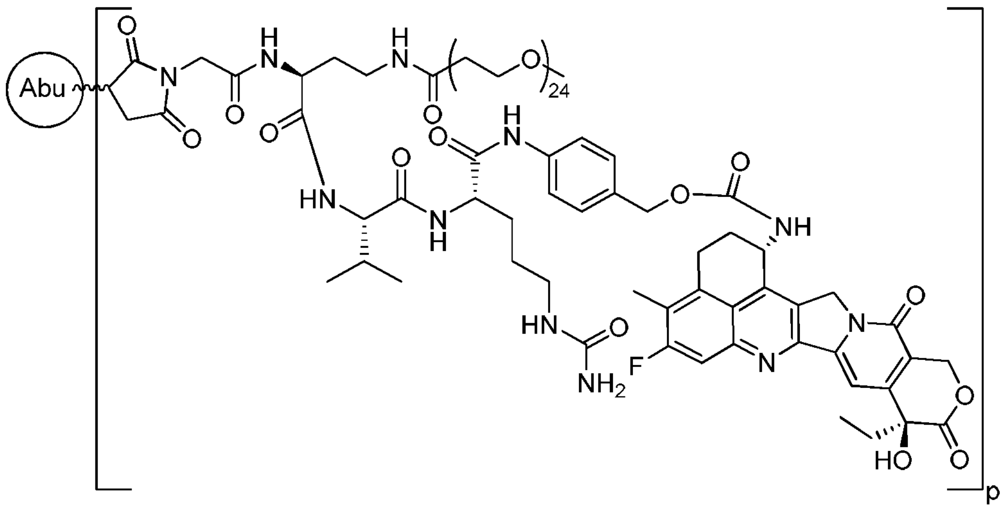

Formula I-A-18

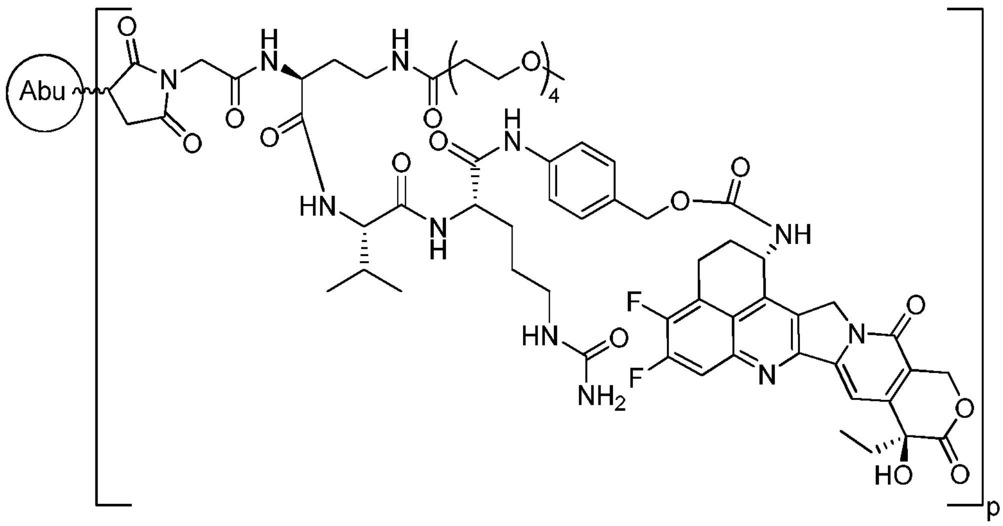

Formula I-A-19

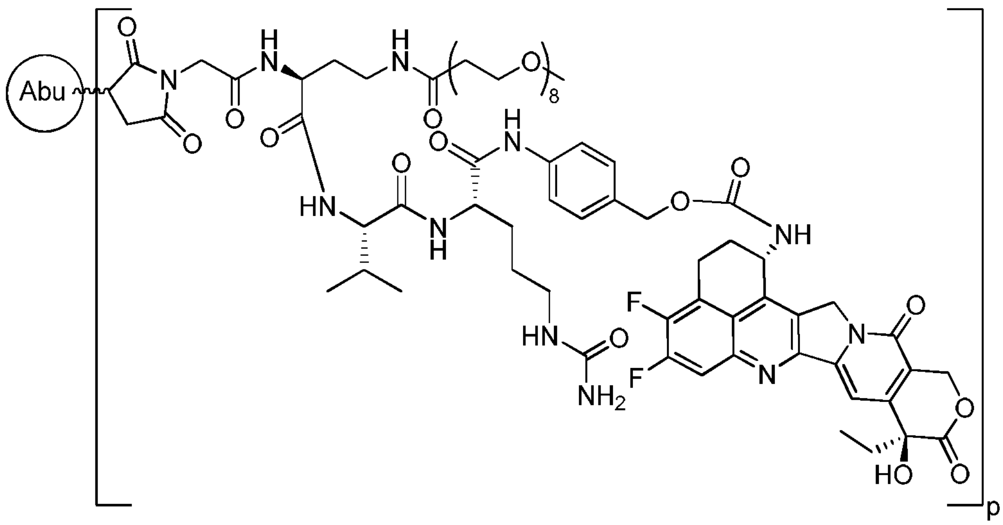

Formula I-A-20

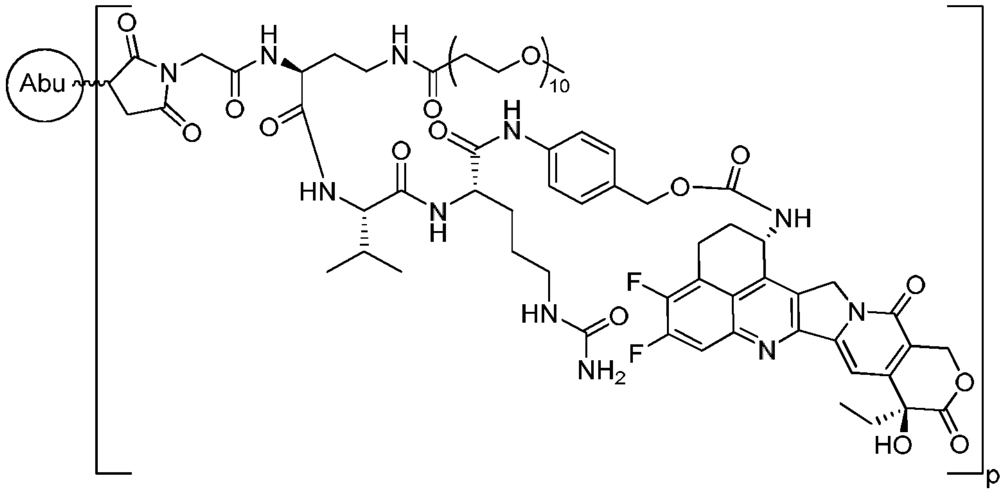

Formula I-A-21

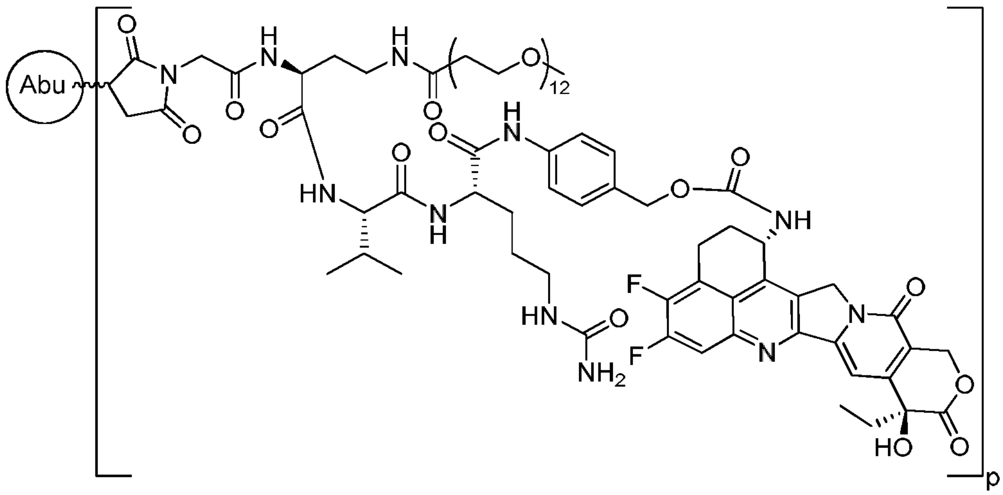

Formula I-A-22

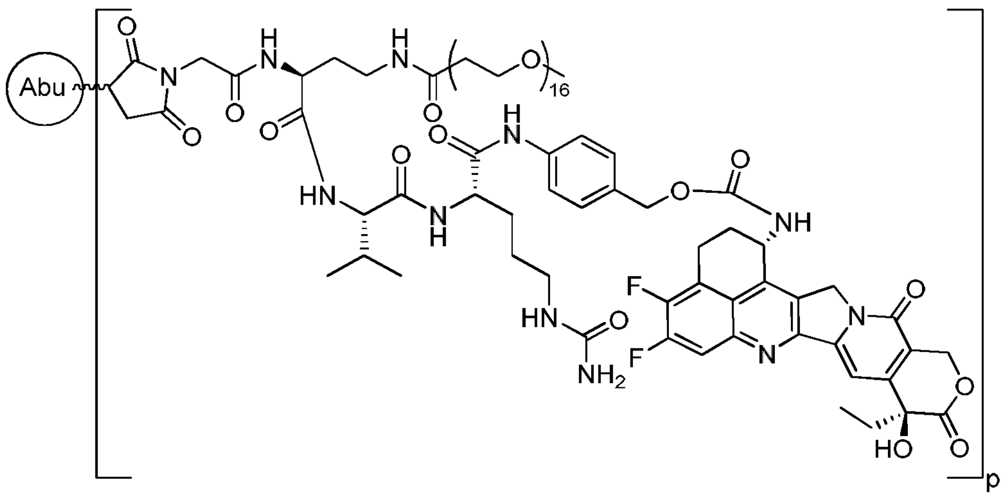

Formula I-A-23

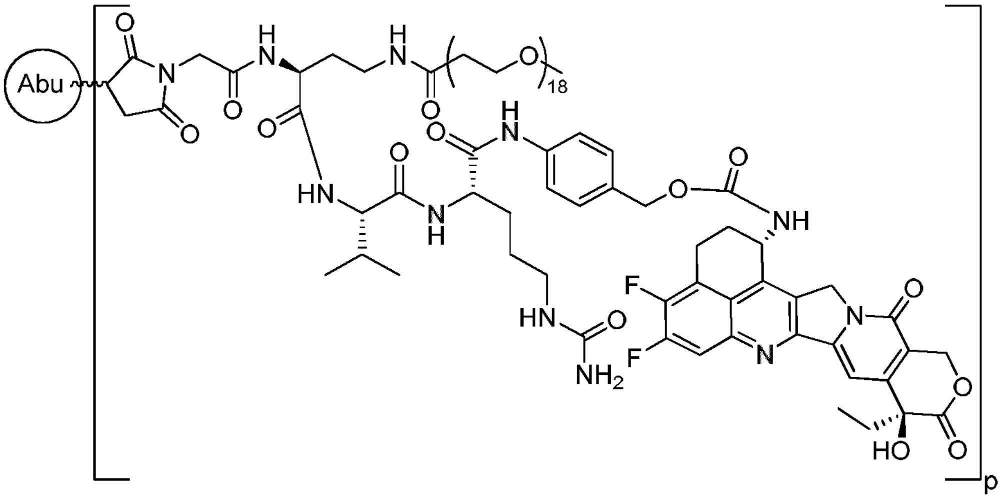

Formula I-A-24

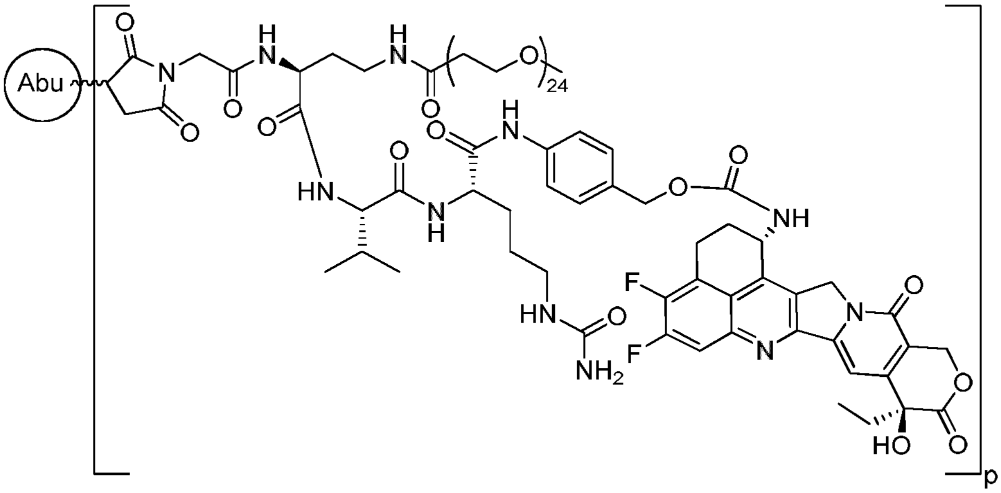

Formula I-A-25

wherein

Abu is the anti-Nectin-4 antibody or antigen-binding unit, or the polypeptide specifically binding to Nectin-4 as described in the application;

p is 1-10.

In one or more embodiments, p is 2-8.

In one or more embodiments, p is 4-8.

In one or more embodiments, p is 6-8.

In one or more embodiments, p is 7-8.

In one or more embodiments, provided is an antibody-drug conjugate having a structure shown in Formula I-B-1 or a stereoisomer thereof, or a pharmaceutically acceptable salt or solvate thereof, wherein the Formulas I-B-1 is:

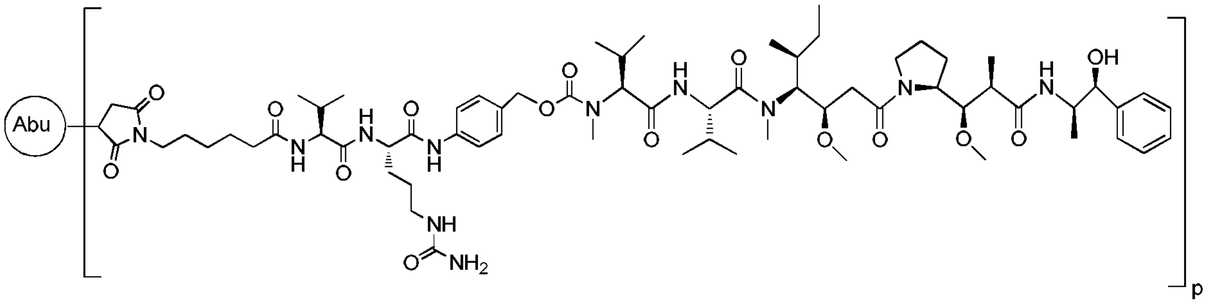

Formulas I-B-1

wherein

Abu is the anti-Nectin-4 antibody or antigen-binding unit, or the polypeptide specifically binding to Nectin-4 as described in the application;

p is 1-10.

In one or more embodiments, p is 2-8.
In one or more embodiments, p is 4-8.
In one or more embodiments, p is 6-8.
In one or more embodiments, p is 7-8.
In one or more embodiments, provided is an antibody-drug conjugate having a structure shown in Formula I-C-1 or a stereoisomer thereof, or a pharmaceutically acceptable salt or solvate thereof, wherein the Formulas I-C-1 is:

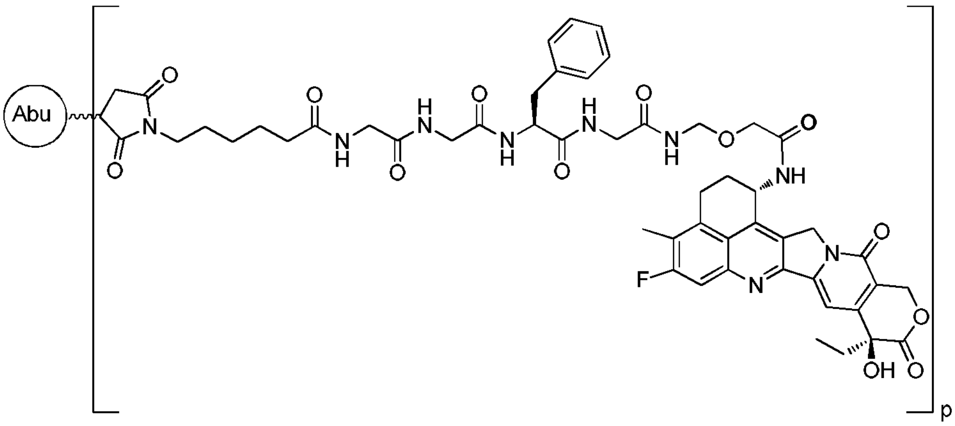

Formulas I-C-1

wherein

Abu is the anti-Nectin-4 antibody or antigen-binding unit, or the polypeptide specifically binding to Nectin-4 as described in the application;

p is 1-10.

In one or more embodiments, p is 2-8.
In one or more embodiments, p is 4-8.
In one or more embodiments, p is 6-8.
In one or more embodiments, p is 7-8.
In one or more embodiments, a pharmaceutical composition is provided comprising the anti-Nectin-4 antibody or antigen-binding unit, the biomaterial, or the antibody-drug conjugate or the pharmaceutically acceptable salt or solvate thereof, and a pharmaceutically acceptable carrier, excipient, and/or adjuvant material as described in the application. In one or more embodiments, the pharmaceutical composition further comprises an additional drug. The pharmaceutical

composition may be administered by any convenient route, e.g., by infusion or bolus injection, by absorption through epithelial or cutaneous mucosa (e.g., oral mucosa or rectal and intestinal mucosa), and may be co-administered with other biologically active agents. Therefore, the pharmaceutical composition may be administered subcutaneously, intravenously, orally, rectally, parenterally, intracisternally, intravaginally, intraperitoneally, topically (e.g. through powder, ointment, drops or transdermal patch), buccally or by oral or nasal spray.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

In one or more embodiments, provided is use of the anti-Nectin-4 antibody or antigen-binding unit, the biomaterial, the antibody-drug conjugate or the pharmaceutically acceptable salt or solvate thereof, or the pharmaceutical composition described herein in preparing a medicament for treating a disease. In one or more embodiments, the medicament is also used in combination with an additional drug.

In one or more embodiments, provided is use of the antibody or antigen-binding unit described herein, the biomaterial described herein, the antibody-drug conjugate or the pharmaceutically acceptable salt or solvate thereof described herein, or the pharmaceutical composition described herein in treating a disease. In one or more embodiments, the use includes combined use with an additional anti-cancer drug.

In one or more embodiments, provided is a method for treating a disease, comprising: administering to a patient in need an effective amount of the anti-Nectin-4 antibody or antigen-binding unit, the antibody-drug conjugate or the pharmaceutically acceptable salt or solvate thereof, or the pharmaceutical composition described herein. The effective amount refers to an amount of an active compound or drug that results in a biological or pharmacological response in tissues, systems, animals, individuals, and humans which is being sought by researchers, vets, doctors, or other clinical physicians, including the treatment of a disease. In general, the effective amount can be about 0.1-100 mg/kg, and the administration frequency can be, for example, once a month. The administration method can be intravenous infusion, intravenous push injection, subcutaneous injection, intramuscular injection, etc. In one or more embodiments, the method further comprises administering additional anti-cancer drugs to the patient.

In one or more embodiments, the disease is a disease associated with the expression or overexpression of Nectin-4. In one or more embodiments, the disease is a disease associated with the abnormal expression of Nectin-4. In one or more embodiments, the disease is a cancer or tumor. In one or more embodiments, the disease is a tumor expressing or overexpressing Nectin-4. In one or more embodiments, the disease is a cancer expressing or overexpressing Nectin-4. In one or more embodiments, the disease is a solid tumor or a hematologic cancer. In one or more embodiments, the disease is selected from breast cancer (e.g., triple-negative breast cancer (TNBC); topical or metastatic TNBC), pancreatic cancer, bladder cancer, urothelial carcinoma, melanoma, lung cancer (e.g., non-small cell lung cancer, squamous cell cancer, or lung adenocarcinoma), head and neck cancer (e.g., head and neck squamous cell carcinoma), cervical cancer (e.g., cervical squamous cell carcinoma), ovarian cancer, choriocarcinoma, skin cancer, esophageal cancer (e.g., esophageal adenocarcinoma), gastric cancer, uterine cancer (e.g., endometrial cancer), gallbladder cancer, liver cancer, hepatocellular carcinoma, urethral carcinoma, renal pelvic carcinoma, ureteral cancer, colorectal cancer, colon cancer, and prostate cancer.

In one or more embodiments, the antibody-drug conjugate or the pharmaceutically acceptable salt or solvate thereof is the antibody-drug conjugate of Formula I-A, Formula I-B or Formula I-C of the present application or the pharmaceutically acceptable salt or solvate thereof.

The pharmaceutically acceptable salts include the antibody-drug conjugates, with a variety of organic and inorganic counterions well known in the art, and salts as examples only include organic or inorganic salts such as sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine piperazine, piperidine, N-ethyl, piperidine, polyamine resin, and tetraalkylammonium salt, etc., when the molecule contains an acidic functional group; and organic or inorganic acid salts such as hydrochloride salt, hydrobromide salt, tartrate salt, mesylate salt, acetate salt, maleate salt and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid cinnamic acid, mandelic acid, methanesulfonic acid, ethanesulfonic acid, p-toluenesulfonic acid salicylic acid, etc. The solvate includes hydrates.

One or more embodiments provide a product comprising the anti-Nectin-4 antibody or antigen-binding unit, the antibody-drug conjugate, or the pharmaceutically acceptable salt or the solvate thereof, or the pharmaceutical composition described herein;

a container; and

a package insert, instruction or label indicating that the anti-Nectin-4 antibody or antigen-binding unit, the antibody-drug conjugate, or the pharmaceutically acceptable salt or the solvate thereof, or the pharmaceutical composition is for use in treating a disease.

## BRIEF DESCRIPTION OF THE DRAWINGS

FIG. 1 shows the affinity assay of anti-Nectin-4 antibodies.

FIG. 2 shows the binding activity of anti-Nectin-4 antibodies to T-47D cells.

FIG. 3 shows the specificity assay results of anti-Nectin-4 antibodies for human Nectin-4 homologous proteins.

FIG. 4 shows the binding of anti-Nectin-4 antibodies to Nectin-4 of different species.

FIG. 5 shows the proliferation inhibitory effects of ADCs on OVCAR-3 and MDA-MB-468 cells, wherein FIGs. 5A and 5B show the proliferation inhibitory effects of ADCs on OVCAR-3 cells; FIG. 5C shows the proliferation inhibitory effect of 1F3-1E1-MMAE on MDA-MB-468 cells.

FIG. 6 shows the concentration-time curves of 10F4-3-ExaD6 and 10F4-3-ExaD8 total antibodies and ADCs in rats, wherein, D6-Tab denotes the 10F4-3-ExaD6 total antibody, D6-ADC denotes the 10F4-3-ExaD6 ADC, D8-Tab denotes the 10F4-3-ExaD8 total antibody, and D8-ADC denotes the 10F4-3-ExaD8 ADC.

FIG. 7 shows the bystander killing effect of 10F4-3-Exa6, wherein FIG. 7A shows the direct killing effect of 10F4-3-Exa6 on CHO-N4 and CHO-K1; FIG. 7B shows the killing effect of 10F4-3-Exa6 on CHO-K1 after the transfer of the culture supernatant, wherein CM denotes the culture supernatant transfer, CHO-N4 and CHO-K1 denote the transferred culture supernatants, and D2, D3, and D4 denote the days of culture at the time of CHO-N4 and CHO-K1 transfer.

FIG. 8 shows the growth curves of tumor volume (mean ± standard error) in all groups of mice in the human breast cancer MDA-MB-468 nude mouse subcutaneous xenograft tumor model receiving ADCs.

FIG. 9 shows the growth curves of tumor volume (mean ± standard error) in all groups of mice in the JEG-3 xenograft model receiving 10F4-3-ExaD8.

FIG. 10 illustrates the growth curves of tumor volume (mean ± standard error) in all groups of mice in the HuPrime® bladder cancer BL0597 xenograft BALB/c nude mouse animal model receiving ADCs.

## DETAILED DESCRIPTION

Unless otherwise defined, scientific and technical terms used in the present invention have the meanings that are commonly understood by those skilled in the art.

### Definition

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of..." means that the antibodies, compositions, methods or the like exclude other elements that have a fundamental impact on

the characteristics of the combination, but do not exclude elements that do not substantially affect the characteristics of the antibodies, compositions, methods or the like. "Consisting of..." means that elements not specifically listed are excluded.

The term "antibody" as used herein refers to immunoglobulin (Ig) molecules and immunologically active portions of immunoglobulin molecules, i.e., molecules that contain an antigen-binding site that specifically binds to (immunoreacts with) an antigen. Antibodies include, but are not limited to, monoclonal antibodies, chimeric antibodies, dAbs (domain antibodies), single-chain antibodies, Fab, Fab- and F(ab')2 fragments, Fv and Fab expression libraries.

The term "antibody" includes a wide variety of polypeptides that can be biochemically distinguished.

The antibodies, antigen-binding units or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and F(ab')2), and single-chain Fv (scFv).

The term "monoclonal antibody" (mAb) refers to a population of antibody molecules that contain only one molecular species of the antibody molecules consisting of a unique light chain gene product and a unique heavy chain gene product. In particular, the complementarity determining regions (CDRs) of the monoclonal antibody are identical in all the molecules of the population. MAbs contain an antigen-binding site capable of immunoreacting with a particular epitope of an antigen.

The term "single-chain antibody" (scFv) refers to an antibody in which its heavy chain variable region (VH) and light chain variable region (VL) are linked by a linker of 15-20 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

Those skilled in the art will appreciate that the classes of heavy chains include gamma, mu, alpha, delta, or epsilon (γ, μ, α, δ, or ε), and some subclasses (e.g., γ1-γ4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In one or more embodiments, the type of immunoglobulin molecule is IgG. Two heavy chains and two light chains are connected in a "Y" configuration through disulfide bonds, wherein the light chain starts at the opening of "Y" configuration and extends through the variable region to surround the heavy chain.Light chains can be classified into kappa (κ) or lambda (λ). Each heavy chain may bind to a κ or λ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; and the immunoglobulin λ light chain variable region is Vλ.

The light chain variable region (VL) and the heavy chain variable region (VH) of the antibody determine the antigen recognition and specificity. The light chain constant region (CL) and the heavy chain constant region (CH) impart important biological properties such as secretion, transplacental movement, Fc receptor binding, complement fixation, etc.. By convention, the numbering of amino acids in the constant regions increases as they become further away from the antigen-binding site of the antibody or amino terminus. The N-terminal portion is the variable region, and the C-terminal portion is the constant region; the CH3 and CL domains of the IgG1 antibody actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region ("FR"), exhibit little intermolecular variability. Most of the framework regions adopt a β-sheet conformation, with the CDRs forming a loop structure connected to, or in some cases forming part of the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. Generally, in an antibody molecule, the heavy and light chains each have three CDRs, referred to as VH CDR1, VH CDR2, VH CDR3, VL CDR1, VL CDR2, and VL CDR3, respectively. In positional order, the heavy chain variable region typically comprises VH FR1, VH CDR1, VH FR2, VH CDR2, VH FR3, VH CDR3, and VH FR4, and the light chain variable region comprises VL FR1, VL CDR1, VL FR2, VL CDR2, VL FR3, VL CDR3, and VL FR4. For a given heavy or light chain variable region, amino acids in the CDRs and the framework regions may be identified by those of ordinary skill in the art according to known methods (see, Kabat, E., et al., U.S. Department of Health and Human Services, Sequences of Proteins of Immunological Interest, (1983) and Chothia and Lesk, J. Mol. Biol., 196: 901-917 (1987)).

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. Usually, at least 80%, at least 85%, at least 90% or at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entirety.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the antibody may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant $\kappa$ domain or a constant $\lambda$ domain. "Light chain-heavy chain pair" refers to a collection of light and heavy chains that can form dimers through disulfide bonds between the CL domain of the light chain and the CH1 domain of the heavy chain.

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

The term "epitope" as used herein includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

As used herein, the term "specific bind to" or "immune response" refers to a non-covalent interaction between an immunoglobulin molecule and one or more antigenic determinants of its target antigen. The strength or affinity of an immunological binding interaction can be expressed in terms of the equilibrium dissociation constant (KD) of the interaction, where a smaller KD represents a greater affinity. The immunological binding properties of the selected polypeptides may be quantified using methods well known in the art. One such method requires the measurement of the rates of antigen-binding site/antigen complex formation and dissociation, where those rates depend on the concentration of the complex partners, the affinity of the interaction and geometric parameters that affect the rates equally in both directions. Thus, both "association rate constant" ($k_{on}$) and "dissociation rate constant" ($k_{off}$) can be determined by calculating the concentration and the actual association and dissociation rates (see Malmqvist, M.,Nature 361: 186-87 (1993)). The ratio $k_{off}/k_{on}$ is capable of eliminating all the parameters that are independent of affinity and is equal to the equilibrium dissociation constant KD (see Davies et al., (1990) Annual Rev Biochem 59:439-473). Specific binding can be

measured by radioligand binding assays, surface plasmon resonance (SPR), flow cytometry binding assay or similar assays known to those skilled in the art.

The term "isolated" as used herein with respect to cells, nucleic acids, polypeptides and the like, e.g., "isolated" DNA, RNA or polypeptides, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides and the like are usually prepared by at least one purification step. In one or more embodiments, the purity of the isolated nucleic acids, polypeptides or the like is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Amino acid" refers to an organic compound containing both an amino group and a carboxyl group, such as an α-amino acid or β-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids.

As used herein, the twenty conventional amino acids and their abbreviations follow conventional usage. See Immunology-A Synthesis (second edition, Eds. E. S. Golub and D. R. Gren, Sinauer Associates, Sunderland Mass. (1991)). Stereoisomers (e.g., D-amino acids) of the twenty conventional amino acids, unnatural amino acids (such as α- or α-disubstituted amino acids), N-alkyl amino acids, lactic acid, and other unconventional amino acids can also be suitable components for the polypeptides of the present disclosure. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylaiginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V) etc.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence. It may be produced in any manner, including chemical synthesis.

The term "substantially identical" when applied to polypeptides means that two peptide sequences, when optimally aligned, such as by the program GAP or BESTFIT using default GAP weights, share at least 80% sequence identity, preferably at least 90% sequence identity, more preferably at least 95% sequence identity, and most preferably at least 99% sequence identity.

A polynucleotide consists of a specific sequence of four bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may

have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, DNA, RNA, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

A polynucleotide or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), *Current Protocols in Molecular Biology.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

Minor variations in the amino acid sequences of antibodies or immunoglobulin molecules are contemplated by the present disclosure, provided that the identity of the amino acid sequences is maintained to be at least 90%, such as at least 92%, 95%, 98% or 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984*));* Introduction to Protein Structure (Eds. C. Branden and J. Tooze, Garland Publishing, New York, N.Y. (1991)); and Thornton et al., Nature 354:105 (1991).

The number of amino acids of conservative amino acid substitutions of VL or VH may be about 1, about 2, about 3, about 4, about 5, about 6, about 8, about 9, about 10, about 11, about 13, about 14, about 15, or a range between any two of these values (inclusive) or any value therein. The number of amino acids of conservative amino acid substitutions of a heavy chain constant region, a light chain constant region, a heavy chain or a light chain may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a

range between any two of these values (inclusive) or any value therein.

The term "agent" as used herein means a chemical compound, a mixture of chemical compounds, a biological macromolecule, or an extract made from biomaterial.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$, $^{125}I$ and $^{131}I$), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance. The term "medicament" or "drug" refers to a compound or composition that is capable of inducing the desired therapeutic effect when properly administered to a patient.

"About" refers to a general error range for corresponding values as readily understood by those skilled in the relevant art. In some embodiments, "about" mentioned herein refers to the values described and ranges thereof of $\pm$ 10%, $\pm$5% or $\pm$1%.

"$EC_{50}$", i.e., concentration for 50% of maximal effect, refers to a concentration that causes 50% of maximal effect.

"$IC_{50}$" represents 50% inhibitory concentration, i.e., a concentration of drug or inhibitor required to inhibit half of a given biological process.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "tumor" means or is intended to describe the physiological state of a mammal, which is typically characterized by uncontrolled cell growth, including benign tumors and malignant tumors such as cancer. Examples of cancers include, but are not limited to, carcinoma, lymphoma, blastoma, sarcoma or leukemia. More specific examples of such cancers include, but are not limited to, colorectal cancer, lung cancer, ovarian cancer, uterine cancer, endometrial cancer, colon cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, rectal cancer, prostate cancer, vulval cancer, testicular cancer, squamous cell carcinoma, small cell lung cancer, cervical cancer, bladder cancer, retinoblastoma, glioblastoma, mesothelioma, oral epithelioid cancer, choriocarcinoma and head and neck cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression may be by 1%, 5%, 10%, 20%, 30%, 50%, 60%, 70%, 80%, 90% or more in comparison to a normal cell or control cell. In some embodiments, the anti-Nectin-4 antibody and the antibody-drug conjugate of the present invention is used for treating a solid tumor expressing or overexpressing Nectin-4.

As used herein, the term "tumor overexpressing Nectin-4" refers to a tumor that overexpresses Nectin-4 (including benign tumors and cancers). In some embodiments, Nectin-4 expression in a tumor sample above the immunohistochemical background level (e.g., as determined by immunohistochemical staining) indicates that the tumor is a tumor overexpressing Nectin-4. Methods for detecting the expression of Nectin-4 in a tumor are known in the art, such as immunohistochemical assays.

As used herein, the terms "give" and "administer" are used interchangeably and refer to the delivery of a substance (e.g., anti-Nectin-4 antibody or ADC) for therapeutic purposes (e.g., treating a disease associated with the expression or overexpression of Nectin-4). The mode of administration may be parenteral, enteral, and topical. Parenteral administration is typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

As used herein, the term "effective amount" or "therapeutically effective amount" refers to the amount of a drug, e.g., an antibody or ADC, that is sufficient to reduce or ameliorate the severity and/or duration of a disorder (e.g., cancer) or one or more symptoms thereof, prevent the progression of a disorder, cause regression of a disorder, prevent the recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The terms "patient" and "subject" are used interchangeably and refer to any mammal in need of diagnosis, prognosis or treatment, including, but not limited to, humans, dogs, cats, guinea pigs, rabbits, rats, mice, horses, cattle, and the like.

As used herein, the term "in need" means that the patient has been identified as in need of a particular method or treatment. In some embodiments, identification may be made by any diagnostic mode. The patient may be in need of any methods and treatments described herein.

The term "drug for treating a tumor" as used herein refers to an agent having the functional property of inhibiting in a human the development or progression of a tumor, particularly a malignant (cancerous) lesion, such as cancer, sarcoma, lymphoma or leukemia. Inhibition of metastasis is a property of antineoplastic drugs in many cases.

The term "antibody-drug conjugate" or "ADC" refers to a binding protein (e.g., an antibody or an antigen-binding unit) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC are a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor) and a radiosensitizer.

The terms "antibody-drug conjugate" and "ADC" are used interchangeably. The terms "anti-Nectin-4 antibody-drug conjugate" and "anti-Nectin-4 ADC" are used interchangeably to refer to an ADC comprising an antibody that specifically binds to Nectin-4, wherein the antibody is conjugated to one or more drugs. In one or more embodiments, the anti-Nectin-4 ADC comprises an antibody conjugated to exatecan. In one or more embodiments, the anti-Nectin-4 antibody or ADC binds to Nectin-4 (e.g., human Nectin-4).

The term "drug to antibody ratio" or "DAR" refers to the number of drugs (e.g., exatecan) that are attached to the antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino and aryl) is indicated by the prefix "Cx-Cy" or "Cx-y", wherein x is the minimum number and y is the maximum number of carbon atoms. Thus, for example, "C1-C6 alkyl" refers to an alkyl containing 1 to 6 carbon atoms. If a substituent is described as "substituted with... ", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. To illustrate, monofluoroalkyl is an alkyl substituted with a fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, sulfhydryl, alkylthio, amino, C1-C6 aminoalkyl, C1-C6 aminoalkylamino, C1-C6 alkyl linking to a heterocycle, C1-C6 alkylamino linking to a heterocycle, heterocyclyl, amino-substituted heterocyclyl, heterocyclylamino, carbamoyl, morpholin-1-yl, piperidin-1-yl, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -

arylene-$(CH_2)_q$-$CH_3$, -$(CH_2)_q$-(C3-C8 carbocyclyl)-$CH_3$, -(C3-C8 carbocyclyl)-$(CH_2)_q$-$CH_3$, C3-C8 heterocyclyl, -$(CH_2)_q$-(C3-C8 heterocyclyl)-$CH_3$, -(C3-C8 heterocyclyl)-$(CH_2)_q$-$CH_3$, - $(CH_2)_qC(O)NR^n(CH_2)_q$-$CH_3$, -$(CH_2CH_2O)_q$-$CH_3$, -$(CH_2CH_2O)_q$-$CH_2$-$CH_3$, -$(CH_2)_qC(O)NR^n(CH_2CH_2O)_q$-$CH_3$, -$(CH_2)_qC(O)NR^n(CH_2CH_2O)_q$-$CH_2$-$CH_3$, -$(CH_2CH_2O)_qC(O)NR^n(CH_2CH_2O)_q$-$CH_3$, - $(CH_2CH_2O)_qC(O)NR^n(CH_2CH_2O)_q$-$CH_2$-$CH_3$ or -$(CH_2CH_2O)_qC(O)NR^n(CH_2)_q$-$CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, *n*-propyl, isopropyl, *n*-butyl, isobutyl, *sec*-butyl, *tert*-butyl, *n*-pentyl, neopentyl, *n*-hexyl, and the like. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphonyl, and the like.

The "alkoxy" refers to a group formed by replacing at least one carbon atom in the alkyl group with an oxygen atom. Non-limiting examples of alkoxy are methoxy, ethoxy, *n*-propoxy, 1-methylethoxy (isopropoxy), *n*-butoxy, isobutoxy, *sec*-butoxy, and *tert*-butoxy. Alkoxy may be substituted or unsubstituted. The definition of alkyl is the same as the definition of "alkyl" mentioned above.

"Alkenyl" refers to a linear or branched unsaturated aliphatic hydrocarbon group containing 1 to 10 (e.g., 1, 2, 3, 4, 5, 6, 7, 8, 9 or 10) carbon-carbon double bonds and consisting of 2 to 20 carbon atoms, preferably an alkenyl group consisting of 2 to 12 (e.g., 2, 3, 4, 5, 6, 7, 8, 9, 10, 11 or 12) carbon atoms, more preferably an alkenyl group consisting of 2 to 8 carbon atoms, and further preferably an alkenyl group consisting of 2 to 6 carbon atoms. Non-limiting examples include vinyl, propen-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9 or 10) carbon-carbon triple bonds and consisting of 2 to 20 carbon atoms, preferably an alkynyl group consisting of 2 to 12 (e.g., 2, 3, 4, 5, 6, 7, 8, 9, 10, 11 or 12) carbon atoms, more preferably an alkynyl group consisting of 2 to 8 carbon atoms, and further preferably an alkynyl group consisting of 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with one or more substituents.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically stated in the specification, the carbocyclyl may be optionally substituted with one or more substituents independently selected from the following: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphonyl. Examples of unsubstituted aryl include, but are not limited to, phenyl, naphthyl, and anthracenyl.

"Heteroaryl" refers to a substituted or unsubstituted aromatic ring. It may be a 3-8 membered (e.g., 3, 4, 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 6 (e.g., 1, 2, 3, 4, 5 or 6) heteroatoms selected from N, O and S, and is preferably 5-8 membered heteroaryl. 1 to 4 (e.g., 1, 2, 3 or 4) N and S optionally substituted in the ring of the heteroaryl can be oxidized to various oxidation states. Heteroaryl may be attached to a heteroatom or carbon atom and it may be a bridged ring or a spiro ring. Non-limiting examples include cyclic pyridinyl, furanyl, thienyl, pyranyl, pyrrolyl, pyrimidinyl, pyrazinyl, pyridazinyl, imidazolyl, piperidinyl, benzimidazolyl, benzopyridinyl and pyrrolopyridinyl. Heteroaryl is optionally further substituted with one or more substituents.

"Cycloalkyl" refers to a saturated cyclic hydrocarbon group, the ring of which may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-20 membered (e.g., 10, 11, 12, 13, 14, 15, 16, 17, 18, 19 or 20 membered) polycyclic ring system. The ring carbon atoms are preferably 3 to 10 carbon atoms, further preferably 3 to 8 carbon atoms. Non-limiting

examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, and the like. When the cycloalkyl is substituted, it may be optionally further substituted with one or more substituents.

"Heterocycloalkyl" refers to a substituted or unsubstituted saturated non-aromatic cyclic group. It may be a 3-8 membered (e.g., 3, 4, 5, 6, 7 or 8 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1, 2 or 3 heteroatoms selected from N, O and S, and is preferably 3-8 membered heterocyclyl. 1, 2 or 3 N and S optionally substituted in the ring of "heterocycloalkyl" can be oxidized to various oxidation states; "heterocycloalkyl" may be attached to a heteroatom or a carbon atom and may be a bridged ring or a spiro ring. Non-limiting examples of "heterocycloalkyl" include epoxyethyl, aziridinyl, oxetanyl, azetidinyl, 1,3-dioxolanyl, 1,4-dioxolanyl, 1,3-dioxanyl, azepanyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, azaadamantyl and oxaspiro[3.3]heptyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated in the specification, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4H)-ylidene, tetrahydrofuranyl, trioxacyclohexyl, trithianyl, triazinanyl, tetrahydropyranyl, thiomorpholinyl, thiamorpholinyl, 1-oxo-thiomorpholinyl and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, n-propoxy, 1-methylethoxy (isopropoxy), n-butoxy, isobutoxy, sec-butoxy, and tert-butoxy. Alkoxy may be substituted or unsubstituted.

The "halogen" refers to fluoro (F), chloro (Cl), bromo (Br), or iodo (I).

The "amino" refers to $-NH_2$.

The "cyano" refers to -CN.

The "nitro" refers to $-NO_2$.

The "hydroxy" refers to -OH.

The "carboxyl" refers to -COOH.

The "thiol" refers to -SH.

The "carbonyl" refers to C=O.

When the "alkyl", "alkoxy", "alkenyl", "alkynyl", "aryl", "heteroaryl", "carbocyclyl", "carbocycle", "heterocyclyl", "heterocycle", "cycloalkyl", "heterocycloalkyl", or "heterocyclyl" described above is substituted, it may be further substituted with 0, 1, 2, 3, 4, 5, 6, 7, 8, 9, or 10 substituents selected from F, Cl, Br, I, hydroxy, sulfhydryl, nitro, cyano, amino, $C_{1-6}$ alkylamino, =O, $C_{1-6}$ alkyl, $C_{1-6}$ alkoxy, $C_{2-6}$ alkenyl, $C_{2-6}$ alkynyl, $-NR_{q4}R_{q5}$, $=NR_{q6}$, $-C(=O)OC_{1-6}$ alkyl, $-OC(=O)C_{1-6}$ alkyl, $-C(=O)NR_{q4}R_{q5}$, $C_{3-8}$ cycloalkyl, $C_{3-8}$ heterocycloalkyl, $C_{6-10}$ aryl, $C_{5-10}$ heteroaryl, $-C(=O)OC_{6-10}$ aryl, $-OC(=O)C_{6-10}$ aryl, $-OC(=O)C_{5-10}$ heteroaryl, $-C(=O)OC_{5-10}$ heteroaryl, $-OC(=O)C_{3-8}$ heterocycloalkyl, $-C(=O)OC_{3-8}$ heterocycloalkyl, - $OC(=O)C_{3-8}$ cycloalkyl, $-C(=O)OC_{3-8}$ cycloalkyl, $-NHC(=O)C_{3-8}$ heterocycloalkyl, $-NHC(=O)C_{6-10}$ aryl, - $NHC(=O)C_{5-10}$ heteroaryl, $-NHC(=O)C_{3-8}$ cycloalkyl, $-NHC(=O)C_{3-8}$ heterocycloalkyl, $-NHC(=O)C_{2-6}$ alkenyl, and $-NHC(=O)C_{2-6}$ alkynyl, wherein the substituent $C_{1-6}$ alkyl, $C_{1-6}$ alkoxy, $C_{2-6}$ alkenyl, $C_{2-6}$ alkynyl, $C_{3-8}$ cycloalkyl, $C_{3-8}$ heterocycloalkyl, $C_{6-10}$ aryl, $C_{5-10}$ heteroaryl, $-NHC(=O)C_{6-10}$ aryl, - $NHC(=O)C_{5-10}$ heteroaryl, $-NHC(=O)C_{3-8}$ heterocycloalkyl, or $-NHC(=O)C_{3-8}$ cycloalkyl is optionally further substituted with 1 to 3 substituents selected from OH, F, Cl, Br, I, $C_{1-6}$ alkyl, $C_{1-6}$ alkoxy, $-NR_{q4}R_{q5}$, and =O; $R_{q1}$ is selected from $C_{1-6}$ alkyl, $C_{1-6}$ alkoxy, and $C_{6-10}$ aryl; $R_{q2}$ and $R_{q3}$ are selected from H and $C_{1-6}$ alkyl, wherein $R_{q4}$ and $R_{q5}$ are selected from H, $C_{1-6}$ alkyl, $-NH(C=NR_{q1})NR_{q2}R_{q3}$, $-S(=O)_2NR_{q2}R_{q3}$, - $C(=O)R_{q1}$, and $-C(=O)NR_{q2}R_{q3}$, wherein $C_{1-6}$ alkyl is optionally further substituted with 1 or more substituents selected from OH, F, Cl, Br, I, $C_{1-6}$ alkyl, $C_{1-6}$ alkoxy, $C_{6-10}$ aryl, $C_{5-10}$ heteroaryl, $C_{3-8}$ cycloalkyl, and $C_{3-8}$ heterocycloalkyl; or $R_{q4}$ and $R_{q5}$, together with the N atom, form a 3- to 8-membered heterocyclyl, wherein the heterocyclyl may contain 1 or more heteroatoms selected from N, O, and S.

The "pharmaceutically acceptable salt" or "pharmaceutically acceptable salt thereof" refers to a salt that maintains the bioavailability and properties of the free acid or free base form of a compound, and is obtained by reacting the free acid with a non-toxic inorganic or organic base, or the free base with a non-toxic inorganic or organic acid.

The "pharmaceutical composition" refers to a mixture of one or more compounds or pharmaceutically acceptable salts or prodrugs thereof, and an additional chemical component, wherein the "additional chemical component" refers to a pharmaceutically acceptable carrier or excipient and/or one or more additional therapeutic agents.

The "carrier" refers to a material that does not cause significant irritation to living organisms and does not eliminate the biological activity and characteristics of the compound administered, i.e., a diluent, adjuvant, excipient, or carrier that can be administered to a patient along with the active ingredient. Such pharmaceutical carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. "Excipient" refers to an inert substance added to a pharmaceutical composition to facilitate administration of a compound. Non-limiting examples include calcium carbonate, calcium phosphate, starches, cellulose derivatives (including microcrystalline cellulose), gelatin, vegetable oils, polyethylene glycols, diluents, granulating agents, lubricants, binders and disintegrants. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or an antigen-binding fragment, preferably in purified form, together with an appropriate amount of a carrier, to provide a form suitable for administration to a patient. The formulation should be suitable for the administration mode. The formulation may be encapsulated in an ampoule bottle, a disposable syringe, or a multi-dose vial made of glass or plastic.

The "stereoisomer" refers to an isomer that results from different spatial arrangements of atoms within a molecule, including a cis-trans isomer, an enantiomer, and a conformational isomer.

The "optional", "optionally", "selective", or "selectively" means that the subsequent event or condition may, but does not necessarily, occur. This description encompasses instances where the event or condition occurs and instances where it does not. For example, "a heterocyclic group that is selectively substituted with an alkyl group" means that the alkyl group may be, but is not necessarily, present. This description encompasses instances where the heterocyclic group is substituted with an alkyl group and instances where the heterocyclic group is not substituted with an alkyl group.

**Anti-Nectin-4 Antibody**

The present disclosure provides an antibody or an antigen-binding unit. In one or more embodiments, the antibody or the antigen-binding unit of the present invention is capable of specifically binding to Nectin-4 (e.g., human Nectin-4). In one or more embodiments, the antibody or the antigen-binding unit of the present invention has one or more of the following properties: binding to Nectin-4 (e.g., human Nectin-4), binding to cells expressing Nectin-4, high affinity, and endocytosis.

In one or more embodiments, the antigen-binding unit of the antibody is Fab, Fab', $F(ab')_2$, Fv, disulfide-stabilized Fv, scFv, or single domain antibody.

In one or more embodiments, the antibody may be a monoclonal antibody.

The binding specificity of the antibody or the antigen-binding unit disclosed herein can be detected by *in-vitro* assays, such as co-immunoprecipitation, radioimmunoassay (RIA), surface plasmon resonance, flow cytometry (Facs), or enzyme-linked immunosorbent assay (ELISA).

The present invention further encompasses an antibody that binds to the same epitope as the antibody disclosed herein. For example, the antibody of the present invention specifically binds to an epitope comprising one or more amino acid residues on human Nectin-4.

In one or more embodiments, the antibody comprises a heavy chain constant region, such as an IgG, IgA, IgE, IgM, or IgD constant region. In one or more embodiments, the antibody or the antigen-binding unit comprises an immunoglobulin heavy chain constant domain selected from a human IgG constant domain, a human IgA constant domain, a human IgE constant domain, a human IgM constant domain, and a human IgD constant domain. In one or more embodiments, the antibody or the antigen-binding unit comprises an IgG1 heavy chain constant region, an IgG2 heavy chain constant region, an IgG3 heavy chain constant region, or an IgG4 heavy chain constant region. In one or more embodiments, the heavy chain constant region is an IgG1 heavy chain constant region or an IgG4 heavy chain constant region. In one or more embodiments, the antibody or the antigen-binding unit comprises a light chain constant region, e.g., a κ light chain constant region or a λ light chain constant region.

In one or more embodiments, the antibody or the antigen-binding unit of the present invention is connected to a detectable reagent, e.g., by incorporation of a radiolabeled amino acid or attachment to a polypeptide of biotinyl moieties that can be detected by marked avidin (e.g., streptavidin containing a fluorescent marker or having enzymatic activity that

can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$, $^{125}I$ and $^{131}I$), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In one or more embodiments, the labels are connected by spacer arms of various lengths to reduce potential steric hindrance.

In one or more embodiments, the antibody or the antigen-binding unit of the present invention can be used to detect whether Nectin-4 (e.g., human Nectin-4) is present in a sample or not. In one or more embodiments, the antibody comprises a detectable agent. The antibody is a polyclonal antibody, or more preferably, a monoclonal antibody. An intact antibody or antigen-binding unit (e.g., Fab, scFv, or $F(ab')_2$) can be used. The detection method can be used to detect an analyte mRNA, protein, or genomic DNA in a biological sample *in vitro* and *in vivo.* For example, *in-vitro* techniques for the detection of an analyte mRNA include Northern hybridization and *in situ* hybridization. *In-vitro* techniques for the detection of an analyte protein include enzyme-linked immunosorbent assay (ELISA), Western blots, immunoprecipitation, and immunofluorescence. *In-vitro* techniques for the detection of an analyte genomic DNA include Southern hybridization. In addition, *in-vivo* techniques for the detection of an analyte protein include introducing a labeled anti-analyte protein antibody into a patient. For example, the antibody can be labeled with a radioactive marker, the presence and location of which in the patient can then be detected by standard imaging techniques.

In one or more embodiments, Nectin-4 may be detected in a biological sample as part of a clinical testing procedure, e.g., to determine the efficacy of a given treatment regimen. Conjugating (e.g., physically linking) the antibody to a detectable substance may facilitate the detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, or phycoerythrin; one example of the luminescent material includes luminol; examples of bioluminescent materials include luciferase, luciferin, and aequorin; examples of suitable radioactive materials include $^{125}I$, $^{131}I$, $^{35}S$, or$^{3}H$.

In one or more embodiments, the antibody or antigen-binding unit of the present invention binds to Nectin-4 with an equilibrium dissociation constant (KD) of about 1 μM or less. In one or more embodiments, the antibody or antigen-binding unit of the present invention binds to Nectin-4 with a KD of about 100 nM to about 1 pM or less. In one or more embodiments, the antibody or antigen-binding unit of the present invention binds to Nectin-4 with a KD of about 10 nM to about 1 pM or less. In one or more embodiments, the antibody or antigen-binding unit of the present invention binds to Nectin-4 with a KD of about 10 nM to about 1 nM or less.

In one or more embodiments, the antibody or antigen-binding unit of the present invention binds to human Nectin-4 (e.g., the antigen hNectin-4-His set forth in SEQ ID NO: 2) with a KD of about 100 nM to about 1 pM or less. In one or more embodiments, the antibody or antigen-binding unit of the present invention binds to Nectin-4 with a KD of about 10 nM to about 1 pM or less, or about 10 nM to about 1 nM or less.

**Antibody-Drug Conjugate**

The antibody or the antigen-binding unit of the present invention can be conjugated to a drug to form an anti-Nectin-4 antibody-drug conjugate (anti-Nectin-4 ADC). Antibody-drug conjugates (ADCs) can increase the therapeutic potency of antibodies in treating a disease (e.g., a cancer) due to their ability to selectively deliver one or more drugs to the target tissue (e.g., a tumor expressing or overexpressing Nectin-4). In one or more embodiments, the antibody-drug conjugate (ADC) of the present invention comprises the anti-Nectin-4 antibody or the antigen-binding unit described herein and at least one drug (e.g., exatecan). The ADC of the present invention has one or more of the following properties: binding to Nectin-4 (e.g., human Nectin-4), binding to cells expressing Nectin-4, high affinity, endocytosis, and reducing or inhibiting the growth of cancer cells or tumors.

The antigen-binding unit of the present invention may be conjugated to the drug described herein. In one or more embodiments, the antigen-binding unit described herein is conjugated to a drug via a linker to form the anti-Nectin-4 ADC.

The anti-Nectin-4 ADC of the present invention comprises an antibody or an antigen-binding unit that is linked to one or more drugs and specifically binds to Nectin-4 (e.g., human Nectin-4). The specificity of the ADC may be determined by the specificity of the antibody (e.g., anti-Nectin-4 antibody) or the antigen-binding unit. In one or more embodiments, the anti-Nectin-4 antibody is linked to one or more drugs (e.g., DNA topoisomerase inhibitors) that are delivered to cells expressing or overexpressing Nectin-4, particularly cancer cells expressing or overexpressing Nectin-4. In one or more embodiments, the anti-Nectin-4 antibody-drug conjugate comprises an anti-Nectin-4 antibody conjugated to a drug (e.g.,

exatecan) via a linker. The anti-Nectin-4 antibody or the antigen-binding unit described herein provides the ADC with the ability to bind to Nectin-4, such that the drug attached to the antibody can be delivered to cells expressing or overexpressing Nectin-4, particularly cancer cells expressing or overexpressing Nectin-4.

In one or more embodiments, the ADC has a structure of Formula I-A or a stereoisomer thereof, or a pharmaceutically acceptable salt or solvate thereof,

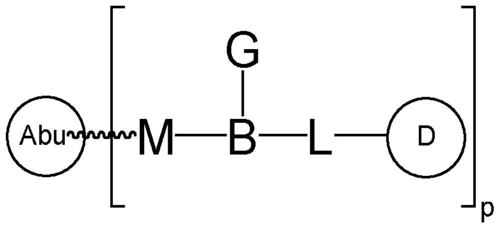

Formula I-A

wherein Abu, M, B, G, L, D, and p are as defined herein.

In one or more embodiments, the ADC is 10F4-3-ExaD8, 10F4-3-ExaD4, 10F4-3-ExaD6, or a pharmaceutically acceptable salt or solvate thereof.

In one or more embodiments, the ADC has a structure of Formula I-B or a stereoisomer thereof, or a pharmaceutically acceptable salt or solvate thereof,

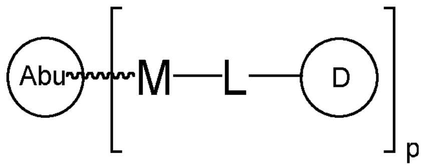

Formula I-B

wherein Abu, M, L, D, and p are as defined herein.

In one or more embodiments, the ADC is ASG-22-MMAE, 10F4-3-MMAE, 10F4-5-MMAE, 1F3-1E1-MMAE, 1F3-2B9-MMAE, or a pharmaceutically acceptable salt or solvate thereof.

In one or more embodiments, the ADC has a structure of Formula I-C or a stereoisomer thereof, or a pharmaceutically acceptable salt or solvate thereof,

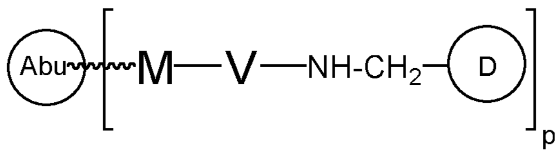

Formula I-C

wherein Abu, M, V, D, and p are as defined herein.

**Pharmaceutical composition**

The antibody or the antigen-binding unit of the present invention or the antibody-drug conjugate comprising the antibody or the antigen-binding unit of the present invention may be incorporated into a pharmaceutical composition suitable for administration. The principles and considerations involved in preparing such compositions, as well as guidelines for selecting components, are well known in the art.

Such compositions typically comprise the antibody or the antigen-binding unit or the antibody-drug conjugate, and a pharmaceutically acceptable carrier.

In one or more embodiments, the antigen-binding unit is the smallest inhibitory fragment that specifically binds to the target protein. For example, a peptide that is based on the variable region sequence of an antibody and retains the ability to bind to a target protein sequence is used.

In one or more embodiments, the pharmaceutical composition further comprises an anti-cancer agent (e.g., an immune checkpoint inhibitor).

As used herein, the term "pharmaceutically acceptable carrier" is intended to include any and all solvents, stabilizers, buffers, dispersion media, coatings, antibacterial agents, isotonic, absorption delaying agents, and the like that are compatible with the administration. Suitable carriers are described in *Remington's Pharmaceutical Sciences.* Such carriers or diluents can selectively include, but are not limited to, water, normal saline, Ringer's solution, glucose solution, and 5% human serum albumin.

In one or more embodiments, the formulation to be administered *in vivo* must be sterile, which can be readily achieved by filtration through sterile filter membranes.

The pharmaceutical compositions can be formulated in dosage unit form for ease of administration and uniformity of doses. As used herein, the dosage unit form refers to physically separable units that are suitable as unitary doses for a patient to be treated; each unit contains a predetermined amount of one or more of the antibodies, the antigen-binding units, or the antibody-drug conjugates calculated to produce the desired therapeutic effect in association with the required pharmaceutical carrier.

The pharmaceutical compositions can be placed in a container or dispenser and packaged together with instructions for administration.

The pharmaceutical compositions of the present invention may, depending on the particular condition to be treated, further comprise additional active ingredients, preferably those with complementary activities that do not adversely affect each other. In one or more embodiments, the composition can comprise a reagent that enhances its functionality, such as a cytotoxic reagent, a cytokine, a chemotherapeutic agent, or a growth inhibitory agent. Such active ingredients are properly present in combination in amounts that are effective for the intended purpose.

The composition disclosed herein may be formulated in a neutral or salt form.

**Treatment Method and Use**

The antibody or the antigen-binding unit or the ADC described herein may be used in a variety of applications including, but not limited to, treatment methods, such as the treatment of a tumor. In one or more embodiments, the antibody or the antigen-binding unit or the ADC may be used to inhibit tumor growth, reduce tumor volume, and/or reduce the tumorigenicity of a tumor. The method of use may be *in vitro, ex vivo,* or *in vivo.*

In one or more embodiments, the method for inhibiting tumor growth comprises contacting a tumor *in vitro* with an antibody, an antigen-binding unit, or an ADC, or a pharmaceutical composition comprising same. For example, an immortalized cell line or cancer cell expressing Nectin-4 is cultured in a medium supplemented with the antibody, the antigen-binding unit, or the ADC, or the pharmaceutical composition comprising same. In one or more embodiments, tumor cells are isolated from a patient sample and transferred to the medium containing the antibody, the antigen-binding unit, or the ADC, or the pharmaceutical composition comprising same for culture. In one or more embodiments, the method for inhibiting tumor growth comprises contacting a tumor or a tumor cell *in vivo* with an antibody, an antigen-binding unit, or an ADC, or a pharmaceutical composition comprising same.

The antibody, the antigen-binding unit, or the ADC, or the pharmaceutical composition comprising same of the present invention can be used for the diagnosis, prognosis, monitoring, treatment, alleviation, and/or prevention of a disease or disorder associated with the abnormal expression of Nectin-4 in a patient. When the disease or disorder associated with the abnormal expression of Nectin-4 in the patient is identified by using a conventional method, the antibody, the antigen-binding unit, or the ADC, or the pharmaceutical composition comprising same of the present invention may be administered. In one or more embodiments, the extent of Nectin-4 expression is detected by immunohistochemistry (IHC), flow cytometry, nucleic acid hybridization, or the like.

In one or more embodiments, the present invention relates to: a method for treating an associated disease with Nectin-4 as the treatment target, thereby ameliorating, slowing, inhibiting, treating, or preventing any disease or disorder associated with the abnormal expression of Nectin-4 (e.g., Nectin-4 overexpression); a method for treating tumors (including benign tumors and cancers) in a patient, a method for alleviating a symptom of a tumor (including benign tumors and cancers) in a patient, and a method for avoiding the recurrence of a tumor (including benign tumors and cancers) in a patient, comprising: administering to the patient an effective amount of the antibody or the antigen-binding unit or the ADC described herein.

In one or more embodiments, the present disclosure provides a method for preventing, treating, or ameliorating a disease, comprising: administering to a patient in need an effective amount of the antibody or the antigen-binding unit or the ADC, or the pharmaceutical composition comprising same described herein. In one or more embodiments, the present invention provides use of the antibody or the antigen-binding unit or the ADC in preparing a medicament for preventing, treating, or ameliorating a disease. In one or more embodiments, the disease is a disease associated with the expression of Nectin-4. In one or more embodiments, the disease is a disease associated with the abnormal expression of Nectin-4. In one or more embodiments, the disease is a disease associated with the overexpression of Nectin-4. In one or more embodiments, the disease is a tumor expressing Nectin-4. In one or more embodiments, the disease is a tumor overexpressing Nectin-4. In one or more embodiments, the disease is a cancer overexpressing Nectin-4. In one or more

embodiments, the disease is a cancer overexpressing human Nectin-4.

In one or more embodiments, the present invention provides a method for treating a tumor (including benign tumors and cancers), comprising: administering to a patient in need an effective amount of the antibody or the antigen-binding unit or the ADC, or the pharmaceutical composition comprising the same. Examples of the cancer include, but are not limited to, a solid tumor, a hematological cancer, and a metastatic lesion. Specific examples of such cancers include, but are not limited to, breast cancer (e.g., triple-negative breast cancer (TNBC); topical or metastatic TNBC), pancreatic cancer, bladder cancer, urothelial carcinoma, melanoma, lung cancer (e.g., non-small cell lung cancer, squamous cell cancer, or lung adenocarcinoma), head and neck cancer (e.g., head and neck squamous cell carcinoma), cervical cancer (e.g., cervical squamous cell carcinoma), ovarian cancer, choriocarcinoma, skin cancer, esophageal cancer (e.g., esophageal adenocarcinoma), gastric cancer, uterine cancer (e.g., endometrial cancer), gallbladder cancer, liver cancer, hepatocellular carcinoma, urethral cancer, renal pelvic carcinoma, ureteral cancer, colorectal cancer, colon cancer, and prostate cancer. In one or more embodiments, the cancer is a metastatic or advanced cancer. In one or more embodiments, the patient is a patient with an increased expression level of Nectin-4. In one or more embodiments, the patient is a human. The antibody or the antigen-binding unit or the ADC of the present invention may be administered to a human patient for therapeutic purposes. Furthermore, the antibody or the antigen-binding unit or the ADC of the present invention may be administered to a non-human mammal expressing Nectin-4 (for veterinary purposes or as an animal model of a human disease). Such an animal model of a human disease can be used to assess the therapeutic efficacy (e.g., dose testing and time course of administration) of the antibody or the antigen-binding unit or the ADC disclosed herein.

The specific dose and treatment regimen for any particular patient will depend on a variety of factors including the particular antibody or derivative thereof (e.g., ADC or pharmaceutical composition) used, the age and body weight, general health condition, sex and diet of the patient, the time of administration, frequency of metabolism, drug combination, and the severity of the particular disease being treated. These factors are judged by medical caregivers included within the scope of those of ordinary skill in the art. The dose will also depend on the individual patient to be treated, the route of administration, the type of the formulation, the nature of the compound used, the severity of the disease, and the efficacy desired. The dose employed can be determined by pharmacological and pharmacokinetic principles well known in the art. In one or more embodiments, an effective dose ranges from about 0.1 mg/kg to about 100 mg/kg, and the frequency of administration may be, for example, once a month. For example, the route of administration may be intravenous infusion, intravenous bolus injection, subcutaneous injection, intramuscular injection, and the like. It should be noted that the dose may vary with the type and severity of the condition to be alleviated. It will be further appreciated that for any specific patient, specific dosage regimens should be adjusted over time according to the patient's need and the professional judgment by the person administering the composition or supervising the administration of the composition, and that dose ranges illustrated herein are exemplary only and are not intended to limit the scope or practice of the claimed composition.

The modes of administration for the antibody or the antigen-binding unit or the ADC include, but are not limited to, intradermal, intramuscular, intraperitoneal, intravenous, subcutaneous, nasal, epidural, and oral administration. The mode of administration may be systemic or local. In addition, it may be desirable to introduce the antibody or the antigen-binding unit or the ADC of the present invention into the central nervous system by any suitable route, including intracerebroventricular and intrathecal injections; the intracerebroventricular injection may be assisted by an intracerebroventricular catheter connected to, for example, a reservoir (which may be an Ommaya reservoir). Pulmonary administration is also possible, for example, by the use of an inhaler or nebulizer, and by the use of nebulized formulations.

Various known delivery systems may be used to administer the antibody or the antigen-binding unit, or the polynucleotide encoding same, or the ADC of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), or the construction of nucleic acids as part of a retrovirus or other vectors.

## Combination Therapy

In one or more embodiments, the antibody or the antigen-binding unit or the ADC of the present invention may be administered in combination with an additional therapeutic or prophylactic modality, including the administration of one or more of the antibody or the antigen-binding unit or the ADC of the present invention together with or in combination with one or more additional therapeutic agents or modalities. For a combination therapy, the antibody or the antigen-binding unit or the ADC may be administered simultaneously or separately with the additional therapeutic agent. When administered separately, the antibody or the antigen-binding unit or the ADC of the present invention may be administered prior to or subsequent to the administration of the additional therapeutic agent.

In one or more embodiments, when the antibody, the antigen-binding unit, or the ADC of the present invention is administered to a patient, the antibody or the antigen-binding unit, the ADC, the pharmaceutical composition or the immunoconjugate disclosed herein, and one or more additional therapies, such as therapeutic modalities and/or other formulations (e.g., a therapeutic agent), may also be administered in combination to the patient.

In one or more embodiments, the antibody or the antigen-binding unit or the ADC of the present invention can be

used with an immune checkpoint inhibitor. In one or more embodiments, the antibody or the antigen-binding unit or the ADC of the present invention is administered in combination with an additional therapeutic or prophylactic modality, such as radiotherapy.

Such combination therapies encompass both combined administration (wherein two or more agents are contained in the same formulation or separate formulations) and separate administration (wherein the antibody or the antigen-binding unit or the ADC of the present invention may be administered prior to, concurrently with, and/or subsequent to the administration of the additional therapy, e.g., therapeutic modalities or therapeutic agents). The antibody or the antigen-binding unit or the ADC, and/or the additional therapy, e.g., therapeutic agents or therapeutic modalities, can be administered when a disease is active or when the disease is in alleviation or less active. The antibody or the antigen-binding unit or the ADC can be administered prior to, concurrently with, or subsequent to the additional therapy, or during the alleviation of the disease.

**Antibody preparation method**

Using conventional recombinant DNA techniques, one or more CDRs of the antibody of the present invention can be inserted into framework regions. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of a target antigen. One or more amino acid substitutions are made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

A gene of the antibody (e.g., anti-Nectin-4 antibody) or the antigen-binding unit thereof may be inserted into an expression vector by standard methods (e.g., linking the antibody gene fragment to complementary restriction sites on the vector, or blunt end ligation if no restriction sites are present). The expression vector may be a plasmid, a retrovirus, a YAC, an EBV-derived episome, or the like. To express the antibody (e.g., anti-Nectin-4 antibody), the DNA encoding the full-length light and heavy chains can be inserted into an expression vector, such that the genes are operably linked to transcriptional and translational control sequences. The "operably link" means that the antibody genes are linked into a vector such that transcriptional and translational control sequences within the vector perform their intended functions of regulating the transcription and translation of the antibody genes.

The consensus sequence J region of the heavy and light chains can be used to design oligonucleotides for use as primers to introduce useful restriction sites into the J region for subsequent linkage of V region fragments to C region fragments. The C region cDNA may be modified by site-directed mutagenesis to place a restriction site at a similar position in the human sequence. In one or more embodiments, the expression vector already carries antibody constant region sequences prior to the insertion of the antibody-related light or heavy chain variable region gene sequences. For example, one method for converting the anti-Nectin-4 antibody-related VH and VL sequences into full-length antibody genes is to insert them into an expression vector that already encodes a heavy chain constant region and a light chain constant region, such that the VH segment is operably linked to the CH segment within the vector and the VL segment is operably linked to the CL segment within the vector.

The recombinant expression vector may encode a signal peptide that facilitates the secretion of the heavy and light chains of an antibody from a host cell. Alternatively, the antibody heavy and light chain genes may be cloned into a vector encoding a signal peptide that facilitates the secretion of the antibody heavy and light chains from a host cell, such that the signal peptide is linked in frame to the amino termini of the antibody heavy and light chain genes. The signal peptide may be an immunoglobulin signal peptide or a heterologous signal peptide (i.e., a signal peptide from a non-immunoglobulin protein), for example, MELGLCWVFLVAILEGVQC (shown in SEQ ID NO: 3), MDMRVPAQLLGLLLLWFPGSRC (shown in SEQ ID NO: 4), MEWSWVFLFFLSVTTGVHS (shown in SEQ ID NO: 39), MDMRVPAQLLGLLLLWLPGARC(shown in SEQ ID NO: 40).

In addition to the antibody heavy and light chain genes, the recombinant expression vector may also carry regulatory sequences that control the expression of the antibody chain genes in the host cell. The regulatory sequences include promoters, enhancers, and other expression control elements (e.g., polyadenylation signals) that control the transcription or translation of antibody chain genes. Such regulatory sequences are described, for example, in Goeddel,

Gene Expression Technology: Methods in Enzymology 185, Academic Press, San Diego, CA, 1990. Those skilled in the art will appreciate that the design of an expression vector, including the selection of regulatory sequences, may depend on such factors as the selection of host cells to be transformed, the expression level of the desired protein, and the like. Suitable regulatory sequences for the expression in mammalian host cells include viral elements that direct high-level protein expression in mammalian cells, such as promoters and/or enhancers derived from cytomegalovirus (CMV) (e.g., the CMV promoter/enhancer), simian virus 40 (SV40) (e.g., the SV40 promoter/enhancer), adenovirus (e.g., the adenovirus major late promoter (AdMLP)), and polyoma. For further description of viral regulatory elements and sequences thereof, see, e.g., U.S. Pat. Nos. 5,168,062, 4,510,245, and 4,968,615.

In addition to the antibody chain genes and regulatory sequences, the recombinant expression vector may carry other sequences, such as a sequence that regulates the replication of the vector in the host cell (e.g., an origin of replication) and a selectable marker gene. The selectable marker gene facilitates the selection of host cells into which the vector has been introduced (see, e.g., U.S. Pat. Nos. 4,399,216, 4,634,665, and 5,179,017). For example, generally, a marker gene that imparts resistance to drugs (e.g., G418, hygromycin, or methotrexate) to the host cell into which the vector has been introduced can be selected. Suitable selectable marker genes include the dihydrofolate reductase (DHFR) gene (for use in DHFR-host cells with methotrexate selection/amplification), the neo gene (for G418 selection), and the GS gene. For the expression of heavy and light chains, an expression vector encoding the heavy and light chains is transfected into a host cell by standard techniques. In one or more embodiments, the inserted gene fragment should comprise a selection marker, and common selection marker include screening genes such as dihydrofolate reductase, glutamine synthetase, neomycin resistance, and hygromycin resistance, to facilitate the screening and separation of cells that have been successfully transfected. The constructed plasmid is transfected to a host cell without the above genes, and the successfully transfected cells are expanded in a large quantity in a selective culture medium to produce the desired target protein. The antibody will be purified by one or more purification procedures. The purification may be conducted by conventional methods, for example, by centrifuging a cell suspension and collecting the supernatant, and centrifuging again to further remove impurities. Protein A affinity column, ion exchange column, and other methods may be used for purifying the antibody protein.

The antibody (e.g., anti-Nectin-4 antibody) of the present invention can be prepared by the recombinant expression of the heavy and light chain genes of the antibody in a host cell. For example, the host cell is transfected with one or more recombinant expression vectors carrying heavy and light chain DNA fragments encoding the antibody such that the heavy and light chains are expressed in the host cell, the expressed antibody can be secreted into the medium in which the host cell is cultured, and the antibody can be isolated from the medium. The "transfect" refers to a wide variety of techniques commonly used to introduce a foreign DNA into eukaryotic host cells, such as electroporation, lipofection, calcium phosphate precipitation, DEAE-dextran transfection, and the like. Standard recombinant DNA methods for obtaining the antibody heavy and light chain genes, incorporating these genes into expression vectors, and introducing the vectors into host cells are well known in the art, such as those described in U.S. Pat. No. 4,816,397. The DNAs expressing the heavy chain and the light chain of the antibody may be placed in the same vector or placed in different vectors; if placed in different vectors, the vectors expressing the heavy and light chains of the antibody may transfect host cells in a proper ratio (e.g., Tihomir S. Dodev et al., A tool kit for rapid cloning and expression of recombinant antibodies, Scientific Reports volume 4, Article number: 5885 (2014)). In one or more embodiments, the antibody expression vector comprises at least one promoter element, an antibody coding sequence, a transcription termination signal, and a polyA tail. Other elements may include enhancers, Kozak sequences, and donor and recipient sites flanking the inserted sequence for RNA splicing. Efficient transcription can be achieved by early and late promoters of SV40, long terminal repeats from retroviruses such as RSV, HTLV1, and HIVI, and early promoters of cytomegalovirus, and promoters from other cells such as actin promoter can also be used. Proper expression vectors may include pIRES1neo, pRetro-Off, pRetro-On, pLXSN, pLNCX, pcDNA3.1(+/-), pcDNA/Zeo(+/-), pcDNA3.1/Hygro(+/-), pSVL, pMSG, pRSVcat, pSV2dhfr, pBC12MI, pCS2, pCHO1.0, or the like.

The antibody of the present invention (e.g., anti-Nectin-4 antibody) can be expressed in eukaryotic host cells. In some certain embodiments, the antibody is expressed in eukaryotic cells, such as mammalian host cells. Exemplary host cells for expressing the antibody of the present invention include Chinese hamster ovary cells (CHO cells) or CHO-S, CHO-dhfr-, CHO/DG44 or ExpiCHO obtained by the modification of CHO cells, NSO myeloma cells, COS cells, Cos1 cells, Cos7 cells, SP2 cells, CV1 cells, murine L cells, or human embryonic kidney HEK293 cells or HEK293T, HEK293F or HEK293E cells obtained by modification of HEK293 cells. After a recombinant expression vector encoding an antibody chain gene is introduced into a host cell, the host cell is cultured in a medium for a period of time sufficient to allow the antibody to be expressed in the host cell or allow the antibody to be secreted into the medium to produce the antibody. The antibody can be isolated from the medium using standard protein purification methods.

For the recombinant expression of the antibody (e.g., anti-Nectin-4 antibody) of the present invention, the host cell can be co-transfected with two recombinant expression vectors, a first recombinant expression vector encoding the heavy chain of the antibody and a second recombinant expression vector encoding the light chain of the antibody. The two recombinant expression vectors may contain the same selectable marker, or they may each contain separate selectable

markers. Alternatively, a host cell may be transfected with a recombinant expression vector encoding the heavy and light chains of the antibody.

The antibody (e.g., anti-Nectin-4 antibody) of the present invention may also be produced by chemical synthesis (e.g., by the methods described in Solid Phase Peptide Synthesis, second edition, 1984, The Pierce Chemical Co., Rockford, Ill.). Antibody variants may also be generated using a cell-free platform (see, e.g., Chu et al., Biochemia No. 2, 2001 (Roche Molecular Biologicals) and Murray et al., 2013, Current Opinion in Chemical Biology, 17: 420-426).

The antibody (e.g., anti-Nectin-4 antibody) produced by recombinant expression may be purified by any method known in the art for purifying immunoglobulin molecules, for example, by chromatography (e.g., ion exchange, affinity, and sizing column chromatography), centrifugation, differential solubility, or any other standard technique for purifying proteins. For example, protein A or protein G affinity chromatography may be used, which provides primarily the IgG fraction in immune serum. In addition, the specific antigen targeted by the immunoglobulin, or an epitope thereof, may be immobilized on a column to purify the immune-specific antibody by immunoaffinity chromatography. The antibody (e.g., anti-Nectin-4 antibody) of the present invention can be fused to heterologous polypeptide sequences known in the art to facilitate the purification. For the purification of immunoglobulins, reference can be made to D. Wilkinson's article (The Scientist, published by the Scientist, Inc., Philadelphia Pa., Vol. 14, No. 8 (Apr. 17, 2000), pp. 25-28).

In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids, or a substitution of less than 2 amino acids relative to the original heavy chain variable region and the original light chain variable region. Alternatively, mutations may be introduced randomly along all or part of the encoding sequence, for example, by saturation mutagenesis, and the resulting mutants may be screened for the biological activity to identify mutants that retain the activity. In one or more embodiments, the substitutions described herein are conservative amino acid substitutions.

## Preparation Method

The present invention further provides preparation method for drug conjugates, such as antibody-drug conjugates, and intermediates. The drug conjugates, such as antibody-drug conjugates, and intermediates of the present invention can be prepared by using known formulations and methods. In one or more embodiments, the methods for preparing are described below.

Preparation method of

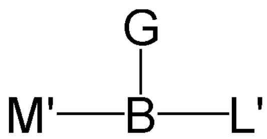

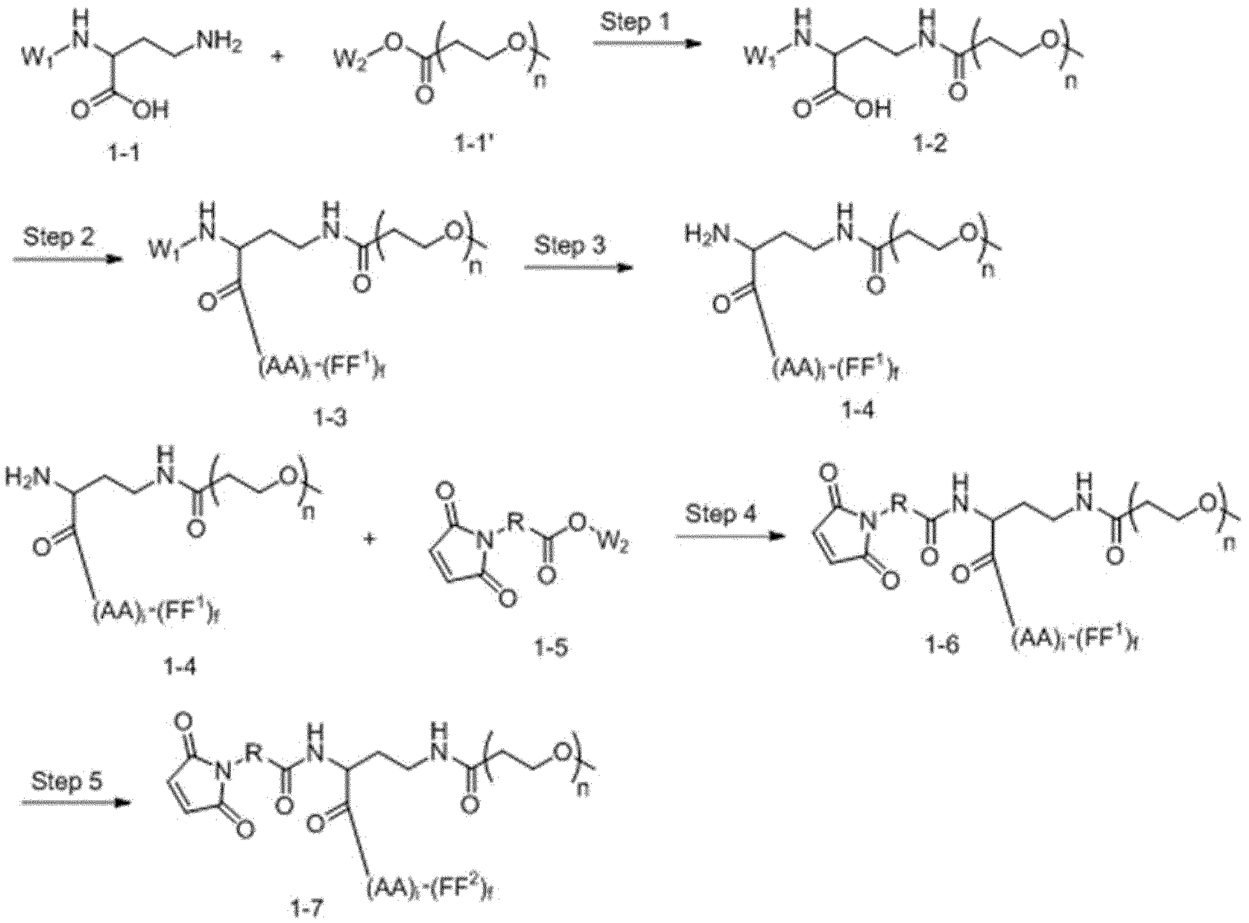


Step 1: reacting a compound of general Formula 1-1 with a compound of general Formula 1-1' under a basic condition to obtain a compound of general Formula 1-2;

Step 2: reacting the compound of general Formula 1-2 with general Formula $(AA)_i$-$(FF^1)_f$ in the presence of a condensing agent under a basic condition to obtain a compound of general Formula 1-3;

Step 3: removing the amino-protecting group $W_1$ of the compound of general Formula 1-3 to obtain a compound of general Formula 1-4;

Step 4: reacting the compound of general Formula 1-4 with a compound of general Formula 1-5 under a basic condition to obtain a compound of general Formula 1-6; and

Step 5: reacting the compound of general Formula 1-6 with bis(*p*-nitrophenyl)carbonate under a basic condition to obtain a compound of general Formula 1-7.

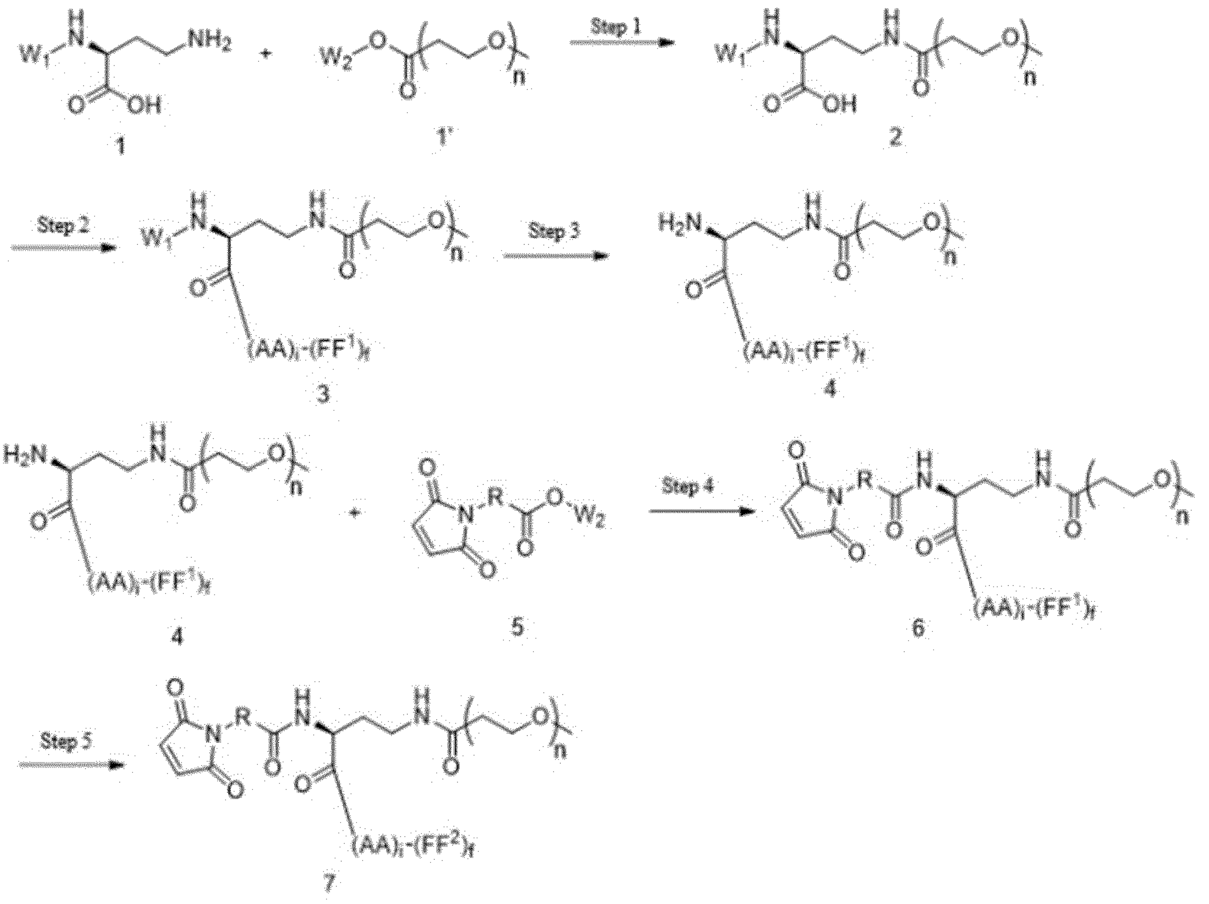


1 1' 2 3 4 4 5 6 7

Step 1: reacting a compound of general Formula 1 with a compound of general Formula 1' under a basic condition to obtain a compound of general Formula 2;

Step 2: reacting the compound of general Formula 2 with general Formula $(AA)_i$-$(FF^1)_f$ in the presence of a condensing agent under a basic condition to obtain a compound of general Formula 3;

Step 3: removing the amino-protecting group $W_1$ of the compound of general Formula 3 to obtain a compound of general Formula 4;

Step 4: reacting the compound of general Formula 4 with a compound of general Formula 5 under a basic condition to obtain a compound of general Formula 6; and

Step 5: reacting the compound of general Formula 6 with bis(*p*-nitrophenyl)carbonate under a basic condition to obtain a compound of general Formula 7;
wherein

$W_1$ is an amino-protecting group, e.g., 9-fluorenylmethyloxycarbonyl; $W_2$ is a carboxylic acid active ester, for example, a succinimidyl ester;

wherein B, G, n, AA, R, i, and f are described as herein;

M' is

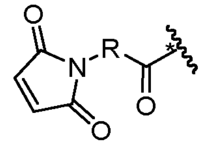

,

wherein * links to B, R is selected from: $-(CH_2)_r-$, $-(CHR^m)_r-$, C3-C8 carbocyclyl, - $O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, - $(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, - $(CH_2)_rC(O)NR^m(CH_2CH_2O)_r-CH_2-$, $-(CH_2CH_2O)_rC(O)NR^m(CH_2CH_2O)_r-$, - $(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl; and each r is independently 1, 2, 3, 4, 5, 6, 7, 8, 9 or 10; or R is $-(CH_2)_r-$, or r is 1 or 5;

L' is $-(AA)_i-(FF')_f-$, wherein AA is amino acid or polypeptide, i is 1, 2, 3, 4, 5, 6, 7, 8, 9, 10, 11, 12, 13, 14, 15, 16, 17, 18, 19 or 20; or each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly; or AA is Val-Cit, i is 1; each FF' is independently

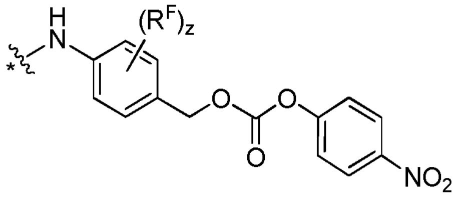
,
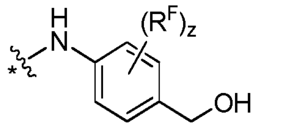
,
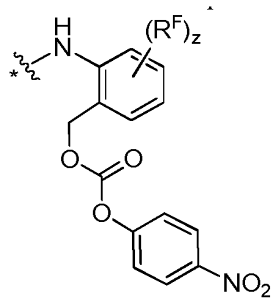
,

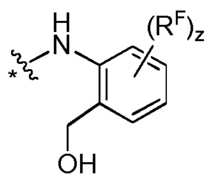
,
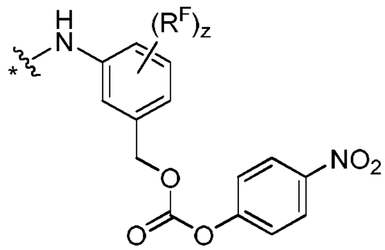
or
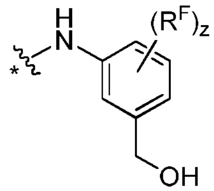
,

wherein each $R^F$ is independently C1-C6 alkyl, C1-C6 alkoxy, $-NO_2$ or halogen; wherein * links to AA, or z is 0 or $R^F$ is F, or z is 0, 1, 2, 3 or 4, or z is 1 or 2; or each FF' is independently

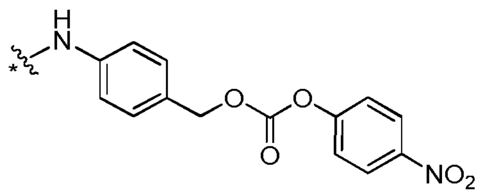
,

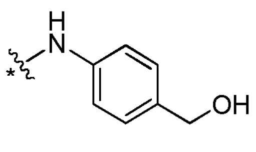
,
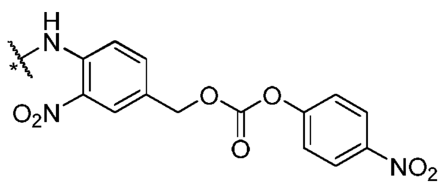
,
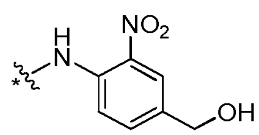
,

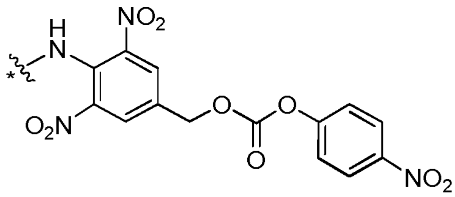 ,

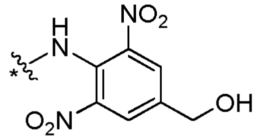 ,

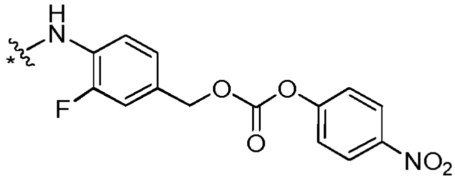 ,

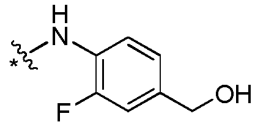 ,

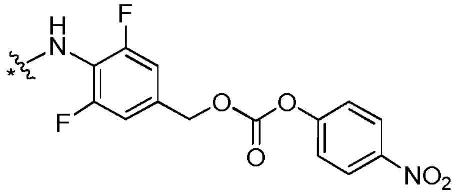 ,

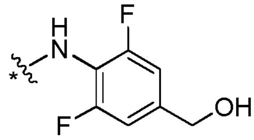 ,

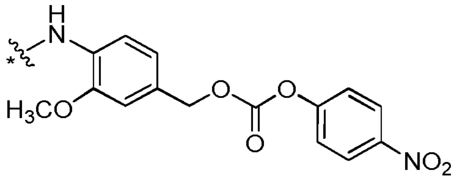 ,

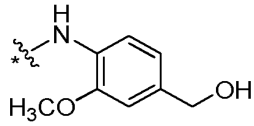 ,

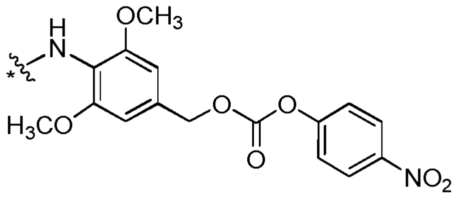 ,

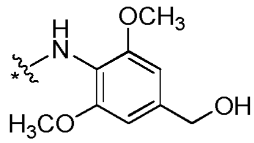 ,

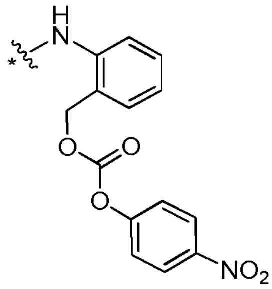 ,

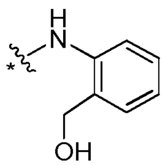 ,

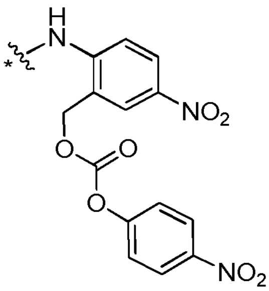 ,

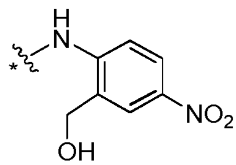 ,

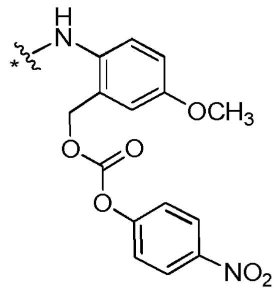 ,

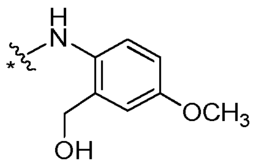, 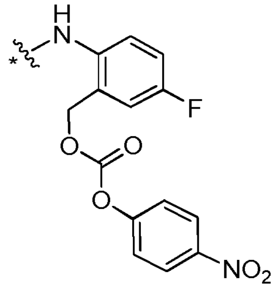, 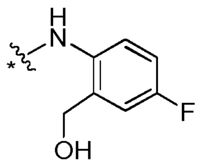, 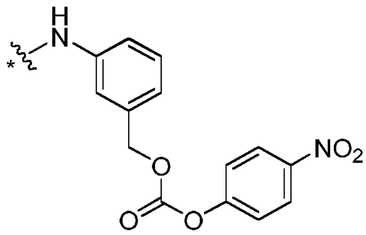,

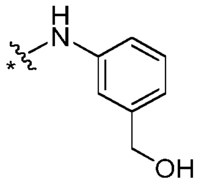, 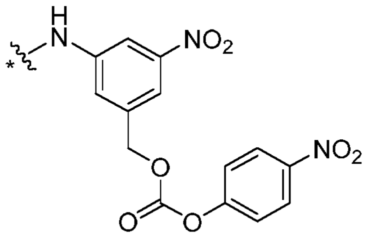, 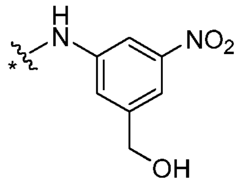,

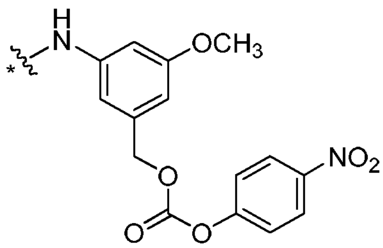, 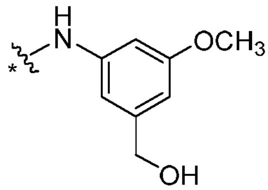, 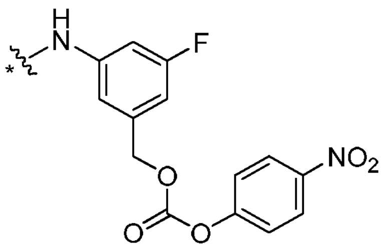,

or

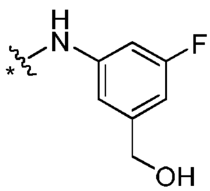,

wherein * links to AA, f is 1, 2, 3, 4, 5, 6, 7, 8, 9 or 10; or FF' is p-aminophenyl (4-nitrophenyl) carbonate or p-aminobenzyl alcohol, f is 1 or L' is

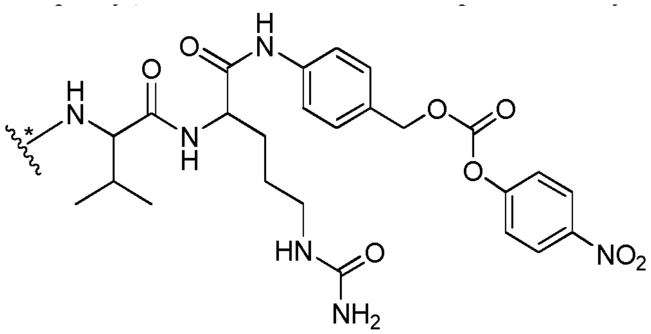 or 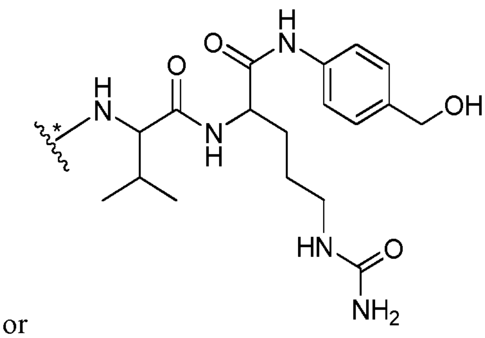,

wherein

* links to B, or L' is

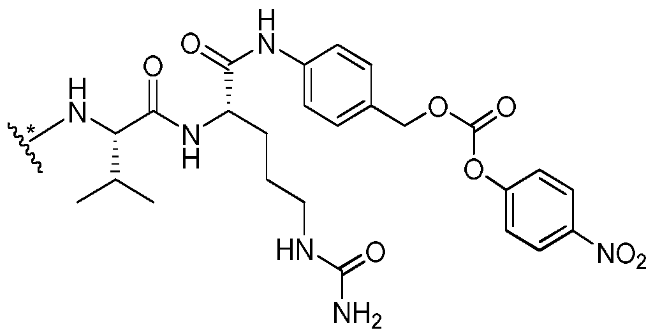

or

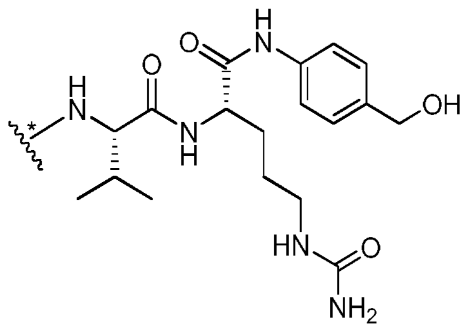

wherein * links to B;

each $FF^1$ is independently

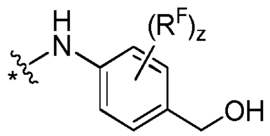 , 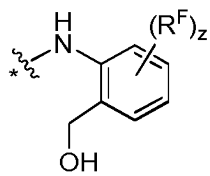 or 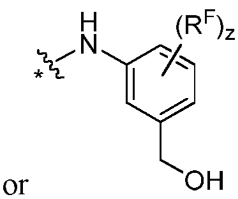 ,

wherein each $R^F$ is independently C1-C6 alkyl, C1-C6 alkoxy, $-NO_2$ or halogen; z is 0, 1, 2, 3 or 4; wherein * links to AA;

each $FF^2$ is independently

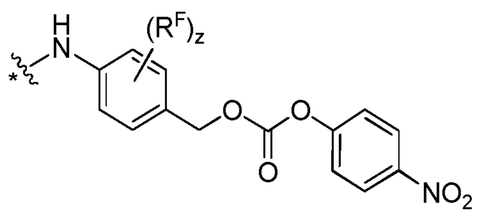 , 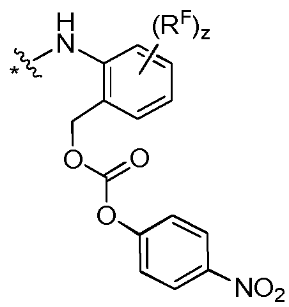

or

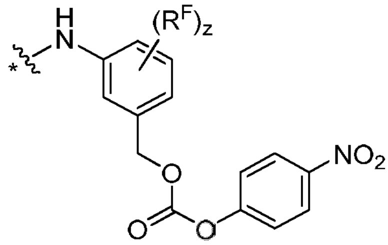

,

wherein each $R^F$ is independently C1-C6 alkyl, C1-C6 alkoxy, $-NO_2$ or halogen; z is 0, 1, 2, 3 or 4; wherein * links to AA.

The basic conditions described above can be provided using reagents including organic bases and inorganic bases, wherein the organic bases include, but are not limited to, triethylamine, diethylamine, *N*-methylmorpholine, pyridine, piperidine, *N,N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N,N,N',N'*-tetramethyl-O-(7-azabenzotriazol-1-yl) uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N,N*-dicyclohexylcarbodiimide, *N,N'*-diisopropylcarbodiimide, *O*-benzotriazol-*N,N,N',N'*-tetramethyluronium tetrafluoroborate, 1-hydroxybenzotriazole, 1-hydroxy-7-azobenzotriazol, O-benzotriazol-*N,N,N,N*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method of

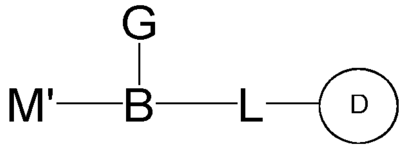

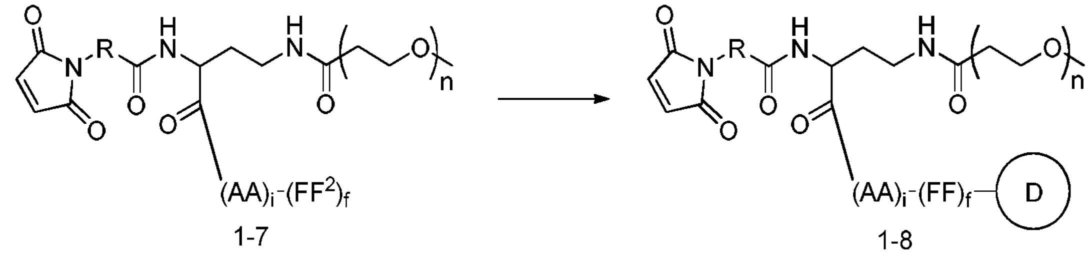

1-7 1-8

The compound of general Formula 1-7 and D are reacted in the presence of a condensing agent under a basic condition to obtain a compound of general Formula 1-8.

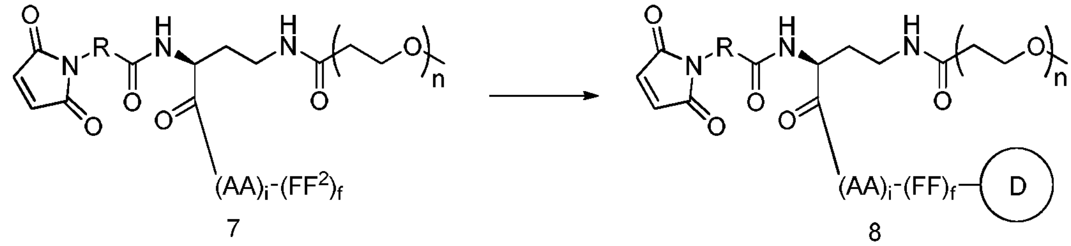

7 8

The compound of general Formula 7 and D are reacted in the presence of a condensing agent under a basic condition to obtain a compound of general Formula 8.

Wherein M', B, L, G, n, AA, R, i, f, FF, $FF^2$, D are as described herein.

The basic conditions described above can be provided using reagents including organic bases and inorganic bases, wherein the organic bases include, but are not limited to, triethylamine, diethylamine, *N*-methylmorpholine, pyridine, piperidine, *N,N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N,N,N',N'*-tetramethyl-O-(7-azabenzotriazol-1-yl) uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N,N*-dicyclohexylcarbodiimide, *N,N'*-diisopropylcarbodiimide, *O*-benzotriazol-*N,N,N',N'*-tetramethyluronium tetrafluoroborate, 1-hydroxybenzotriazole, 1-hydroxy-7-azobenzotriazol, *O*-benzotriazol-*N,N,N,N*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method of

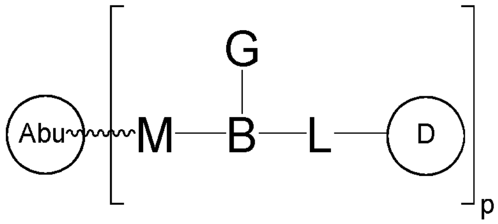

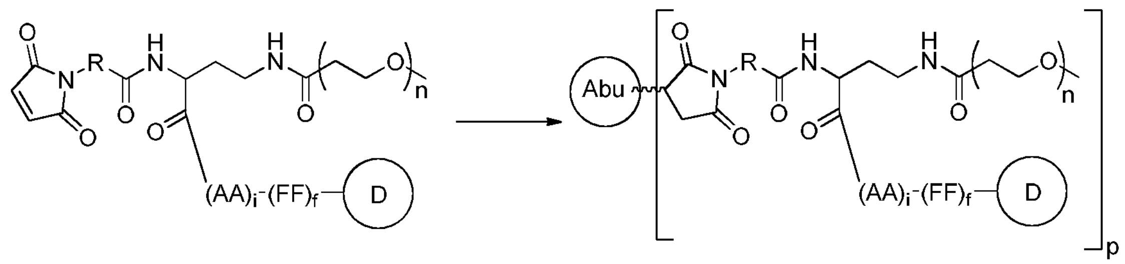

1-8 1-9

The compound of general Formula 1-8 is conjugated to Abu under a weakly acidic condition to obtain a compound of general Formula 1-9.

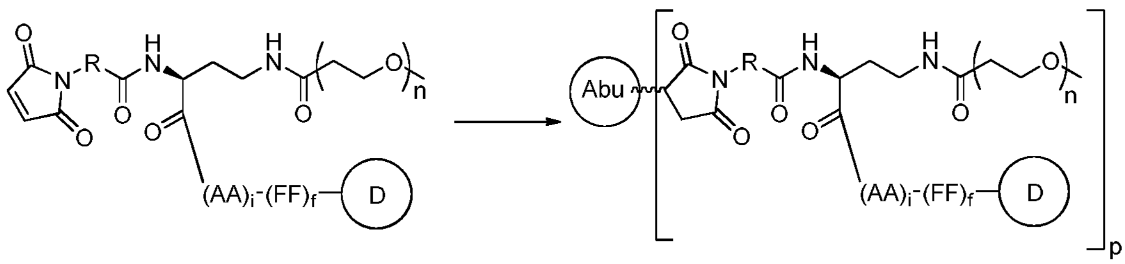

8 9

The compound of general Formula 8 is conjugated to Abu under a weakly acidic condition to obtain a compound of general Formula 9.

Wherein Abu, M, B, L, G, D, p, n, AA, R, i, f, FF are as described as Formula I-A.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, hydrosulfuric acid, silicic acid, metasilicic acid, phosphoric acid, metaphosphoric acid, sodium bicarbonate, and sodium bisulfite.

The drug conjugate can be purified by a conventional method, e.g., preparative high-performance liquid chromatography (prep-HPLC) or the like.

The present application will be illustrated with reference to specific examples, but the content of the present application is not limited thereto.

The reagents and instruments used in the following methods are those conventional in the art and commercially available unless otherwise specified; the methods used are all conventional in the art, and those skilled in the art can

undoubtedly implement the methods and acquire the corresponding results according to the content described in the examples.

**Example 1. Expression of human Nectin-4 extracellular antigen**

The human Nectin-4 extracellular antigen (hNectin-4-His) was prepared according to conventional methods: the signal peptide sequence of the human Nectin-4 extracellular sequence (sequence source: GenBank No. Q96NY8) was replaced with an albumin signal peptide (MKWVTFISLLFLFSSAYS, as set forth in SEQ ID NO: 1), and a histidine tag 8×His was added to the C-terminus of the albumin signal peptide to give the antigen protein (the sequence is set forth in SEQ ID NO: 2, and denoted as hNectin-4-His). The DNA sequence of the antigen protein hNectin-4-His was cloned to an expression vector. Eukaryotic cells, i.e., HEK293F cells, were transiently transfected, or CHO cells were stably transfected. Stable cell lines were selected, and purification and expression were performed.
MKWVTFISLLFLFSSAYSGELETSDVVTVVLGQDAKLPCFYRGDSGEQVGQVAWARVDAGEGAQ ELALLHSKYGLHVSPAYEGRVEQPPPPRNPLDGSVLLRNAVQADEGEYECRVSTFPAGSFQARLRL RVLVPPLPSLNPGPALEEGQGLTLAASCTAEGSPAPSVTWDTEVKGTTSSRSFKHSRSAAVTSEFHL VPSRSMNGQPLTCVVSHPGLLQDQRITHILHVSFLAEASVRGLEDQNLWHIGREGAMLKCLSEGQ PPPSYNWTRLDGPLPSGVRVDGDTLGFPPLTTEHSGIYVCHVSNEFSSRDSQVTVDVLDPQEDSGK QVDLVSASHHHHHHHH (SEQ ID NO: 2, with the albumin signal peptide underlined).

**Example 2. Expression of reference antibody**

The amino acid sequences of the heavy and light chains of the reference antibody ASG-22 are shown in Table 1. For the convenience of expression in a host cell, signal peptides were added to N-termini of the heavy and light chains, where the signal peptide sequence linked to the N-terminus of the heavy chain was MELGLCWVFLVAILEGVQC (as set forth in SEQ ID NO: 3) and the signal peptide sequence linked to the N-terminus of the light chain was MDMRVPAQLLGLLLLWFPGSRC (as set forth in SEQ ID NO: 4). DNA sequences of the heavy and light chains were synthesized by conventional methods, and the antibody normally bound to hNectin-4-His expressed in Example 1.

Table 1. Amino acid sequences of reference antibody ASG-22

| | Amino acid sequence (with CDR sequences underlined, according to the Kabat numbering system) | SEQ ID NO: |
|---|---|---|
| Heavy chain | EVQLVESGGGLVQPGGSLRLSCAASGFTFSSYNMNWVRQAPGKGLEWVSYISSSSSTIYYADSVKGRFTISRDNAKNSLSLQMNSLRDEDTAVYYCARAYYYGMDVWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK | 5 |
| Light chain | DIQMTQSPSSVSASVGDRVTITCRASQGISGWLAWYQQKPGKAPKFLIYAASTLQSGVPSRFSGSGSGTDFTLTISSLQPEDFATYYCQQANSFPPTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 6 |

**Example 3. Preparation of anti-Nectin-4 antibodies**

**1) Antibody sequences**

Table 2 shows the light and heavy chain variable regions of scFv, where the light chain variable regions have identical amino acid sequences.

Table 2: Sequences of heavy chain variable region and light chain variable region

| Clone No. | Type | Amino acid sequence |
|---|---|---|
| 1B4 | VH | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARQTEDRYYGYDLDYWGQGTLVTVSS (SEQ ID NO:7)<br>HCDR1 : SYAMS (SEQ ID NO:8)<br>HCDR2 : AISGSGGSTYYADSVKG (SEQ ID NO:9)<br>HCDR3 : QTEDRYYGYDLDY (SEQ ID NO:10) |
| 1G8 | VH | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARQTEDRSYGYDLDYWGQGTLVTVSS (SEQ ID NO:11)<br>HCDR1 : SYAMS (SEQ ID NO:8)<br>HCDR2 : AISGSGGSTYYADSVKG (SEQ ID NO:9)<br>HCDR3 : QTEDRSYGYDLDY (SEQ ID NO:12) |
| 1F3 | VH | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARAELLAFDVYTASDYWGQGTLVTVSS (SEQ ID NO:13)<br>HCDR1 : SYAMS (SEQ ID NO:8)<br>HCDR2 : AISGSGGSTYYADSVKG (SEQ ID NO:9)<br>HCDR3 : AELLAFDVYTASDY (SEQ ID NO:14) |
| 10F4 | VH | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCAREYSDVVDYVSVRTPLDYWGQGTLVTVSS (SEQ ID NO:15)<br>HCDR1 : SYAMS (SEQ ID NO:8)<br>HCDR2 : AISGSGGSTYYADSVKG (SEQ ID NO:9)<br>HCDR3 : EYSDVVDYVSVRTPLDY (SEQ ID NO:16) |
| 3A10 | VH | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARDRSDVYSYSYFPTPLDYWGQGTLVTVSS (SEQ ID NO:17)<br>HCDR1 : SYAMS (SEQ ID NO:8)<br>HCDR2 : AISGSGGSTYYADSVKG (SEQ ID NO:9)<br>HCDR3 : DRSDVYSYSYFPTPLDY (SEQ ID NO:18) |

(continued)

| Clone No. | Type | Amino acid sequence |
|---|---|---|
| 10A4 | VH | EVQLVESGGGLVQPGGSLRLSCAASGFTFSSYAMSWVRQAPGKGLEWVSAISGSGGS TYYADSVKGRFTISRDNSKNTLYLQMNSLRAEDTAVYYCARQTEDRSYGYDMDYWG QGTLVTVSS (SEQ ID NO:19)<br>HCDR1 : SYAMS (SEQ ID NO:8)<br>HCDR2 : AISGSGGSTYYADSVKG (SEQ ID NO:9)<br>HCDR3 : QTEDRSYGYDMDY (SEQ ID NO:20) |
| 1G7 | VH | EVQLVESGGGLVQPGGSLRLSCAASGFTFSSYAMSWVRQAPGKGLEWVSAISGSGGS TYYADSVKGRFTISRDNSKNTLYLQMNSLRAEDTAVYYCARERSDATYLRTPLDYWG QGTLVTVSS (SEQ ID NO:21)<br>HCDR1 : SYAMS (SEQ ID NO:8)<br>HCDR2 : AISGSGGSTYYADSVKG (SEQ ID NO:9)<br>HCDR3 : ERSDATYLRTPLDY (SEQ ID NO:22) |
| 1G12 | VH | EVQLVESGGGLVQPGGSLRLSCAASGFTFSSYAMSWVRQAPGKGLEWVSAISGSGGS TYYADSVKGRFTISRDNSKNTLYLQMNSLRAEDTAVYYCARHTEDRYYGYYLDYWG QGTLVTVSS (SEQ ID NO:23)<br>HCDR1 : SYAMS (SEQ ID NO:8)<br>HCDR2 : AISGSGGSTYYADSVKG (SEQ ID NO:9)<br>HCDR3 : HTEDRYYGYYLDY (SEQ ID NO:24) |
| Common light chain variable region | VL | DIQMTQSPSSLSASVGDRVTITCRASQGISSYLAWYQQKPGKAPKLLIYAASSLQSGVP SRFSGSGSGTDFTLTISSLQPEDFATYYCQQHYTTPPTFGQGTKVEIKR (SEQ ID NO:25)<br>LCDR1 : RASQGISSYLA (SEQ ID NO:26)<br>LCDR2 : AASSLQS (SEQ ID NO:27)<br>LCDR3 : QQHYTTPPT (SEQ ID NO:28) |

The sequences of the framework regions HFR1-HFR4 in the VH are set forth in SEQ ID NOs: 29-32, and the sequences of the framework regions LFR1-LFR4 in the VL are set forth in SEQ ID NOs: 33-36.

Framework regions in VH:

HFR1 : EVQLVESGGGLVQPGGSLRLSCAASGFTFS (SEQ ID NO:29)

HFR2 : WVRQAPGKGLEWVS (SEQ ID NO:30)

HFR3 : RFTISRDNSKNTLYLQMNSLRAEDTAVYYCAR (SEQ ID NO:31)

HFR4 : WGQGTLVTVSS (SEQ ID NO:32)

Framework regions in VL:

LFR1 : DIQMTQSPSSLSASVGDRVTITC (SEQ ID NO:33)

LFR2 : WYQQKPGKAPKLLIY (SEQ ID NO:34)

LFR3 : GVPSRFSGSGSGTDFTLTISSLQPEDFATYYC (SEQ ID NO:35)

LFR4 : FGQGTKVEIKR (SEQ ID NO:36)

**2) Preparation of scFv**

The HCDR3 of the above 1F3, 1G8, and 10F4 were cloned into scFv phage libraries of HCDR1 and HCDR2, and supernatants of phage clones were screened and selected for detection. The supernatants were diluted 50-fold, and the binding OD values of the diluted supernatants to hNectin-4-His were measured by ELISA. The samples were serially 3-fold diluted, and the $EC_{50}$ titer for the binding of the supernatants to the antigen was measured by ELISA. Clones with higher OD values were selected for sequencing to give the heavy chain variable region sequences and the HCDR1 and HCDR2 sequences of all the clones, as shown in Tables 3-8. The light chain variable region sequence is set forth in SEQ ID NO: 25. The OD value is the measurement of ELISA, and the $EC_{50}$ value denotes the $EC_{50}$ value of the binding activity titer of the supernatant to the antigen.

**3) Preparation of antibody**

Construction of full-length antibody: the heavy chain variable regions shown in Tables 2-5 and a heavy chain constant regions (CH) were combined to give the heavy chains of the antibodies, and the light chain variable region set forth in SEQ ID NO: 25 and a light chain constant region (CL) were combined to give the light chain of the antibodies; the amino acid sequence of CH is set forth in SEQ ID NO: 37, and the amino acid sequence of CL is set forth in SEQ ID NO: 38. For the convenience of expression in a host cell, signal peptides were added to N-termini of the heavy and light chains, where the signal peptide sequence linked to the N-terminus of the heavy chain was MEWSWVFLFFLSVTTGVHS (as set forth in SEQ ID NO: 39) and the signal peptide sequence linked to the N-terminus of the light chain was MDMRVPAQLLGLLLLWLPGARC (as set forth in SEQ ID NO: 40). The antibody numbers are consistent with the clone numbers corresponding to the heavy chain variable regions. The amino acid sequence of the light chain is set forth in SEQ ID NO: 250, and the nucleic acid sequence of the light chain is set forth in SEQ ID NO: 359. The nucleic acid sequence was synthesized according to the antibody sequences. For example, for antibody 1F3, the amino acid sequence of the heavy chain is set forth in SEQ ID NO: 232; for antibody 1G8, the amino acid sequence of the heavy chain is set forth in SEQ ID NO: 233; for antibody 10F4, the amino acid sequence of the heavy chain is set forth in SEQ ID NO: 249; for antibody 10F4-3, the amino acid sequence of the heavy chain is set forth in SEQ ID NO: 360, and the nucleic acid sequence of the heavy chain is set forth in SEQ ID NO: 364; for antibody 1F3-1E4, the amino acid sequence of the heavy chain is set forth in SEQ ID NO: 361, and the nucleic acid sequence of the heavy chain is set forth in SEQ ID NO: 365; for antibody 1F3-2B9, the amino acid sequence of the heavy chain is set forth in SEQ ID NO: 362, and the nucleic acid sequence of the heavy chain is set forth in SEQ ID NO: 366; for antibody 1G8-1C10, the amino acid sequence of the heavy chain is set forth in SEQ ID NO: 363, and the nucleic acid sequence of the heavy chain is set forth in SEQ ID NO: 367. The light and heavy chain expression vectors were constructed according to conventional operation methods. HEK293F cells were transformed, and the acquired antibodies were subjected to further detection.

Table 3: Heavy chain variable region sequences of 1F3-series

| Clone No. | VH sequences (with CDR sequences underlined, according to the Kabat numbering system) | OD value | $EC_{50}$ |
|---|---|---|---|
| 1F3 | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARAELLAFDVYTASDYWGQGTLVTVSS (SEQ ID NO:13) | NA | NA |
| 1F3-1A9 | EVQLVESGGGLVQPGGSLRLSCAASGYSFDNYAMSWVRQAPGKGLEWVSRISPTGGYASYADSVKGRFTISRDNSKNTLYLQMNSLRAEDTAVYYCARAELLAFDVYTASDYWGQGTLVTVSS (SEQ ID NO:41) | 1.635 | 309. 2 |
| 1F3-1B1 | EVQLVESGGGLVQPGGSLRLSCAASGFTFGTYGMSWVRQAPGKGLEWVSGIKPGDSTTYYADSVKGRFTISRDNSKNTLYLQMNSLRAEDTAVYYCARAELLAFDVYTASDYWGQGTLVTVSS (SEQ ID NO:42) | 1.651 | 195. 1 |
| 1F3-1C11 | EVQLVESGGGLVQPGGSLRLSCAASGFNFSSYGMSWVRQAPGKGLEWVSAISGSGGSTYYADSVKGRFTISRDNSKNTLYLQMNSLRAEDTAVYYCARAELLAFDVYTASDYWGQGTLVTVSS(SEQ ID NO:43) | 1.633 | 78.5 1 |
| 1F3-1C4 | EVQLVESGGGLVQPGGSLRLSCAASGFTFGDYGMSWVRQAPGKGLEWVSGIKPWGSGTYYADSVKGRFTISRDNSKNTLYLQMNSLRAEDTAVYYCARAELLAFDVYTASDYWGQGTLVTVSS(SEQ ID NO:44) | 1.591 | 238. 3 |
| 1F3-1D1 | EVQLVESGGGLVQPGGSLRLSCAASGDIFTNYSMSWVRQAPGKGLEWVSGIYPSGDNNRYADSVKGRFTISRDNSKNTLYLQMNSLRAEDTAVYYCARAELLAFDVYTASDYWGQGTLVTVSS(SEQ ID NO:45) | 1.287 | 78.3 3 |
| 1F3-1D12 | EVQLVESGGGLVQPGGSLRLSCAASGYNFTDYGMSWVRQAPGKGLEWVSGISGSGDNKYYADSVKGRFTISRDNSKNTLYLQMNSLRAEDTAVYYCARAELLAFDVYTASDYWGQGTLVTVSS(SEQ ID NO:46) | 1.633 | 323. 6 |
| 1F3-1D6 | EVQLVESGGGLVQPGGSLRLSCAASGGSFYGFGMSWVRQAPGKGLEWVSGIYSHDADKRYADSVKGRFTISRDNSKNTLYLQMNSLRAEDTAVYYCARAELLAFDVYTASDYWGQGTLVTVSS(SEQ ID NO:47) | 1.441 | 97.8 8 |
| 1F3-1E1 | EVQLVESGGGLVQPGGSLRLSCAASGFSFDDYAMSWVRQAPGKGLEWVSIIYPTGSTNYYADSVKGRFTISRDNSKNTLYLQMNSLRAEDTAVYYCARAELLAFDVYTASDYWGQGTLVTVSS(SEQ ID NO:48) | 1.643 | 214. 1 |

(continued)

| Clone No. | VH sequences (with CDR sequences underlined, according to the Kabat numbering system) | OD value | $EC_{50}$ |
|---|---|---|---|
| 1F3-1E10 | EVQLVESGGGLVQPGGSLRLSCAASGFNFSSYGMSWVRQAPGKGLEWVSRISPYTDNKYYADSVKGRFTISRDNSKNTLYLQMNSLRAEDTAVYYCARAELLAFDVYTASDYWGQGTLVTVSS(SEQ ID NO:49) | 1.545 | 187. 9 |
| 1F3-1E4 | EVQLVESGGGLVQPGGSLRLSCAASGFSFGTYGMSWVRQAPGKGLEWVSRISGDSANIRYADSVKGRFTISRDNSKNTLYLQMNSLRAEDTAVYYCARAELLAFDVYTASDYWGQGTLVTVSS(SEQ ID NO:50) | 1.615 | 386. 4 |
| 1F3-1E9 | EVQLVESGGGLVQPGGSLRLSCAASGFTFDDYAMSWVRQAPGKGLEWVSGIKPSSWTTYYADSVKGRFTISRDNSKNTLYLQMNSLRAEDTAVYYCARAELLAFDVYTASDYWGQGTLVTVSS(SEQ ID NO:51) | 1.555 | 192 |
| 1F3-1F4 | EVQLVESGGGLVQPGGSLRLSCAASGDIFTGSSMSWVRQAPGKGLEWVSGIYASGSDKRYADSVKGRFTISRDNSKNTLYLQMNSLRAEDTAVYYCARAELLAFDVYTASDYWGQGTLVTVSS(SEQ ID NO:52) | 1.421 | 147. 1 |
| 1F3-1F8 | EVQLVESGGGLVQPGGSLRLSCAASGFNFGNYGMSWVRQAPGKGLEWVSRISGDGSNINYADSVKGRFTISRDNSKNTLYLQMNSLRAEDTAVYYCARAELLAFDVYTASDYWGQGTLVTVSS(SEQ ID NO:53) | 1.671 | 677 |
| 1F3-1G11 | EVQLVESGGGLVQPGGSLRLSCAASGYSFTGYGMSWVRQAPGKGLEWVSGISPSGGTIGYADSVKGRFTISRDNSKNTLYLQMNSLRAEDTAVYYCARAELLAFDVYTASDYWGQGTLVTVSS(SEQ ID NO:54) | 1.652 | 417. 3 |
| 1F3-1G2 | EVQLVESGGGLVQPGGSLRLSCAASGYTFGDYGMSWVRQAPGKGLEWVSRIDPWGASIRYADSVKGRFTISRDNSKNTLYLQMNSLRAEDTAVYYCARAELLAFDVYTASDYWGQGTLVTVSS(SEQ ID NO:55) | 1.598 | 164. 2 |
| 1F3-1G5 | EVQLVESGGGLVQPGGSLRLSCAASGFSFGSYGMSWVRQAPGKGLEWVSGIKPSTSTTYYADSVKGRFTISRDNSKNTLYLQMNSLRAEDTAVYYCARAELLAFDVYTASDYWGQGTLVTVSS(SEQ ID NO:56) | 1.565 | 122. 6 |
| 1F3-1G7 | EVQLVESGGGLVQPGGSLRLSCAASGDIFSNYSMSWVRQAPGKGLEWVSGIYPSGDSRRYADSVKGRFTISRDNSKNTLYLQMNSLRAEDTAVYYCARAELLAFDVYTASDYWGQGTLVTVSS(SEQ ID NO:57) | 1.56 | 198. 3 |

(continued)

| Clone No. | VH sequences (with CDR sequences underlined, according to the Kabat numbering system) | OD value | $EC_{50}$ |
|---|---|---|---|
| 1F3-1H11 | EVQLVESGGGLVQPGGSLRLSCAASGFNFTDYGMSWVRQAPGKGLEWVSRISQWGDTSNYADSVKGRFTISRDNSKNTLYLQMNSLRAEDTAVYYCARAELLAFDVYTASDYWGQGTLVTVSS(SEQ ID NO:58) | 1.65 | 162 |
| 1F3-1H3 | EVQLVESGGGLVQPGGSLRLSCAASGFNFYNYGMSWVRQAPGKGLEWVSGIKPHSDVAHYADSVKGRFTISRDNSKNTLYLQMNSLRAEDTAVYYCARAELLAFDVYTASDYWGQGTLVTVSS(SEQ ID NO:59) | 1.725 | 313. 6 |
| 1F3-2A11 | EVQLVESGGGLVQPGGSLRLSCAASGDNFYTFYMSWVRQAPGKGLEWVSGIKPTGSYINYADSVKGRFTISRDNSKNTLYLQMNSLRAEDTAVYYCARAELLAFDVYTASDYWGQGTLVTVSS(SEQ ID NO:60) | 1.525 | 108. 6 |
| 1F3-2A12 | EVQLVESGGGLVQPGGSLRLSCAASGYSFDSYGMSWVRQAPGKGLEWVSGIKPTGASTYYADSVKGRFTISRDNSKNTLYLQMNSLRAEDTAVYYCARAELLAFDVYTASDYWGQGTLVTVSS(SEQ ID NO:61) | 1.646 | 264. 7 |
| 1F3-2B1 | EVQLVESGGGLVQPGGSLRLSCAASGYNFDNYAMSWVRQAPGKGLEWVSGIKPDSATTYYADSVKGRFTISRDNSKNTLYLQMNSLRAEDTAVYYCARAELLAFDVYTASDYWGQGTLVTVSS(SEQ ID NO:62) | 1.617 | 268. 7 |
| 1F3-2B2 | EVQLVESGGGLVQPGGSLRLSCAASGYNFYSYGMSWVRQAPGKGLEWVSIISPDGGYKRYADSVKGRFTISRDNSKNTLYLQMNSLRAEDTAVYYCARAELLAFDVYTASDYWGQGTLVTVSS(SEQ ID NO:63) | 1.574 | 171. 7 |
| 1F3-2B2-2 | EVQLVESGGGLVQPGGSLRLSCAASGYSFSDFGMSWVRQAPGKGLEWVSRIDPSGWSTSYADSVKGRFTISRDNSKNTLYLQMNSLRAEDTAVYYCARAELLAFDVYTASDYWGQGTLVTVSS(SEQ ID NO:64) | 1.593 | 217 |
| 1F3-2B9 | EVQLVESGGGLVQPGGSLRLSCAASGFNFDSYAMSWVRQAPGKGLEWVSGIKPHTDDIYYADSVKGRFTISRDNSKNTLYLQMNSLRAEDTAVYYCARAELLAFDVYTASDYWGQGTLVTVSS (SEQ ID NO:65) | 1.568 | 289 |
| 1F3-2C6 | EVQLVESGGGLVQPGGSLRLSCAASGDIFTNYSMSWVRQAPGKGLEWVSGIYASGSDKRYADSVKGRFTISRDNSKNTLYLQMNSLRAEDTAVYYCARAELLAFDVYTASDYWGQGTLVTVSS(SEQ ID NO:66) | 1.382 | 65.3 7 |

(continued)

| Clone No. | VH sequences (with CDR sequences underlined, according to the Kabat numbering system) | OD value | $EC_{50}$ |
|---|---|---|---|
| 1F3-2C9 | EVQLVESGGGLVQPGGSLRLSCAASGFNFGGYGMSWVRQAPGKGLEWVSRISPGDSTPRYADSVKGRFTISRDNSKNTLYLQMNSLRAEDTAVYYCARAELLAFDVYTASDYWGQGTLVTVSS(SEQ ID NO:67) | 1.586 | 191. 4 |
| 1F3-2D2 | EVQLVESGGGLVQPGGSLRLSCAASGFNFSSYGMSWVRQAPGKGLEWVSRISPYTGTTHYADSVKGRFTISRDNSKNTLYLQMNSLRAEDTAVYYCARAELLAFDVYTASDYWGQGTLVTVSS(SEQ ID NO:68) | 1.589 | 180. 9 |
| 1F3-2D8 | EVQLVESGGGLVQPGGSLRLSCAASGYSFSDYGMSWVRQAPGKGLEWVSRISPSKAVKDYADSVKGRFTISRDNSKNTLYLQMNSLRAEDTAVYYCARAELLAFDVYTASDYWGQGTLVTVSS(SEQ ID NO:69) | 1.56 | 155. 8 |
| 1F3-2E12 | EVQLVESGGGLVQPGGSLRLSCAASGGNFTNSGMSWVRQAPGKGLEWVSGIKWSSDETYYADSVKGRFTISRDNSKNTLYLQMNSLRAEDTAVYYCARAELLAFDVYTASDYWGQGTLVTVSS(SEQ ID NO:70) | 1.519 | 95.0 6 |
| 1F3-2E8 | EVQLVESGGGLVQPGGSLRLSCAASGYSFSDYGMSWVRQAPGKGLEWVSWISPSKGSASYADSVKGRFTISRDNSKNTLYLQMNSLRAEDTAVYYCARAELLAFDVYTASDYWGQGTLVTVSS(SEQ ID NO:71) | 1.556 | 209. 9 |
| 1F3-2F1 | EVQLVESGGGLVQPGGSLRLSCAASGDIFTGYSMSWVRQAPGKGLEWVSGIYQTGSNTRYADSVKGRFTISRDNSKNTLYLQMNSLRAEDTAVYYCARAELLAFDVYTASDYWGQGTLVTVSS(SEQ ID NO:72) | 1.025 | NA |
| 1F3-2F3 | EVQLVESGGGLVQPGGSLRLSCAASGFNFSDYGMSWVRQAPGKGLEWVSRIDWDGGNTRYADSVKGRFTISRDNSKNTLYLQMNSLRAEDTAVYYCARAELLAFDVYTASDYWGQGTLVTVSS(SEQ ID NO:73) | 1.567 | 325. 3 |
| 1F3-2F6 | EVQLVESGGGLVQPGGSLRLSCAASGFTFSDYGMSWVRQAPGKGLEWVSRISQYGGGIRYADSVKGRFTISRDNSKNTLYLQMNSLRAEDTAVYYCARAELLAFDVYTASDYWGQGTLVTVSS(SEQ ID NO:74) | 1.519 | 129. 4 |
| 1F3-2G10 | EVQLVESGGGLVQPGGSLRLSCAASGFNFSNYGMSWVRQAPGKGLEWVSRIKPTDGSTHYADSVKGRFTISRDNSKNTLYLQMNSLRAEDTAVYYCARAELLAFDVYTASDYWGQGTLVTVSS(SEQ ID NO:75) | 1.54 | 120. 2 |

(continued)

| Clone No. | VH sequences (with CDR sequences underlined, according to the Kabat numbering system) | OD value | $EC_{50}$ |
|---|---|---|---|
| 1F3-2G9 | EVQLVESGGGLVQPGGSLRLSCAASGFTFGGYGMSWVRQAPGKGLEWVSWISAWDGSIYYADSVKGRFTISRDNSKNTLYLQMNSLRAEDTAVYYCARAELLAFDVYTASDYWGQGTLVTVSS(SEQ ID NO:76) | 1.38 | 49.3 3 |
| 1F3-2H12 | EVQLVESGGGLVQPGGSLRLSCAASGDSFDSSGMSWVRQAPGKGLEWVSGIKWITGESYYADSVKGRFTISRDNSKNTLYLQMNSLRAEDTAVYYCARAELLAFDVYTASDYWGQGTLVTVSS(SEQ ID NO:77) | 1.59 | 130. 5 |
| 1F3-2H6 | EVQLVESGGGLVQPGGSLRLSCAASGYTFSSYGMSWVRQAPGKGLEWVSRIKSDGGDTHYADSVKGRFTISRDNSKNTLYLQMNSLRAEDTAVYYCARAELLAFDVYTASDYWGQGTLVTVSS(SEQ ID NO:78) | 1.311 | 53.4 9 |

Table 4. Heavy chain variable region sequences of 1G8-series

| Clone No. | VH sequences (with CDR sequences underlined, according to the Kabat numbering system) | OD value | $EC_{50}$ |
|---|---|---|---|
| 1G8 | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCARQTEDRSYGYDLDYWGQGTLVTVSS(SEQ ID NO:11) | NA | NA |
| 1G8-1A12 | EVQLVESGGGLVQPGGSLRLSCAASGGTFDTNAMSWVRQAPGKGLEWVSAISTGGDNKRYADSVKGRFTISRDNSKNTLYLQMNSLRAEDTAVYYCARQTEDRSYGYDLDYWGQGTLVTVSS(SEQ ID NO:79) | 1.612 | 518.8 |
| 1G8-1A3 | EVQLVESGGGLVQPGGSLRLSCAASGYNFGSYAMSWVRQAPGKGLEWVSRIYPGSWSPDYADSVKGRFTISRDNSKNTLYLQMNSLRAEDTAVYYCARQTEDRSYGYDLDYWGQGTLVTVSS(SEQ ID NO:80) | 1.625 | 316.4 |
| 1G8-1A7 | EVQLVESGGGLVQPGGSLRLSCAASGYTFDSYAMSWVRQAPGKGLEWVSRIKTTDDGKSYADSVKGRFTISRDNSKNTLYLQMNSLRAEDTAVYYCARQTEDRSYGYDLDYWGQGTLVTVSS(SEQ ID NO:81) | 1.573 | 307.6 |
| 1G8-1B11 | EVQLVESGGGLVQPGGSLRLSCAASGYNFSNYAMSWVRQAPGKGLEWVSAIDASGGDTRYADSVKGRFTISRDNSKNTLYLQMNSLRAEDTAVYYCARQTEDRSYGYDLDYWGQGTLVTVSS(SEQ ID NO:82) | 1.582 | 597.6 |
| 1G8-1B7 | EVQLVESGGGLVQPGGSLRLSCAASGYSFSNYAMSWVRQAPGKGLEWVSRISAWGSTNNYADSVKGRFTISRDNSKNTLYLQMNSLRAEDTAVYYCARQTEDRSYGYDLDYWGQGTLVTVSS(SEQ ID NO:83) | 1.558 | 295.9 |
| 1G8-1C1 | EVQLVESGGGLVQPGGSLRLSCAASGYIFSNYAMSWVRQAPGKGLEWVSRISTGFAGPDYADSVKGRFTISRDNSKNTLYLQMNSLRAEDTAVYYCARQTEDRSYGYDLDYWGQGTLVTVSS(SEQ ID NO:84) | 1.502 | 294.5 |
| 1G8-1C10 | EVQLVESGGGLVQPGGSLRLSCAASGFNFSDYSMSWVRQAPGKGLEWVSRIKASSGGSDYADSVKGRFTISRDNSKNTLYLQMNSLRAEDTAVYYCARQTEDRSYGYDLDYWGQGTLVTVSS(SEQ ID NO:85) | 1.606 | 618.1 |
| 1G8-1C12 | EVQLVESGGGLVQPGGSLRLSCAASGGSFYGFGMSWVRQAPGKGLEWVSGIYSHDADKRYADSVKGRFTISRDNSKNTLYLQMNSLRAEDTAVYYCARQTEDRSYGYDLDYWGQGTLVTVSS(SEQ ID NO:86) | 1.415 | 24.26 |
| 1G8-1C8 | EVQLVESGGGLVQPGGSLRLSCAASGFSFGSSAMSWVRQAPGKGLEWVSAISGSSDSPRYADSVKGRFTISRDNSKNTLYLQMNSLRAEDTAVYYCARQTEDRSYGYDLDYWGQGTLVTVSS(SEQ ID NO:87) | 1.591 | 601.6 |
| 1G8-1D11 | EVQLVESGGGLVQPGGSLRLSCAASGDIFTGYAMSWVRQAPGKGLEWVSRIDGTGDSKFYADSVKGRFTISRDNSKNTLYLQMNSLRAEDTAVYYCARQTEDRSYGYDLDYWGQGTLVTVSS(SEQ ID NO:88) | 1.591 | 326.2 |
| 1G8-1D7 | EVQLVESGGGLVQPGGSLRLSCAASGGSFSNYAMSWVRQAPGKGLEWVSWIDTTSAYASYADSVKGRFTISRDNSKNTLYLQMNSLRAEDTAVYYCARQTEDRSYGYDLDYWGQGTLVTVSS(SEQ ID NO:89) | 1.491 | 190.4 |
| 1G8-1E10 | EVQLVESGGGLVQPGGSLRLSCAASGDSFSTYAMSWVRQAPGKGLEWVSRISGSGDGKDYADSVKGRFTISRDNSKNTLYLQMNSLRAEDTAVYYCARQTEDRSYGYDLDYWGQGTLVTVSS(SEQ ID NO:90) | 1.602 | 512.5 |

(continued)

| Clone No. | VH sequences (with CDR sequences underlined, according to the Kabat numbering system) | OD value | $EC_{50}$ |
|---|---|---|---|
| 1G8-1E12 | EVQLVESGGGLVQPGGSLRLSCAASGFNFTSYAMSWVRQAPGKGLEWVSRISAHKWDASYADSVKGRFTISRDNSKNTLYLQMNSLRAEDTAVYYCARQTEDRSYGYDLDYWGQGTLVTVSS(SEQ ID NO:91) | 1.613 | 272.3 |
| 1G8-1E6 | EVQLVESGGGLVQPGGSLRLSCAASGYIFTDYSMSWVRQAPGKGLEWVSRIYTDGWEKDYADSVKGRFTISRDNSKNTLYLQMNSLRAEDTAVYYCARQTEDRSYGYDLDYWGQGTLVTVSS(SEQ ID NO:92) | 1.481 | 225 |
| 1G8-1E8 | EVQLVESGGGLVQPGGSLRLSCAASGFTFDGYAMSWVRQAPGKGLEWVSRISSYSWNPDYADSVKGRFTISRDNSKNTLYLQMNSLRAEDTAVYYCARQTEDRSYGYDLDYWGQGTLVTVSS(SEQ ID NO:93) | 1.546 | 303.5 |
| 1G8-1F11 | EVQLVESGGGLVQPGGSLRLSCAASGDTFSSYAMSWVRQAPGKGLEWVSRISQTGGTTDYADSVKGRFTISRDNSKNTLYLQMNSLRAEDTAVYYCARQTEDRSYGYDLDYWGQGTLVTVSS(SEQ ID NO:94) | 1.601 | 580.7 |
| 1G8-1F5 | EVQLVESGGGLVQPGGSLRLSCAASGDIFDTYMSWVRQAPGKGLEWVSRIYTDGSSDYADSVKGRFTISRDNSKNTLYLQMNSLRAEDTAVYYCARQTEDRSYGYDLDYWGQGTLVTVSS(SEQ ID NO:95) | 1.564 | 551.9 |
| 1G8-1F7 | EVQLVESGGGLVQPGGSLRLSCAASGYSFYGYAMSWVRQAPGKGLEWVSAIDAIGGNKRYADSVKGRFTISRDNSKNTLYLQMNSLRAEDTAVYYCARQTEDRSYGYDLDYWGQGTLVTVSS(SEQ ID NO:96) | 1.576 | 423 |
| 1G8-1G12 | EVQLVESGGGLVQPGGSLRLSCAASGYNFTSSAMSWVRQAPGKGLEWVSRIYSISGNTDYADSVKGRFTISRDNSKNTLYLQMNSLRAEDTAVYYCARQTEDRSYGYDLDYWGQGTLVTVSS(SEQ ID NO:97) | 1.546 | 204.2 |
| 1G8-1G3 | EVQLVESGGGLVQPGGSLRLSCAASGFNFGTYAMSWVRQAPGKGLEWVSRISGISAGTNYADSVKGRFTISRDNSKNTLYLQMNSLRAEDTAVYYCARQTEDRSYGYDLDYWGQGTLVTVSS(SEQ ID NO:98) | 1.581 | 355.4 |
| 1G8-1G5 | EVQLVESGGGLVQPGGSLRLSCAASGYNFTTSAMSWVRQAPGKGLEWVSRIYADTAYNDYADSVKGRFTISRDNSKNTLYLQMNSLRAEDTAVYYCARQTEDRSYGYDLDYWGQGTLVTVSS(SEQ ID NO:99) | 1.482 | 170.1 |
| 1G8-1G9 | EVQLVESGGGLVQPGGSLRLSCAASGFNFGDFAMSWVRQAPGKGLEWVSRIYSGDDNASYADSVKGRFTISRDNSKNTLYLQMNSLRAEDTAVYYCARQTEDRSYGYDLDYWGQGTLVTVSS(SEQ ID NO:100) | 1.546 | 348 |
| 1G8-1H1 | EVQLVESGGGLVQPGGSLRLSCAASGDTFSGYAMSWVRQAPGKGLEWVSRIYPYSSVRDYADSVKGRFTISRDNSKNTLYLQMNSLRAEDTAVYYCARQTEDRSYGYDLDYWGQGTLVTVSS(SEQ ID NO:101) | 1.398 | 89.93 |
| 1G8-1H6 | EVQLVESGGGLVQPGGSLRLSCAASGFNFSGFAMSWVRQAPGKGLEWVSRIYPTSSESDYADSVKGRFTISRDNSKNTLYLQMNSLRAEDTAVYYCARQTEDRSYGYDLDYWGQGTLVTVSS(SEQ ID NO:102) | 1.584 | 386.9 |
| 1G8-2A1 | EVQLVESGGGLVQPGGSLRLSCAASGYSFDGFAMSWVRQAPGKGLEWVSRISGHDWSSSYADSVKGRFTISRDNSKNTLYLQMNSLRAEDTAVYYCARQTEDRSYGYDLDYWGQGTLVTVSS(SEQ ID NO:103) | 1.597 | 338.7 |

(continued)

| Clone No. | VH sequences (with CDR sequences underlined, according to the Kabat numbering system) | OD value | $EC_{50}$ |
|---|---|---|---|
| 1G8-2A11 | EVQLVESGGGLVQPGGSLRLSCAASGDSFYNYSMSWVRQAPGKGLEWVSRISSDGWETFYADSVKGRFTISRDNSKNTLYLQMNSLRAEDTAVYYCARQTEDRSYGYDLDYWGQGTLVTVSS(SEQ ID NO:104) | 1.538 | 352.9 |
| 1G8-2A4 | EVQLVESGGGLVQPGGSLRLSCAASGDTFSSNAMSWVRQAPGKGLEWVSRIYGGSWDTDYADSVKGRFTISRDNSKNTLYLQMNSLRAEDTAVYYCARQTEDRSYGYDLDYWGQGTLVTVSS(SEQ ID NO:105) | 1.543 | 217.4 |
| 1G8-2A7 | EVQLVESGGGLVQPGGSLRLSCAASGYSFSDYAMSWVRQAPGKGLEWVSRISAYGWGKSYADSVKGRFTISRDNSKNTLYLQMNSLRAEDTAVYYCARQTEDRSYGYDLDYWGQGTLVTVSS(SEQ ID NO:106) | 1.602 | 311.7 |
| 1G8-2B10 | EVQLVESGGGLVQPGGSLRLSCAASGFSFTNNAMSWVRQAPGKGLEWVSRIDTIGGTSSYADSVKGRFTISRDNSKNTLYLQMNSLRAEDTAVYYCARQTEDRSYGYDLDYWGQGTLVTVSS(SEQ ID NO:107) | 1.559 | 344 |
| 1G8-2B12 | EVQLVESGGGLVQPGGSLRLSCAASGDTFGTSSMSWVRQAPGKGLEWVSRIDTWGWYKFYADSVKGRFTISRDNSKNTLYLQMNSLRAEDTAVYYCARQTEDRSYGYDLDYWGQGTLVTVSS(SEQ ID NO:108) | 1.392 | NA |
| 1G8-2B3 | EVQLVESGGGLVQPGGSLRLSCAASGYNFTTSAMSWVRQAPGKGLEWVSRIYQDSSTTDYADSVKGRFTISRDNSKNTLYLQMNSLRAEDTAVYYCARQTEDRSYGYDLDYWGQGTLVTVSS(SEQ ID NO:109) | 1.569 | 396.5 |
| 1G8-2B5 | EVQLVESGGGLVQPGGSLRLSCAASGYNFGNYAMSWVRQAPGKGLEWVSRIKSHDSTSHYADSVKGRFTISRDNSKNTLYLQMNSLRAEDTAVYYCARQTEDRSYGYDLDYWGQGTLVTVSS(SEQ ID NO:110) | 1.578 | NA |
| 1G8-2B6 | EVQLVESGGGLVQPGGSLRLSCAASGGSFGGYAMSWVRQAPGKGLEWVSRIYAYKSENDYADSVKGRFTISRDNSKNTLYLQMNSLRAEDTAVYYCARQTEDRSYGYDLDYWGQGTLVTVSS(SEQ ID NO:111) | 1.565 | 225.1 |
| 1G8-2C6 | EVQLVESGGGLVQPGGSLRLSCAASGDNFSSYAMSWVRQAPGKGLEWVSRIYGSDSTPDYADSVKGRFTISRDNSKNTLYLQMNSLRAEDTAVYYCARQTEDRSYGYDLDYWGQGTLVTVSS(SEQ ID NO:112) | 1.578 | 615.7 |
| 1G8-2C7 | EVQLVESGGGLVQPGGSLRLSCAASGYSFTNFAMSWVRQAPGKGLEWVSRISQDGGNKNYADSVKGRFTISRDNSKNTLYLQMNSLRAEDTAVYYCARQTEDRSYGYDLDYWGQGTLVTVSS(SEQ ID NO:113) | 1.585 | 404.3 |
| 1G8-2D11 | EVQLVESGGGLVQPGGSLRLSCAASGYSFSGNAMSWVRQAPGKGLEWVSRIDGWGSYTDYADSVKGRFTISRDNSKNTLYLQMNSLRAEDTAVYYCARQTEDRSYGYDLDYWGQGTLVTVSS(SEQ ID NO:114) | 1.53 | 298.6 |
| 1G8-2D4 | EVQLVESGGGLVQPGGSLRLSCAASGYNFGGYAMSWVRQAPGKGLEWVSRISGTSGNNDYADSVKGRFTISRDNSKNTLYLQMNSLRAEDTAVYYCARQTEDRSYGYDLDYWGQGTLVTVSS(SEQ ID NO:115) | 1.577 | 492.1 |
| 1G8-2D6 | EVQLVESGGGLVQPGGSLRLSCAASGFSFDGYAMSWVRQAPGKGLEWVSRISAHSWNKDYADSVKGRFTISRDNSKNTLYLQMNSLRAEDTAVYYCARQTEDRSYGYDLDYWGQGTLVTVSS(SEQ ID NO:116) | 1.629 | 523.9 |

(continued)

| Clone No. | VH sequences (with CDR sequences underlined, according to the Kabat numbering system) | OD value | $EC_{50}$ |
|---|---|---|---|
| 1G8-2D7 | EVQLVESGGGLVQPGGSLRLSCAASGDSFTSYAMSWVRQAPGKGLEWVSRISADSASPSYADSVKGRFTISRDNSKNTLYLQMNSLRAEDTAVYYCARQTEDRSYGYDLDYWGQGTLVTVSS(SEQ ID NO:117) | 1.544 | 418.6 |
| 1G8-2D9 | EVQLVESGGGLVQPGGSLRLSCAASGDIFDGYSMSWVRQAPGKGLEWVSRIDTGGSVKNYADSVKGRFTISRDNSKNTLYLQMNSLRAEDTAVYYCARQTEDRSYGYDLDYWGQGTLVTVSS(SEQ ID NO:118) | 1.537 | 436.6 |
| 1G8-2E7 | EVQLVESGGGLVQPGGSLRLSCAASGYTFGNYAMSWVRQAPGKGLEWVSRISPSTWNIDYADSVKGRFTISRDNSKNTLYLQMNSLRAEDTAVYYCARQTEDRSYGYDLDYWGQGTLVTVSS(SEQ ID NO:119) | 1.556 | 599.5 |
| 1G8-2F11 | EVQLVESGGGLVQPGGSLRLSCAASGYTFGSNAMSWVRQAPGKGLEWVSRISPDSSGISYADSVKGRFTISRDNSKNTLYLQMNSLRAEDTAVYYCARQTEDRSYGYDLDYWGQGTLVTVSS(SEQ ID NO:120) | 1.574 | 617 |
| 1G8-2F2 | EVQLVESGGGLVQPGGSLRLSCAASGDIFGSYAMSWVRQAPGKGLEWVSRISSHGASRDYADSVKGRFTISRDNSKNTLYLQMNSLRAEDTAVYYCARQTEDRSYGYDLDYWGQGTLVTVSS(SEQ ID NO:121) | 1.599 | 566.8 |
| 1G8-2F9 | EVQLVESGGGLVQPGGSLRLSCAASGYSFSNYAMSWVRQAPGKGLEWVSRISAWGSTNNYADSVKGRFTISRDNSKNTLYLQMNSLRAEDTAVYYCARQTEDRSYGYDLDYWGQGTLVTVSS(SEQ ID NO:122) | 1.565 | 358.8 |
| 1G8-2G12 | EVQLVESGGGLVQPGGSLRLSCAASGFTFSNNAMSWVRQAPGKGLEWVSAIYASTDGRSYADSVKGRFTISRDNSKNTLYLQMNSLRAEDTAVYYCARQTEDRSYGYDLDYWGQGTLVTVSS(SEQ ID NO:123) | 1.505 | 300.9 |
| 1G8-2G3 | EVQLVESGGGLVQPGGSLRLSCAASGFIFGGNAMSWVRQAPGKGLEWVSRISSDKGTTNYADSVKGRFTISRDNSKNTLYLQMNSLRAEDTAVYYCARQTEDRSYGYDLDYWGQGTLVTVSS(SEQ ID NO:124) | 1.583 | 272.1 |
| 1G8-2G4 | EVQLVESGGGLVQPGGSLRLSCAASGFTFGGYSMSWVRQAPGKGLEWVSRISTDGAYTNYADSVKGRFTISRDNSKNTLYLQMNSLRAEDTAVYYCARQTEDRSYGYDLDYWGQGTLVTVSS(SEQ ID NO:125) | 1.58 | 311.9 |
| 1G8-2G7 | EVQLVESGGGLVQPGGSLRLSCAASGGTFYTYAMSWVRQAPGKGLEWVSRIYSTKGTPDYADSVKGRFTISRDNSKNTLYLQMNSLRAEDTAVYYCARQTEDRSYGYDLDYWGQGTLVTVSS(SEQ ID NO:126) | 1.489 | 136 |
| 1G8-2G8 | EVQLVESGGGLVQPGGSLRLSCAASGFNFTNYAMSWVRQAPGKGLEWVSRISTYGDTTHYADSVKGRFTISRDNSKNTLYLQMNSLRAEDTAVYYCARQTEDRSYGYDLDYWGQGTLVTVSS(SEQ ID NO:127) | 1.584 | 370.9 |
| 1G8-2G9 | EVQLVESGGGLVQPGGSLRLSCAASGYIFTNYAMSWVRQAPGKGLEWVSRISQSGGTNDYADSVKGRFTISRDNSKNTLYLQMNSLRAEDTAVYYCARQTEDRSYGYDLDYWGQGTLVTVSS(SEQ ID NO:128) | 1.545 | 285.1 |
| 1G8-2H1 | EVQLVESGGGLVQPGGSLRLSCAASGYSFYSYAMSWVRQAPGKGLEWVSRISQGGGVTHYADSVKGRFTISRDNSKNTLYLQMNSLRAEDTAVYYCARQTEDRSYGYDLDYWGQGTLVTVSS(SEQ ID NO:129) | 1.585 | 277.5 |

(continued)

| Clone No. | VH sequences (with CDR sequences underlined, according to the Kabat numbering system) | OD value | $EC_{50}$ |
|---|---|---|---|
| 1G8-2H10 | EVQLVESGGGLVQPGGSLRLSCAASGYNFTNSAMSWVRQAPGKGLEWVSSIYYHSGVPRYADSVKGRFTISRDNSKNTLYLQMNSLRAEDTAVYYCARQTEDRSYGYDLDYWGQGTLVTVSS(SEQ ID NO:130) | 1.535 | 214.2 |
| 1G8-2H11 | EVQLVESGGGLVQPGGSLRLSCAASGFNFDNYAMSWVRQAPGKGLEWVSRIYGWDWYASYADSVKGRFTISRDNSKNTLYLQMNSLRAEDTAVYYCARQTEDRSYGYDLDYWGQGTLVTVSS(SEQ ID NO:131) | 1.347 | NA |
| 1G8-2H3 | EVQLVESGGGLVQPGGSLRLSCAASGYSFGGYAMSWVRQAPGKGLEWVSRISGIKAYNDYADSVKGRFTISRDNSKNTLYLQMNSLRAEDTAVYYCARQTEDRSYGYDLDYWGQGTLVTVSS(SEQ ID NO:132) | 1.531 | 208.9 |
| 1G8-2H4 | EVQLVESGGGLVQPGGSLRLSCAASGDSFTGYAMSWVRQAPGKGLEWVSAISGSGGSTYYADSVKGRFTISRDNSKNTLYLQMNSLRAEDTAVYYCARQTEDRSYGYDLDYWGQGTLVTVSS(SEQ ID NO:133) | 1.578 | 440.5 |
| 1G8-2H8 | EVQLVESGGGLVQPGGSLRLSCAASGDIFGSYAMSWVRQAPGKGLEWVSAISGSGGSTYYADSVKGRFTISRDNSKNTLYLQMNSLRAEDTAVYYCARQTEDRSYGYDLDYWGQGTLVTVSS(SEQ ID NO:134) | 1.574 | 487.2 |
| 1G8-2H9 | EVQLVESGGGLVQPGGSLRLSCAASGFNFGNYAMSWVRQAPGKGLEWVSAIYSYFDEPRYADSVKGRFTISRDNSKNTLYLQMNSLRAEDTAVYYCARQTEDRSYGYDLDYWGQGTLVTVSS(SEQ ID NO:135) | 1.459 | 176.2 |

Table 5. Heavy chain variable region sequences of 10F4-series antibodies

| Clone No. | VH sequences (with CDR sequences underlined, according to the Kabat numbering system) | OD value |
|---|---|---|
| 10F4 | EVQLVESGGGLVQPGGSLRLSCAASGFTFSSYAMSWVRQAPGKGLEWVSAISGSGGSTYYADSVKGRFTISRDNSKNTLYLQMNSLRAEDTAVYYCAREYSDVVDYVSVRTPLDYWGQGTLVTVSS(SEQ ID NO:15) | |
| 10F4-1A1 | EVQLVESGGGLVQPGGSLRLSCAASGFSFDNYDMSWVRQAPGKGLEWVSSIKPGSAYKFYADSVKGRFTISRDNSKNTLYLQMNSLRAEDTAVYYCAREYSDVVDYVSVRTPLDYWGQGTLVTVSS(SEQ ID NO:136) | 1.691 |
| 10F4-1A3 | EVQLVESGGGLVQPGGSLRLSCAASGYSFSNYDMSWVRQAPGKGLEWVSGIKGWKSYTGYADSVKGRFTISRDNSKNTLYLQMNSLRAEDTAVYYCAREYSDVVDYVSVRTPLDYWGQGTLVTVSS(SEQ ID NO:137) | 1.534 |
| 10F4-1B2 | EVQLVESGGGLVQPGGSLRLSCAASGFSFGNYWMSWVRQAPGKGLEWVSGIDGHSSYAFYADSVKGRFTISRDNSKNTLYLQMNSLRAEDTAVYYCAREYSDVVDYVSVRTPLDYWGQGTLVTVSS(SEQ ID NO:138) | 1.635 |
| 10F4-1B5 | EVQLVESGGGLVQPGGSLRLSCAASGFNFTDYWMSWVRQAPGKGLEWVSSIIGDDSTNHYADSVKGRFTISRDNSKNTLYLQMNSLRAEDTAVYYCAREYSDVVDYVSVRTPLDYWGQGTLVTVSS(SEQ ID NO:139) | 1.526 |
| 10F4-1B7 | EVQLVESGGGLVQPGGSLRLSCAASGFNFSGFDMSWVRQAPGKGLEWVSGIKAYKSTADYADSVKGRFTISRDNSKNTLYLQMNSLRAEDTAVYYCAREYSDVVDYVSVRTPLDYWGQGTLVTVSS(SEQ ID NO:140) | 1.605 |

(continued)

| Clone No. | VH sequences (with CDR sequences underlined, according to the Kabat numbering system) | OD value |
|---|---|---|
| 10F4-1B9 | EVQLVESGGGLVQPGGSLRLSCAASGFNFDNYYMSWVRQAPGKGLEWVSSIYQTDASIFYADSVKGRFTISRDNSKNTLYLQMNSLRAEDTAVYYCAREYSDVVDYVSVRTPLDYWGQGTLVTVSS(SEQ ID NO:141) | 1.503 |
| 10F4-1C3 | EVQLVESGGGLVQPGGSLRLSCAASGYSFSDYWMSWVRQAPGKGLEWVSSIKQTGGVKHYADSVKGRFTISRDNSKNTLYLQMNSLRAEDTAVYYCAREYSDVVDYVSVRTPLDYWGQGTLVTVSS(SEQ ID NO:142) | 1.586 |
| 10F4-1C4 | EVQLVESGGGLVQPGGSLRLSCAASGFNFTDYWMSWVRQAPGKGLEWVSSIIGDDSTNHYADSVKGRFTISRDNSKNTLYLQMNSLRAEDTAVYYCAREYSDVVDYVSVRTPLDYWGQGTLVTVSS(SEQ ID NO:143) | 1.458 |
| 10F4-1C7 | EVQLVESGGGLVQPGGSLRLSCAASGDSFDNYWMSWVRQAPGKGLEWVSAIDTHSDNSFYADSVKGRFTISRDNSKNTLYLQMNSLRAEDTAVYYCAREYSDVVDYVSVRTPLDYWGQGTLVTVSS(SEQ ID NO:144) | 1.573 |
| 10F4-1C10 | EVQLVESGGGLVQPGGSLRLSCAASGYNFGSYDMSWVRQAPGKGLEWVSGIKTYGGNTHYADSVKGRFTISRDNSKNTLYLQMNSLRAEDTAVYYCAREYSDVVDYVSVRTPLDYWGQGTLVTVSS(SEQ ID NO:145) | 1.518 |
| 10F4-1D3 | EVQLVESGGGLVQPGGSLRLSCAASGFSFYDYDMSWVRQAPGKGLEWVSSIIQSGSTAFYADSVKGRFTISRDNSKNTLYLQMNSLRAEDTAVYYCAREYSDVVDYVSVRTPLDYWGQGTLVTVSS(SEQ ID NO:146) | 1.521 |
| 10F4-1D11 | EVQLVESGGGLVQPGGSLRLSCAASGFIFSDFDMSWVRQAPGKGLEWVSGIKQSSAGINYADSVKGRFTISRDNSKNTLYLQMNSLRAEDTAVYYCAREYSDVVDYVSVRTPLDYWGQGTLVTVSS(SEQ ID NO:147) | 1.46 |
| 10F4-1E1 | EVQLVESGGGLVQPGGSLRLSCAASGDIFGNYWMSWVRQAPGKGLEWVSSIDASSGNRFYADSVKGRFTISRDNSKNTLYLQMNSLRAEDTAVYYCAREYSDVVDYVSVRTPLDYWGQGTLVTVSS(SEQ ID NO:148) | 1.627 |
| 10F4-1E10 | EVQLVESGGGLVQPGGSLRLSCAASGDTFDTYAMSWVRQAPGKGLEWVSGISQWGAYASYADSVKGRFTISRDNSKNTLYLQMNSLRAEDTAVYYCAREYSDVVDYVSVRTPLDYWGQGTLVTVSS(SEQ ID NO:149) | 1.289 |
| 10F4-1E12 | EVQLVESGGGLVQPGGSLRLSCAASGYNFSSYDMSWVRQAPGKGLEWVSGISDTGDYANYADSVKGRFTISRDNSKNTLYLQMNSLRAEDTAVYYCAREYSDVVDYVSVRTPLDYWGQGTLVTVSS(SEQ ID NO:150) | 1.521 |
| 10F4-1F1 | EVQLVESGGGLVQPGGSLRLSCAASGFSFSNYWMSWVRQAPGKGLEWVSAISPGDGTIFYADSVKGRFTISRDNSKNTLYLQMNSLRAEDTAVYYCAREYSDVVDYVSVRTPLDYWGQGTLVTVSS(SEQ ID NO:151) | 1.632 |
| 10F4-1F2 | EVQLVESGGGLVQPGGSLRLSCAASGYNFTNYYMSWVRQAPGKGLEWVSSISAYSDNRNYADSVKGRFTISRDNSKNTLYLQMNSLRAEDTAVYYCAREYSDVVDYVSVRTPLDYWGQGTLVTVSS(SEQ ID NO:152) | 1.397 |
| 10F4-1F8 | EVQLVESGGGLVQPGGSLRLSCAASGYTFTDYDMSWVRQAPGKGLEWVSSIITDGGGAFYADSVKGRFTISRDNSKNTLYLQMNSLRAEDTAVYYCAREYSDVVDYVSVRTPLDYWGQGTLVTVSS(SEQ ID NO:153) | 1.492 |

(continued)

| Clone No. | VH sequences (with CDR sequences underlined, according to the Kabat numbering system) | OD value |
|---|---|---|
| 10F4-1F9 | EVQLVESGGGLVQPGGSLRLSCAASGFSFGGFDMSWVRQAPGKGLEWVSGIKTYGGYRSYADSVKGRFTISRDNSKNTLYLQMNSLRAEDTAVYYCAREYSDVVDYVSVRTPLDYWGQGTLVTVSS(SEQ ID NO:154) | 1.484 |
| 10F4-1G5 | EVQLVESGGGLVQPGGSLRLSCAASGYTFDSSWMSWVRQAPGKGLEWVSSIDAYGDYNFYADSVKGRFTISRDNSKNTLYLQMNSLRAEDTAVYYCAREYSDVVDYVSVRTPLDYWGQGTLVTVSS(SEQ ID NO:155) | 1.408 |
| 10F4-1G7 | EVQLVESGGGLVQPGGSLRLSCAASGYNFGDYDMSWVRQAPGKGLEWVSSIIGHGDTKFYADSVKGRFTISRDNSKNTLYLQMNSLRAEDTAVYYCAREYSDVVDYVSVRTPLDYWGQGTLVTVSS(SEQ ID NO:156) | 1.545 |
| 10F4-1G8 | EVQLVESGGGLVQPGGSLRLSCAASGFNFSDFDMSWVRQAPGKGLEWVSVIKPSGDYINYADSVKGRFTISRDNSKNTLYLQMNSLRAEDTAVYYCAREYSDVVDYVSVRTPLDYWGQGTLVTVSS(SEQ ID NO:157) | 1.52 |
| 10F4-1H1 | EVQLVESGGGLVQPGGSLRLSCAASGYSFGNFDMSWVRQAPGKGLEWVSGIKQYKGYTSYADSVKGRFTISRDNSKNTLYLQMNSLRAEDTAVYYCAREYSDVVDYVSVRTPLDYWGQGTLVTVSS(SEQ ID NO:158) | 1.611 |
| 10F4-1H4 | EVQLVESGGGLVQPGGSLRLSCAASGFNFTNYWMSWVRQAPGKGLEWVSAISGTGGYTYYADSVKGRFTISRDNSKNTLYLQMNSLRAEDTAVYYCAREYSDVVDYVSVRTPLDYWGQGTLVTVSS(SEQ ID NO:159) | 1.375 |
| 10F4-1H6 | EVQLVESGGGLVQPGGSLRLSCAASGFNFYDYDMSWVRQAPGKGLEWVSGIKTHSGGNGYADSVKGRFTISRDNSKNTLYLQMNSLRAEDTAVYYCAREYSDVVDYVSVRTPLDYWGQGTLVTVSS(SEQ ID NO:160) | 1.478 |
| 10F4-1H7 | EVQLVESGGGLVQPGGSLRLSCAASGYSFDTYDMSWVRQAPGKGLEWVSGIKQYKGYTSYADSVKGRFTISRDNSKNTLYLQMNSLRAEDTAVYYCAREYSDVVDYVSVRTPLDYWGQGTLVTVSS(SEQ ID NO:161) | 1.49 |
| 10F4-1H11 | EVQLVESGGGLVQPGGSLRLSCAASGYTFTGFWMSWVRQAPGKGLEWVSSISPDSGTKHYADSVKGRFTISRDNSKNTLYLQMNSLRAEDTAVYYCAREYSDVVDYVSVRTPLDYWGQGTLVTVSS(SEQ ID NO:162) | 1.562 |
| 10F4-2A1 | EVQLVESGGGLVQPGGSLRLSCAASGYTFDDYDMSWVRQAPGKGLEWVSGIKAHSDNKRYADSVKGRFTISRDNSKNTLYLQMNSLRAEDTAVYYCAREYSDVVDYVSVRTPLDYWGQGTLVTVSS(SEQ ID NO:163) | 1.758 |
| 10F4-2A5 | EVQLVESGGGLVQPGGSLRLSCAASGYSFTDYDMSWVRQAPGKGLEWVSGIKTYKSDARYADSVKGRFTISRDNSKNTLYLQMNSLRAEDTAVYYCAREYSDVVDYVSVRTPLDYWGQGTLVTVSS(SEQ ID NO:164) | 1.59 |
| 10F4-2A9 | EVQLVESGGGLVQPGGSLRLSCAASGYTFTDYDMSWVRQAPGKGLEWVSGIKQYKGYTSYADSVKGRFTISRDNSKNTLYLQMNSLRAEDTAVYYCAREYSDVVDYVSVRTPLDYWGQGTLVTVSS(SEQ ID NO:165) | 1.567 |
| 10F4-2B11 | EVQLVESGGGLVQPGGSLRLSCAASGGSFTNYWMSWVRQAPGKGLEWVSVIDSTGSNRFYADSVKGRFTISRDNSKNTLYLQMNSLRAEDTAVYYCAREYSDVVDYVSVRTPLDYWGQGTLVTVSS(SEQ ID NO:166) | 1.548 |

(continued)

| Clone No. | VH sequences (with CDR sequences underlined, according to the Kabat numbering system) | OD value |
|---|---|---|
| 10F4-2C2 | EVQLVESGGGLVQPGGSLRLSCAASGFNFSNYWMSWVRQAPGKGLEWVS VIDAHSANIRYADSVKGRFTISRDNSKNTLYLQMNSLRAEDTAVYYCARE YSDVVDYVSVRTPLDYWGQGTLVTVSS(SEQ ID NO:167) | 1.584 |
| 10F4-2C4 | EVQLVESGGGLVQPGGSLRLSCAASGYSFGDYWMSWVRQAPGKGLEWVS VIDSGSGNKRYADSVKGRFTISRDNSKNTLYLQMNSLRAEDTAVYYCARE YSDVVDYVSVRTPLDYWGQGTLVTVSS(SEQ ID NO:168) | 1.534 |
| 10F4-2C6 | EVQLVESGGGLVQPGGSLRLSCAASGFNFGGFDMSWVRQAPGKGLEWVS GIKQYKGYTSYADSVKGRFTISRDNSKNTLYLQMNSLRAEDTAVYYCARE YSDVVDYVSVRTPLDYWGQGTLVTVSS(SEQ ID NO:169) | 1.517 |
| 10F4-2C12 | EVQLVESGGGLVQPGGSLRLSCAASGYNFGDYSMSWVRQAPGKGLEWVS VIDTTSAYTRYADSVKGRFTISRDNSKNTLYLQMNSLRAEDTAVYYCARE YSDVVDYVSVRTPLDYWGQGTLVTVSS(SEQ ID NO:170) | 1.447 |
| 10F4-2E1 | EVQLVESGGGLVQPGGSLRLSCAASGFTFTTYDMSWVRQAPGKGLEWVS GIKYSKYYTHYADSVKGRFTISRDNSKNTLYLQMNSLRAEDTAVYYCARE YSDVVDYVSVRTPLDYWGQGTLVTVSS(SEQ ID NO:171) | 1.596 |
| 10F4-2E4 | EVQLVESGGGLVQPGGSLRLSCAASGYTFDSYWMSWVRQAPGKGLEWVS AIDGTDSVAYYADSVKGRFTISRDNSKNTLYLQMNSLRAEDTAVYYCARE YSDVVDYVSVRTPLDYWGQGTLVTVSS(SEQ ID NO:172) | 1.429 |
| 10F4-2E6 | EVQLVESGGGLVQPGGSLRLSCAASGFSFGDYWMSWVRQAPGKGLEWVS AIDQGKATSFYADSVKGRFTISRDNSKNTLYLQMNSLRAEDTAVYYCARE YSDVVDYVSVRTPLDYWGQGTLVTVSS(SEQ ID NO:173) | 1.454 |
| 10F4-2E8 | EVQLVESGGGLVQPGGSLRLSCAASGFNFGNYDMSWVRQAPGKGLEWVS SIIPHGDYSFYADSVKGRFTISRDNSKNTLYLQMNSLRAEDTAVYYCAREY SDVVDYVSVRTPLDYWGQGTLVTVSS(SEQ ID NO:174) | 1.376 |
| 10F4-2F11 | EVQLVESGGGLVQPGGSLRLSCAASGYNFTSYDMSWVRQAPGKGLEWVS GIKTGGGSARYADSVKGRFTISRDNSKNTLYLQMNSLRAEDTAVYYCARE YSDVVDYVSVRTPLDYWGQGTLVTVSS(SEQ ID NO:175) | 1.455 |
| 10F4-2G2 | EVQLVESGGGLVQPGGSLRLSCAASGYNFTDYDMSWVRQAPGKGLEWVS SIIASGSGNYYADSVKGRFTISRDNSKNTLYLQMNSLRAEDTAVYYCAREY SDVVDYVSVRTPLDYWGQGTLVTVSS(SEQ ID NO:176) | 1.485 |
| 10F4-2H5 | EVQLVESGGGLVQPGGSLRLSCAASGYIFTNYWMSWVRQAPGKGLEWVS SIDQGSGNIFYADSVKGRFTISRDNSKNTLYLQMNSLRAEDTAVYYCAREY SDVVDYVSVRTPLDYWGQGTLVTVSS(SEQ ID NO:177) | 1.442 |
| 10F4-1 | EVQLVESGGGLVQPGGSLRLSCAASGDSFTNYWMSWVRQAPGKGLEWVS GISQGGSYAYYADSVKGRFTISRDNSKNTLYLQMNSLRAEDTAVYYCARE YSDVVDYVSVRTPLDYWGQGTLVTVSS(SEQ ID NO:178) | 1.389 |
| 10F4-3 | EVQLVESGGGLVQPGGSLRLSCAASGFSFGDFAMSWVRQAPGKGLEWVS VIDGHTAYNSYADSVKGRFTISRDNSKNTLYLQMNSLRAEDTAVYYCARE YSDVVDYVSVRTPLDYWGQGTLVTVSS(SEQ ID NO:179) | 1.325 |

(continued)

| Clone No. | VH sequences (with CDR sequences underlined, according to the Kabat numbering system) | OD value |
|---|---|---|
| 10F4-5 | EVQLVESGGGLVQPGGSLRLSCAASGFIFDGYDMSWVRQAPGKGLEWVSVIKDHGGYKFYADSVKGRFTISRDNSKNTLYLQMNSLRAEDTAVYYCAREYSDVVDYVSVRTPLDYWGQGTLVTVSS(SEQ ID NO:180) | 1.308 |
| Note: NA indicates not available | | |

Table 6. CDR sequences of heavy chain variable region of 1F3-series antibodies

| No. | HCDR1 | | | | | HCDR2 | | | | | | | | | | | | HCDR3 |
|---|---|---|---|---|---|---|---|---|---|---|---|---|---|---|---|---|---|---|
| 1F3 | S | Y | A | M S | SEQ ID NO:8 | A | I | S | G | S | G | G | S | T | Y | YADSVK G | SEQ ID NO:9 | AELLAFDVYTAS DY (SEQ ID NO:14) |
| 1F3-1A9 | N | Y | A | M S | SEQ ID NO:18 1 | R | I | S | P | T | G | G | Y | A | S | YADSVK G | SEQ ID NO:19 6 | |
| 1F3-1B1 | T | Y | G | M S | SEQ ID NO:18 2 | G | I | K | P | G | D | S | T | T | Y | YADSVK G | SEQ ID NO: 197 | |
| 1F3-1C1 1 | S | Y | G | M S | SEQ ID NO:18 3 | A | I | S | G | S | G | G | S | T | Y | YADSVK G | SEQ ID NO:9 | |
| 1F3-1C4 | D | Y | G | M S | SEQ ID NO:18 4 | G | I | K | P | W | G | S | G | T | Y | YADSVK G | SEQ ID NO:19 9 | |
| 1F3-1D1 | N | Y | S | M S | SEQ ID NO: 185 | G | I | Y | P | S | G | D | N | N | R | YADSVK G | SEQ ID NO:20 0 | |
| 1F3-1D1 2 | D | Y | G | M S | SEQ ID NO:18 4 | G | I | S | G | S | G | D | N | K | Y | YADSVK G | SEQ ID NO:20 1 | |
| 1F3-1D6 | G | F | G | M S | SEQ ID NO:18 6 | G | I | Y | S | H | D | A | D | K | R | YADSVK G | SEQ ID NO:20 2 | |
| 1F3-1E1 | D | Y | A | M S | SEQ ID NO:18 7 | I | I | Y | P | T | G | S | T | N | Y | YADSVK G | SEQ ID NO:20 3 | |
| 1F3-1E1 0 | S | Y | G | M S | SEQ ID NO:18 3 | R | I | S | P | Y | T | D | N | K | Y | YADSVK G | SEQ ID NO:20 4 | |

(continued)

| No. | HCDR1 | | | | | HCDR2 | | | | | | | | | | | | HCDR3 |
|---|---|---|---|---|---|---|---|---|---|---|---|---|---|---|---|---|---|---|
| 1F3-1E4 | T | Y | G | M S | SEQ ID NO:18 2 | R | I | S | G | D | S | A | N | I | R | YADSVK G | SEQ ID NO:20 5 | |
| 1F3-1E9 | D | Y | A | M S | SEQ ID NO:18 7 | G | I | K | P | S | S | W | T | T | Y | YADSVK G | SEQ ID NO:20 6 | |
| 1F3-1F4 | G | S | S | M S | SEQ ID NO:18 8 | G | I | Y | A | S | G | S | D | K | R | YADSVK G | SEQ ID NO:20 7 | |
| 1F3-1F8 | N | Y | G | M S | SEQ ID NO:18 9 | R | I | S | G | D | G | S | N | I | N | YADSVK G | SEQ ID NO:20 8 | |
| 1F3-1G1 1 | G | Y | G | M S | SEQ ID NO:19 0 | G | I | S | P | S | G | G | T | I | G | YADSVK G | SEQ ID NO:20 9 | |
| 1F3-1G2 | D | Y | G | M S | SEQ ID NO:18 4 | R | I | D | P | W | G | A | S | I | R | YADSVK G | SEQ ID NO:21 0 | |
| 1F3-1G5 | S | Y | G | M S | SEQ ID NO:18 3 | G | I | K | P | S | T | S | T | T | Y | YADSVK G | SEQ ID NO:21 1 | |
| 1F3-1G7 | N | Y | S | M S | SEQ ID NO:18 5 | G | I | Y | P | S | G | D | S | R | R | YADSVK G | SEQ ID NO:21 2 | |
| 1F3-1H1 1 | D | Y | G | M S | SEQ ID NO:18 4 | R | I | S | Q | W | G | D | T | S | N | YADSVK G | SEQ ID NO:21 3 | |
| 1F3-1H3 | N | Y | G | M S | SEQ ID NO:18 9 | G | I | K | P | H | S | D | V | A | H | YADSVK G | SEQ ID NO:21 4 | |
| 1F3-2A1 1 | T | F | Y | M S | SEQ ID NO:19 1 | G | I | K | P | T | G | S | Y | I | N | YADSVK G | SEQ ID NO:21 5 | |
| 1F3-2A1 2 | S | Y | G | M S | SEQ ID NO:18 3 | G | I | K | P | T | G | A | S | T | Y | YADSVK G | SEQ ID NO:21 6 | |
| 1F3-2B1 | N | Y | A | M S | SEQ ID NO:18 1 | G | I | K | P | D | S | A | T | T | Y | YADSVK G | SEQ ID NO:21 7 | |
| 1F3-2B2 | S | Y | G | M S | SEQ ID NO:18 3 | I | I | S | P | D | G | G | Y | K | R | YADSVK G | SEQ ID NO:21 8 | |
| 1F3-2B2 -2 | D | F | G | M S | SEQ ID NO:19 2 | R | I | D | P | S | G | W | S | T | S | YADSVK G | SEQ ID NO:21 9 | |
| 1F3-2B9 | S | Y | A | M S | SEQ ID NO:8 | G | I | K | P | H | T | D | D | I | Y | YADSVK G | SEQ ID NO:22 0 | |
| 1F3-2C6 | N | Y | S | M S | SEQ ID | G | I | Y | A | S | G | S | D | K | R | YADSVK G | SEQ ID | |

(continued)

| No. | HCDR1 | | | | | HCDR2 | | | | | | | | | | | | HCDR3 |
|---|---|---|---|---|---|---|---|---|---|---|---|---|---|---|---|---|---|---|
| | | | | | NO:18 5 | | | | | I | | | | | | | NO:20 7 | |
| 1F3-2C9 | G | Y | G | M S | SEQ ID NO:19 0 | R | I | S | P | G | D | S | T | P | R | YADSVK G | SEQ ID NO:22 2 | |
| 1F3-2D2 | s | Y | G | M S | SEQ ID NO:18 3 | R | I | S | P | Y | T | G | T | T | H | YADSVK G | SEQ ID NO:22 3 | |
| 1F3-2D8 | D | Y | G | M S | SEQ ID NO:18 4 | R | I | S | P | S | K | A | V | K | D | YADSVK G | SEQ ID NO:22 4 | |
| 1F3-2E1 2 | N | s | G | M S | SEQ ID NO:19 3 | G | I | K | W | S | S | D | E | T | Y | YADSVK G | SEQ ID NO:22 5 | |
| 1F3-2E8 | D | Y | G | M S | SEQ ID NO:18 4 | W | I | S | P | S | K | G | S | A | S | YADSVK G | SEQ ID NO:22 6 | |
| 1F3-2F1 | G | Y | S | M S | SEQ ID NO:19 4 | G | I | Y | Q | T | G | S | N | T | R | YADSVK G | SEQ ID NO:22 7 | |
| 1F3-2F3 | D | Y | G | M S | SEQ ID NO:18 4 | R | I | D | W | D | G | G | N | T | R | YADSVK G | SEQ ID NO:22 8 | |
| IF3-2F6 | D | Y | G | M S | SEQ ID NO:18 4 | R | I | S | Q | Y | G | G | G | I | R | YADSVK G | SEQ ID NO:22 9 | |
| 1F3-2G1 0 | N | Y | G | M S | SEQ ID NO:18 9 | R | I | K | P | T | D | G | S | T | H | YADSVK G | SEQ ID NO:23 0 | |
| 1F3-2G9 | G | Y | G | M S | SEQ ID NO:19 0 | W | I | S | A | W | D | G | S | I | Y | YADSVK G | SEQ ID NO:23 1 | |
| 1F3-2H1 2 | S | s | G | M S | SEQ ID NO:19 5 | G | I | K | W | I | T | G | E | S | Y | YADSVK G | SEQ ID NO:19 8 | |
| 1F3-2H6 | s | Y | G | M S | SEQ ID NO:18 3 | R | I | K | S | D | G | G | D | T | H | YADSVK G | SEQ ID NO:22 1 | |

Table 7. CDR sequences of heavy chain variable region of IG8-series antibodies

| No. | HCDR1 | | | | | HCDR2 | | | | | | | | | | | | HCDR3 |
|---|---|---|---|---|---|---|---|---|---|---|---|---|---|---|---|---|---|---|
| 1G8 | s | Y | A | MS | SEQ ID NO:8 | A | I | S | G | S | G | G | S | T | Y | YADSVK G | SEQ ID NO:9 | QTEDRSYG YDLDY SEQ ID NO:12 |
| 1G8-1A12 | T | N | A | MS | SEQ ID NO:234 | A | I | S | T | G | G | D | N | K | R | YADSVK G | SEQ ID NO:251 | |
| 1G8-1A3 | S | Y | A | MS | SEQ ID NO:8 | R | I | Y | P | G | S | W | S | P | D | YADSVK G | SEQ ID NO:252 | |
| 1G8-1A7 | S | Y | A | MS | SEQ ID NO:8 | R | I | K | T | T | D | D | G | K | S | YADSVK G | SEQ ID NO:253 | |
| 1G8-1B11 | N | Y | A | MS | SEQ ID NO:181 | A | I | D | A | S | G | G | D | T | R | YADSVK G | SEQ ID NO:254 | |
| 1G8-1B7 | N | Y | A | MS | SEQ ID NO:181 | R | I | S | A | W | G | S | T | N | N | YADSVK G | SEQ ID NO:255 | |
| 1G8-1C1 | N | Y | A | MS | SEQ ID NO:181 | R | I | S | T | G | F | A | G | P | D | YADSVK G | SEQ ID NO:256 | |
| 1G8-1C10 | D | Y | S | MS | SEQ ID NO:235 | R | I | K | A | S | S | G | G | S | D | YADSVK G | SEQ ID NO:257 | |
| 1G8-1C12 | G | F | G | MS | SEQ ID NO:186 | G | I | Y | S | H | D | A | D | K | R | YADSVK G | SEQ ID NO:202 | |
| 1G8-1C8 | S | S | A | MS | SEQ ID NO:236 | A | I | S | G | S | S | D | S | P | R | YADSVK G | SEQ ID NO:258 | |
| 1G8-1D11 | G | Y | A | MS | SEQ ID NO:237 | R | I | D | G | T | G | D | S | K | F | YADSVK G | SEQ ID NO:259 | |
| 1G8-1D7 | N | Y | A | MS | SEQ ID NO:181 | W | I | D | T | T | S | A | Y | A | S | YADSVK G | SEQ ID NO:260 | |
| 1G8-1E10 | T | Y | A | MS | SEQ ID NO:238 | R | I | S | G | S | G | D | G | K | D | YADSVK G | SEQ ID NO:261 | |
| 1G8-1E12 | S | Y | A | MS | SEQ ID NO:8 | R | I | S | A | H | K | W | D | A | S | YADSVK G | SEQ ID NO:262 | |
| 1G8-1E6 | D | Y | S | MS | SEQ ID NO:235 | R | I | Y | T | D | G | W | E | K | D | YADSVK G | SEQ ID NO:263 | |
| 1G8-1E8 | G | Y | A | MS | SEQ ID NO:237 | R | I | S | S | Y | S | W | N | P | D | YADSVK G | SEQ ID NO:264 | |

(continued)

| No. | HCDR1 | | | | | HCDR2 | | | | | | | | | | | | HCDR3 |
|---|---|---|---|---|---|---|---|---|---|---|---|---|---|---|---|---|---|---|
| 1G8-1F11 | S | Y | A | MS | SEQ ID NO:8 | R | I | S | Q | T | G | G | T | T | D | YADSVK G | SEQ ID NO:265 | |
| 1G8-1F5 | T | Y | | MS | SEQ ID NO:239 | R | I | Y | T | D | G | | S | S | D | YADSVK G | SEQ ID NO:266 | |
| 1G8-1F7 | G | Y | A | MS | SEQ ID NO:237 | A | I | D | A | I | G | G | N | K | R | YADSVK G | SEQ ID NO:267 | |
| 1G8-1G12 | S | S | A | MS | SEQ ID NO:236 | R | I | Y | S | I | S | G | N | T | D | YADSVK G | SEQ ID NO:268 | |
| 1G8-1G3 | T | Y | A | MS | SEQ ID NO:238 | R | I | S | G | I | S | A | G | T | N | YADSVK G | SEQ ID NO:269 | |
| 1G8-1G5 | T | S | A | MS | SEQ ID NO:240 | R | I | Y | A | D | T | A | Y | N | D | YADSVK G | SEQ ID NO:270 | |
| 1G8-1G9 | D | F | A | MS | SEQ ID NO:241 | R | I | Y | S | G | D | D | N | A | S | YADSVK G | SEQ ID NO:271 | |
| 1G8-1H1 | G | Y | A | MS | SEQ ID NO:237 | R | I | Y | P | Y | S | S | V | R | D | YADSVK G | SEQ ID NO:272 | |
| 1G8-1H6 | G | F | A | MS | SEQ ID NO:242 | R | I | Y | P | T | S | S | E | S | D | YADSVK G | SEQ ID NO:273 | |
| 1G8-2A1 | G | F | A | MS | SEQ ID NO:242 | R | I | S | G | H | D | W | S | S | S | YADSVK G | SEQ ID NO:274 | |
| 1G8-2A11 | N | Y | S | MS | SEQ ID NO:185 | R | I | S | S | D | G | W | E | T | F | YADSVK G | SEQ ID NO:275 | |
| 1G8-2A4 | S | N | A | MS | SEQ ID NO:243 | R | I | Y | G | G | S | W | D | T | D | YADSVK G | SEQ ID NO:276 | |
| 1G8-2A7 | D | Y | A | MS | SEQ ID NO:187 | R | I | S | A | Y | G | W | G | K | S | YADSVK G | SEQ ID NO:277 | |
| 1G8-2B10 | N | N | A | MS | SEQ ID NO:244 | R | I | D | T | I | G | G | T | S | S | YADSVK G | SEQ ID NO:278 | |
| 1G8-2B12 | T | S | S | MS | SEQ ID NO:245 | R | I | D | T | W | G | W | Y | K | F | YADSVK G | SEQ ID NO:279 | |
| 1G8-2B3 | T | S | A | MS | SEQ ID NO:240 | R | I | Y | Q | D | S | S | T | T | D | YADSVK G | SEQ ID NO:280 | |
| 1G8-2B5 | N | Y | A | MS | SEQ ID NO:181 | R | I | K | S | H | D | S | T | S | H | YADSVK G | SEQ ID NO:281 | |
| 1G8-2B6 | G | Y | A | MS | SEQ ID NO:237 | R | I | Y | A | Y | K | S | E | N | D | YADSVK G | SEQ ID NO:282 | |
| 1G8-2C6 | S | Y | A | MS | SEQ ID NO:8 | R | I | Y | G | S | D | S | T | P | D | YADSVK G | SEQ ID NO:283 | |
| 1G8-2C7 | N | F | A | MS | SEQ ID NO:246 | R | I | S | Q | D | G | G | N | K | N | YADSVK G | SEQ ID NO:284 | |
| 1G8-2D11 | G | N | A | MS | SEQ ID NO:247 | R | I | D | G | W | G | S | Y | T | D | YADSVK G | SEQ ID NO:285 | |
| 1G8-2D4 | G | Y | A | MS | SEQ ID NO:237 | R | I | S | G | T | S | G | N | N | D | YADSVK G | SEQ ID NO:286 | |
| 1G8-2D6 | G | Y | A | MS | SEQ ID NO:237 | R | I | S | A | H | S | W | N | K | D | YADSVK G | SEQ ID NO:287 | |
| 1G8-2D7 | S | Y | A | MS | SEQ ID NO:8 | R | I | S | A | D | S | A | S | P | S | YADSVK G | SEQ ID NO:288 | |
| 1G8-2D9 | G | Y | S | MS | SEQ ID NO:194 | R | I | D | T | G | G | S | V | K | N | YADSVK G | SEQ ID NO:289 | |

(continued)

| No. | HCDR1 | | | | | HCDR2 | | | | | | | | | | | | HCDR3 |
|---|---|---|---|---|---|---|---|---|---|---|---|---|---|---|---|---|---|---|
| 1G8-2E7 | N | Y | A | MS | SEQ ID NO:181 | R | I | S | P | S | T | W | N | I | D | YADSVK G | SEQ ID NO:290 | |
| 1G8-2F11 | S | N | A | MS | SEQ ID NO:243 | R | I | S | P | D | S | S | G | I | S | YADSVK G | SEQ ID NO:291 | |
| 1G8-2F2 | S | Y | A | MS | SEQ ID NO:8 | R | I | S | S | H | G | A | S | R | D | YADSVK G | SEQ ID NO:292 | |
| 1G8-2F9 | N | Y | A | MS | SEQ ID NO:181 | R | I | S | A | W | G | S | T | N | N | YADSVK G | SEQ ID NO:255 | |
| 1G8-2G12 | N | N | A | MS | SEQ ID NO:244 | A | I | Y | A | S | T | D | G | R | S | YADSVK G | SEQ ID NO:293 | |
| 1G8-2G3 | G | N | A | MS | SEQ ID NO:247 | R | I | S | S | D | K | G | T | T | N | YADSVK G | SEQ ID NO:294 | |
| 1G8-2G4 | G | Y | S | MS | SEQ ID NO:194 | R | I | S | T | D | G | A | Y | T | N | YADSVK G | SEQ ID NO:295 | |
| 1G8-2G7 | T | Y | A | MS | SEQ ID NO:238 | R | I | Y | S | T | K | G | T | P | D | YADSVK G | SEQ ID NO:296 | |
| 1G8-2G8 | N | Y | A | MS | SEQ ID NO:181 | R | I | S | T | Y | G | D | T | T | H | YADSVK G | SEQ ID NO:297 | |
| 1G8-2G9 | N | Y | A | MS | SEQ ID NO:181 | R | I | S | Q | S | G | G | T | N | D | YADSVK G | SEQ ID NO:298 | |
| 1G8-2H1 | S | Y | A | MS | SEQ ID NO:8 | R | I | S | Q | G | G | G | V | T | H | YADSVK G | SEQ ID NO:299 | |
| 1G8-2H10 | N | S | A | MS | SEQ ID NO:248 | S | I | Y | Y | H | S | G | V | P | R | YADSVK G | SEQ ID NO:300 | |
| 1G8-2H11 | N | Y | A | MS | SEQ ID NO:181 | R | I | Y | G | W | D | W | Y | A | S | YADSVK G | SEQ ID NO:301 | |
| 1G8-2H3 | G | Y | A | MS | SEQ ID NO:237 | R | I | S | G | I | K | A | Y | N | D | YADSVK G | SEQ ID NO:302 | |
| 1G8-2H4 | G | Y | A | MS | SEQ ID NO:237 | A | I | S | G | S | G | G | S | T | Y | YADSVK G | SEQ ID NO:9 | |
| 1G8-2H8 | S | Y | A | MS | SEQ ID NO:8 | A | I | S | G | S | G | G | S | T | Y | YADSVK G | SEQ ID NO:9 | |
| 1G8-2H9 | N | Y | A | MS | SEQ ID NO:181 | A | I | Y | S | Y | F | D | E | P | R | YADSVK G | SEQ ID NO:303 | |

Table 8. CDR sequences of heavy chain variable region of 10F4-series antibodies

| No. | HCDR1 | | | | | HCDR2 | | | | | | | | | | | | HCDR3 |
|---|---|---|---|---|---|---|---|---|---|---|---|---|---|---|---|---|---|---|
| 10F4 | S | Y | A | MS | SEQ ID NO: 8 | A | I | S | G | S | G | G | S | T | Y | YADSVKG | SEQ ID NO:9 | EYSDVVDY VSVRTPLDY SEQ ID NO:16 |
| 10F4-1A1 | N | Y | D | MS | SEQ ID NO:304 | S | I | K | P | G | S | A | Y | K | F | YADSVKG | SEQ ID NO:318 | |
| 10F4-1A3 | N | Y | D | MS | SEQ ID NO:304 | G | I | K | G | W | K | S | Y | T | G | YADSVKG | SEQ ID NO:319 | |
| 10F4-1B2 | N | Y | W | MS | SEQ ID NO:305 | G | I | D | G | H | S | S | Y | A | F | YADSVKG | SEQ ID NO:320 | |
| 10F4-1B5 | D | Y | W | MS | SEQ ID NO:306 | S | I | I | G | D | D | S | T | N | H | YADSVKG | SEQ ID NO:321 | |
| 10F4-1B7 | G | F | D | MS | SEQ ID NO:307 | G | I | K | A | Y | K | S | T | A | D | YADSVKG | SEQ ID NO:322 | |
| 10F4-1B9 | N | Y | Y | MS | SEQ ID NO:308 | S | I | Y | Q | T | D | A | S | I | F | YADSVKG | SEQ ID NO:323 | |
| 10F4-1C3 | D | Y | W | MS | SEQ ID NO:306 | S | I | K | Q | T | G | G | V | K | H | YADSVKG | SEQ ID NO:324 | |
| 10F4-1C4 | D | Y | W | MS | SEQ ID NO:306 | S | I | I | G | D | D | S | T | N | H | YADSVKG | SEQ ID NO:321 | |
| 10F4-1C7 | N | Y | W | MS | SEQ ID NO:305 | A | I | D | T | H | S | D | N | S | F | YADSVKG | SEQ ID NO:325 | |
| 10F4-1C10 | s | Y | D | MS | SEQ ID NO:309 | G | I | K | T | Y | G | G | N | T | H | YADSVKG | SEQ ID NO326 | |
| 10F4-1D3 | D | Y | D | MS | SEQ ID NO:310 | S | I | I | Q | S | G | S | T | A | F | YADSVKG | SEQ ID NO:327 | |
| 10F4-1D11 | D | F | D | MS | SEQ ID NO:311 | G | I | K | Q | S | S | A | G | I | N | YADSVKG | SEQ ID NO:328 | |
| 10F4-1E1 | N | Y | W | MS | SEQ ID NO:305 | S | I | D | A | S | S | G | N | R | F | YADSVKG | SEQ ID NO:329 | |
| 10F4-1E10 | T | Y | A | MS | SEQ ID NO:238 | G | I | S | Q | W | G | A | Y | A | S | YADSVKG | SEQ ID NO:330 | |
| 10F4-1E12 | S | Y | D | MS | SEQ ID NO:309 | G | I | S | D | T | G | D | Y | A | N | YADSVKG | SEQ ID NO:331 | |
| 10F4-1F1 | N | Y | W | MS | SEQ ID NO:305 | A | I | S | P | G | D | G | T | I | F | YADSVKG | SEQ ID NO:332 | |
| 10F4-1F2 | N | Y | Y | MS | SEQ ID NO:308 | S | I | S | A | Y | S | D | N | R | N | YADSVKG | SEQ ID NO:333 | |
| 10F4-1F8 | D | Y | D | MS | SEQ ID NO:310 | S | I | I | T | D | G | G | G | A | F | YADSVKG | SEQ ID NO:334 | |
| 10F4-1F9 | G | F | D | MS | SEQ ID NO:307 | G | I | K | T | Y | G | G | Y | R | S | YADSVKG | SEQ ID NO:335 | |
| 10F4-1G5 | S | S | W | MS | SEQ ID NO:312 | S | I | D | A | Y | G | D | Y | N | F | YADSVKG | SEQ ID NO:336 | |
| 10F4-1G7 | D | Y | D | MS | SEQ ID NO:310 | S | I | I | G | H | G | D | T | K | F | YADSVKG | SEQ ID NO:337 | |
| 10F4-1G8 | D | F | D | MS | SEQ ID NO:311 | V | I | K | P | S | G | D | Y | I | N | YADSVKG | SEQ ID NO:338 | |
| 10F4-1H1 | N | F | D | MS | SEQ ID NO:313 | G | I | K | Q | Y | K | G | Y | T | S | YADSVKG | SEQ ID NO:339 | |

(continued)

| No. | HCDR1 | | | | | HCDR2 | | | | | | | | | | | | HCDR3 |
|---|---|---|---|---|---|---|---|---|---|---|---|---|---|---|---|---|---|---|
| 10F4-1H4 | N | Y | W | MS | SEQ ID NO:305 | A | I | S | G | T | G | G | Y | T | Y | YADSVKG | SEQ ID NO:340 | |
| 10F4-1H6 | D | Y | D | MS | SEQ ID NO:310 | G | I | K | T | H | S | G | G | N | G | YADSVKG | SEQ ID NO:341 | |
| 10F4-1H7 | T | Y | D | MS | SEQ ID NO:314 | G | I | K | Q | Y | K | G | Y | T | S | YADSVKG | SEQ ID NO:339 | |
| 10F4-1H11 | G | F | W | MS | SEQ ID NO:315 | S | I | S | P | D | S | G | T | K | H | YADSVKG | SEQ ID NO:342 | |
| 10F4-2A1 | D | Y | D | MS | SEQ ID NO:310 | G | I | K | A | H | S | D | N | K | R | YADSVKG | SEQ ID NO:343 | |
| 10F4-2A5 | D | Y | D | MS | SEQ ID NO:310 | G | I | K | T | Y | K | S | D | A | R | YADSVKG | SEQ ID NO:344 | |
| 10F4-2A9 | D | Y | D | MS | SEQ ID NO:310 | G | I | K | Q | Y | K | G | Y | T | S | YADSVKG | SEQ ID NO:339 | |
| 10F4-2B11 | N | Y | W | MS | SEQ ID NO:305 | V | I | D | S | T | G | S | N | R | F | YADSVKG | SEQ ID NO:345 | |
| 10F4-2C2 | N | Y | W | MS | SEQ ID NO:305 | V | I | D | A | H | S | A | N | I | R | YADSVKG | SEQ ID NO:346 | |
| 10F4-2C4 | D | Y | W | MS | SEQ ID NO:306 | V | I | D | S | G | S | G | N | K | R | YADSVKG | SEQ ID NO:347 | |
| 10F4-2C6 | G | F | D | MS | SEQ ID NO:307 | G | I | K | Q | Y | K | G | Y | T | S | YADSVKG | SEQ ID NO:339 | |
| 10F4-2C12 | D | Y | S | MS | SEQ ID NO:235 | V | I | D | T | T | S | A | Y | T | R | YADSVKG | SEQ ID NO:348 | |
| 10F4-2E1 | T | Y | D | MS | SEQ ID NO:314 | G | I | K | Y | S | K | Y | Y | T | H | YADSVKG | SEQ ID NO:349 | |
| 10F4-2E4 | S | Y | W | MS | SEQ ID NO:316 | A | I | D | G | T | D | S | V | A | Y | YADSVKG | SEQ ID NO:350 | |
| 10F4-2E6 | D | Y | W | MS | SEQ ID NO:306 | A | I | D | Q | G | K | A | T | S | F | YADSVKG | SEQ ID NO:351 | |
| 10F4-2E8 | N | Y | D | MS | SEQ ID NO:304 | S | I | I | P | H | G | D | Y | S | F | YADSVKG | SEQ ID NO:352 | |
| 10F4-2F11 | S | Y | D | MS | SEQ ID NO:309 | G | I | K | T | G | G | G | S | A | R | YADSVKG | SEQ ID NO:353 | |
| 10F4-2G2 | D | Y | D | MS | SEQ ID NO:310 | S | I | I | A | S | G | S | G | N | Y | YADSVKG | SEQ ID NO:354 | |
| 10F4-2H5 | N | Y | W | MS | SEQ ID NO:305 | S | I | D | Q | G | S | G | N | I | F | YADSVKG | SEQ ID NO:355 | |
| 10F4-1 | N | Y | W | MS | SEQ ID NO:305 | G | I | S | Q | G | G | S | Y | A | Y | YADSVKG | SEQ ID NO:356 | |
| 10F4-3 | D | F | A | MS | NO:241 | V | I | D | G | H | T | A | Y | N | S | YADSVKG | SEQ ID NO:357 | |
| 10F4-5 | G | Y | D | MS | SEQ ID NO:317 | V | I | K | D | H | G | G | Y | K | F | YADSVKG | SEQ ID NO:358 | |

Amino acid sequence of antibody heavy chain constant region (CH):

ASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLS
PGK（SEQ ID NO:37）

Amino acid sequence of antibody light chain constant region (CL):

TVAAPSVFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO:38)

**The amino acid sequence of the heavy chain of antibody 1F3 is as follows:**

EVQLVESGGGLVQPGGSLRLSCAASGFTFSSYAMSWVRQAPGKGLEWVSAISGSGGSTYYADSV
KGRFTISRDNSKNTLYLQMNSLRAEDTAVYYCARAELLAFDVYTASDYWGQGTLVTVSSASTKG
PSVFPLAPSSKSTSGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQKSLSLSPGK
（SEQ ID NO:232）

The amino acid sequence of the heavy chain of antibody 1G8 is as follows:

EVQLVESGGGLVQPGGSLRLSCAASGFTFSSYAMSWVRQAPGKGLEWVSAISGSGGSTYYADSV
KGRFTISRDNSKNTLYLQMNSLRAEDTAVYYCARQTEDRSYGYDLDYWGQGTLVTVSSASTKGP
SVFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSLSPGK
（SEQ ID NO:233）

The amino acid sequence of the heavy chain of antibody 10F4 is as follows:

EVQLVESGGGLVQPGGSLRLSCAASGFTFSSYAMSWVRQAPGKGLEWVSAISGSGGSTYYADSV
KGRFTISRDNSKNTLYLQMNSLRAEDTAVYYCAREYSDVVDYVSVRTPLDYWGQGTLVTVSSAS
TKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK
（SEQ ID NO:249）

The amino acid sequence of the heavy chain of antibody 10F4-3 is as follows:

EVQLVESGGGLVQPGGSLRLSCAASGFSFGDFAMSWVRQAPGKGLEWVSVIDGHTAYNSYADSVKGRFTISRDNSKNTLYLQMNSLRAEDTAVYYCAREYSDVVDYVSVRTPLDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

（SEQ ID NO:360）

The nucleic acid sequence of the heavy chain of antibody 10F4-3 is as follows:

gaagtgcaattggtggaaagcggcggcggcctggtgcagccgggcggcagcctgcgcctgagctgcgcggcgtccggattctcttttggtgacttcgctatgagctgggtgcgccaggcaccgggcaaaggcctcgaatgggtgagcgttattgacggtcatactgcttacaactcttatgcggatagcgtgaaaggccgctttaccattagccgcgataattcgaaaaacaccctgtatctgcaaatgaacagcctgcgtgcggaagatactgcagtgtattattgcgcgcgtgaatactctgacgttgttgactacgtttctgttcgtactccgctggattattggggccaaggcaccctggtgacggtgagcagcgcctctaccaagggcccctctgtgtttcctctggctccctccagcaagtctacctctggtggaacagctgccctgggctgcctggtcaaggattactttcctgagcctgtcaccgtgtcctggaactctggcgctctgacatctggcgtgcacacctttccagctgtgctccagtcctccggcctgtactctctgtcctctgtcgtgaccgtgccttctagctctctgggcacccagacctacatctgcaatgtgaaccacaagccttccaacaccaaggtggacaagaaggtggaacccaagtcctgcgacaagacccacacctgtcctccatgtcctgctccagaactgctcggcggaccttccgtgttcctgtttcctccaaagcctaaggacaccctgatgatctctcggacccctgaagtgacctgcgtggtggtggatgtgtctcacgaagatcccgaagtgaagttcaactggtacgtggacggcgtggaagtgcacaacgccaagaccaagcctagagaggaacagtacaactccacctacagagtggtgtccgtgctgaccgtgctgcaccaggattggctgaacggcaaagagtacaagtgcaaggtgtccaacaaggccctgcctgctcctatcgaaaagaccatctccaaggccaagggccagcctagggaaccccaggtttacaccttgcctccatctcgggacgagctgaccaagaaccaggtgtccctgacctgtctcgtgaagggcttctacccctccgacatcgccgtggaatgggagtctaatggccagcctgagaacaactacaagacaacccctcctgtgctggactccgacggctcattcttcctgtactccaagctgacagtggacaagtccagatggcagcagggcaacgtgttctcctgctccgtgatgcacgaggccctgcacaatcactacacccagaagtccctgtctctgtcccctggcaaataa (SEQ ID NO:364)

**The amino acid sequence of the heavy chain of antibody 1F3-1E4 is as follows:**

EVQLVESGGGLVQPGGSLRLSCAASGFSFGTYGMSWVRQAPGKGLEWVSRISGDSANIRYADSVKGRFTISRDNSKNTLYLQMNSLRAEDTAVYYCARAELLAFDVYTASDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

（SEQ ID NO:361）

**The nucleic acid sequence of the heavy chain of antibody 1F3-1E4 is as follows:**

gaagtgcaattggtggaaagcggcggcggcctggtgcagccgggcggcagcctgcgcctgagctgcgcggcgtccggattctcttttggtacttacggtatgagctgggtgcgccaggcaccgggcaaaggcctcgaatgggtgagccgtatttctggtgactctgctaacatccgttatgcggatagcgtgaaaggccgctttaccattagccgcgataattcgaaaaacaccctgtatctgcaaatgaacagcctgcgtgcggaagatactgcagtgtattattgcgcgcgtgctgaactgctggctttcgacgtttacactgcttctgattattggggccaaggcaccctggtgacggtgagcagcgcctctaccaagggcccctctgtgtttcctctggctccctccagcaagtctacctctggtggaacagctgccctgggctgcctggtcaaggattactttcctgagcctgtcaccgtgtcctggaactctggcgctctgacatctggcgtgcacacctttccagctgtgctccagtcctccggcctgtactctctgtcctctgtcgtgaccgtgccttctagctctctgggcacccagacctacatctgcaatgtgaaccacaagccttccaacaccaaggtggacaagaaggtggaacccaagtcctgcgacaagacccacacctgtcctccatgtcctgctccagaactgctcggcggaccttccgtgttcctgtttcctccaaagcctaaggacaccctgatgatctctcggacccctgaagtgacctgcgtggtggtggatgtgtctcacgaagatcccgaagtgaagttcaactggtacgtggacggcgtggaagtgcacaacgccaagaccaagcctagagaggaacagtacaactccacctacagagtggtgtccgtgctgaccgtgctgcaccaggattggctgaacggcaaagagtacaagtgcaaggtgtccaacaaggccctgcctgctcctatcgaaaagaccatctccaaggccaagggccagcctagggaaccccaggtttacaccttgcctccatctcgggacgagctgaccaagaaccaggtgtccctgacctgtctcgtgaagggcttctacccctccgacatcgccgtggaatgggagtctaatggccagcctgagaacaactacaagacaacccctcctgtgctggactccgacggctcattcttcctgtactccaagctgacagtggacaagtccagatggcagcagggcaacgtgttctcctgctccgtgatgcacgaggccctgcacaatcactacacccagaagtccctgtctctgtcccctggcaaataa (SEQ ID NO:365)

**The amino acid sequence of the heavy chain of antibody 1F3-2B9 is as follows:**

EVQLVESGGGLVQPGGSLRLSCAASGFNFDSYAMSWVRQAPGKGLEWVSGIKPHTDDIYYADSV
KGRFTISRDNSKNTLYLQMNSLRAEDTAVYYCARAELLAFDVYTASDYWGQGTLVTVSSASTKG
PSVFPLAPSSKSTSGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDIAVEWESN
GQPENNYKTTPPVLDSDGSFFLYSKLTVDKSRWQQGNVFSCSVMHEALHNHYTQKSLSLSPGK
（SEQ ID NO:362）

**The nucleic acid sequence of the heavy chain of antibody 1F3-2B9 is as follows:**

gaagtgcaattggtggaaagcggcggcggcctggtgcagccgggcggcagcctgcgcctgagctgcgcggcgtccggattcaactttgactcttacgc
tatgagctgggtgcgccaggcaccgggcaaaggcctcgaatgggtgagcggtattaaaccgcatactgacgacatctactatgcggatagcgtgaaag
gccgctttaccattagccgcgataattcgaaaaacaccctgtatctgcaaatgaacagcctgcgtgcggaagatactgcagtgtattattgcgcgcgtgctg

aactgctggctttcgacgtttacactgcttctgattattggggccaaggcaccctggtgacggtgagcagcgcctctaccaagggcccctctgtgtttcctct
ggctccctccagcaagtctacctctggtggaacagctgccctgggctgcctggtcaaggattactttcctgagcctgtcaccgtgtcctggaactctggcgc
tctgacatctggcgtgcacacctttccagctgtgctccagtcctccggcctgtactctctgtcctctgtcgtgaccgtgccttctagctctctgggcacccaga
cctacatctgcaatgtgaaccacaagccttccaacaccaaggtggacaagaaggtggaacccaagtcctgcgacaagacccacacctgtcctccatgtc
ctgctccagaactgctcggcggaccttccgtgttcctgtttcctccaaagcctaaggacaccctgatgatctctcggacccctgaagtgacctgcgtggtgg
tggatgtgtctcacgaagatcccgaagtgaagttcaactggtacgtggacggcgtggaagtgcacaacgccaagaccaagcctagagaggaacagtac
aactccacctacagagtggtgtccgtgctgaccgtgctgcaccaggattggctgaacggcaaagagtacaagtgcaaggtgtccaacaaggccctgcct
gctcctatcgaaaagaccatctccaaggccaagggccagcctagggaaccccaggtttacaccttgcctccatctcgggacgagctgaccaagaaccag
gtgtccctgacctgtctcgtgaagggcttctacccctccgacatcgccgtggaatgggagtctaatggccagcctgagaacaactacaagacaacccctc
ctgtgctggactccgacggctcattcttcctgtactccaagctgacagtggacaagtccagatggcagcagggcaacgtgttctcctgctccgtgatgcac
gaggccctgcacaatcactacacccagaagtccctgtctctgtcccctggcaaataa (SEQ ID NO:366)

**The nucleic acid sequence of the heavy chain of antibody 1G8-1C10 is as follows:**

EVQLVESGGGLVQPGGSLRLSCAASGFNFSDYSMSWVRQAPGKGLEWVSRIKASSGGSDYADSV
KGRFTISRDNSKNTLYLQMNSLRAEDTAVYYCARQTEDRSYGYDLDYWGQGTLVTVSSASTKGP
SVFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSLSPGK
（SEQ ID NO:363）

**The nucleic acid sequence of the heavy chain of antibody 1G8-1C10 is as follows:**

gaagtgcaattggtggaaagcggcggcggcctggtgcagccgggcggcagcctgcgcctgagctgcgcggcgtccggattcaacttttctgactactct atgagctgggtgcgccaggcaccgggcaaaggcctcgaatgggtgagccgtattaaagcttcttctggtggttctgactatgcggatagcgtgaaaggcc gctttaccattagccgcgataattcgaaaaacaccctgtatctgcaaatgaacagcctgcgtgcggaagatactgcagtgtattattgcgcgcgtcagactg aagaccgttcttacggttacgacctggattattggggccaaggcaccctggtgacggtgagcagcgcctctaccaagggcccctctgtgtttcctctggctc cctccagcaagtctacctctggtggaacagctgccctgggctgcctggtcaaggattactttcctgagcctgtcaccgtgtcctggaactctggcgctctga catctggcgtgcacacctttccagctgtgctccagtcctccggcctgtactctctgtcctctgtcgtgaccgtgccttctagctctctgggcacccagacctac atctgcaatgtgaaccacaagccttccaacaccaaggtggacaagaaggtggaacccaagtcctgcgacaagacccacacctgtcctccatgtcctgctc cagaactgctcggcggaccttccgtgttcctgtttcctccaaagcctaaggacaccctgatgatctctcggacccctgaagtgacctgcgtggtggtggatg tgtctcacgaagatcccgaagtgaagttcaactggtacgtggacggcgtggaagtgcacaacgccaagaccaagcctagagaggaacagtacaactcc acctacagagtggtgtccgtgctgaccgtgctgcaccaggattggctgaacggcaaagagtacaagtgcaaggtgtccaacaaggccctgcctgctcct atcgaaaagaccatctccaaggccaagggccagcctagggaaccccaggtttacaccttgcctccatctcgggacgagctgaccaagaaccaggtgtc cctgacctgtctcgtgaagggcttctaccccctccgacatcgccgtggaatgggagtctaatggccagcctgagaacaactacaagacaacccctcctgtg ctggactccgacggctcattcttcctgtactccaagctgacagtggacaagtccagatggcagcagggcaacgtgttctcctgctccgtgatgcacgaggc cctgcacaatcactacacccagaagtccctgtctctgtcccctggcaaataa (SEQ ID NO:367)

**The amino acid sequence of the common light chain is as follows:**

DIQMTQSPSSLSASVGDRVTITCRASQGISSYLAWYQQKPGKAPKLLIYAASSLQSGVPSRFSGSGS GTDFTLTISSLQPEDFATYYCQQHYTTPPTFGQGTKVEIKRTVAAPSVFIFPPSDEQLKSGTASVVCL LNNFYPREAKVQWKVDNALQSGNSQESVTEQDSKDSTYSLSSTLTLSKADYEKHKVYACEVTHQ GLSSPVTKSFNRGEC ( SEQ ID NO:250 )

**The nucleic acid sequence of the common light chain is as follows:**

gatatccagatgacccagtcccccagctccctgagcgctagcgtgggcgaccgggtgaccatcacctgcagggcctcccagggcatcagctcctacct ggcttggtatcagcagaagcctggcaaggcccctaagctgctgatctatgccgcttcctccctgcagtccggcgtgccttccaggttcagcggctccggc agcggcaccgacttcaccctgaccatctcctccctgcagcctgaggacttcgccacctattattgccagcagcactataccacccctcctacctttggccag ggcaccaaggtggagatcaagcgtacggtggctgcaccatctgtcttcatcttcccgccatctgatgagcagttgaaatctggaactgcctctgttgtgtgc ctgctgaataacttctatcccagagaggccaaagtacagtggaaggtggataacgccctccaatcgggtaactcccaggagagtgtcacagagcaggac agcaaggacagcacctacagcctcagcagcaccctgacgctgagcaaagcagactacgagaaacacaaagtctacgcctgcgaagtcacccatcagg gcctgagctcgcccgtcacaaagagcttcaacaggggagagtgttga (SEQ ID NO:359)

**Example 4. Binding activity assay**

The binding activity of the antibodies was detected by ELISA. The procedures are briefly described as follows: the hNectin-4-His antigen protein of Example 1 was immobilized at a concentration of 1 μg/mL on a plate; the plate was blocked with skimmed milk powder overnight, and then, serially diluted antibodies of interest were added for co-incubation (antibodies in Table 9 were serially 2-fold diluted from 2 μg/mL; antibodies in Tables 10 and 11 were serially 3-fold diluted from 10 μg/mL) at room temperature for 2 h; then an HRP-labeled anti-human-kappa (Sigma, A7164) secondary antibody was added for co-incubation for 1 h, followed by the addition of TMB (Huzhou InnoReagents, TMB-S-001) for chromogenic reaction, and the reaction was stopped with 0.1 M H2SO4; after the absorbance at OD450 was read on a microplate reader, the binding EC50 value for each antibody was calculated by four-parameter fitting, and the binding activities of the antibodies were compared. The results are shown in Tables 9-11.

Table 9. Binding activity of anti-Nectin-4 antibodies

| Antibody No. | $EC_{50}$ ( μg/mL ) | Antibody No. | $EC_{50}$ ( μg/mL ) |
|---|---|---|---|
| ASG-22 | 0.042 | 1G8 | 0.068 |
| 3A10 | 0.068 | 1G7 | 0.022 |
| 10F4 | 0.038 | 1B4 | 0.025 |
| 10A4 | 0.041 | 1F3 | 0.041 |
| 1G12 | 0.073 | | |

Table 10. Binding activity of antibodies

| Antibody No. | $EC_{50}$ ( μg/mL ) | Antibody No. | $EC_{50}$ ( μg/mL ) |
|---|---|---|---|
| 1G8-1E10 | 0.006 | 1F3-1C4 | 0.011 |
| 1G8-1C10 | 0.005 | 1F3-1D7 | 0.015 |
| 1G8-2B3 | 0.005 | 1F3-1E1 | 0.017 |
| 1G8-1G3 | 0.004 | 1F3-1F4 | 0.009 |
| 1G8-2C6 | 0.005 | 1F3-1F8 | 0.008 |
| 1G8-2G8 | 0.006 | 1F3-1G7 | 0.008 |
| 1G8-2F2 | 0.012 | 1F3-1G11 | 0.007 |
| 1G8-2F11 | 0.004 | 1F3-1D12 | 0.007 |
| 1G8-2H8 | 0.005 | 1F3-1H3 | 0.008 |
| 1F3-1A9 | 0.007 | 1F3-2B9 | 0.009 |
| 1G8-2H4 | 0.039 | 1F3-2D2 | 0.015 |
| ASG-22 | 0.019 | 1F3-2E8 | 0.009 |
| 1F3 | 0.005 | 1F3-2F3 | 0.011 |
| 1F3-1B1 | 0.016 | 1F3-2B1 | 0.014 |

Table 11. Binding activity of antibodies

| Antibody No. | $EC_{50}$(μg/mL) | Antibody No. | $EC_{50}$(μg/mL) |
|---|---|---|---|
| ASG-22 | 0.042 | ASG-22 | 0.046 |
| 10F4-1A1 | 0.051 | 10F4-1G7 | 0.068 |
| 10F4-1C3 | 0.053 | 10F4-1H6 | 0.063 |
| 10F4-1C10 | 0.058 | 10F4-1H11 | 0.059 |
| 10F4-1F1 | 0.057 | 10F4-2C6 | 0.09 |
| 10F4-1E1 | 0.067 | 10F4-2C12 | 0.06 |
| 10F4-1F8 | 0.054 | 10F4-2F11 | 0.078 |
| 10F4 | 0.041 | 10F4 | 0.043 |

**Example 5: Affinity assay**

In this study, the affinity of the antibodies for the human Nectin-4 extracellular antigen was detected by surface plasmon resonance (SPR) technique (BIAcore: GE Healthcare, T200).

Capture was performed using a Protein A chip (Cat#29127556, GE Healthcare), and 5 μg/mL antibody dilutions (in HBS-EP+(10×): GE Healthcare, Cat# BR-1006-69, with a working concentration of 1×) were directed through the experimental flow channels (Fc2 and Fc4) at a flow rate of 10 μL/min for 10 s; then, the flow rate was adjusted to 30 μL/min, the hNectin-4-His dilutions at different concentrations (0 nM, 1.23 nM, 3.7 nM, 11.1 nM, 33.3 nM, and 100 nM) were sequentially added, and simultaneously directed through the surfaces of the experimental flow channels (Fc2 and Fc4) and reference flow channels (Fc1 and Fc3) with an association time of 120 s and a dissociation time of 180 s, and finally, the chip was regenerated in Glycine 1.5. Fitting analysis was performed using BiaEvaluation 3.2 with a 1:1 fitting model, and the fitting results are shown in Tables 12-13 and FIG. 1.

Table 12. Affinity of anti-Nectin-4 antibodies to hNectin-4-His

| Antibody No. | KD(M) | Ka ( 1/Ms ) | Kd ( 1/s ) | $Chi^2(RU^2)$ |
|---|---|---|---|---|
| 1G8 | 4.97E-09 | 6.05E+05 | 3.00E-03 | 0.626 |
| ASG-22 | 6.03E-09 | 7.23E+05 | 4.36E-03 | 4.9 |

(continued)

| Antibody No. | KD(M) | Ka ( 1/Ms ) | Kd ( 1/s ) | $Chi^2(RU^2)$ |
|---|---|---|---|---|
| 1G12 | 8.61E-09 | 5.39E+05 | 4.64E-03 | 0.891 |
| 1B4 | 5.33E-09 | 6.00E+05 | 3.20E-03 | 0.873 |
| 1G7 | 1.67E-08 | 3.77E+05 | 6.31E-03 | 0.124 |
| 10F4 | 2.77E-09 | 3.73E+05 | 1.03E-03 | 3.52 |
| 10A4 | 6.71E-09 | 3.46E+05 | 2.32E-03 | 1.15 |
| 3A10 | 2.1E-09 | 4.13E+05 | 8.67E-04 | 3.24 |
| 1F3 | 3.21E-09 | 8.13E+05 | 2.61E-03 | 8.81 |

Table 13. Affinity of antibodies

| Sample | ka (1/Ms) | kd (1/s) | KD (M) | Rmax (RU) | $Chi^2$ ($RU^2$) |
|---|---|---|---|---|---|
| 1F3 | 7.85E+05 | 1.95E-03 | 2.49E-09 | 127.6 | 0.795 |
| 1F3-1B1 | 1.20E+06 | 2.04E-04 | 1.7E-10 | 142.5 | 0.974 |
| 1F3-1A9 | 1.55E+06 | 9.94E-04 | 6.41E-10 | 120.8 | 0.949 |
| 1F3-1E4 | 1.94E+06 | 8.25E-04 | 4.26E-10 | 132 | 1.16 |
| 1F3-1E1 | 1.51E+06 | 2.12E-04 | 1.41E-10 | 131.5 | 0.172 |
| 1F3-1G7 | 8.02E+05 | 5.96E-04 | 7.43E-10 | 135.8 | 1.96 |
| 1F3-1F8 | 1.71E+06 | 9.66E-04 | 5.64E-10 | 124.2 | 0.701 |
| 1F3-1H3 | 1.69E+06 | 1.05E-03 | 6.2E-10 | 130.8 | 0.972 |
| 1F3-1G11 | 1.41E+06 | 1.15E-03 | 8.15E-10 | 132.6 | 0.89 |
| 1F3-2B9 | 1.82E+06 | 1.01E-03 | 5.52E-10 | 124.4 | 0.91 |
| 1F3-2B1 | 1.32E+06 | 3.17E-04 | 2.41E-10 | 130.2 | 0.399 |
| 1F3-2F3 | 1.40E+06 | 5.19E-04 | 3.7E-10 | 125.4 | 0.694 |
| 1F3-2E8 | 1.17E+06 | 1.02E-03 | 8.72E-10 | 144.8 | 0.864 |
| 1G8 | 6.05E+05 | 3.00E-03 | 4.97E-09 | 48.4 | 0.626 |
| 1G8-1G3 | 4.71E+05 | 1.41E-03 | 3.00E-09 | 135.4 | 1.47 |
| 1G8-1E10 | 6.65E+05 | 8.70E-04 | 1.31E-09 | 109.6 | 1.71 |
| 1G8-2C6 | 7.34E+05 | 1.37E-03 | 1.87E-09 | 122.1 | 1.27 |
| 1G8-2B3 | 7.42E+05 | 1.47E-03 | 1.98E-09 | 102.6 | 0.817 |
| 1G8-2G8 | 7.58E+05 | 1.42E-03 | 1.87E-09 | 100.8 | 0.974 |
| 1G8-2F2 | 7.84E+05 | 1.66E-03 | 2.12E-09 | 53.8 | 0.516 |
| ASG-22 | 5.77E+05 | 5.81E-03 | 1.01E-08 | 84.9 | 0.69 |
| 1G8-1G3 | 4.71E+05 | 1.41E-03 | 3E-09 | 135.4 | 1.47 |
| 10F4 | 3.75E+05 | 1.21E-03 | 3.23E-09 | 29.4 | 0.232 |
| 10F4-1 | 4.15E+05 | 2.26E-04 | 5.45E-10 | 94.8 | 1.16 |
| 10F4-5 | 3.43E+05 | 1.37E-04 | 3.99E-10 | 135.8 | 1.79 |
| 10F4-3 | 3.46E+05 | 1.22E-04 | 3.52E-10 | 110.3 | 1.26 |
| ASG-22 | 5.40E+05 | 5.90E-03 | 1.09E-08 | 78.3 | 0.714 |
| 10F4-1C3 | 3.70E+05 | 2.18E-04 | 5.89E-10 | 123.6 | 1.3 |
| 10F4-1A1 | 4.30E+05 | 1.86E-04 | 4.32E-10 | 106.1 | 1.47 |

(continued)

| Sample | ka (1/Ms) | kd (1/s) | KD (M) | Rmax (RU) | $Chi^2$ ($RU^2$) |
|---|---|---|---|---|---|
| 10F4-1C10 | 2.65E+05 | 1.16E-04 | 4.35E-10 | 108.3 | 0.238 |
| 10F4-1F8 | 4.24E+05 | 2.37E-04 | 5.58E-10 | 95.8 | 0.764 |
| 10F4-1H6 | 2.99E+05 | 1.51E-04 | 5.05E-10 | 118.7 | 0.348 |
| 10F4-1G7 | 3.89E+05 | 1.45E-04 | 3.74E-10 | 91 | 0.711 |
| 10F4-2C6 | 2.94E+05 | 8.36E-05 | 2.85E-10 | 108.6 | 0.927 |
| 10F4-1H11 | 3.50E+05 | 2.12E-04 | 6.05E-10 | 119 | 1.14 |
| 10F4-2F11 | 4.06E+05 | 1.61E-04 | 3.96E-10 | 81 | 1.15 |
| 10F4-2C12 | 5.70E+05 | 3.04E-04 | 5.33E-10 | 89.4 | 2.15 |

**Example 6. Endocytosis efficiency assay**

In this assay, the relative amount of antibodies remaining on the cell surface after co-incubation at 37 °C for a certain period of time was detected by FACS to reflect the endocytosis efficiency. The procedures are briefly described as follows: T-47D cells (human ductal breast cancer cells, derived from the Cell Bank of Chinese Academy of Sciences, Cat.No. TCHu 87); the antibodies of interest were adjusted to 10 μg/mL and co-incubated with the cells on ice for 1 h; the cells were washed 3 times with pre-cooled PBS, and then placed at 37 °C and incubated for 0 h, 2 h and 4 h; 0.2% $N_3Na$ was added to stop the endocytosis reaction; the cells were washed with PBS thrice, followed by the addition of the PE-labeled anti-human Fc secondary antibody (Invitrogen 12-4998-82); the cells were incubated at 4 °C for 30 min; the red fluorescence signal was detected on a flow cytometer. The endocytosis results of the antibodies of interest are shown in Tables 14-15.

Table 14. Endocytosis results of anti-Nectin-4 antibodies

| Antibody No. | MFI (mean fluorescence intensity) | | | Endocytosis rate at 2h | Endocytosis rate at 4 h |
|---|---|---|---|---|---|
| | 0h | 2h | 4h | | |
| ASG-22 | 107097.7 | 53739.06 | 27694.23 | 49.8% | 74.1% |
| 1B4 | 107856.3 | 52035.69 | 26450.5 | 51.8% | 75.5% |
| 1F3 | 121007.3 | 60512.22 | 28271.26 | 50.0% | 76.6% |
| 1G8 | 104517.6 | 56508.13 | 27760.32 | 45.9% | 73.4% |
| 3A10 | 105072.4 | 47336.81 | 20035.45 | 54.9% | 80.9% |
| 10A4 | 107039.3 | 53157.58 | 21358.23 | 50.3% | 80.0% |
| 10F4 | 104284.8 | 56913.51 | 24043.91 | 45.4% | 76.9% |

Table 15. Endocytosis results of anti-Nectin-4 antibodies

| Antibody No. | MFI (mean fluorescence intensity) | | | Endocytosis rate at 2 h | Endocytosis rate at 4 h |
|---|---|---|---|---|---|
| | 0h | 2h | 4h | | |
| ASG-22 | 58,591.2 | 37,973.0 | 37,844.0 | 35.2% | 35.4% |
| 10F4-1 | 58,616.6 | 31,049.2 | 29,267.8 | 47.0% | 50.1% |
| 10F4-3 | 61,231.7 | 34,751.4 | 27,044.8 | 43.2% | 55.8% |
| 10F4-5 | 63,449.1 | 37,060.1 | 20,132.5 | 41.6% | 68.3% |
| 1F3-1B1 | 65,333.1 | 36,442.5 | 33,275.2 | 44.2% | 49.1% |
| 1F3-1E1 | 73,387.8 | 45,103.4 | 31,632.0 | 38.5% | 56.9% |
| 1F3-1G11 | 60,470.8 | 48,303.1 | 42,145.4 | 20.1% | 30.3% |
| 1F3-2B1 | 59,430.8 | 38,232.6 | 30,476.3 | 35.7% | 48.7% |

(continued)

| Antibody No. | MFI (mean fluorescence intensity) | | | Endocytosis rate at 2 h | Endocytosis rate at 4 h |
|---|---|---|---|---|---|
| | 0h | 2h | 4h | | |
| 1F3-2E8 | 58,160.4 | 36,480.4 | 32,490.0 | 37.3% | 44.1% |
| 1F3-2F3 | 68,970.9 | 45,880.7 | 31,065.2 | 33.5% | 55.0% |

**Example 7. Cell binding curves**

In this study, the binding activity of different antibodies to T-47D cells positive for Nectin-4 expression was detected by FACS, and the $EC_{50}$ for the binding of antibodies to the cells was analyzed by a four-parameter method. The procedures are briefly described as follows: T-47D cells (the Cell Bank of Chinese Academy of Sciences, TCHu 87) were trypsinized; the antibodies were serially diluted and co-incubated with T-47D cells at 4 °C for 0.5-1 h, followed by the addition of the secondary PE-labeled anti-human Fc antibody (Invitrogen, 12-4998-82); the signal intensity of red fluorescence was detected on a flow cytometer, and curves were plotted using Graphpad Prism and the $EC_{50}$ was calculated. The binding curves and the $EC_{50}$ results are shown in Table 16 and FIG. 2.

Table 16. $EC_{50}$ value for binding of anti-Nectin-4 antibody to cells

| $EC_{50}$ | ASG-22 | 1F3 | 1G8 | 3A10 | 10F4 |
|---|---|---|---|---|---|
| μg/mL | 0.118 | 0.116 | 0.109 | 0.186 | 0.156 |
| nM | 0.788 | 0.770 | 0.727 | 1.241 | 1.039 |

**Example 8. Specificity assay**

In this assay, the binding of different antibodies to human Nectin-4 homologous proteins hNectin-1 (ACRO, PV1-H5223), hNectin-2 (ACRO, PV2-H52E2), hNectin-3 (ACRO, PV3-H52E4), and hNectin-4-His was detected by ELISA to determine the specificity of the antibodies. For the methodology and procedures, see Example 4. Antigens of interest (1 μg/mL) were immobilized; after blocking, serially diluted antibodies (2-fold serially diluted from 2 μg/mL) were added, and HRP-labeled anti-human-Kappa secondary antibody was added for chromogenic reaction. The results are shown in Table 17. Antibody 3A10 exhibited weaker binding to hNectin-3, while the remaining antibodies exhibited good specificity, which did not bind to the homologous proteins hNectin-1/2/3 but bound to hNectin-4-His. The binding curves are shown in FIG. 3.

Table 17. Binding of anti-Nectin-4 antibodies to human Nectin-4 homologous proteins

| | $EC_{50}$ ( μg/mL) | | | |
|---|---|---|---|---|
| | 1F3 | 1G8 | 3A10 | 10F4 |
| hNectin-1 | NA | NA | NA | NA |
| hNectin-2 | NA | NA | NA | NA |
| hNectin-3 | NA | NA | 0.626 | NA |
| hNectin-4-His | 0.040 | 0.041 | 0.062 | 0.045 |
| Note: NA indicates not available in this assay. | | | | |

**Example 9. Species specificity assay**

Nectin-4 has high homology in different species.

The binding of different antibodies to different species of Nectin-4 proteins was detected by ELISA. hNectin-4-His, monkey cNectin-4 protein (ACRO, NE4-C52H4) and mouse mNectin-4 protein (ACRO, NE4-M52H3) were immobilized; antibodies ASG-22, 1F3, 1G8, 3A10, and 10F4 were serially diluted, and HRP-labeled anti-human-Kappa secondary antibody was added for chromogenic reaction. The results are shown in Table 18 and FIG. 4. The antibodies of interest exhibited similar binding activity to human Nectin-4, cynomolgus monkey Nectin-4, and mouse Nectin-4, while the reference antibody exhibited weak binding to mouse Nectin-4.

Table 18. Binding of anti-Nectin-4 antibody to Nectin-4 of different species

| Sample | $EC_{50}$ ( μg/mL) | | | | |
|---|---|---|---|---|---|
| | ASG-22 | 1F3 | 1G8 | 3A10 | 10F4 |
| hNectin-4-His | 0.035 | 0.036 | 0.043 | 0.037 | 0.041 |
| cNectin-4 | 0.033 | 0.034 | 0.036 | 0.042 | 0.041 |
| mNectin-4 | 1.229 | 0.034 | 0.037 | 0.037 | 0.041 |

**Example 10. Synthesis Procedures for Compound (1S,9S)-1-amino-9-ethyl-4,5-difluoro-9-hydroxy-1,2,3,9,12,15-hexahydro-10*H*,13*H*-benzo[de]pyrano[3',4':6,7]indolizino[1,2-b]quinolin-10,13-dione hydrochloride (D-1)**

1. Synthesis of*N,N'*-(3,4-difluoro-8-oxo-5,6,7,8-tetrahydronaphthalene-1,7-diyl)diacetamide

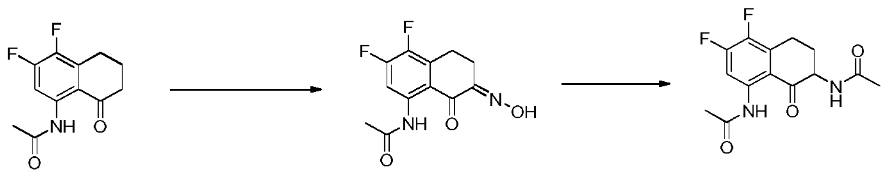

A solution of potassium tert-butoxide in tetrahydrofuran (42 mL, 1 M) was added to a dry reaction flask under a nitrogen atmosphere, stirred, and cooled to 0-5 °C. N-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by tert-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of N-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of $NaCO_3$ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed in vacuo to obtain a gray powder (2.1 g, 33.7%). LC-MS: $[M+H]^+$=297.

2. Synthesis of N-(8-amino-5,6-difluoro-1-oxo-1,2,3,4-tetrahydronaphthalen-2-yl)acetamide

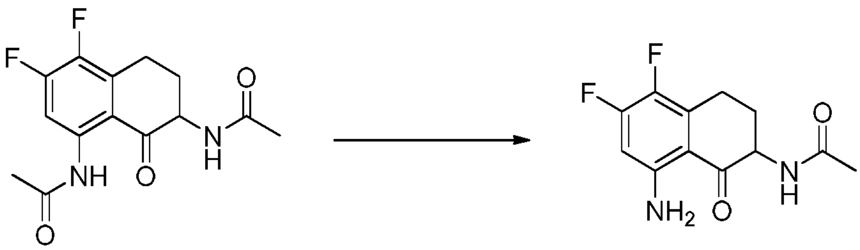

*N,N'*-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$=255.

3. Synthesis of *N*-((9,*S*)-9-ethyl-4,5-difluoro-9-hydroxy-10,13-dioxo-2,3,9,10,13,15-hexahydro-1*H*,12*H*-benzo[de]pyrano[3',4':6,7]indolizino[1,2-b]quinolin-1-yl)acetamide

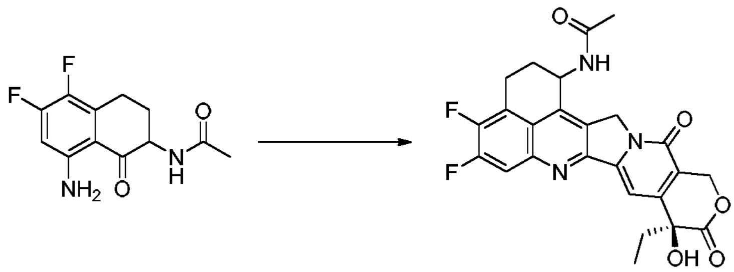

*N*-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$=482.

4. Synthesis of (1*S*,9*S*)-1-amino-9-ethyl-4,5-difluoro-9-hydroxy-1,2,3,9,12,15-hexahydro-10*H*,13*H*-benzo[*de*]pyrano[3',4':6,7]indolizino[1,2-*b*]quinolin-10,13-dione hydrochloride

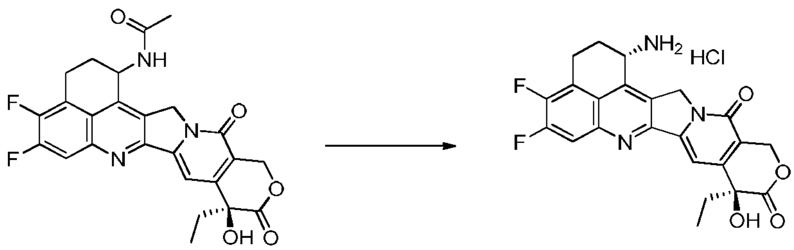

N-((9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)acetamide (1.1 g, 2.28 mmol) and a 6 M aqueous solution of hydrochloric acid (44 mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: $[M+H]^+$=440.

**Example 11. Synthesis Procedures for 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07)**

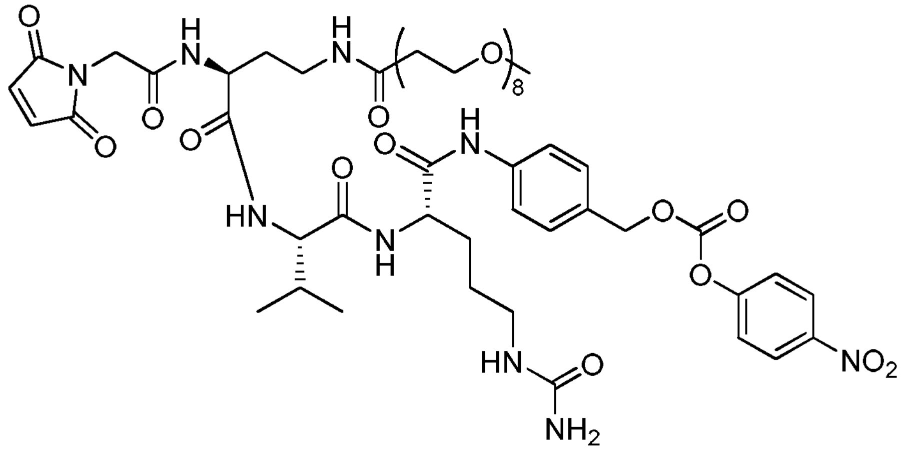

1) Synthesis of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01).

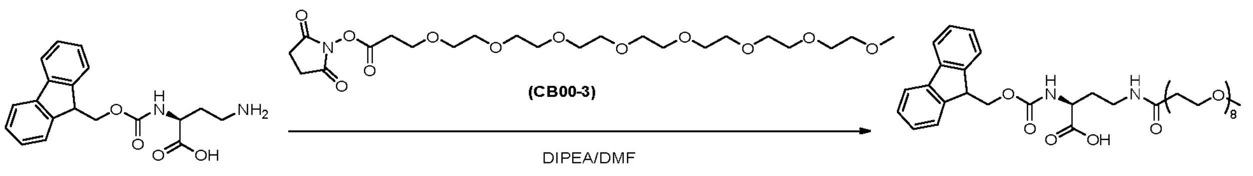


(CB00-2)

(CB01)

Under a nitrogen atmosphere at 0-5 °C, 8.14 g of N2-fluorenylmethoxycarbonyl-L-2,4-diaminobutyric acid (CB00-2) was dissolved in 40 mL of dimethylformamide (DMF), and 10 g of *N*-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

2) Synthesis of (9H-fluoren-9-yl)methyl [(S)-1-[[(S)-1-[[4-(hydroxymethyl)phenyl]amino]-1-oxo-5-ureidopentan-2-yl]amino]-3-methyl-1-oxobutan-2-yl]carbamate (CB02).

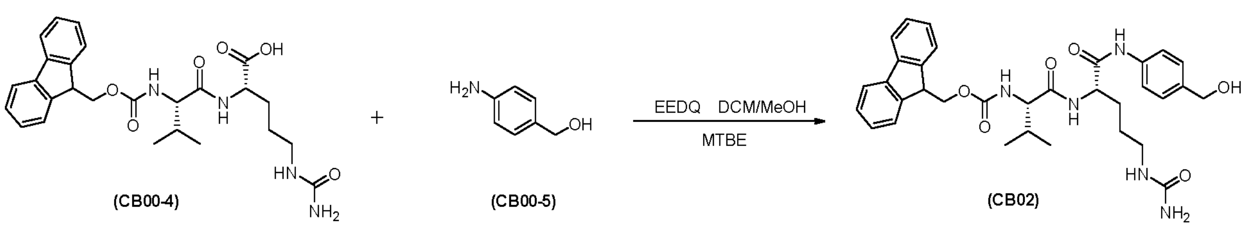


(CB00-4) (CB00-5) (CB02)

11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

3) Synthesis of (*S*)-2-((*S*)-2-amino-3-methylbutanamido)-*N*-(4-(hydroxymethyl)phenyl)-5-ureidopentanamide (CB03)

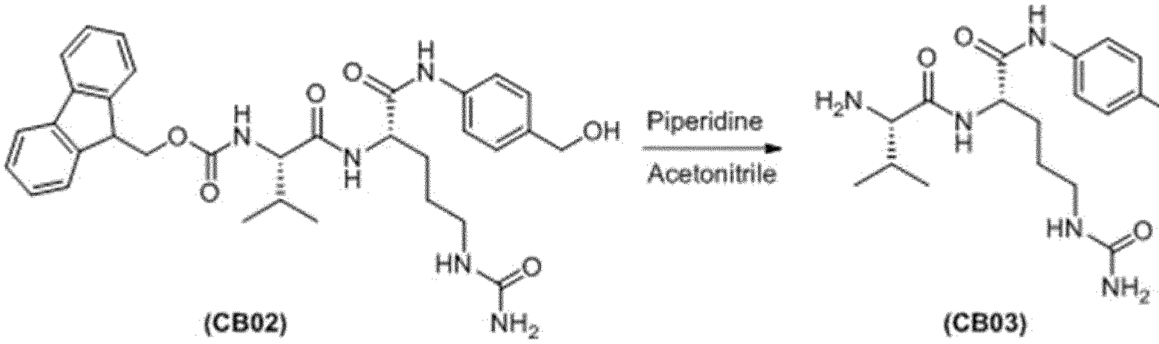


(CB02) (CB03)

Under a nitrogen atmosphere at room temperature, 300 mL of acetonitrile was added to 11.9 g of (9H-fluoren-9-yl)methyl [(*S*)-1-[[(*S*)-1-[[4-(hydroxymethyl)phenyl]amino]-1-oxo-5-ureidopentan-2-yl]amino]-3-methyl-1-oxobutan-2-yl] carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

4) Synthesis of (9*H*-fluoren-9-yl)methyl ((30*S*,33*S*,36*S*)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04).

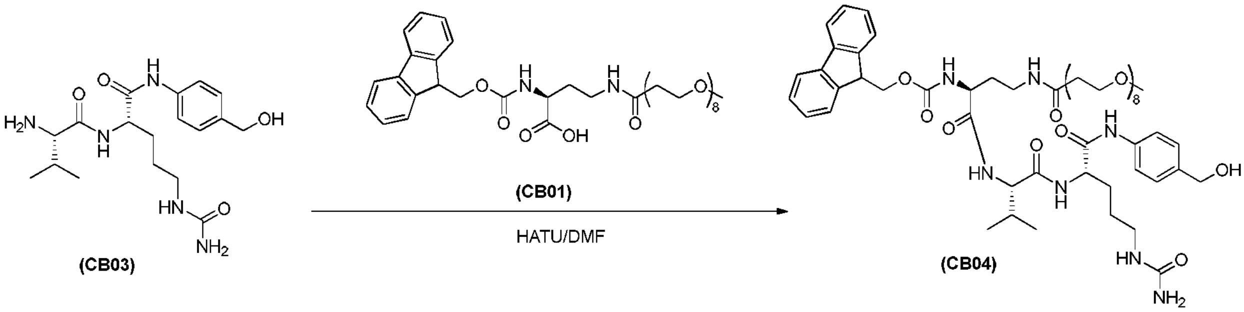


(CB03) (CB04)

At 0 °C under a nitrogen atmosphere, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N,N,N',N'*-tetramethyl-*O*-(7-azabenzotriazol-1-yl)urea hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide (CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10:1 as elution solvents) to obtain CB04 in the form of a solid (9.66 g).

5) Synthesis of *N*-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05).

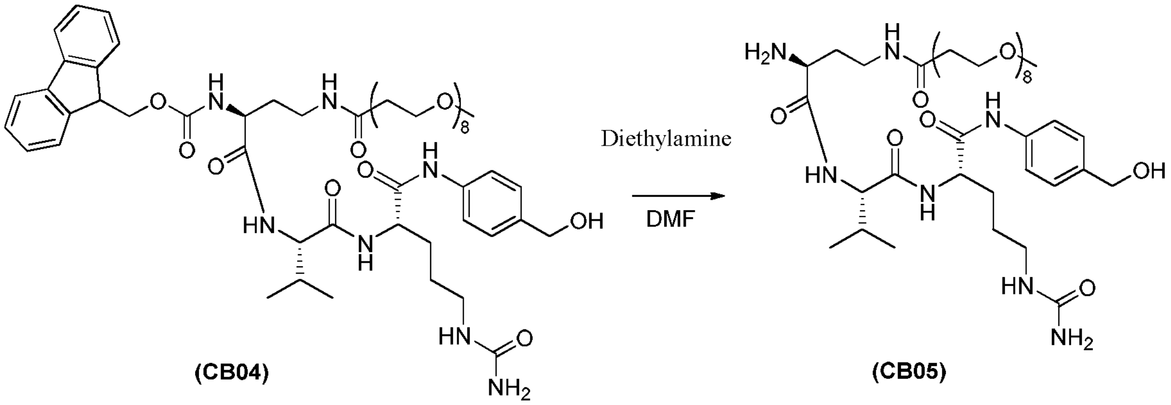


(CB04) (CB05)

9.66 g of (9H-fluoren-9-yl)methyl ((30S,33S,36S)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

6) Synthesis of *N*-((*S*)-3-(2-(2,5-dioxo-2,5-dihydro-1*H*-pyrrol-1-yl)acetamido)-4-(((*S*)-1-(((S)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

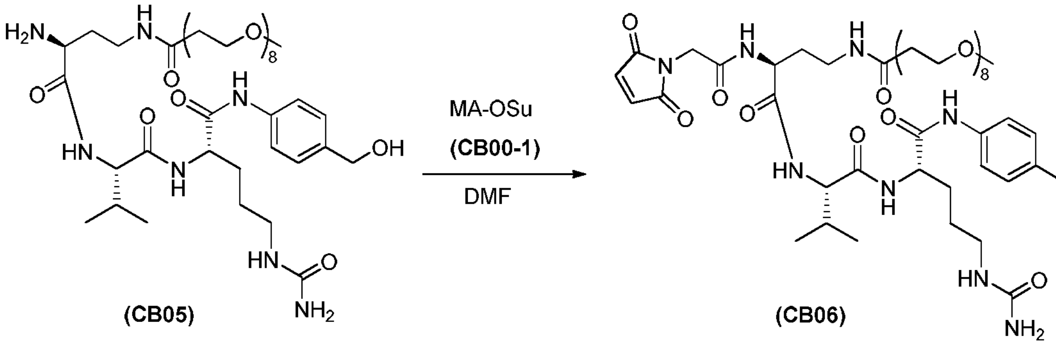


(CB05) (CB06)

7.7 g of N-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5°C. The mixture was allowed to react at 0-5°C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

7) Synthesis of 4-((30S,33S,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07).

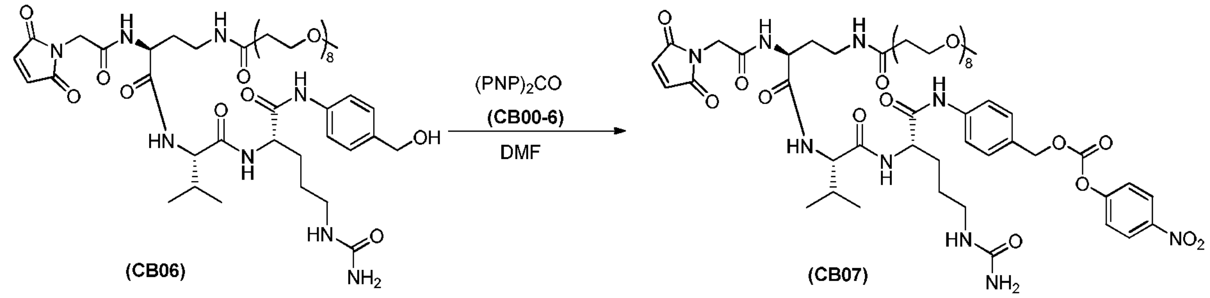


(CB06) (CB07)

9.5 g of *N*-((*S*)-3-(2-(2,5-dioxo-2,5-dihydro-1*H*-pyrrol-1-yl)acetamido)-4-(((*S*)-1-(((S)-1-((4-(hydroxymethyl) phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(p-nitrophenyl)carbonate ((PNP)$_2$CO) was added under a nitrogen atmosphere at 0 °C, followed by 8.2 mL of *N*,*N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 8:1 as elution solvents) to obtain CB07 in the form of a white solid (2.6 g).

**Example 12. Synthesis of 4-((18*S*,21S,24S)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (CB14).**

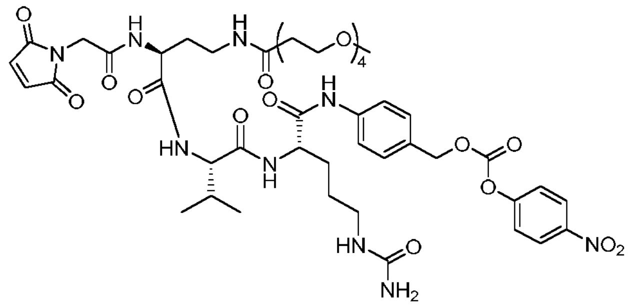

(CB14)

CB14 was prepared by referring to the synthesis of CB07 in Example 11, with *N*-succinimidyl 4,7,10,13,16,19,22,25-octaoxahexacosanoate replaced by N-succinimidyl 4,7,10,13-tetraoxatetradecanoate. CB14 was eventually obtained in the form of a white solid.

**Example 13. Synthesis of Intermediate (CB07-Exatecan)**

Synthesis of 4-((30S,33S,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino [1,2-*b*]quinolin-1-yl) carbamate.

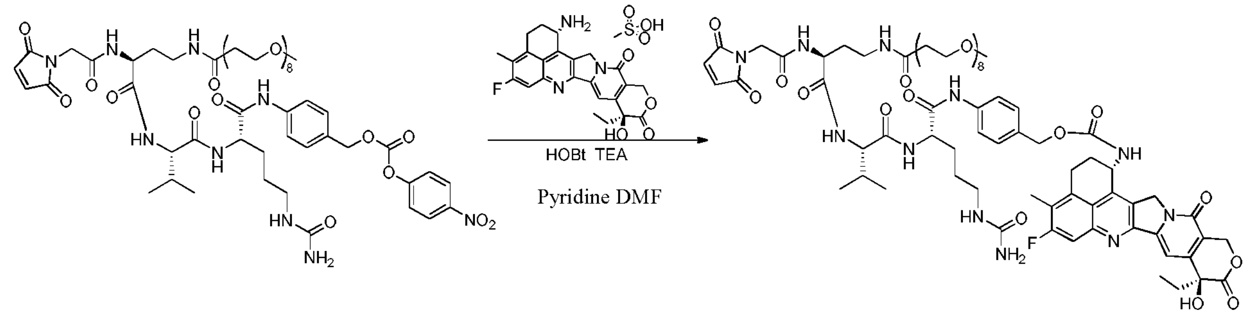


4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (2.6 g, 2.21 mmol) and N,N-dimethylformamide (23 mL) were added to a reaction flask R1, stirred under a nitrogen atmosphere and cooled to 0-5 °C. At the same time, (1S,9S)-1-amino-9-ethyl-5-fluoro-9-hydroxy-4-methyl-1,2,3,9,12,15-hexahydro-10H,13H-benzo[de]pyrano[3',4':6,7]indolizino[1,2-b]quinolin-10,13-dione methanesulfonate (Exatecan methane sulfonate, 0.98 g, 1.84 mmol, Advanced ChemBlocks) and N,N-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with N,N-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5°C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: $[1/2M+H]^+$=737.

**Example 14. Synthesis of Intermediate (CB07-D-1)**

Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-4,5-difluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)carbamat.

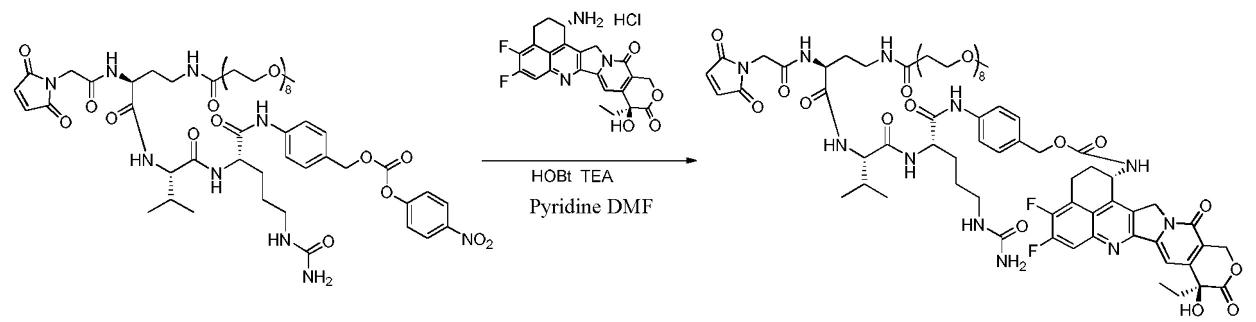


4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (220 mg, 0.189 mmol) and *N*,*N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H*,13*H*-benzo[de]pyrano[3',4':6,7]indolizino[1,2-*b*]quinolin-10,13-dione hydrochloride (D-1) (90 mg, 0.189 mmol) and *N*,*N*-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5°C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: $[1/2M+H]^+$=739.

**Example 15. Synthesis of Intermediate (CB14-Exatecan)**

Synthesis of 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[de]pyrano[3',4':6,7]indolizino[1,2-b]quinolin-1-yl) carbamate.

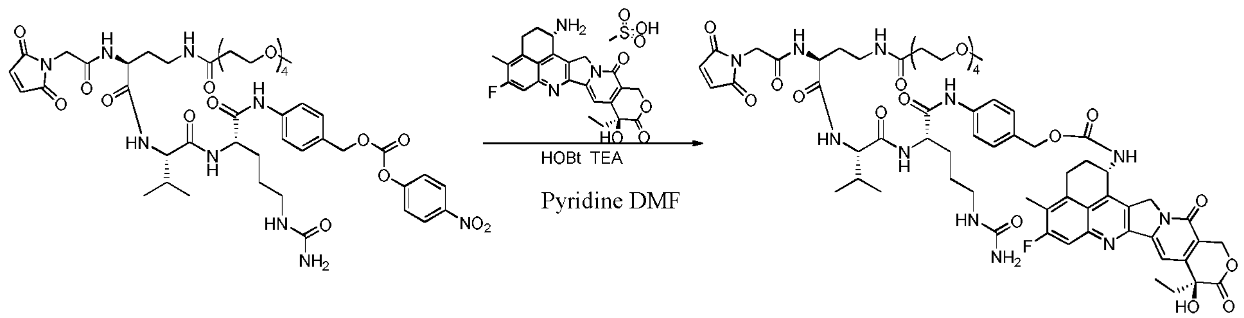


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (190 mg, 0.19 mmol)and N,Ndimethylformamide (23 mL) were added to a reaction flask R1, stirred under a nitrogen atmosphere, and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[de]pyrano[3',4':6,7]indolizino[1,2-*b*]quinolin-10,13-dione methanesulfonate (Exatecan methane sulfonate; 101 mg, 0.19 mmol; Advanced ChemBlocks) and *N*,*N*-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

**Example 16. Preparation of ASG-22-MMAE**

The structural formula of ASG-22-MMAE is as follows:

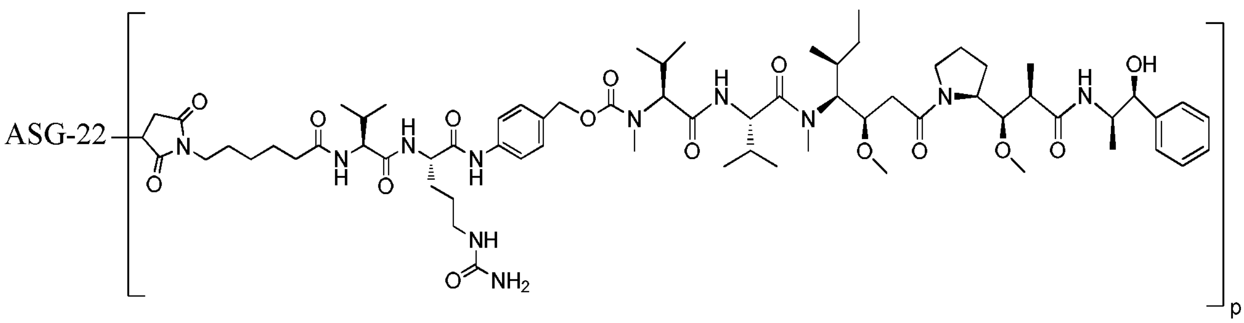

Antibody ASG-22 was adjusted to a concentration of 10 mg/mL using 10 mM succinic acid, and 2.5 molar equivalents of TCEP (tris(2-carboxyethyl)phosphine) was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and

eluted with 10 mM succinic acid in a Sephadex G-25 resin medium.

The DAR value was determined using reversed-phase chromatography, and the DAR value of ASG-22-MMAE was 3.14.

Determination of DAR value:

Procedures

Sample treatment: The test sample was diluted with water to about 1 mg/mL before 0.5 M DTT (dithiothreitol) was added. The sample was reduced in a 37 °C water bath for 30 min and centrifuged at 13,000 g for 5 min, and the supernatant was taken for liquid chromatography analysis.

Instrument conditions: The mobile phases were 0.1% DFA (difluoroacetic acid) in ACN (acetonitrile) and 0.05% DFA in $H_2O$, with a flow rate of 0.5 mL/min. The liquid chromatography separation was performed on a Waters BioResolve column (2.1 × 100 mm, 2.7 μm), and the detection wavelength was set at 214 nm.

Result calculation: After reduction, the sample can be separated by reversed-phase chromatography to give chromatographic peaks of light and heavy chains conjugated to different amounts of drug. The percentage content of each peak was calculated by area normalization, and the drug-to-antibody ratio (DAR) was calculated according to the percentage content of the peaks.

$$\mathrm{DAR} = 2 \times \left( \frac{\mathrm{L1}}{\mathrm{L0} + \mathrm{L1}} + \frac{\mathrm{H1} + 2 \times \mathrm{H2} + 3 \times \mathrm{H3}}{\mathrm{H0} + \mathrm{H1} + \mathrm{H2} + \mathrm{H3}} \right)$$

L0 denotes the percentage content of light chains not conjugated to any small molecule drugs, L1 denotes the percentage content of light chains conjugated to 1 small molecule drug, H0 denotes the percentage content of heavy chains not conjugated to any small molecule drugs, H1 denotes the percentage content of heavy chains conjugated to 1 small molecule drug, H2 denotes the percentage content of heavy chains conjugated to 2 small molecule drugs, and H3 denotes the percentage content of heavy chains conjugated to 3 small molecule drugs.

**Example 17. Preparation of 10F4-3-MMAE**

The structural formula of 10F4-3-MMAE is as follow:

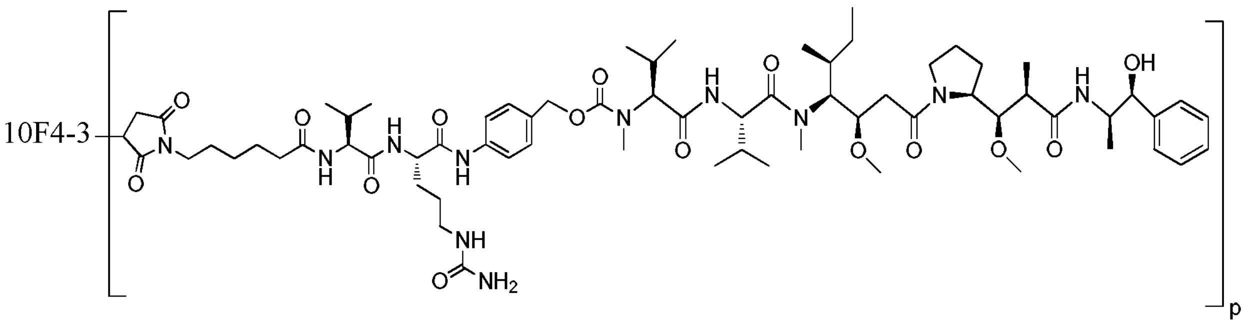

Antibody 10F4-3 was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 10F4-3-MMAE, as determined by reversed-phase chromatography, was 3.22.

**Example 18. Preparation of 10F4-5-MMAE**

The structural formula of 10F4-5-MMAE is as follows:

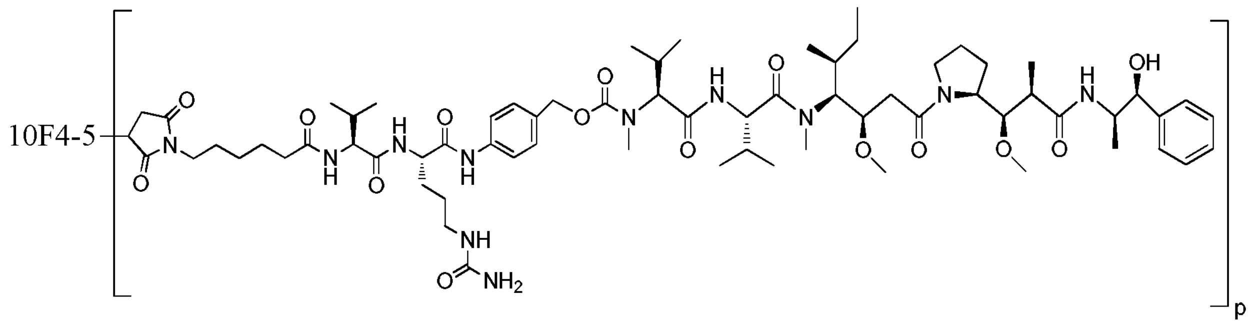

Antibody 10F4-5 was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 10F4-5-MMAE, as determined by reversed-phase chromatography, was 2.97.

**Example 19. Preparation of 1F3-1E1-MMAE**

The structural formula of 1F3-1E1-MMAE is as follows

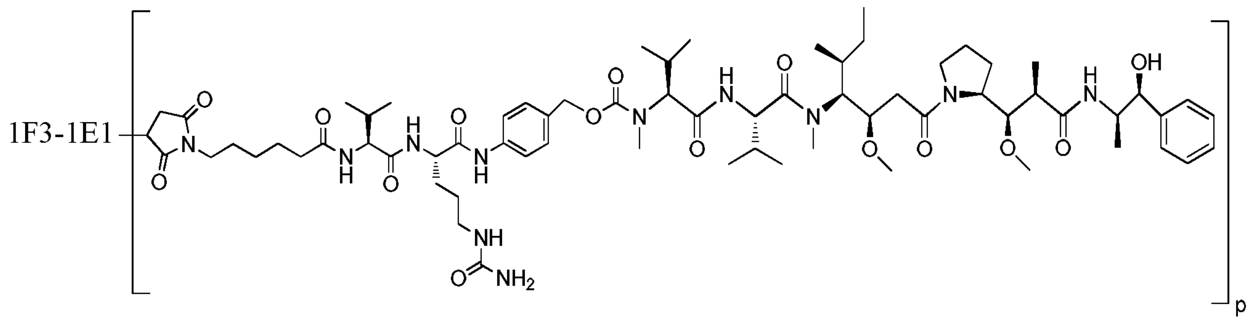

Antibody 1F3-1E1 was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 1F3-1E1-MMAE, as determined by reversed-phase chromatography, was 3.83.

**Example 20. Preparation of 1F3-2B9-MMAE**

The structural formula of 1F3-2B9-MMAE is as follows

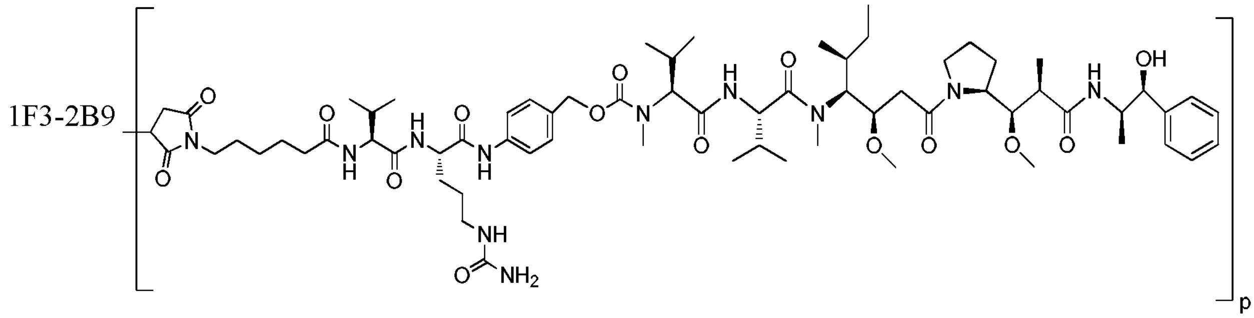

Antibody 1F3-2B9 was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 1F3-2B9-MMAE, as determined by reversed-phase chromatography, was 3.52.

**Example 21. Preparation of 10F4-3-ExaD8**

The structural formula of 10F4-3-ExaD8 was as follows

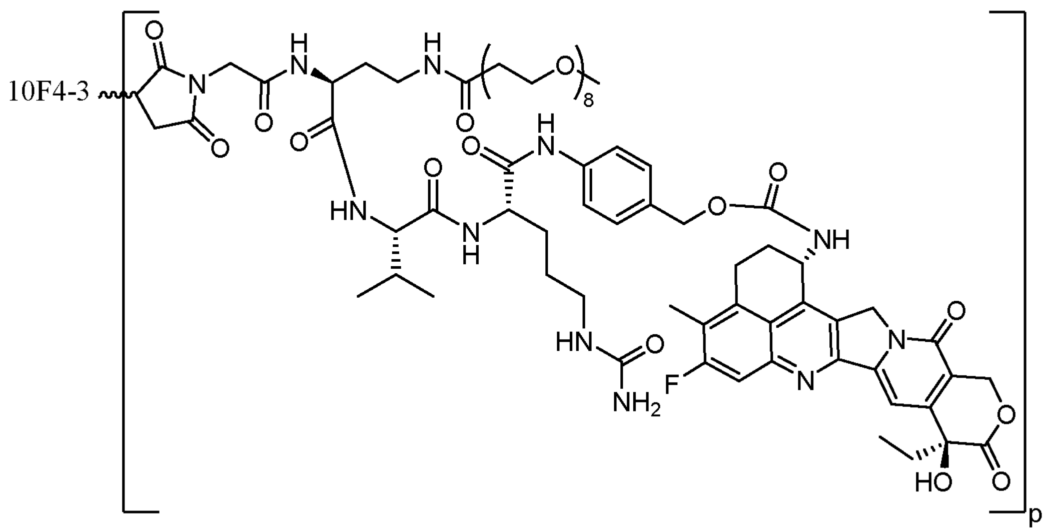

Antibody 10F4-3 was adjusted to a concentration of 18 mg/mL using 10 mM succinic acid, and 4.5 molar equivalents of TCEP (tris(2-carboxyethyl)phosphine) was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 12 molar equivalents of CB07-Exatecan was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 10F4-3-ExaD8, as determined by reversed-phase chromatography, was 7.87.

**Example 22. Preparation of 10F4-3-ExaD4**

The structural formula of 10F4-3-ExaD4 was as follows

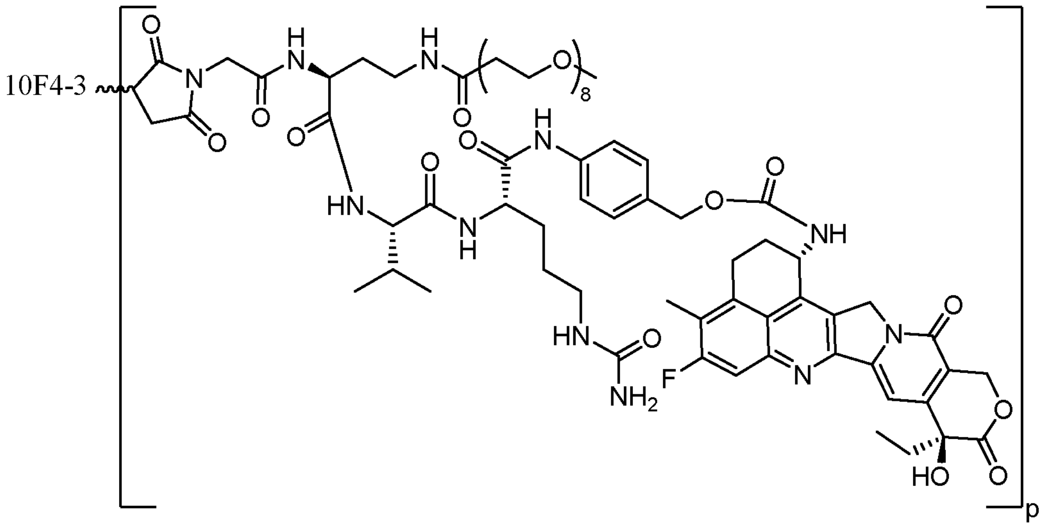

Antibody 10F4-3 was adjusted to a concentration of 18 mg/mL using 10 mM succinic acid, and 2.5 molar equivalents of TCEP (tris(2-carboxyethyl)phosphine) was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of CB07-Exatecan was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value, as determined by reversed-phase chromatography, was 7.63.

**Example 23. Preparation of 10F4-3-ExaD6**

The structural formula of 10F4-3-ExaD6 was as follows

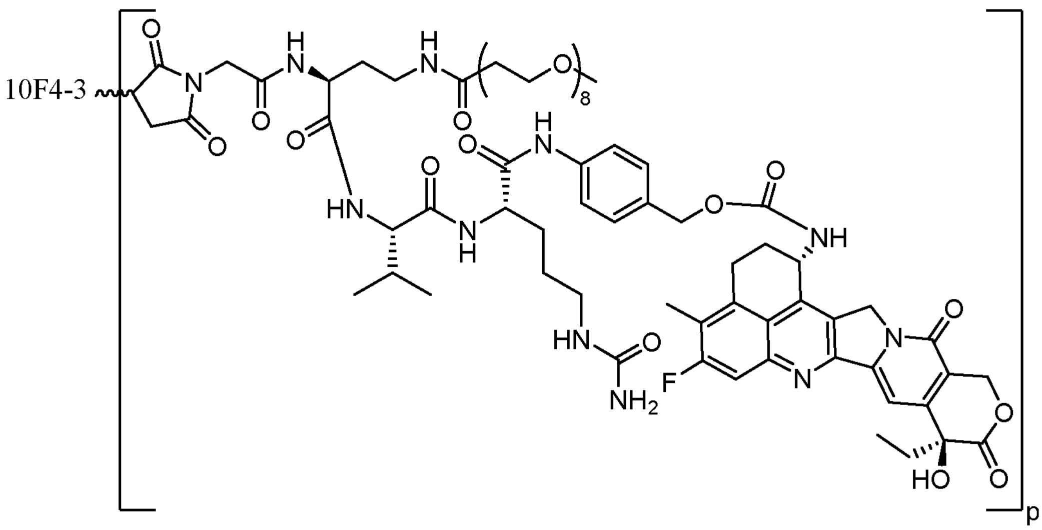

Antibody 10F4-3 was adjusted to a concentration of 18 mg/mL using 10 mM succinic acid, and 3.2 molar equivalents of TCEP (tris(2-carboxyethyl)phosphine) was added. Then the system was adjusted to pH 8 with 1 M Tris

base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 7 molar equivalents of CB07-Exatecan was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of 10F4-3-ExaD6, as determined by reversed-phase chromatography, was 6.1.

**Example 24. *In vitro* efficacy of ADCs**

1) OVCAR-3 cells and MDA-MB-468 cells were used in this experiment. The cells were digested and separately plated on a 96-well plate at a density of 6000 cells/well. After incubation for 4 h in an incubator, the test drugs were added. The test drugs were serially 4-fold diluted from 12.5 μg/mL to a total of 9 concentrations. After a 3-day incubation in the incubator, a relative cell proliferation analysis was conducted using the cell counting kit-8 (CCK-8, Dojindo, Japan) reagents. The results of the experiment (see FIGs. 5A-C) show that ASG-22-MMAE, 10F4-3-MMAE, 10F4-5-MMAE, 1F3-1E1-MMAE, and 1F3-2B9-MMAE exhibited similar *in vitro* efficacies.

2) In this study, the *in vitro* efficacies of 10F4-3-ExaD4 and 10F4-3-ExaD8 on various cells were compared with reference to procedure 1), and the incubation period after treatment was adjusted to 7 days. The $EC_{50}$ results of the ADCs on different cells are shown in Table 19.

Table 19. EC50 (nM) of ADCs on different cells

| Cell | 10F4-3-ExaD4 | 10F4-3-ExaD8 |
|---|---|---|
| MDA-MB-468 | 45.16 | 26.67 |
| OVCAR3 | 28.25 | 8.14 |
| Jeg3 | 57.01 | 21.56 |

**Example 25. *In vivo* pharmacokinetic study in rats**

3 healthy adult Sprague Dawley rats, aged 6-8 weeks, were selected. Each of the mice was given an intravenous bolus injection of 200 μg of 10F4-3-ExaD6 or 10F4-3-ExaD8 at the tail vein. Blood was collected at 3 h, 24 h, 48 h, 72 h, 120 h, 168 h, 240 h, and 336 h post-dose, and the serum was centrifugally isolated within 30-120 min. The concentrations of total antibody (Tab) and ADC in the blood samples were measured by conventional ELISA.

The procedures for total antibody measurement are briefly described as follows. hNectin-4-His extracellular antigen was immobilized at 4 °C overnight at a concentration of 1 μg/mL, and the plate was blocked with 5% skim milk powder at 37 °C for 2 h. The reference standard and quality control samples were added for incubation for 2 h. Then a 1:8000 diluted goat anti-human κ light chain peroxidase secondary antibody (Sigma, Cat. No.: A7164-1ML) was added for incubation for 1 h. After 8 washings with PBST, TMB (InnoReagents, TMB-S-001) was added for chromogenic reaction, and the reaction was terminated with 0.1 M sulfuric acid. The plate was read on an OD450 microplate reader, and the blood sample concentration was calculated at different time points using the analysis software of the microplate reader, SoftMax Pro.

The procedures for ADC measurement are briefly described as follows. hNectin-4-His extracellular antigen was immobilized at 4 °C overnight at a concentration of 1 μg/mL, and the plate was blocked with 5% skim milk powder at 37 °C for 2 h. The reference standard and quality control samples were added for incubation for 2 h. Then a 1:5000 diluted anti-small molecule secondary antibody (Dxd murine mAb 9A8, 9A8-20201221) was added for incubation for 1 h. A 1:5000 diluted HRP-labeled donkey anti-mouse IgG secondary antibody (Sangon, D110085) was added for incubation for 1 h. After 8 washings with PBST, 100 μL of single-component TMB substrate solution (InnoReagents, TMB-S-001) was added for chromogenic reaction, and the reaction was terminated with 0.1 M sulfuric acid. The plate was read on an OD450 microplate reader, and the blood sample concentration was calculated at different time points using the analysis software of the microplate reader, SoftMax Pro. A blood drug concentration curve was plotted by ELISA to illustrate the changes, as

shown in FIG. 6. The ADCs and total antibodies of 10F4-3-ExaD6 and 10F4-3-ExaD8 exhibited substantially consistent concentrations, suggesting their stability in the blood.

**Example 26. Bystander effect**

In this study, the bystander effect of 10F4-3-ExaD6 was assessed by culture supernatant transfer. The procedures are briefly described as follows. CHO-K1, a Nectin-4 negative cell line, and CHO-Nectin-4 (CHO-N4), a cell line overexpressing the full-length human Nectin-4, were used. The test substance 10F4-3-ExaD6 was serially diluted and added to the culture dishes of CHO-K1 and CHO-Nectin-4. After 2-4 days of culture, the culture supernatants from CHO-K1 and CHO-N4 were transferred to CHO-K1 cells that had been plated in advance. Following an additional 3 days of culture, a relative cell proliferation analysis was conducted using the cell counting kit-8 (CCK-8, Dojindo, Japan) reagents. The proliferation inhibitory effects of 10F4-3-ExaD6 on CHO-N4 and CHO-K1 were assessed after 5 days of *in vitro* culture. 10F4-3-ExaD6 exhibited a significant killing effect on CHO-N4 but no effect on CHO-K1. After the CHO-N4 culture supernatant transfer, a relatively significant killing effect on CHO-K1 was observed, whereas after the CHO-K1 culture supernatant transfer, no killing effect on CHO-K1 was observed, suggesting that 10F4-3-ExaD6 released the small molecule exatecan after killing the target cells, which then killed the Nectin-4 negative cells (see FIGs. 7A and 7B).

Construction of CHO-Nectin-4 cell line:

The full-length sequence of human Nectin-4 was synthesized, and an expression vector was constructed.

The plasmid was transfected into CHO-K1 cells via electroporation. After stressed screening and subcloning, a stable CHO-K1 cell line expressing the full-length Nectin-4 was isolated and designated CHO-Nectin-4.

**Example 27. Efficacy of test drugs in MDA-MB-468 xenograft tumor model**

In this study, the *in vivo* anti-tumor activity of the test drugs was evaluated using a human breast cancer MDA-MB-468 nude mouse subcutaneous xenograft tumor model.

In this study, female nude mice were grafted subcutaneously with MDA-MB-468 tumor cells to establish the MDA-MB-468 nude mouse xenograft tumor model. On day 18 after grafting, when the mean tumor volume reached about 140 $mm^3$, the animals were randomized by tumor volume into groups of 6 mice each. The day of grouping was designated as Day 0. In the study, 1 mg/kg and 5 mg/kg treatment groups were set for ASG-22-MMAE, 10F4-3-MMAE, and 10F4-5-MMAE, respectively. On the day of grouping (Day 0), the drugs were given to the mice alone via intravenous (IV) tail vein injection, with a total of 1 dose. In the vehicle control group (normal saline), the solvent was administered alone on the day of grouping via intravenous (IV) tail vein injection, with a total of 1 dose.

The tumor growth in the treatment groups and the control group of the human breast cancer MDA-MB-468 nude mouse subcutaneous xenograft tumor model is shown in Table 20 below and FIG. 8.

On day 21 post-dose, the mean tumor volume of the vehicle control group was 382.85 ± 23.2 $mm^3$, and the experiment ended on Day 21. Compared with the vehicle control group, on Day 21, the relative tumor proliferation rates (T/C) for the ASG-22-MMAE treatment groups (1 mg/kg and 5 mg/kg) were 49.82% (TGI = 49.14%, P < 0.001) and 17.56% (TGI = 81.91%, P < 0.001), respectively.

The relative tumor proliferation rates (T/C) for the 10F4-3-MMAE treatment groups (1 mg/kg and 5 mg/kg) were 59.50% (TGI = 38.68%, P < 0.001) and 24.37% (TGI = 75.07%, P < 0.001), respectively.

The relative tumor proliferation rates (T/C) for the 10F4-5-MMAE treatment groups (1 mg/kg and 5 mg/kg) were 57.35% (TGI = 41.14%, P < 0.01) and 32.97% (TGI = 66.17%, P < 0.001), respectively.

In this experiment, by Day 21, the mean body weight of the animals in the vehicle control group had increased, with an increase of 3.11%. The mean body weight of animals in the low- and high-dose ASG-22-MMAE treatment groups, the low- and high-dose 10F4-3-MMAE treatment groups, and the low- and high-dose 10F4-5-MMAE treatment group, all increased, with increases of 1.03%, 2.42%, 3.04%, 1.16%, 3.28%, and 3.04%, respectively. In the experiment, the number of drug-related deaths was 0, and no other drug-related adverse effects were observed.

Table 20. Effects of test drugs on animal tumor volume in human breast cancer MDA-MB-468 nude mouse xenograft tumor model

| Group | Number of animals | Test drug | Dose mg/kg | Tumor volume ($mm^3$, Mean ± SEM) | | Tumor volume inhibition rate (%TGI) Day 21 | Relative tumor proliferation rate (%T/C) Day 21 |
|---|---|---|---|---|---|---|---|
| | | | | Day 0 | Day 21 | | |
| 1 | 6 | Normal saline | NA | 140.29 ± 9.02 | 382.85 ± 23.21 | N/A | N/A |

(continued)

| Group | Number of animals | Test drug | Dose mg/kg | Tumor volume (mm$^3$, Mean ± SEM) | | Tumor volume inhibition rate (%TGI) Day 21 | Relative tumor proliferation rate (%T/C) Day 21 |
|---|---|---|---|---|---|---|---|
| | | | | Day 0 | Day 21 | | |
| 2 | 6 | ASG-22-MMAE | 1 | 140.84 ± 9.30 | 194.71 ± 24.5 *** | 49.14 | 49.82 |
| 3 | 6 | ASG-22-MMAE | 5 | 140.47 ± 8.46 | 69.26 ± 28.28 *** | 81.91 | 17.56 |
| 6 | 6 | 10F4-3-MMAE | 1 | 142.00 ± 5.76 | 234.63 ± 7.07 *** | 38.68 | 59.50 |
| 7 | 6 | 10F4-3-MMAE | 5 | 141.33 ± 6.05 | 95.43 ± 17.69 *** | 75.07 | 24.37 |
| 8 | 6 | 10F4-5-MMAE | 1 | 140.02 ± 5.97 | 225.34 ± 24.10 *** | 41.14 | 57.35 |
| 9 | 6 | 10F4-5-MMAE | 5 | 139.00 ± 5.66 | 129.5 ± 15.87 *** | 66.17 | 32.97 |
| Note: 1. Day 0 denotes the day of grouping and treatment; 2. *: $P < 0.05$ compared with the vehicle control group; **: $P < 0.01$ compared with the vehicle control group; ***: $P < 0.001$ compared with the vehicle control group. | | | | | | | |

**Example 28. Pharmacological evaluation in JEG-3 cell subcutaneous xenograft tumors**

Objective:

This is a preclinical pharmacodynamic study to evaluate the efficacy of test drug 10F4-3-ExaD8 in a human choriocarcinoma JEG-3 cell subcutaneous xenograft female NOD/SCID mouse animal model.

Design

In the study, the mice were divided into groups of 6 mice each, and the treatment was started on the day of the grouping. The study design is detailed in Table 21.

Table 21. Administration routes, dosages, and regimens in human choriocarcinoma JEG-3

| Group | Number of animals | Treatment | Dose (mg/kg) | Route of administration | Dose volume ( μL/g ) | Treatment cycle |
|---|---|---|---|---|---|---|
| 1 | 6 | Vehicle control | -- | *i.v.* | 10 | Single dose |
| 6 | 6 | 10F4-3-ExaD8 | 5 | *i.v.* | 10 | Single dose |

Procedures

JEG-3 cells were cultured in a MEM medium containing 10% fetal bovine serum (supplemented with 0.01 mM NEAA). JEG-3 cells in the exponential phase were collected and resuspended in PBS to a proper concentration for subcutaneous tumor grafting in mice.

$1 \times 10^7$ JEG-3 cells resuspended in a 1:1 mixture of PBS and matrigel were grafted in the mice subcutaneously at the right dorsum (0.2 mL/mouse). The growth of the tumors was observed periodically. When the mean volume of the tumors reached 129.56 mm$^3$, the mice were randomized by tumor size and the body weight for treatment. The day of grouping was designated as Day 0, and the treatment was started on Day 0.

Results

In the JEG-3 cell line subcutaneous xenograft model, no significant abnormalities or weight loss were observed in

mice in both the treatment and control groups after treatment.

The tumor growth in the treatment groups and the control group of the JEG-3 xenograft model is shown in Table 22 and FIG. 9. After a single dose in the treatment group, significantly inhibited tumor growth can be observed.

Table 22. Efficacy analysis for each group in JEG-3 xenograft model

| Group | Mean tumor volume (Day 0)[a] | Mean tumor volume (Day 13)[a] | TGI (%)[b] | *P* Value (relative to control group)[c] |
|---|---|---|---|---|
| **Group 1**<br>Vehicle, 0 mg/kg, single dose, *i.v.* | 129.61±7.16 | 2072.05±230.27 | - | - |
| **Group 6**<br>10F4-3-ExaD8, 5 mg/kg, | 129.58±7.40 | 95.19±7.17 | 101.77% | 6.88e-10*** |

Note:
a. Data were expressed in "mean ± standard error";
b. TGI% = [1 - (Ti - T0)/(Ci - C0)] × 100, where T0 and C0 are the mean tumor volumes of the treatment group and vehicle control group on the day of grouping (Day 0), respectively, and Ti and Ci are the mean tumor volumes of the treatment group and vehicle control group on Day 13, respectively;
c. **P* < 0.05, ***P* < 0.01, and ****P* < 0.001 compared with the tumor volume of the vehicle control group.

**Example 29. Efficacy evaluation of test drugs in BL0597 bladder cancer subcutaneous xenograft model**

Objective:

Anti-tumor effects of test drugs 10F4-3-ExaD4 and 10F4-5-ExaD8 were evaluated in a HuPrime® bladder cancer BL0597 xenograft BALB/c nude mouse model.

Design:

The experiment included 5 groups, 8 animals per group, and administration was started on the day of the grouping. The study design is detailed in Table 23.

Table 23. Administration routes, dosages, and regimens in HuPrime® bladder cancer BL0597 subcutaneous xenograft model

| Group | Number of animals | Treatment | Dose (mg/kg) | Route of administration | Dose volume ( μL/g ) | Treatment cycle |
|---|---|---|---|---|---|---|
| 1 | 8 | Vehicle control | -- | *i.v.* | 10 | Single dose |
| 2 | 8 | 10F4-3-ExaD4 | 5 | *i.v.* | 10 | Single dose |
| 3 | 8 | 10F4-3-ExaD4 | 8 | *i.v.* | 10 | Single dose |
| 4 | 8 | 10F4-3-ExaD8 | 3 | *i.v.* | 10 | Single dose |
| 5 | 8 | 10F4-3-ExaD8 | 5 | *i.v.* | 10 | Single dose |

Note: The day of grouping was defined as Day 0, and the administration was started on Day 0.

Procedures

Tumor tissues were collected from tumor-bearing mice of HuPrime® bladder cancer xenograft model BL0597, cut into tumor masses of a diameter of 2-3 mm and then subcutaneously grafted into the right anterior scapula of BALB/c nude mice.

When the mean tumor volume of the tumor-bearing mice reached about 143.67 $mm^3$, the mice were randomly grouped according to Table 23. The day of grouping was defined as Day 0, and the administration was started on Day 0.

Results

In the BL0597 subcutaneous xenograft model, there were no significant changes in mouse body weight after the

treatment in the treatment and control groups.

Both 10F4-3-ExaD4 and 10F4-3-ExaD8, after a single dose, exhibited significant tumor inhibitory effects in the BL0597 xenograft model, with no significant differences observed between the treatment groups.

The tumor growth in the treatment and control groups is shown in FIG. 10 and Table 24.

Table 24. Efficacy analysis for each group in BL0597 xenograft model

| Group | Mean tumor volume (Day 0)[a] | Mean tumor volume (Day 28)[a] | TGI (%)[b] | *p* value (relative to control group)[c] |
|---|---|---|---|---|
| **Group 1** Vehicle, 0 mg/kg | 143.66±10.26 | 691.91±67.43 | - | - |
| **Group 2** 10F4-3-ExaD4, 5 mg/kg | 143.60±9.95 | 252.64±48.29 | 80.11 | 3.04e-03** |
| **Group 3** 10F4-3-ExaD4, 8 mg/kg | 143.62±9.72 | 257.08±33.98 | 79.31 | 1.24e-02* |
| **Group 4** 10F4-3-ExaD8, 3 mg/kg | 143.66±10.27 | 296.11±92.30 | 72.19 | 3.73e-03** |
| **Group 5** 10F4-3-ExaD8, 5 mg/kg | 143.71±10.31 | 233.79±29.35 | 83.57 | 5.61e-03** |

Note: a. Data were expressed in "mean ± standard error";
b. TGI% = [1 - (Ti - T0)/(Ci - C0)] × 100, where T0 and C0 are the mean tumor volumes of the treatment group and vehicle control group on the day of grouping (Day 0), respectively, and Ti and Ci are the mean tumor volumes of the treatment group and vehicle control group on Day 28, respectively;
c. **p<0.05, **p*<0.01, and ****p*<0.001 compared with the tumor volume of the vehicle control group.

## Claims

1. An antibody-drug conjugate, or a pharmaceutically acceptable salt or solvate thereof, comprising: the an-Nectin-4 antibody or antigen-binding unit, or the polypeptide specifically binding to Nectin-4 conjugated to a drug via a linker, or the linker is a cleavable linker.

2. An antibody-drug conjugate having a structure shown in Formula I-A, Formula I-B or Formula I-C or a stereoisomer thereof, or a pharmaceutically acceptable salt or solvate thereof:

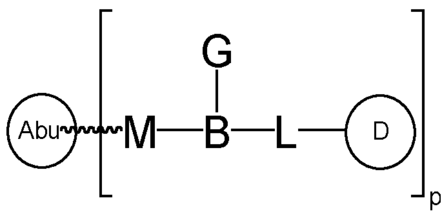

Formula I-A

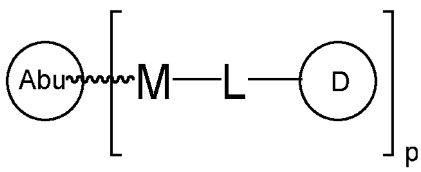

Formula I-B

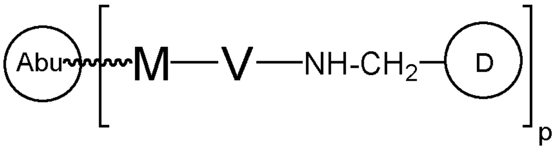

Formula I-C

Abu is the anti-Nectin-4 antibody or antigen-binding unit, or the polypeptide specifically binding to Nectin-4;
D is a drug;
M is

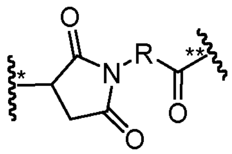

,

R is selected from $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, - $(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$, and - $(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10; in Formula I-A, * links to Abu, and ** links to B; in Formula I-B, * links to Abu, and ** links to L; in Formula I-C, * links to Abu, and ** links to V;
B is

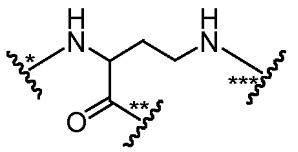

,

wherein * links to M, ** links to L, and *** links to G;
L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19, or 20; each FF is independently

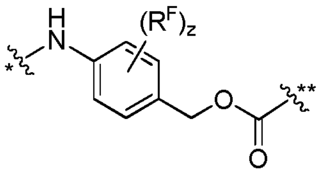

,

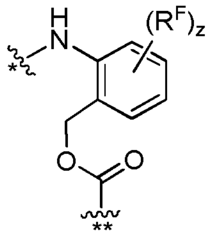

or

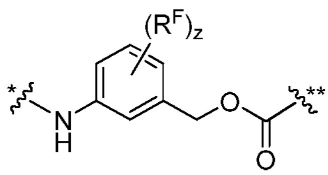

,

wherein each $R^F$ is independently C1-C6 alkyl, C1-C6 alkoxy, $-NO_2$, or halogen, wherein * links to AA, and ** links to D, and z is 0, 1, 2, 3, or 4; f is 1, 2, 3, 4, 5, 6, 7, 8, 9, or 10;
G is

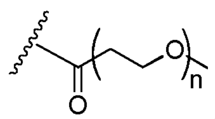

,

wherein n is 1-24;

V is

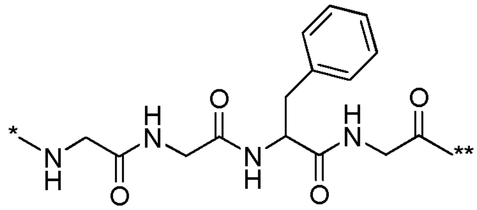

,

or V is

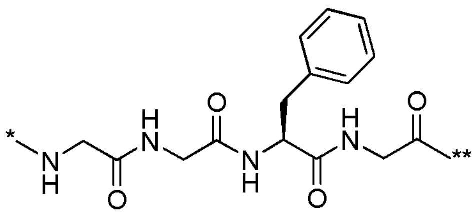

,

wherein * links to M, and ** links to -NH-CH2-;
p is 1-10.

3. The antibody-drug conjugate or the pharmaceutically acceptable salt or solvate thereof according to claim 2, wherein B is

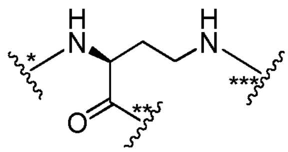

,

wherein * links to M, ** links to L, and *** links to G.

4. The antibody-drug conjugate or the pharmaceutically acceptable salt or solvate thereof according to claim 2 or 3, wherein each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

5. The antibody-drug conjugate or the pharmaceutically acceptable salt or solvate thereof according to claim 4, wherein AA is Val-Cit.

6. The antibody-drug conjugate or the pharmaceutically acceptable salt or solvate thereof according to any one of claims 2-5, wherein i is 1.

7. The antibody-drug conjugate or the pharmaceutically acceptable salt or solvate thereof according to any one of claims 2-6, wherein each FF is independently

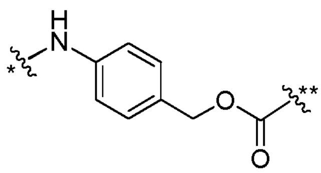

,

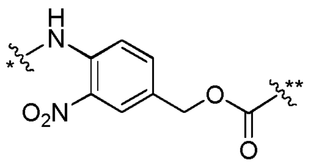 , 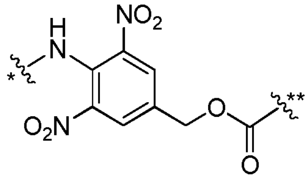 , 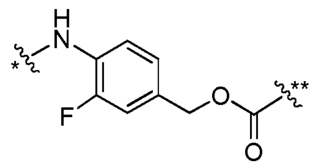 ,

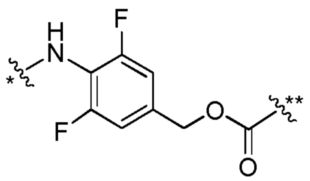 , 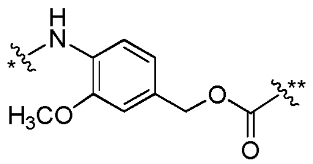 , 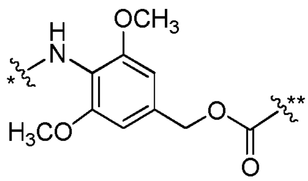 , 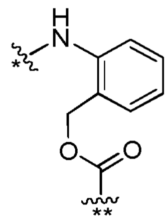 ,

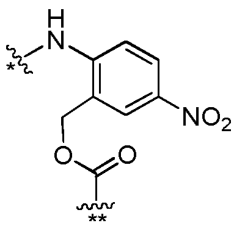 , 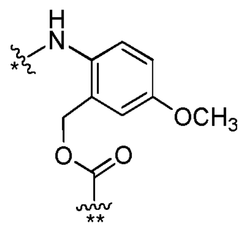 , 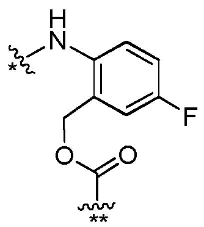 , 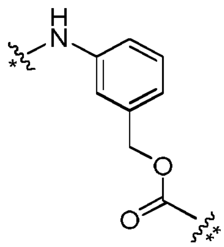 ,

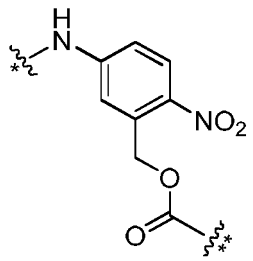 , 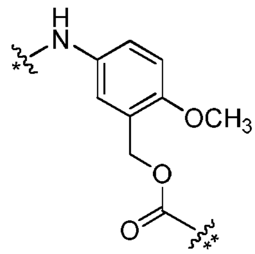 , and 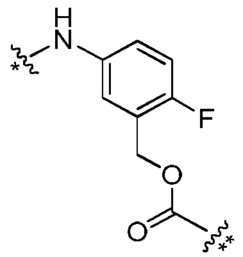 ,

wherein * links to AA, and ** links to D.

**8.** The antibody-drug conjugate or the pharmaceutically acceptable salt or solvate thereof according to claim 7, wherein FF is

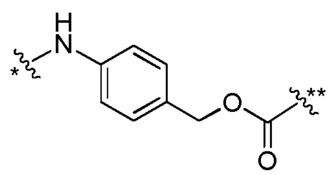 ,

wherein * links to AA, and ** links to D.

**9.** The antibody-drug conjugate or the pharmaceutically acceptable salt or solvate thereof according to any one of claims 2-8, wherein f is 1.

**10.** The antibody-drug conjugate or the pharmaceutically acceptable salt or solvate thereof according to any one of claims 2-9, wherein L is

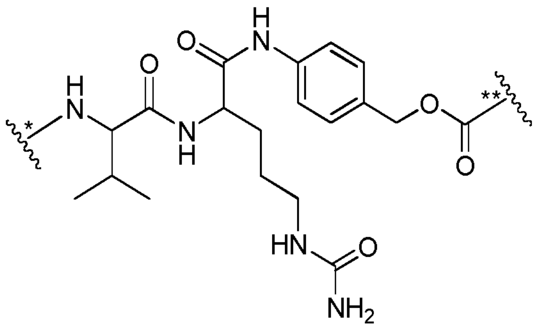

,

or

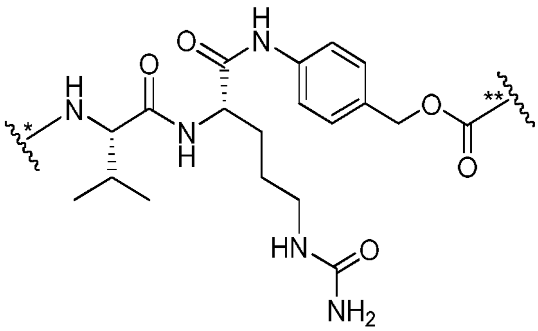

,

wherein in Formula I-A, * links to B, and ** links to D; in Formula I-B, * links to M, and ** links to D.

**11.** An antibody-drug conjugate having a structure shown in Formula I-A-1 or I-A-2 or a stereoisomer thereof, or a pharmaceutically acceptable salt or solvate thereof, wherein the Formulas I-A-1 and I-A-2 are:

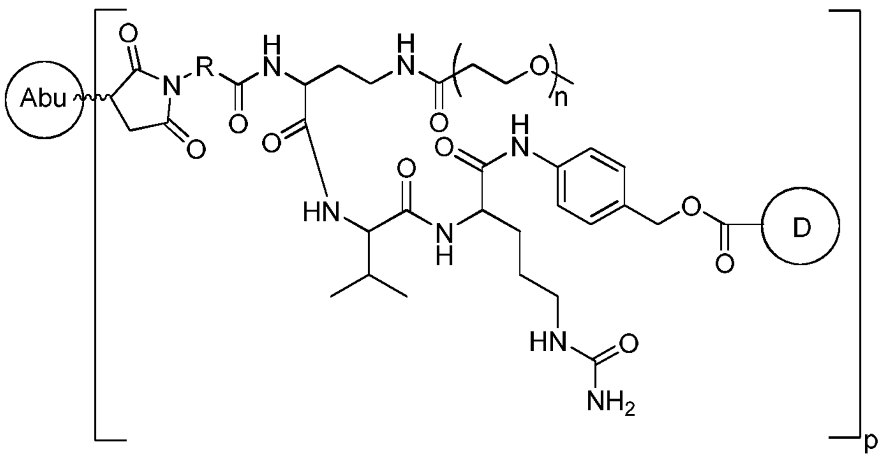

Formula I-A-1

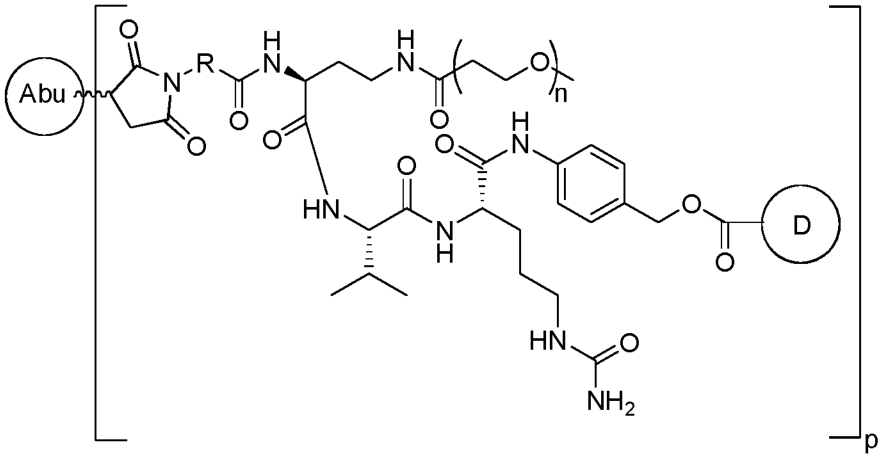

Formula I-A-2

wherein

Abu is the anti-Nectin-4 antibody or antigen-binding unit, or the polypeptide specifically binding to Nectin-4;
R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r$-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_r-C(O)NR^m(CH_2CH_2O)_r-CH_2-$, and $-(CH_2CH_2O)_rC(O)NR^m(CH_2)_r-$, wherein each $R^m$ is independently H, C1-C6 alkyl, C3-C8 carbocyclyl, phenyl, or benzyl, and each r is independently 1, 2, 3, 4, 5, 6, 7, 8, 9, or 10;
D is a drug;
n is an integer of 1-24;
p is 1-10.

**12.** The antibody-drug conjugate, the pharmaceutically acceptable salt thereof, or the solvate thereof according to any one of claims 2-11, wherein R is $-(CH_2)_r-$, and r is 1 or 5.

**13.** An antibody-drug conjugate having a structure shown in Formula I-A-3 or I-A-4 or a stereoisomer thereof, or a pharmaceutically acceptable salt or solvate thereof, wherein the Formulas I-A-3 and I-A-4 are:

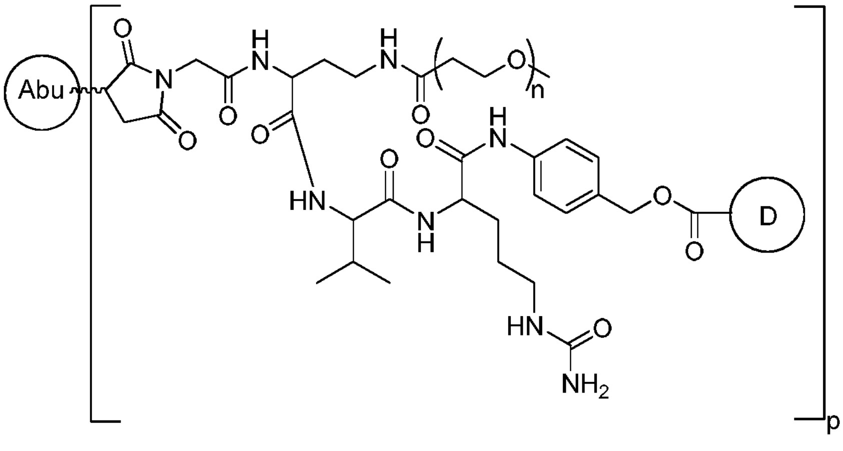

Formula I-A-3

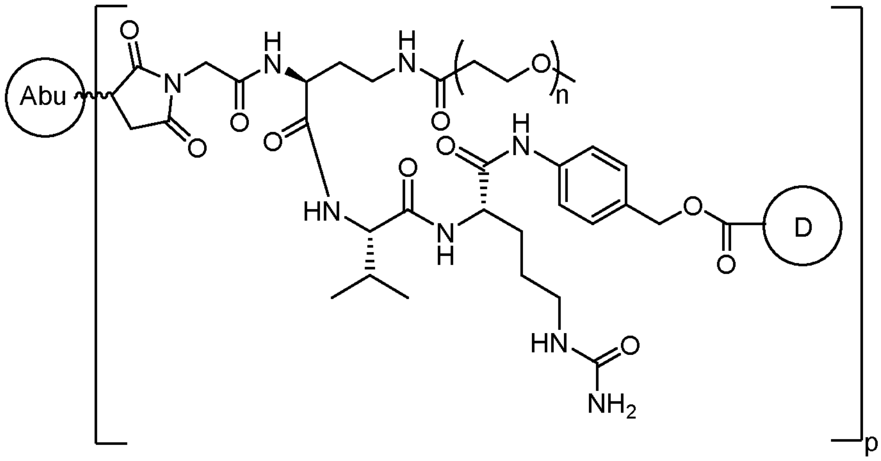

Formula I-A-4

wherein

Abu is the anti-Nectin-4 antibody or antigen-binding unit, or the polypeptide specifically binding to Nectin-4;
D is a drug;
n is an integer of 1-24;
p is 1-10.

**14.** The antibody-drug conjugate or the pharmaceutically acceptable salt or solvate thereof according to any one of claims 2-13, wherein n is 4-12; or n is 4-8; or n is 4 or 8.

**15.** An antibody-drug conjugate having a structure shown in Formula I-A-5, I-A-6, I-A-7, I-A-8, I-A-9, I-A-10 or I-A-11 or a stereoisomer thereof, or a pharmaceutically acceptable salt or solvate thereof, wherein the Formulas I-A-5, I-A-6, I-A-7, I-A-8, I-A-9, I-A-10, and I-A-11 are:

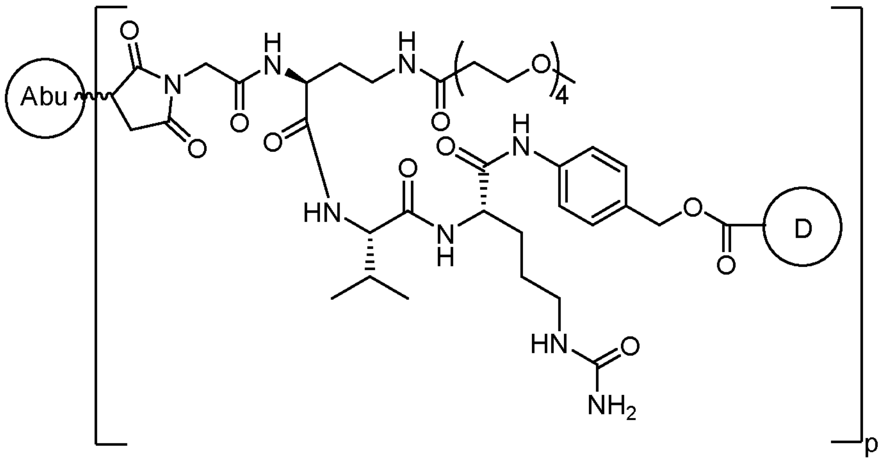

Formula I-A-5

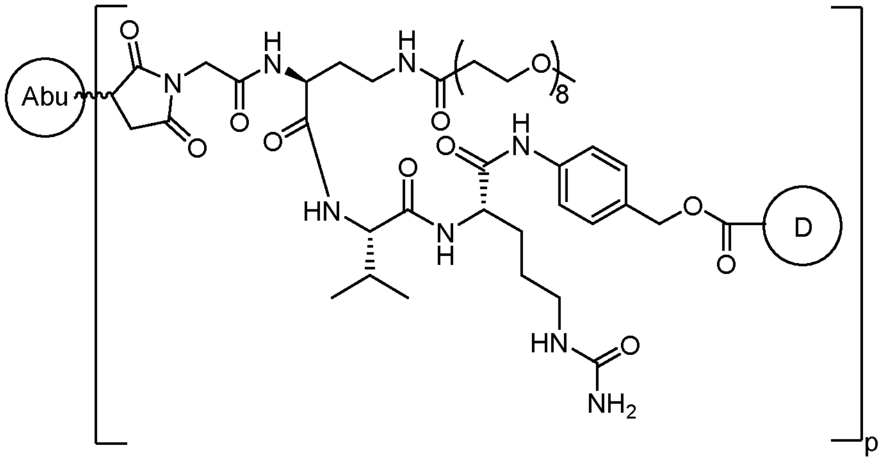

Formula I-A-6

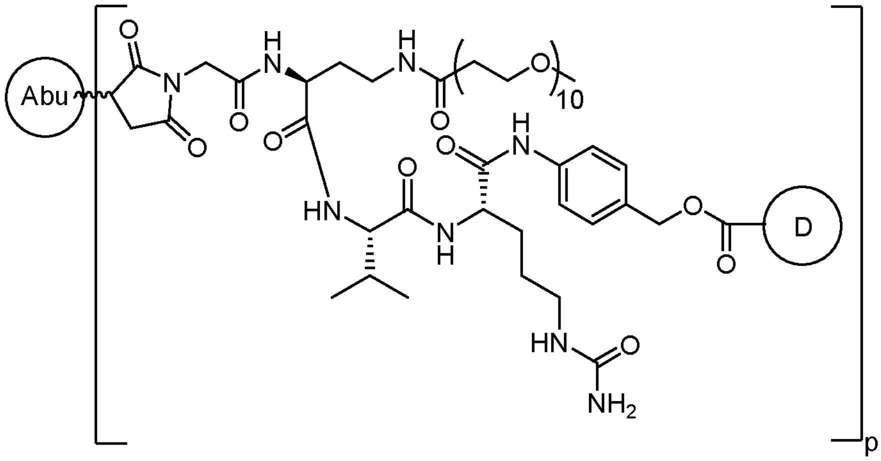

Formula I-A-7

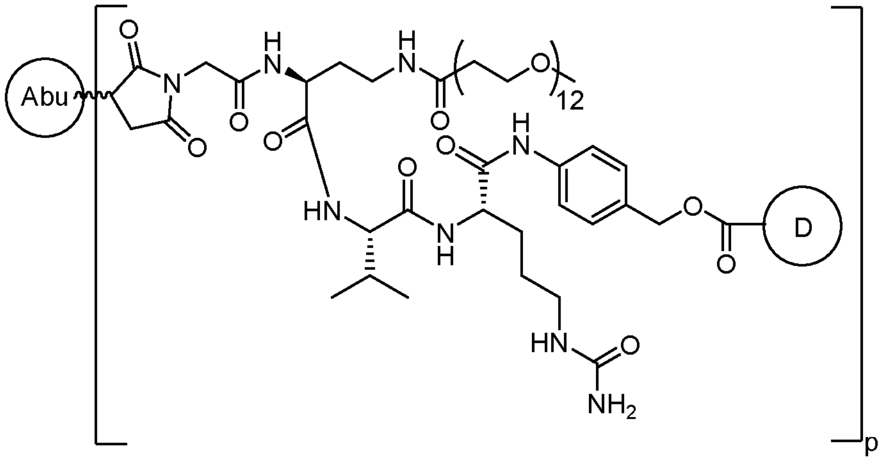

Formula I-A-8

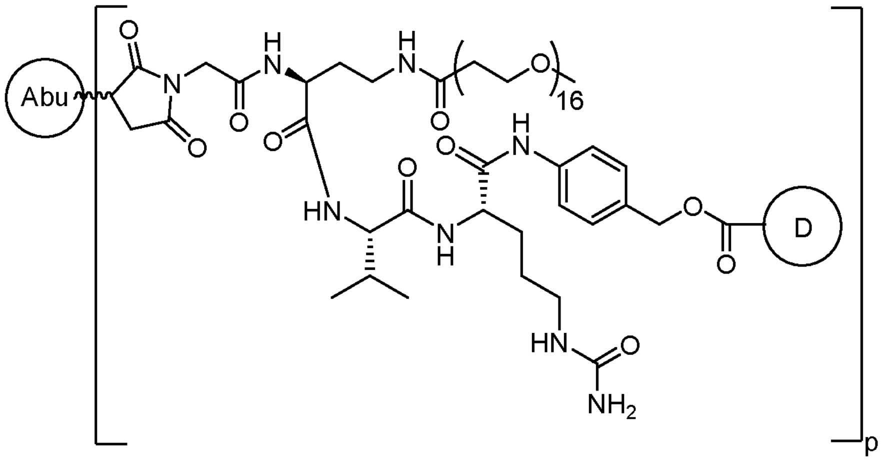

Formula I-A-9

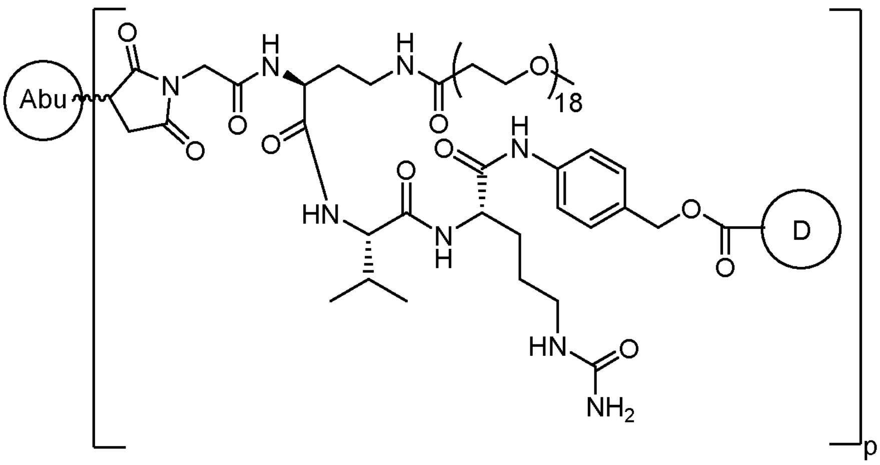

, or

Formula I-A-10

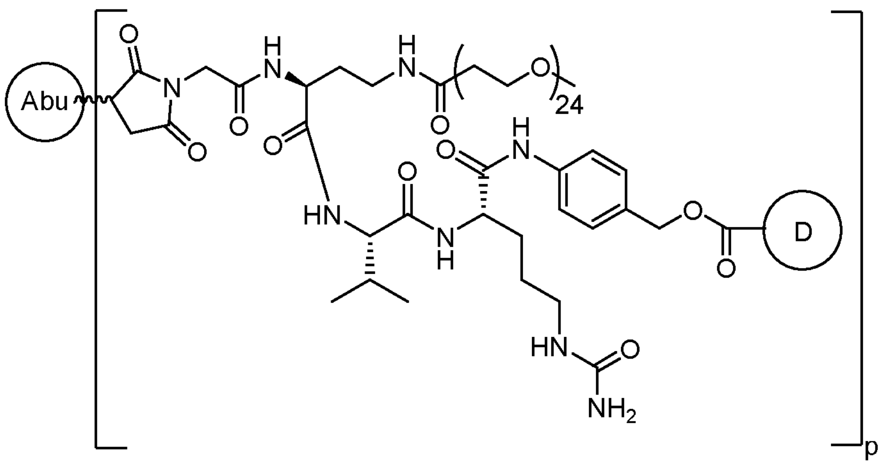

Formula I-A-11

wherein

Abu is the anti-Nectin-4 antibody or antigen-binding unit, or the polypeptide specifically binding to Nectin-4;
D is a drug;
p is 1-10.

**16.** The antibody-drug conjugate or the pharmaceutically acceptable salt or solvate thereof according to any one of claims 1-15, wherein the drug is an anti-cancer drug, a cytotoxic drug, a cell differentiation factor, a stem cell trophic factor, a steroid drug, a drug for treating autoimmune diseases, an anti-inflammatory drug, or a drug for treating an infectious disease; or the drug is an anti-cancer drug; or the drug is a tubulin inhibitor, a DNA damaging agent, or a DNA topoisomerase inhibitor; or the tubulin inhibitor is selected from dolastatin, an auristatin, and a maytansine; or the drug is an auristatin selected from MMAE, MMAF, and AF; or the drug is a DNA damaging agent selected from a calicheamicins, a duocarmycin, and anthramycin derivative PBD (pyrrolobenzodiazepine); or the drug is a DNA topoisomerase inhibitor or a salt thereof selected from irinotecan, irinotecan hydrochloride, camptothecin, 9-aminocamptothecin, 9-nitrocamptothecin, 10-hydroxycamptothecin, 9-chloro-10-hydroxycamptothecin, camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, and N-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan.

**17.** The antibody-drug conjugate or the pharmaceutically acceptable salt or solvate thereof according to any

one of claims 1-15, wherein the drug is
wherein

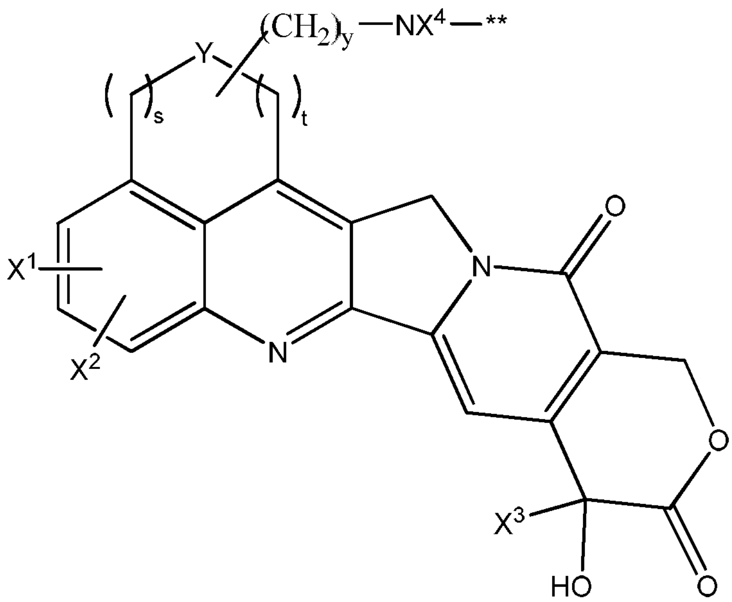

,

$X^1$ and $X^2$ are each independently:

H,
hydroxy,
C1-C6 alkyl,
C1-C6 alkyl substituted with one or more hydroxy, halogen, nitro, or cyano groups,
C2-C6 alkenyl,
C2-C6 alkynyl,
C1-C6 alkoxy,
C1-C6 aminoalkoxy, halogen,
nitro,
cyano,
sulfhydryl,
alkylthio,
amino, amino substituted with an amino-protecting group, C1-C6 aminoalkyl optionally substituted at the

amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclyl, wherein the heterocyclyl is optionally substituted with one or more C1-C6 alkyl, C1-C6 alkoxy, amino, halogen, nitro, or cyano groups,

C1-C6 alkylamino linking to a heterocyclyl, wherein the heterocyclyl is optionally substituted with C1-C6 alkyl or C1-C6 alkoxy, and the amino is optionally substituted with an amino-protecting group, halogen, nitro, cyano, or a protecting group,

amino-substituted heterocyclyl optionally substituted at a nitrogen atom of the heterocyclic moiety or at the amino moiety with a protecting group or one or more C1-C6 alkyl groups,

heterocyclylamino optionally substituted at a nitrogen atom of the heterocyclyl moiety or at the amino moiety with a protecting group or C1-C6 alkyl,

carbamoyl optionally substituted with a carbamoyl-protecting group or C1-C6 alkyl, morpholin-1-yl, or piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, - $(CH_2CH_2O)_qC(O)NR^n(CH_2CH_2O)_q-CH_2-CH_3$, or $-(CH_2CH_2O)_qC(O)NR^n(CH_2)_q-CH_3$, wherein each $R^n$ is independently H, C1-C6 alkyl, C3-C8 carbocyclyl, phenyl, or benzyl, and each q is independently 1, 2, 3, 4, 5, 6, 7, 8, 9, or 10; or $X^4$ is H or C1-C6 alkyl;

** is a linking point;

y is 0, 1, or 2;

Y is O, S, or $CR^{1D}R^{2D}$, wherein $R^{1D}$ and $R^{2D}$ are each independently H or C1-C6 alkyl;

s and t are each independently 0, 1, or 2, but not both 0;

or the drug is

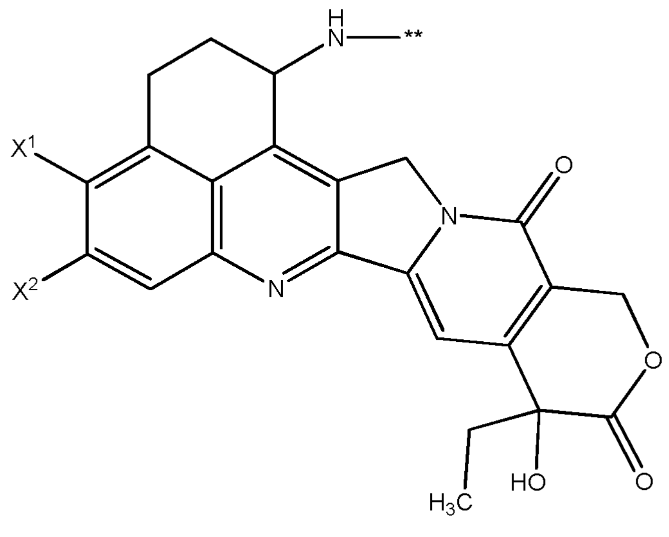

,

wherein $X^1$ and $X^2$ are each independently C1-C6 alkyl, halogen, or -OH; or the C1-C6 alkyl is $-CH_3$; or the halogen is F; ** is a linking point;

or the drug is

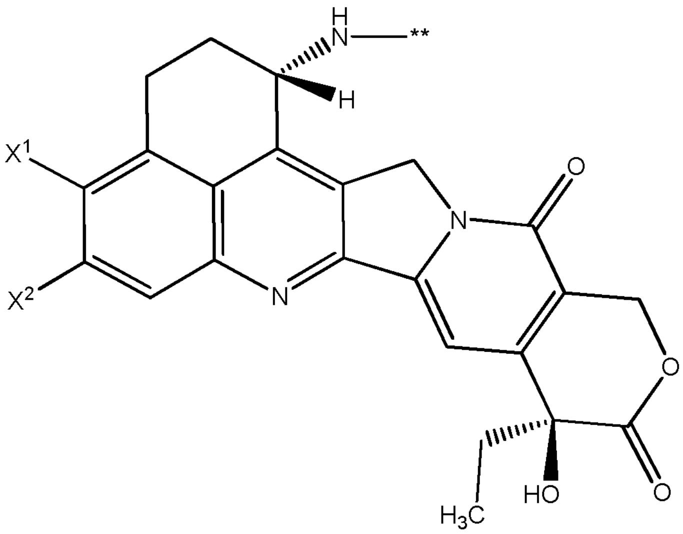

,

wherein $X^1$ and $X^2$ are each independently C1-C6 alkyl, halogen, or -OH; or the C1-C6 alkyl is $-CH_3$; or the halogen is F; ** is a linking point;
or the drug is

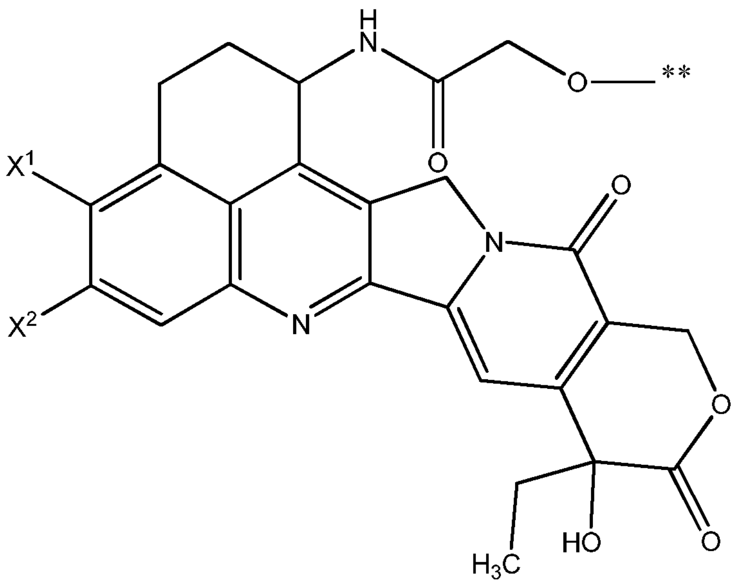

,

wherein $X^1$ and $X^2$ are each independently C1-C6 alkyl, halogen, or -OH; or the C1-C6 alkyl is $-CH_3$; or the halogen is F; ** is a linking point;
or the drug is

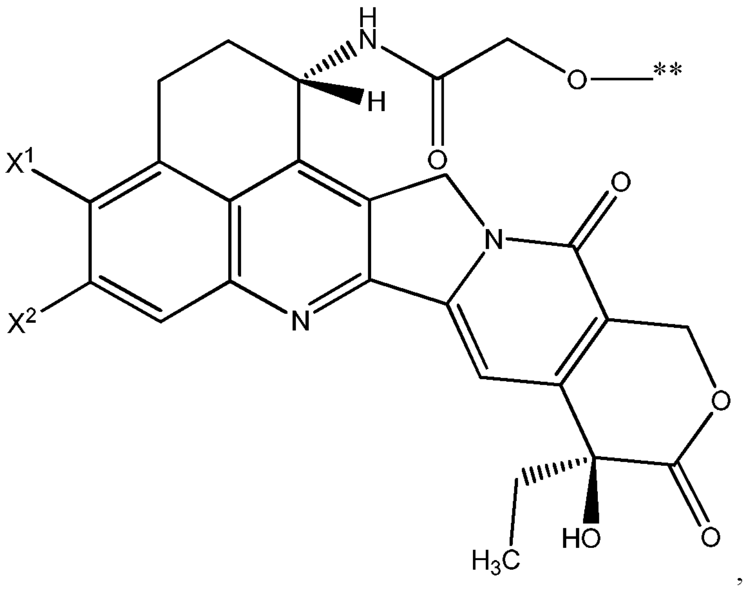

,

wherein $X^1$ and $X^2$ are each independently C1-C6 alkyl, halogen, or -OH; or the C1-C6 alkyl is $-CH_3$; or the halogen is F; ** is a linking point;
or the drug is

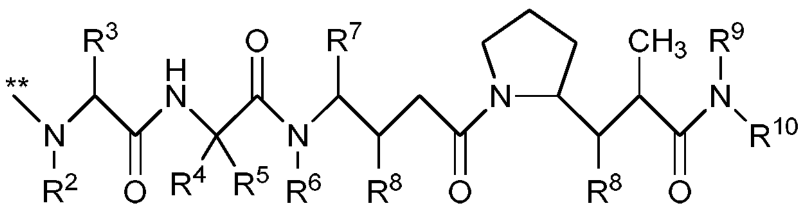

,

wherein ** is a linking point;
$R^2$ is H or C1-C8 alkyl;
$R^3$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);
$R^4$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);
$R^5$ is H or methyl;
or $R^4$ and $R^5$ are linked to form a carbocyclyl having a formula of $-(CR^aR^b)_j-$, wherein $R^a$ and $R^b$ are each independently H, C1-C8 alkyl, or C3-C8 carbocyclyl, and j is 2, 3, 4, 5, or 6;
$R^6$ is H or C1-C8 alkyl;
$R^7$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);
each $R^8$ is independently H, OH, C1-C8 alkyl, C3-C8 carbocyclyl, or O-(C1-C8 alkyl);
$R^9$ is H or C1-C8 alkyl; and
$R^{10}$ is $-C(R^8)_2-C(R^8)_2$-aryl, $-C(R^8)_2-C(R^8)_2$-(C3-C8 heterocyclyl), or $-C(R^8)_2-C(R^8)_2$-(C3-C8 carbocyclyl);
or the drug is

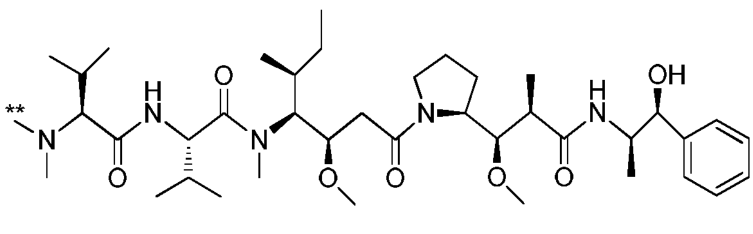

,

wherein ** is a linking point.

**18.** An antibody-drug conjugate having a structure shown in Formula I-A-12, I-A-13, I-A-14, I-A-15, I-A-16, I-A-17, I-A-18, I-A-19, I-A-20, I-A-21, I-A-22, I-A-23, I-A-24, I-A-25, I-B-1 or I-C-1 or a stereoisomer thereof, or a pharmaceutically acceptable salt or solvate thereof, wherein the Formulas I-A-12, I-A-13, I-A-14, I-A-15, I-A-16, I-A-17, I-A-18, I-A-19, I-A-20, I-A-21, I-A-22, I-A-23, I-A-24, I-A-25, I-B-1, and I-C-1 are:

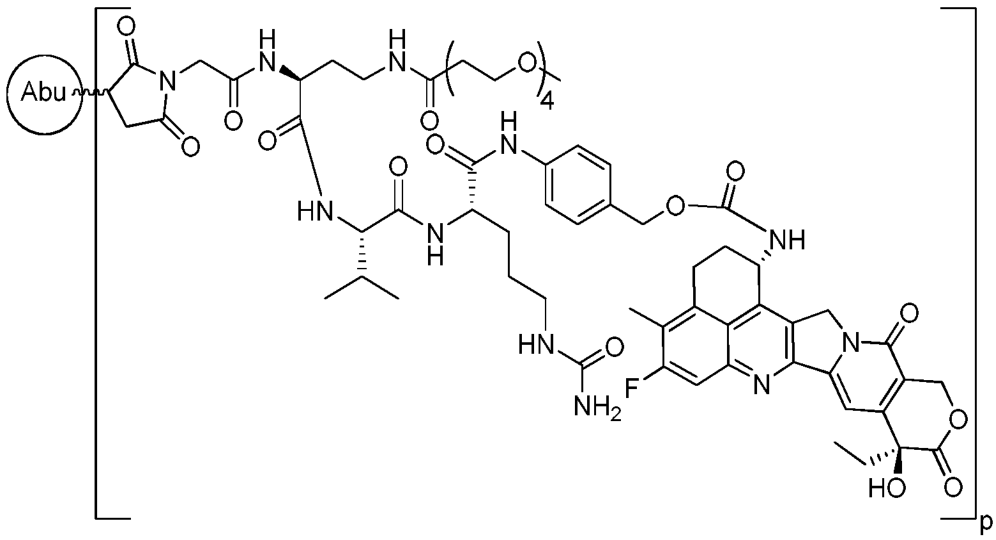

Formula I-A-12

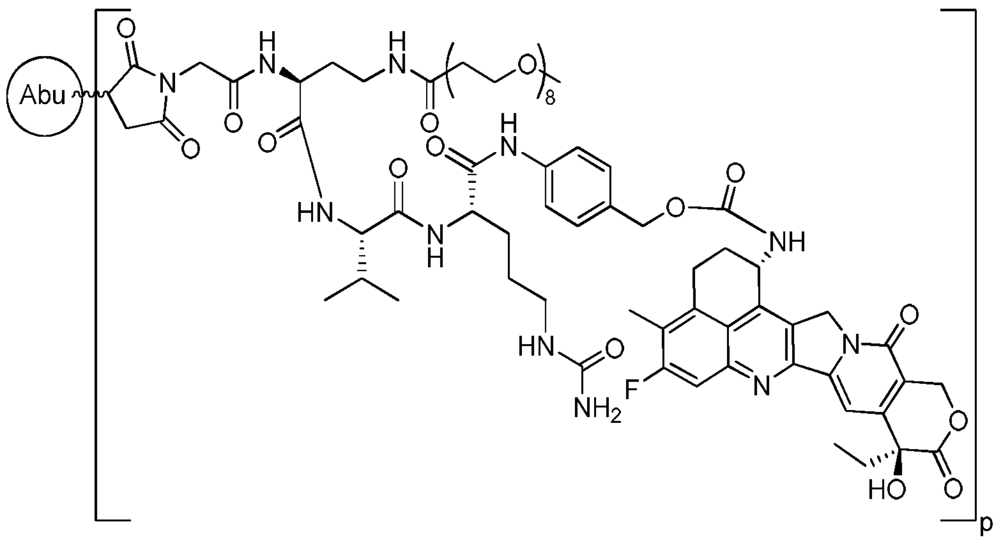

Formula I-A-13

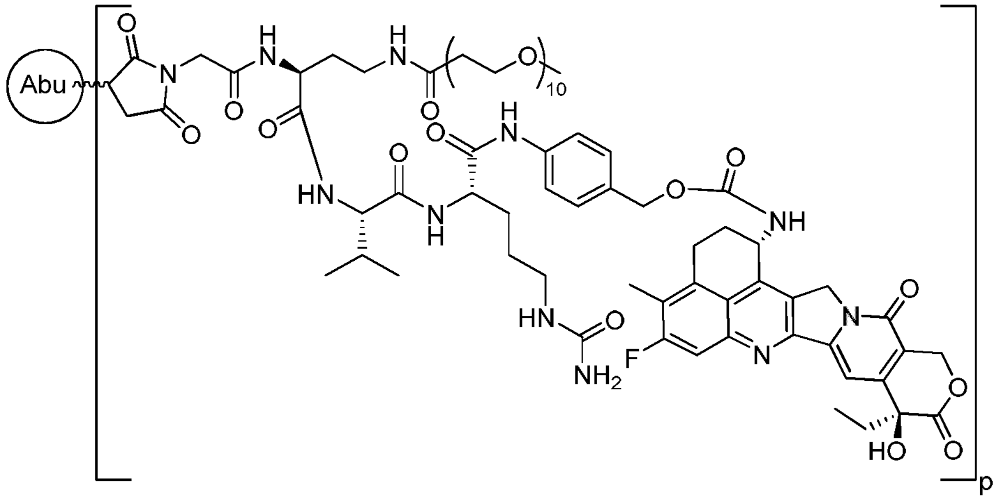

Formula I-A-14

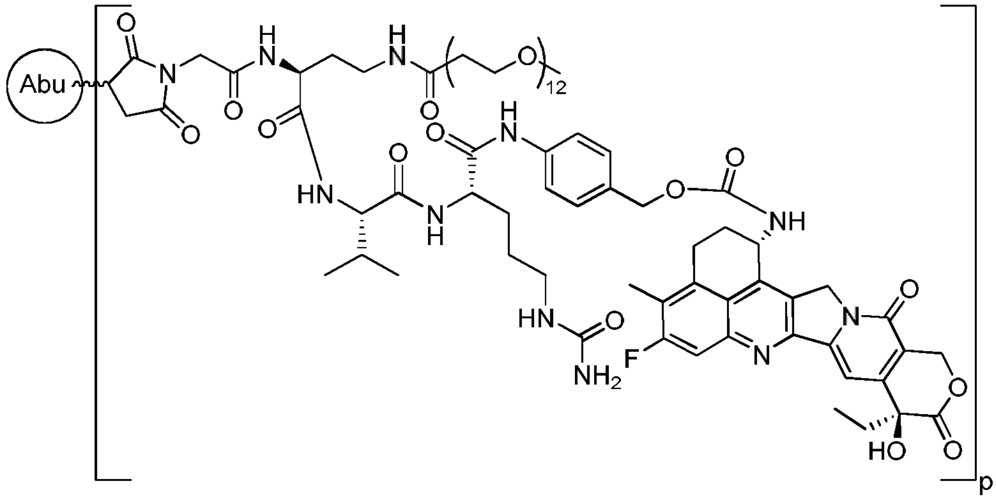

Formula I-A-15

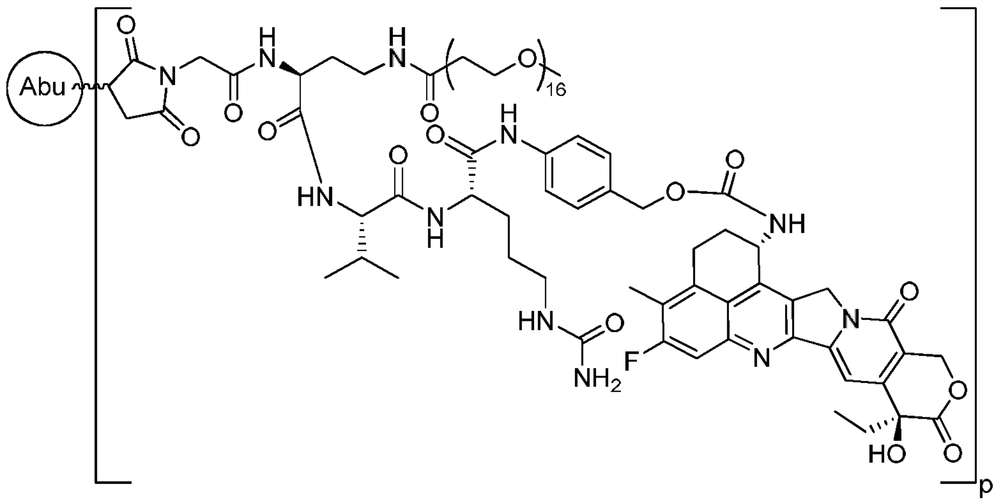

Formula I-A-16

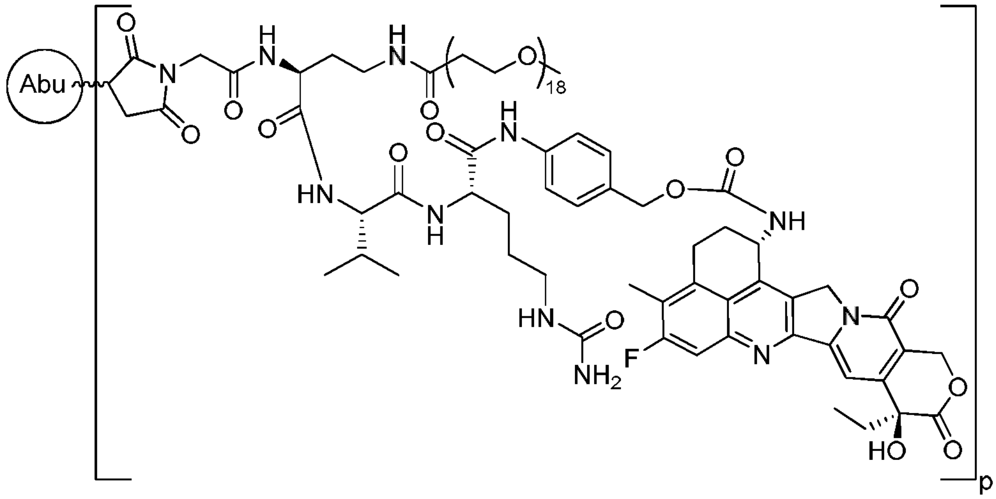

Formula I-A-17

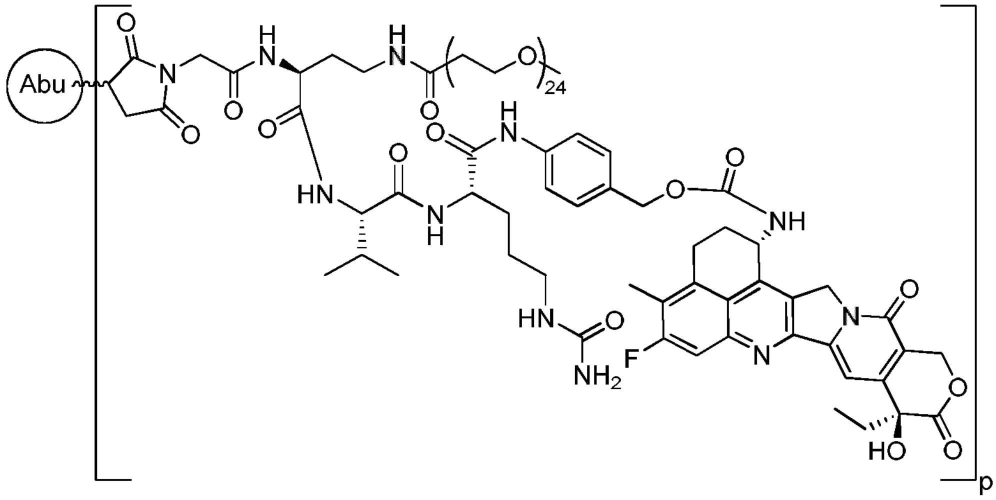

Formula I-A-18

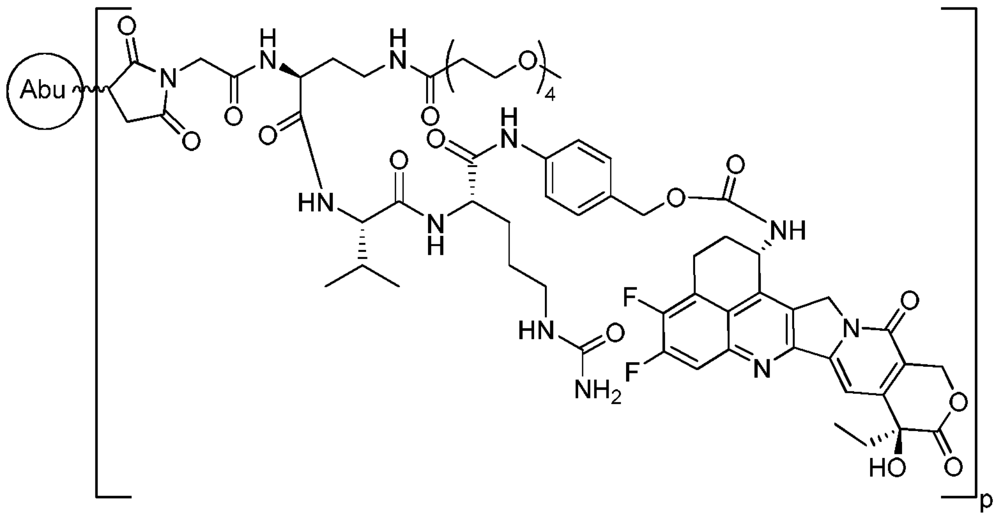

Formula I-A-19

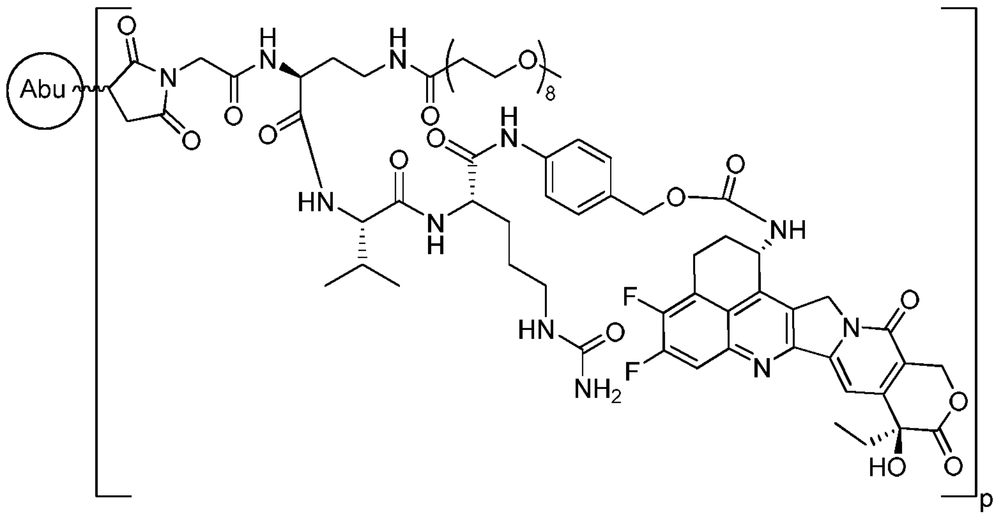

Formula I-A-20

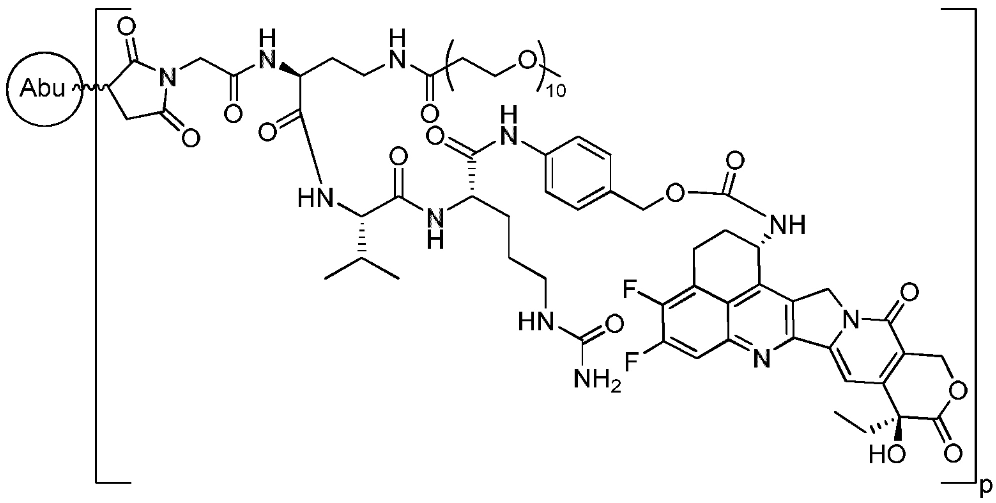

Formula I-A-21

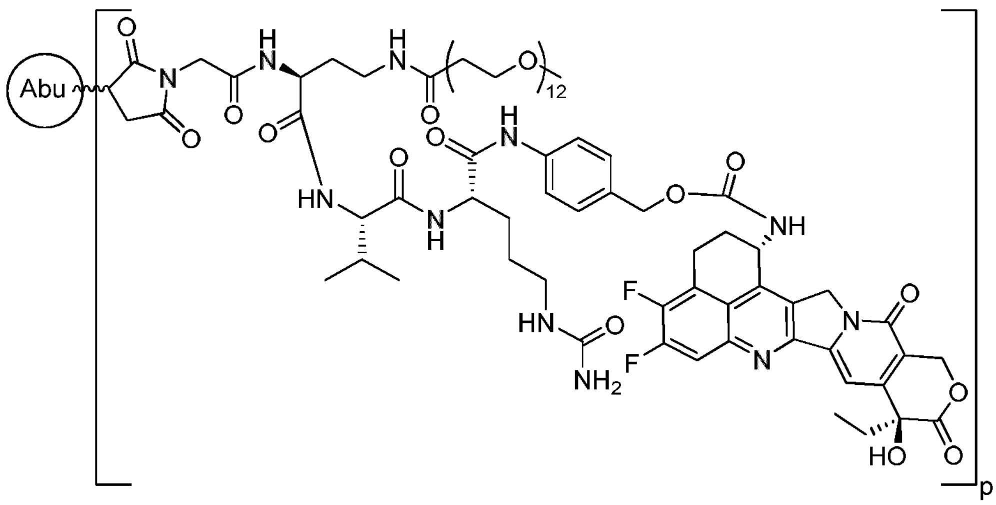

Formula I-A-22

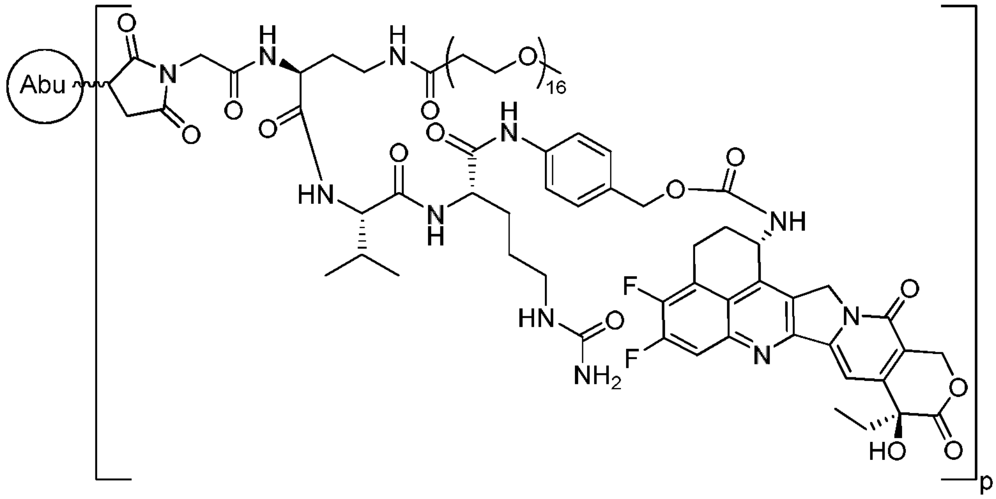

Formula I-A-23

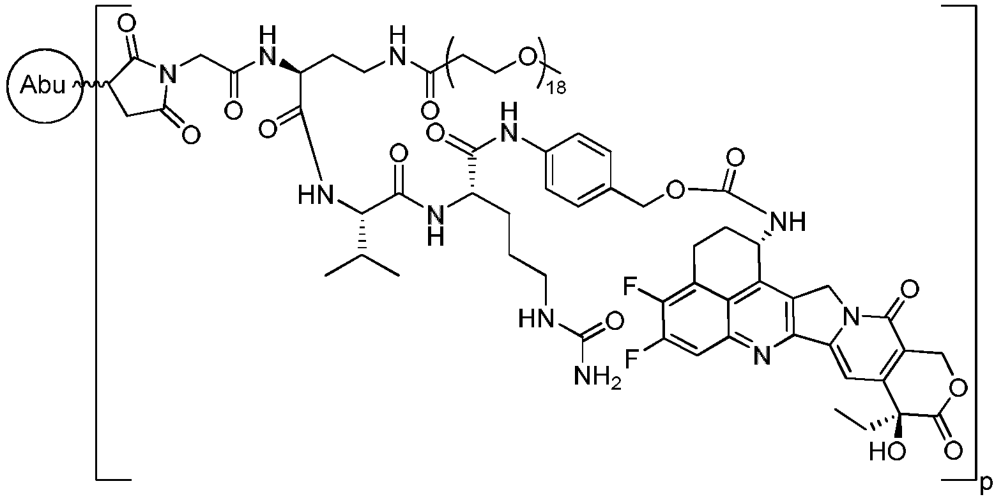

Formula I-A-24

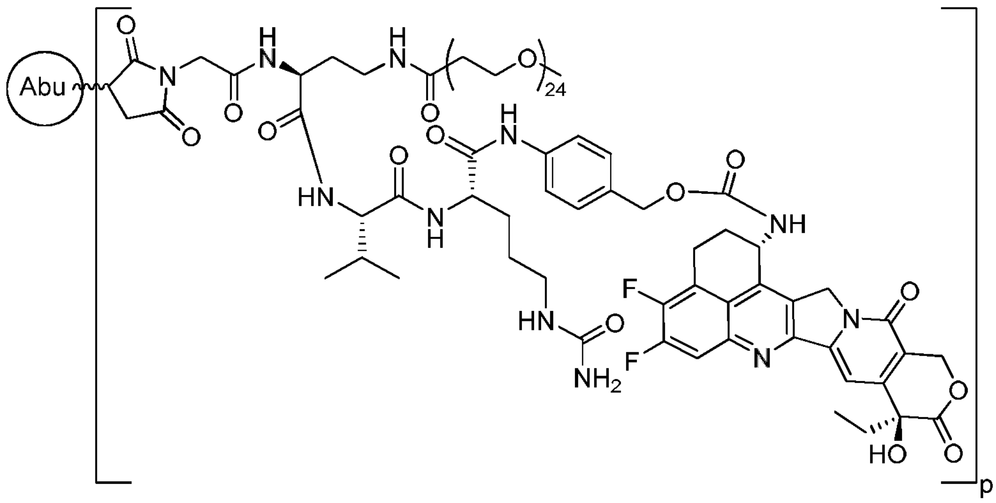

Formula I-A-25

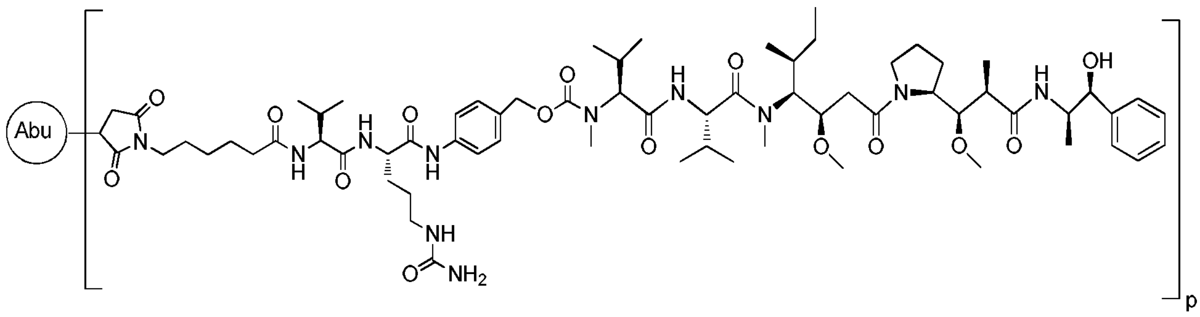

Formula I-B-1

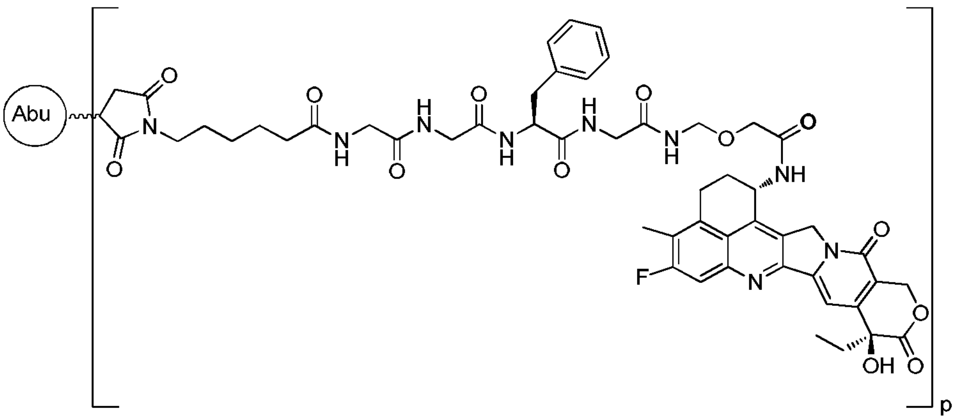

Formula I-C-1

wherein

Abu is the anti-Nectin-4 antibody or antigen-binding unit, or the polypeptide specifically binding to Nectin-4; p is 1-10.

**19.** The antibody-drug conjugate or the pharmaceutically acceptable salt or solvate thereof according to any one of claims 2-18, wherein p is 2-8; or p is 4-8; or p is 6-8; or p is 7-8.

**20.** The antibody-drug conjugate or the pharmaceutically acceptable salt or solvate thereof according to any one of claims 1-19, wherein the anti-Nectin-4 antibody or antigen-binding unit comprises one or more of the following HCDR1, HCDR2 and HCDR3, or variants thereof:

(1) according to the Kabat numbering system, the HCDR1 sequence comprising or consisting of the sequence set forth in any one of SEQ ID NO: 8, SEQ ID NOs: 181-195, SEQ ID NOs: 234-248, and SEQ ID NOs: 304-317, the HCDR2 sequence comprising or consisting of the sequence set forth in any one of SEQ ID NO: 9, SEQ ID NOs: 196-231, SEQ ID NOs: 251-303, and SEQ ID NOs: 318-358, and the HCDR3 sequence comprising or consisting of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
(2) the HCDR1, the HCDR2, and the HCDR3 contained in the heavy chain variable region set forth in SEQ ID NO: 7,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
(3) the HCDR1, the HCDR2, and the HCDR3 contained in the heavy chain variable region set forth in SEQ ID NO: 11,
for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 12;
(4) the HCDR1, the HCDR2, and the HCDR3 contained in the heavy chain variable region set forth in SEQ ID NO:

13,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 14;

(5) the HCDR1, the HCDR2, and the HCDR3 contained in the heavy chain variable region set forth in SEQ ID NO: 15,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 16;

(6) the HCDR1, the HCDR2, and the HCDR3 contained in the heavy chain variable region set forth in SEQ ID NO: 17,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;

(7) the HCDR1, the HCDR2, and the HCDR3 contained in the heavy chain variable region set forth in SEQ ID NO: 19,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;

(8) the HCDR1, the HCDR2, and the HCDR3 contained in the heavy chain variable region set forth in SEQ ID NO: 21,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;

(9) the HCDR1, the HCDR2, and the HCDR3 contained in the heavy chain variable region set forth in SEQ ID NO: 23,

for example, according to the Kabat numbering system, the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;

(10) the HCDR1 sequence comprising or consisting of the sequence set forth in X1X2X3MS, the HCDR2 sequence comprising or consisting of the sequence set forth in X1'IX2'X3'X4'X5'X6'X7'X8'X9'YADSVKG, and the HCDR3 sequence comprising or consisting of the sequence set forth in SEQ ID NO: 12, 14, or 16, wherein X1, X2, X3, X1', X2', X3', X4', X5', X6', X7', X8', X9', and X10' denote amino acids independently selected from any one of G, A, V, L, I, S, T, C, M, N, Q, K, R, D, E, F, Y, H, P and W,

for example, X1 denotes S, N, T, D, or G, X2 denotes Y, F, or S, X3 denotes A, G, S, or Y, X1' denotes A, R, G, I, or W, X2' denotes S, K, Y, or D, X3' denotes G, P, S, A, Q, or W, X4' denotes S, T, G, Y, H, D, W, or I, X5' denotes G, D, T, S, A, or K, X6' denotes G, S, D, A, or W, X7' denotes S, Y, T, N, D, V, E, or G, X8' denotes T, A, N, K, I, R, S, or P, and X9' denotes Y, S, H, R, N, G, F, or D;

alternatively, X1 denotes S, N, T, D, or G, X2 denotes Y, N, F, or S, X3 denotes A, G, S, or absence, X1' denotes A, R, G, W, or S, X2' denotes S, K, Y, or D, X3' denotes G, P, S, T, A, Q, or Y, X4' denotes S, T, G, Y, H, D, W, or I, X5' denotes G, D, T, S, F, or K, X6' denotes G, S, D, A, W, or absence, X7' denotes S, Y, T, N, D, V, E, or G, X8' denotes T, A, N, K, I, R, S, or P, and X9' denotes Y, S, H, R, N, F, or D;

alternatively, X1 denotes S, N, D, G, or T, X2 denotes Y, F, or S, X3 denotes A, D, W, S, or Y, X1' denotes A, S, G, or V, X2' denotes S, K, I, Y, or D, X3' denotes G, P, S, A, Q, Y, T, or D, X4' denotes S, T, G, Y, H, D, or W, X5' denotes G, D, T, S, or K, X6' denotes G, S, D, A, or Y, X7' denotes S, Y, T, N, D, V, or G, X8' denotes T, A, N, K, I, R, or S, and X9' denotes Y, S, H, R, N, G, F, or D;

alternatively, the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;

alternatively, the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202 and SEQ ID NOs: 251-303, and the HCDR3 sequence comprises or consists of the sequence set forth in SEQ ID NO: 12;

alternatively, the HCDR1 sequence comprises or consists of the sequence set forth in any one of SEQ ID NO: 235, SEQ ID NO: 238, SEQ ID NO: 241, and SEQ ID NOs: 304-317, the HCDR2 sequence comprises or

consists of the sequence set forth in any one of SEQ ID NOs: 318-358, and the HCDR3 sequence comprises or consists of the sequence set forth in SEQ ID NO: 16;

wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding CDR sequences, and the variants retain the binding affinity for Nectin-4.

**21.** The antibody-drug conjugate or the pharmaceutically acceptable salt or solvate thereof according to claim 20, wherein the sequences of the framework regions HFR1-HFR4 in the heavy chain variable region are set forth in SEQ ID NOs: 29-32, respectively; or wherein the sequence of the framework region HFR1 in the heavy chain variable region is set forth in the amino acid sequence of positions 1-30 of the sequence set forth in any one of SEQ ID NOs: 41-180, and the sequences of HFR2-HFR4 are set forth in SEQ ID NOs: 30-32, respectively.

**22.** The antibody-drug conjugate or the pharmaceutically acceptable salt or solvate thereof according to any one of claims 1-21, wherein the anti-Nectin-4 antibody or antigen-binding unit comprises a heavy chain variable region comprising or consisting of the sequence set forth in any one of SEQ ID NOs: 7, 11, 13, 15, 17, 19, 21, 23, 41-180, or a variant thereof;

wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding variable region sequences, and the variants retain the binding affinity for Nectin-4.

**23.** The antibody-drug conjugate or the pharmaceutically acceptable salt or solvate thereof according to any one of claims 20-22, wherein the amino acid sequence of the heavy chain constant region of the anti-Nectin-4 antibody is set forth in SEQ ID NO: 37; alternatively, the heavy chain of the anti-Nectin-4 antibody comprises the heavy chain variable region set forth in any one of SEQ ID NO: 7, SEQ ID NO: 11, SEQ ID NO: 13, SEQ ID NO: 15, SEQ ID NO: 17, SEQ ID NO: 19, SEQ ID NO: 21, SEQ ID NO: 23, and SEQ ID NOs: 41-180, and the heavy chain constant region set forth in SEQ ID NO: 37; alternatively, the amino acid sequence of the heavy chain of the anti-Nectin-4 antibody is set forth in any one of SEQ ID NO: 232, SEQ ID NO: 233, SEQ ID NO: 249, and SEQ ID NOs: 360-363.

**24.** The antibody-drug conjugate or the pharmaceutically acceptable salt or solvate thereof according to any one of claims 1-23, wherein the antigen-binding unit is selected from Fab, Fab', $F(ab')_2$, $F(ab)_2$, Fd, Fv, dAb, Fab/c, a complementarity determining region fragment, scFv, scFv multimers, disulfide-stabilized Fv, $(dsFv)_2$, bispecific dsFv, a diabody, a disulfide-stabilized diabody, a multispecific antibody formed from a portion of an antibody comprising one or more CDRs, a single domain antibody, a nanobody, a domain antibody, or a bivalent domain antibody.

**25.** The antibody-drug conjugate or the pharmaceutically acceptable salt or solvate thereof according to any one of claims 20-24, wherein the antibody or the antigen-binding unit further comprises the LCDR1, the LCDR2, and the LCDR3 contained in the light chain variable region set forth in SEQ ID NO: 25, for example, according to the Kabat numbering system, the LCDR1 sequence comprising or consisting of the sequence set forth in SEQ ID NO: 26, the LCDR2 sequence comprising or consisting of the sequence set forth in SEQ ID NO: 27, and the LCDR3 sequence comprising or consisting of the sequence set forth in SEQ ID NO: 28; alternatively, the sequences of the framework regions LFR1-LFR4 in the light chain variable region are set forth in SEQ ID NOs: 33-36, respectively; alternatively, the antibody or antigen-binding fragment comprises the light chain variable region set forth in SEQ ID NO: 25; alternatively, the antibody or antigen-binding fragment further comprises the light chain constant region set forth in SEQ ID NO: 38.

**26.** The antibody-drug conjugate or the pharmaceutically acceptable salt or solvate thereof according to any one of claims 1-25, wherein the anti-Nectin-4 antibody or antigen-binding fragment is derived from a bird and a mammal; or, the anti-Nectin-4 antibody is derived from a human, a mouse, a donkey, a rabbit, a goat, a camel, a llama, a horse, or a chicken origin.

**27.** The antibody-drug conjugate or the pharmaceutically acceptable salt or solvate thereof according to any one of claims 1-19, wherein the polypeptide specifically binding to Nectin-4 is selected from the group consisting of:

(1) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NOs: 181-195, SEQ ID NOs: 234-248, and SEQ ID NOs: 304-317, the HCDR2 sequence comprises or consists of the

sequence set forth in any one of SEQ ID NO: 9, SEQ ID NOs: 196-231, SEQ ID NOs: 251-303, and SEQ ID NOs: 318-358, and the HCDR3 sequence comprises or consists of the sequence set forth in any one of SEQ ID NO: 10, SEQ ID NO: 12, SEQ ID NO: 14, SEQ ID NO: 16, SEQ ID NO: 18, SEQ ID NO: 20, SEQ ID NO: 22, and SEQ ID NO: 24;
(2) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 10;
(3) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 181, SEQ ID NO: 185, SEQ ID NO: 186, SEQ ID NO: 187, SEQ ID NO: 194, and SEQ ID NOs: 234-248, the HCDR2 sequence comprises or consists of the sequence set forth in any one of SEQ ID NO: 9, SEQ ID NO: 202, and SEQ ID NOs: 251-303, and the HCDR3 sequence comprises or consists of the sequence set forth in SEQ ID NO: 12;
(4) a polypeptide, comprising sequences of an HCDR1, an HCDR2 and an HCDR3, or variants thereof, wherein the polypeptide serves as a portion of the anti-Nectin-4 antibody specifically binding to Nectin-4, wherein the HCDR1 sequence comprises or consists of the sequence set forth in any one of SEQ ID NO: 8 and SEQ ID NOs: 181-195, the HCDR2 sequence comprises or consists of the sequence set forth in any one of SEQ ID NO: 9 and SEQ ID NOs: 196-231, and the HCDR3 sequence comprises or consists of the sequence set forth in SEQ ID NO: 14;
(5) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in any one of SEQ ID NO: 8, SEQ ID NO: 235, SEQ ID NO: 238, SEQ ID NO: 241, and SEQ ID NOs: 304-317, the HCDR2 sequence comprises or consists of the sequence set forth in any one of SEQ ID NO: 9 and SEQ ID NOs: 318-358, and the HCDR3 sequence comprises or consists of the sequence set forth in SEQ ID NO: 16;
(6) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 18;
(7) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 20;
(8) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 22;
(9) a polypeptide, comprising sequences of an HCDR1, an HCDR2 and an HCDR3, or variants thereof, wherein the polypeptide serves as a portion of an anti-Nectin-4 antibody specifically binding to Nectin-4, wherein the HCDR1 sequence comprises or consists of the sequence set forth in SEQ ID NO: 8, the HCDR2 sequence comprises or consists of the sequence set forth in SEQ ID NO: 9, and the HCDR3 sequence comprises or consists of the sequence set forth in SEQ ID NO: 24;
(10) a polypeptide, comprising the sequence set forth in any one of SEQ ID NOs: 7, 11, 13, 15, 17, 19, 21, 23, 41-180 or a variant thereof, wherein the polypeptide serves as a portion of an anti-Nectin-4 antibody specifically binding to Nectin-4;

optionally, the polypeptide further comprises the sequences set forth in SEQ ID NO: 26, SEQ ID NO: 27 and SEQ ID NO: 28, or the sequence set forth in SEQ ID NO: 25 or a variant thereof,
wherein the variant sequences have 3, 2, or 1 amino acid difference (preferably a conservative amino acid substitution) from or at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identity to the corresponding sequences, and the variants retain the binding affinity for Nectin-4.

**28.** A pharmaceutical composition, comprising the antibody-drug conjugate or the pharmaceutically acceptable salt or solvate thereof according to any one of claims 1-27, a pharmaceutically acceptable carrier, excipient, and/or adjuvant material; and optionally, an additional anti-cancer drug.

**29.** Use of the antibody-drug conjugate or the pharmaceutically acceptable salt or solvate thereof according to any one of claims 1-27, or the pharmaceutical composition according to claim 28 in treating and/or preventing a disease or in preparing a medicament for treating and/or preventing a disease, wherein alternatively, the use further includes combined use with an additional anti-cancer drug; alternatively, the disease is a disease associated with the abnormal expression of Nectin-4; alternatively, the disease is a tumor expressing or overexpressing Nectin-4; alternatively, the disease is a cancer expressing or overexpressing Nectin-4; alternatively, the disease is a solid tumor or a hematological cancer; alternatively, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral cancer, renal pelvic carcinoma, ureteral cancer, colorectal cancer, colon cancer, and prostate cancer.

**30.** A method for treating a disease, comprising: administering to a patient in need an effective amount of the antibody-drug conjugate or the pharmaceutically acceptable salt or solvate thereof according to any one of claims 1-27, or the pharmaceutical composition according to claim 28; wherein alternatively, the disease is a disease associated with the abnormal expression of Nectin-4; alternatively, the disease is a tumor expressing or overexpressing Nectin-4; alternatively, the disease is a cancer expressing or overexpressing Nectin-4; alternatively, the disease is a solid tumor or a hematologic tumor; and alternatively, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urethral carcinoma, renal pelvis cancer, ureteral cancer, colorectal cancer, colon cancer, and prostate cancer.

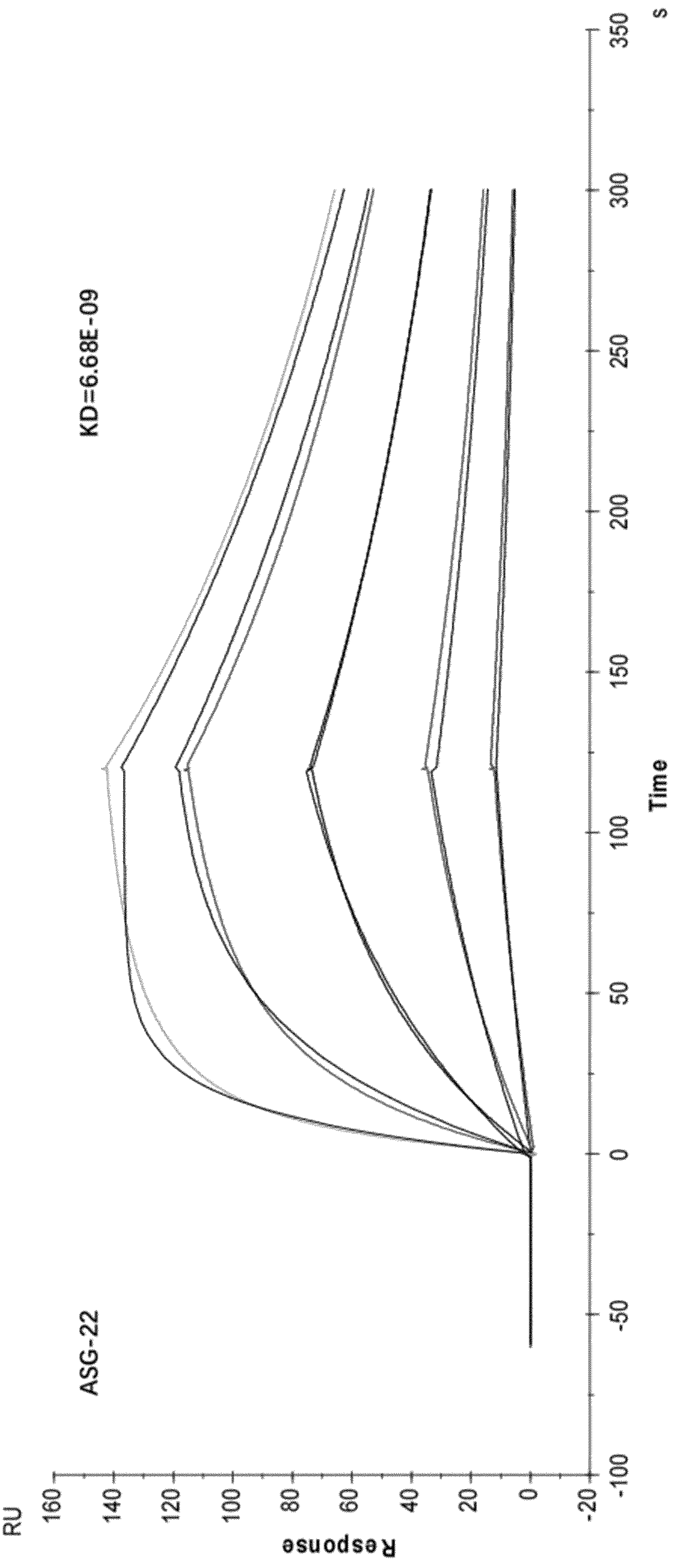
ASG-22
KD=6.68E-09
RU
Response
160
140
120
100
80
60
40
20
0
-20
-100
-50
0
50
100
150
200
250
300
350
s
Time

FIG. 1A

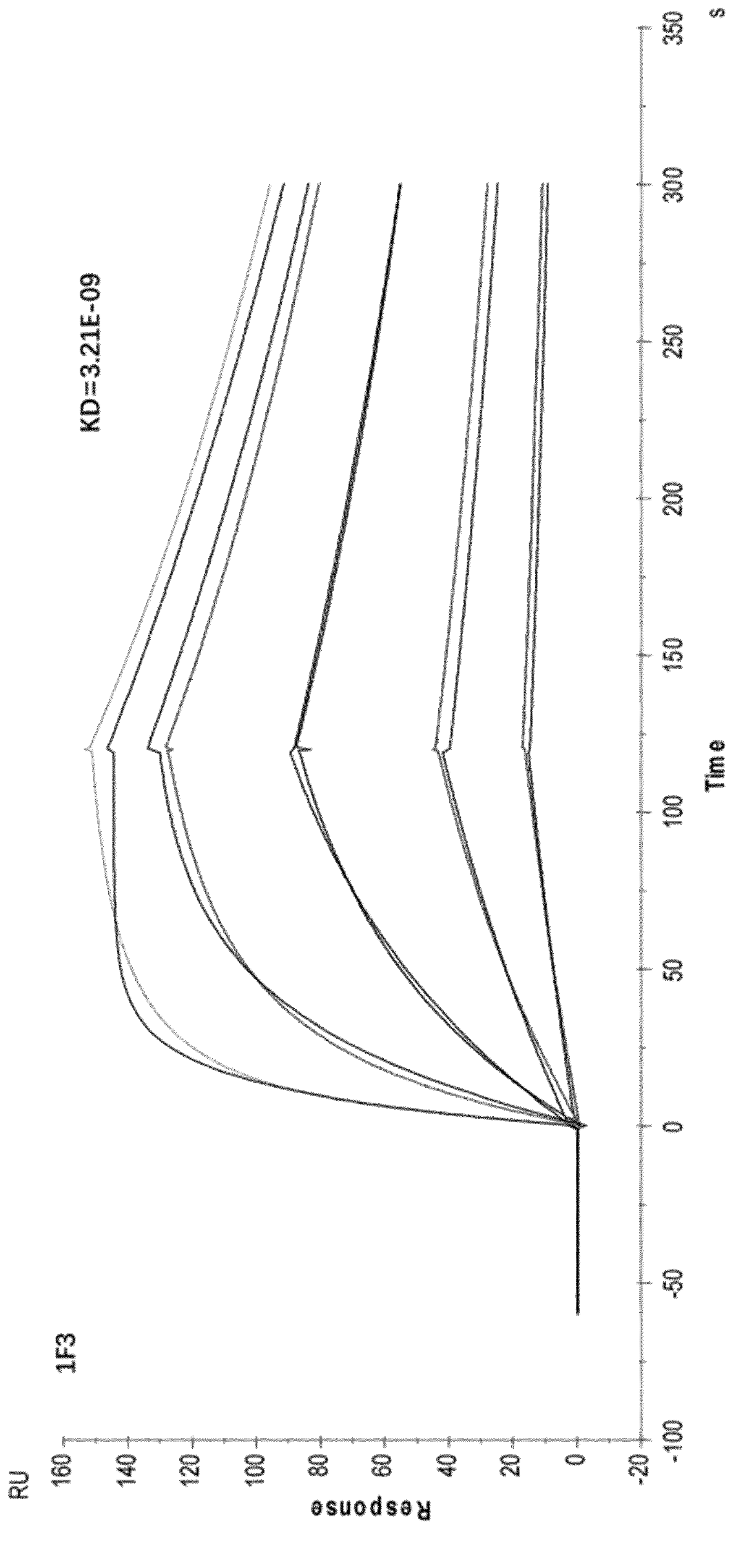
1F3
KD=3.21E-09
RU
Response
160
140
120
100
80
60
40
20
0
-20
-100
-50
0
50
100
150
200
250
300
350
s
Time

FIG. 1B

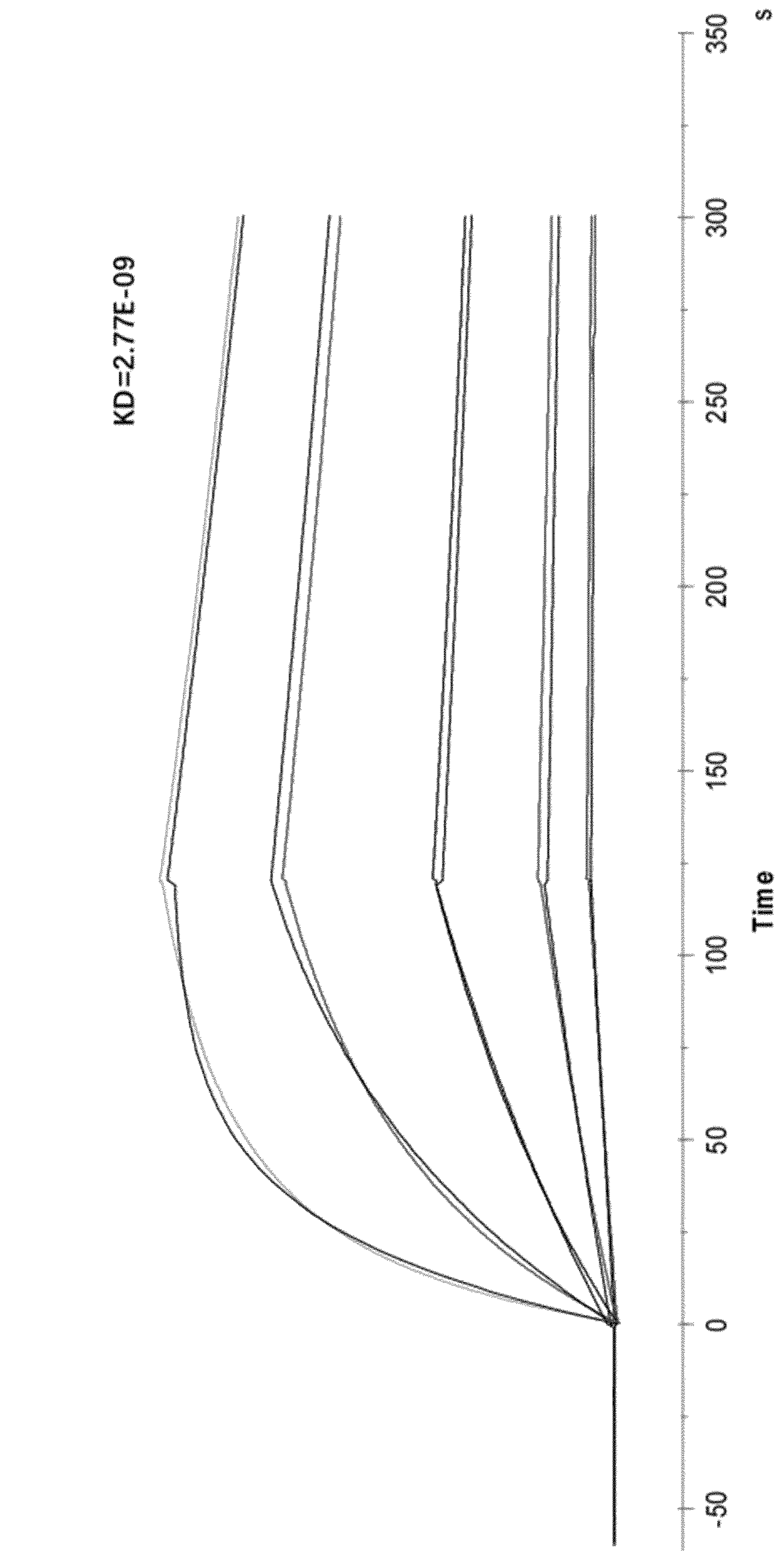
10F4
KD=2.77E-09
RU
Response
Time
s
160
140
120
100
80
60
40
20
0
-20
-100
-50
0
50
100
150
200
250
300
350

FIG. 1C

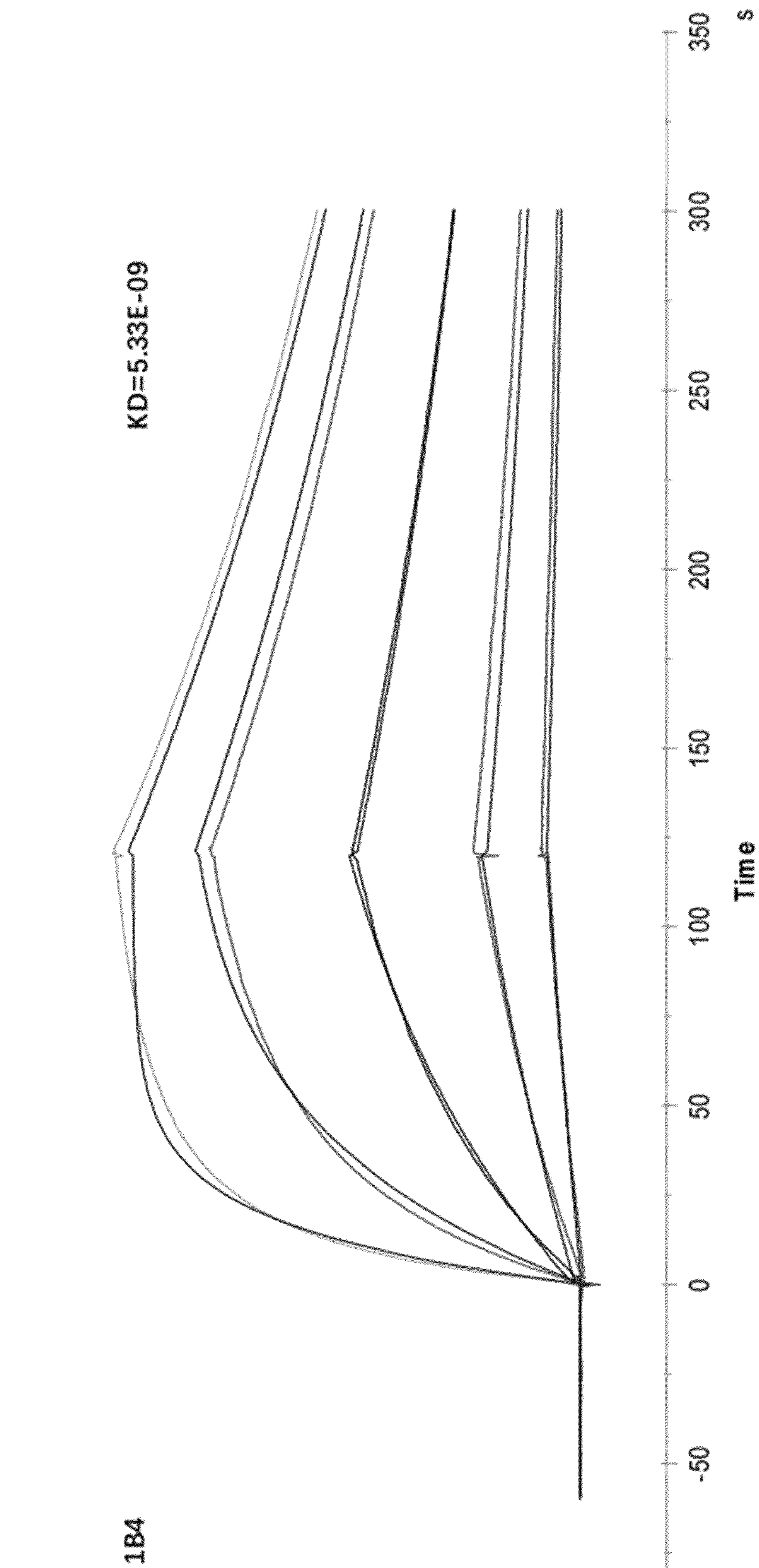
1B4
KD=5.33E-09
RU
Response
60
50
40
30
20
10
0
-10
-100
-50
0
50
100
150
200
250
300
350
s
Time

FIG. 1D

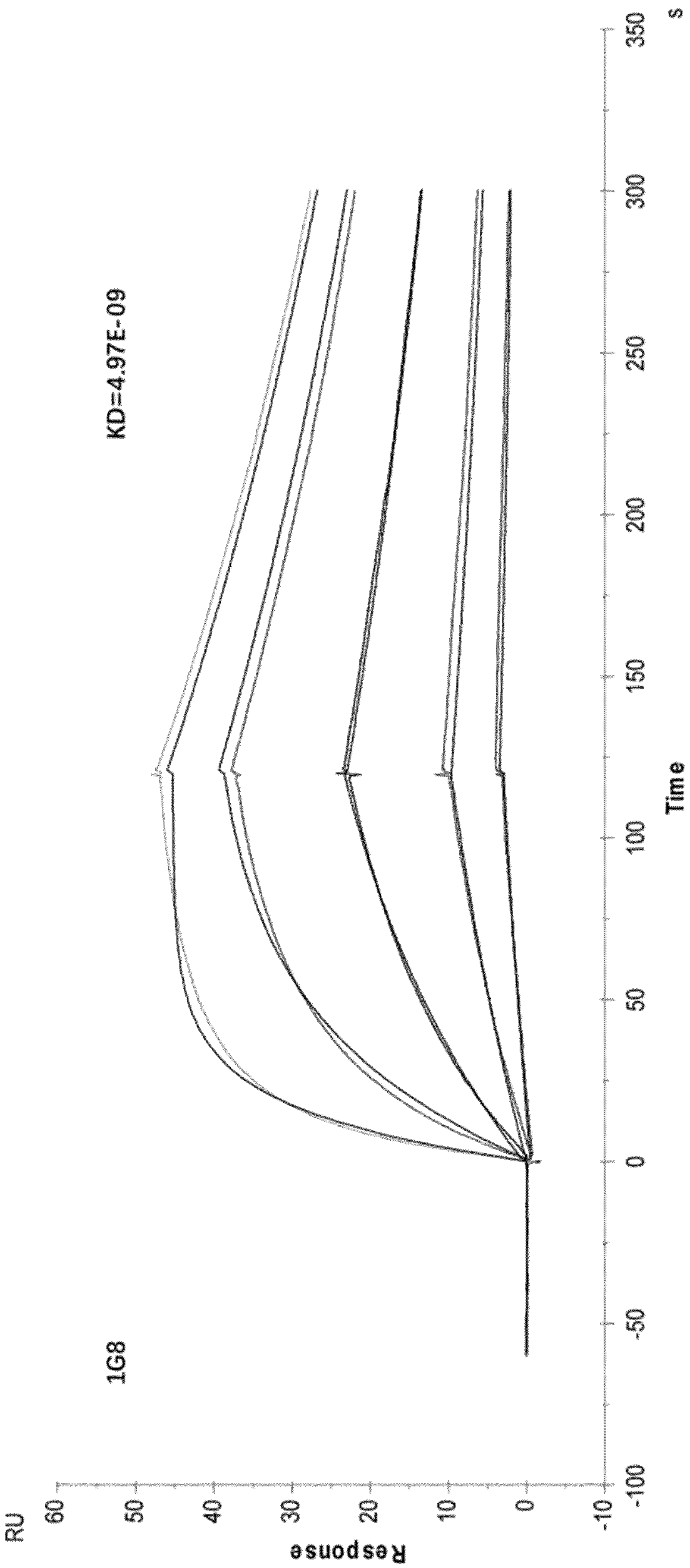
KD=4.97E-09
1G8
RU
Response
60
50
40
30
20
10
0
-10
-100
-50
0
50
100
150
200
250
300
350
s
Time

FIG. 1E

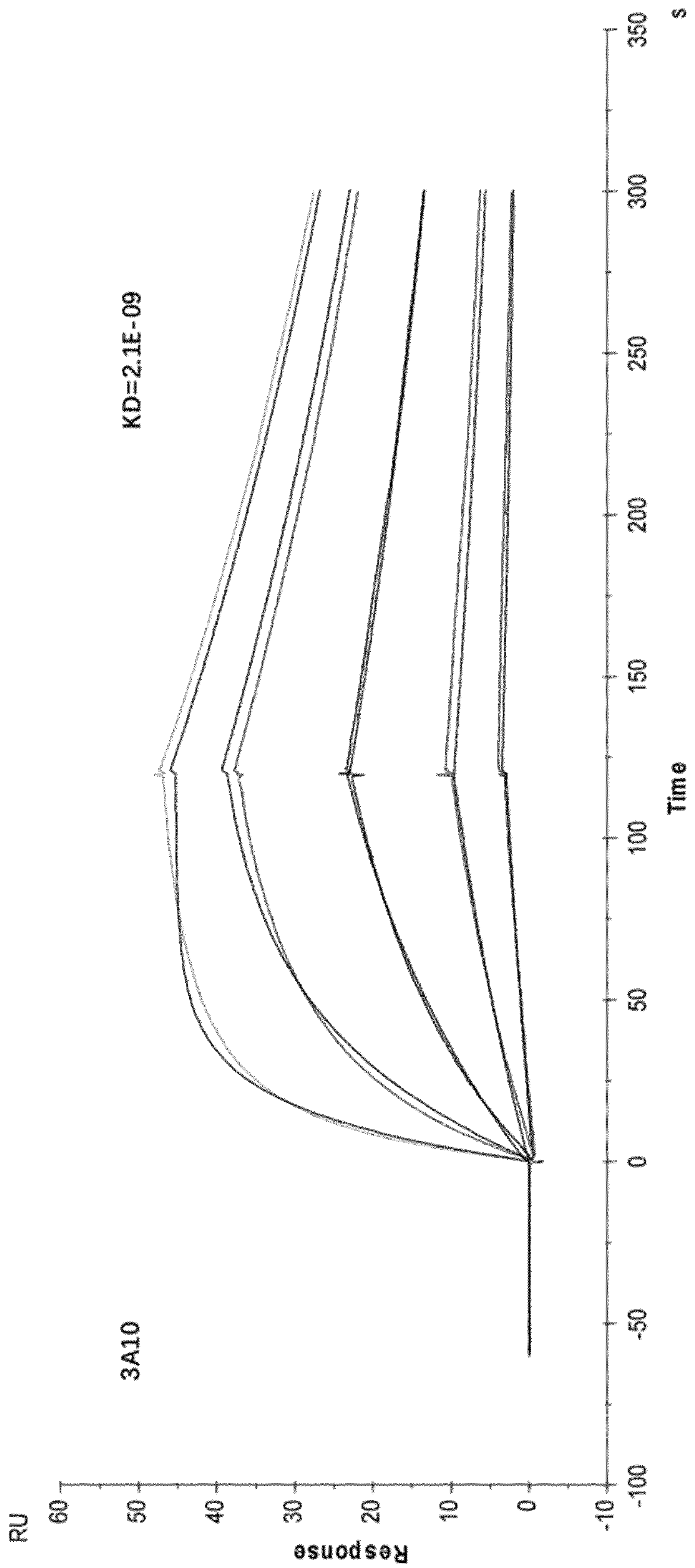
3A10
KD=2.1E-09
RU
Response
60
50
40
30
20
10
0
-10
-100
-50
0
50
100
150
200
250
300
350
s
Time

FIG. 1F

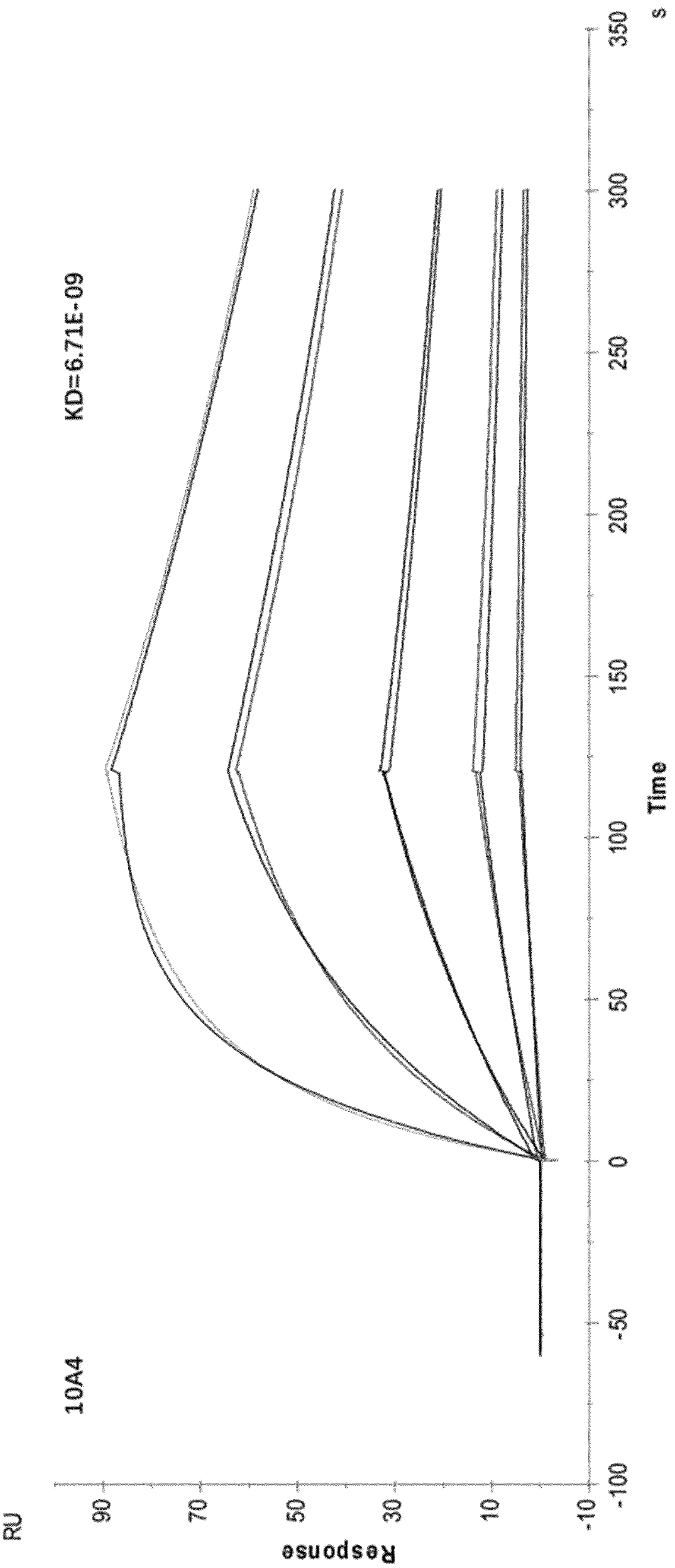
10A4
KD=6.71E-09
RU
Response
90
70
50
30
10
-10
-100
-50
0
50
100
150
200
250
300
350
s
Time

FIG. 1G

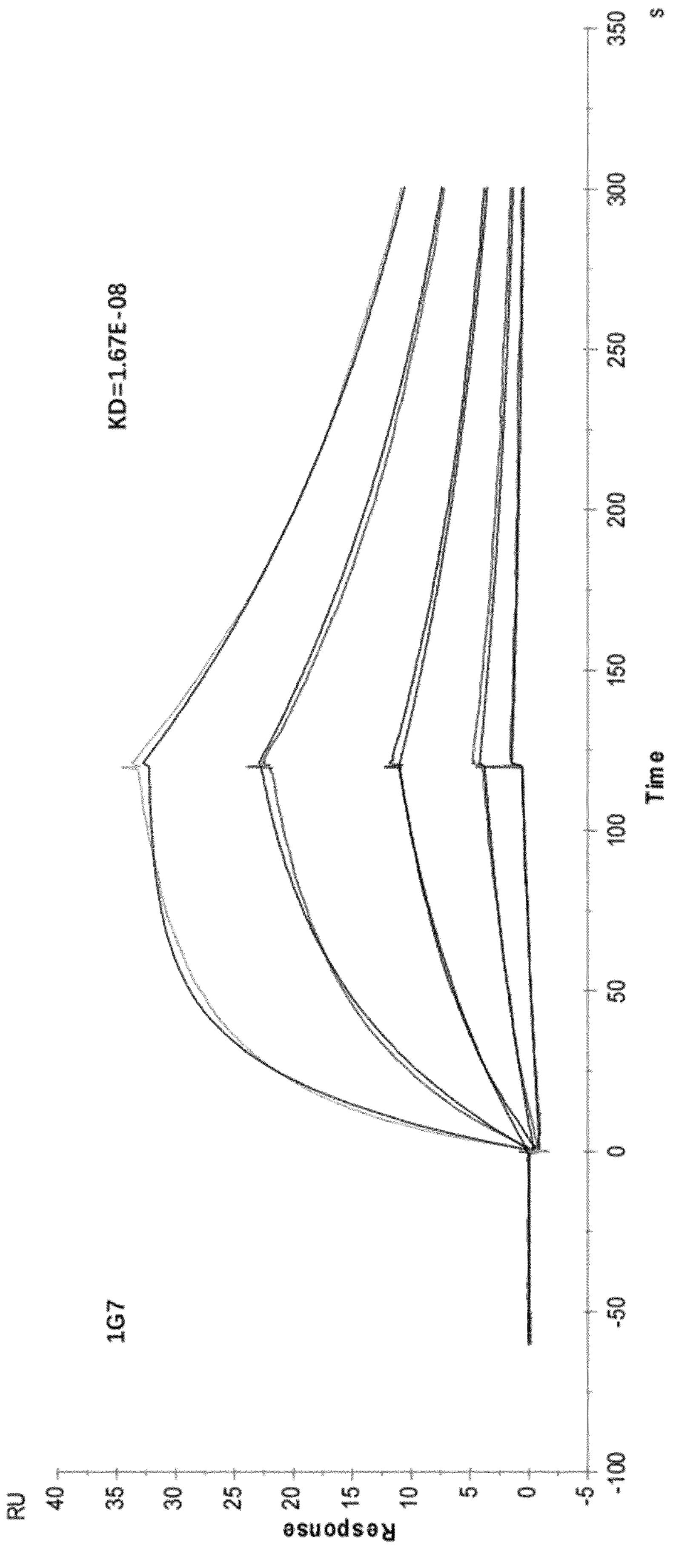
KD=1.67E-08
1G7
Response
RU
40
35
30
25
20
15
10
5
0
-5
Time
s
-100
-50
0
50
100
150
200
250
300
350

FIG. 1H

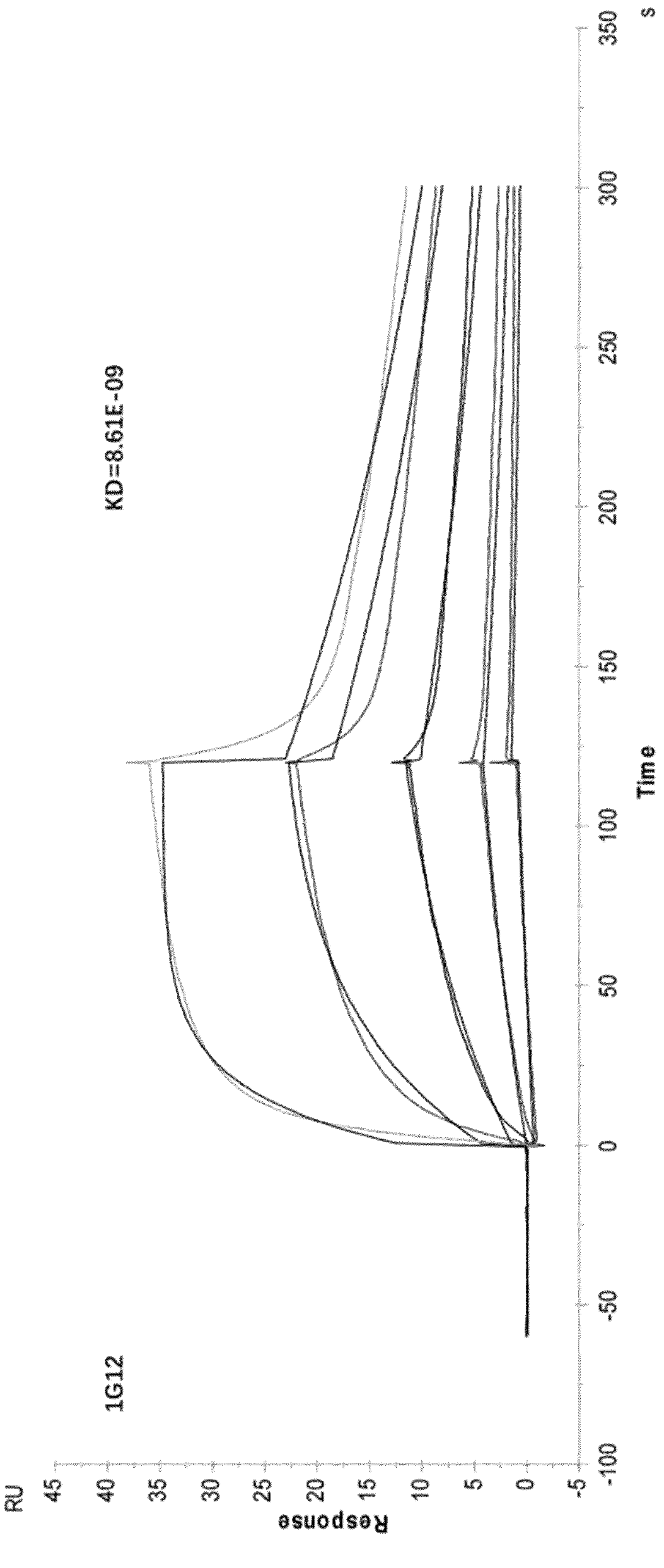
1G12
KD=8.61E-09
Response
RU
Time
s
45
40
35
30
25
20
15
10
5
0
-5
-100
-50
0
50
100
150
200
250
300
350

FIG. 1I

# DRAWINGS OF THE SPECIFICATION

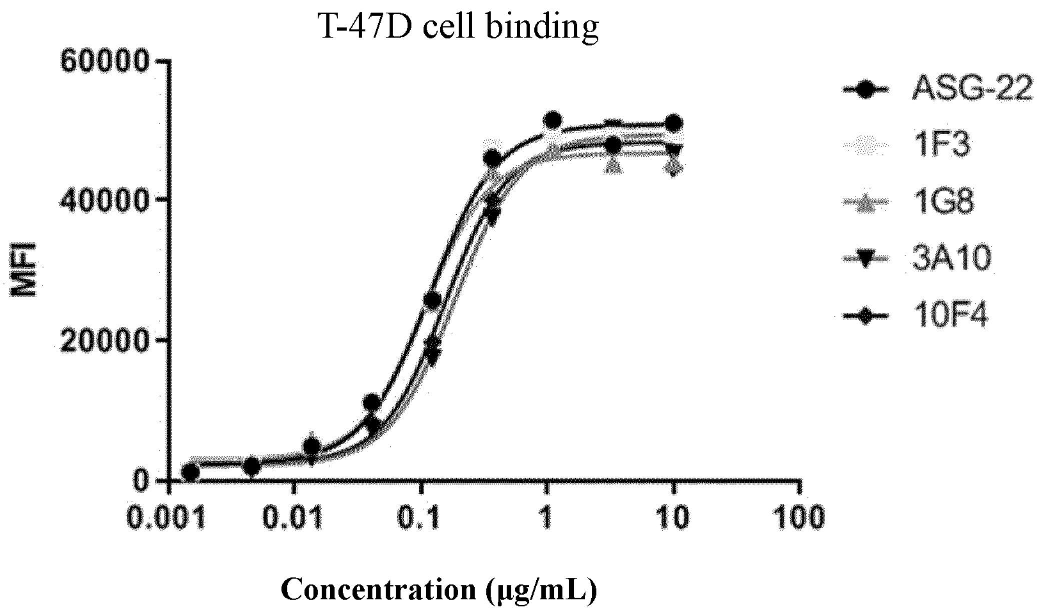


FIG. 2

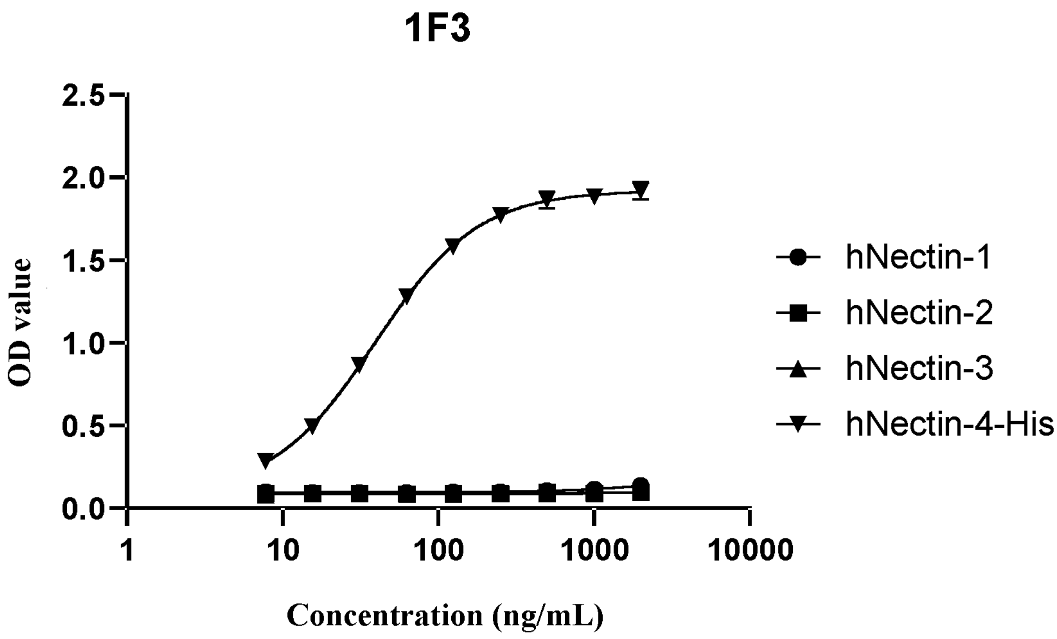
1F3
2.5
2.0
1.5
1.0
0.5
0.0
OD value
1
10
100
1000
10000
Concentration (ng/mL)
hNectin-1
hNectin-2
hNectin-3
hNectin-4-His

FIG. 3A

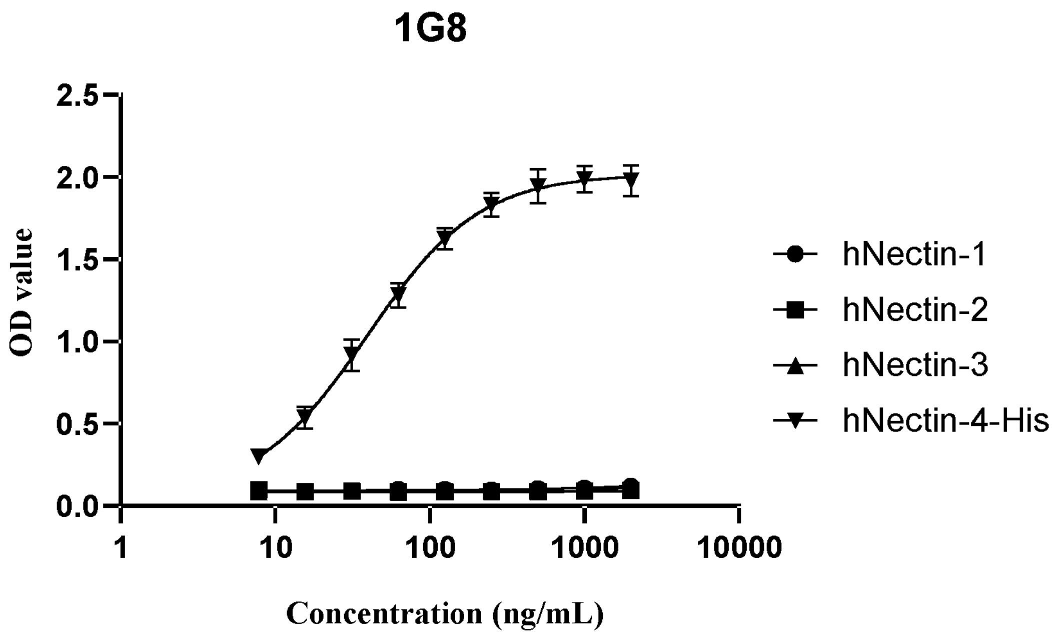
1G8
2.5
2.0
1.5
1.0
0.5
0.0
OD value
1
10
100
1000
10000
Concentration (ng/mL)
hNectin-1
hNectin-2
hNectin-3
hNectin-4-His

FIG. 3B

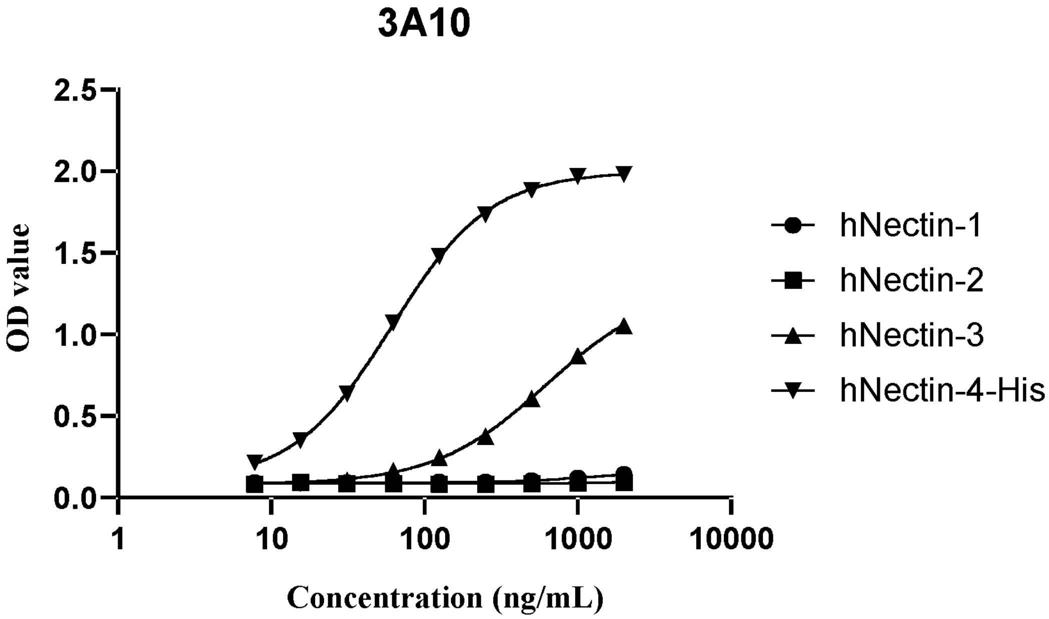
3A10
2.5
2.0
1.5
1.0
0.5
0.0
OD value
1
10
100
1000
10000
Concentration (ng/mL)
hNectin-1
hNectin-2
hNectin-3
hNectin-4-His

FIG. 3C

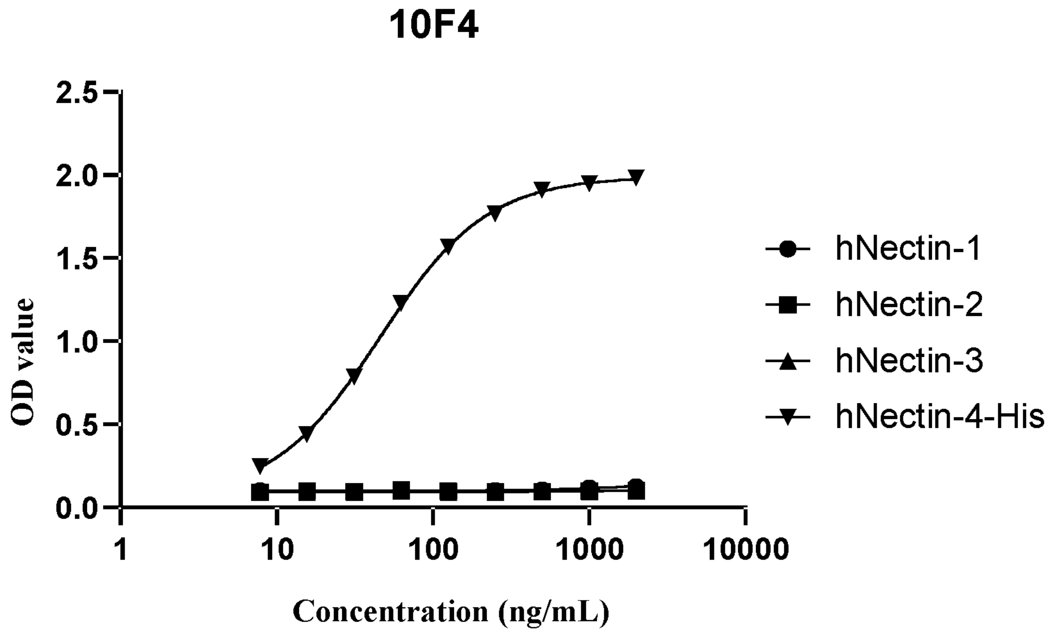
10F4
2.5
2.0
1.5
1.0
0.5
0.0
OD value
1
10
100
1000
10000
Concentration (ng/mL)
hNectin-1
hNectin-2
hNectin-3
hNectin-4-His

FIG. 3D

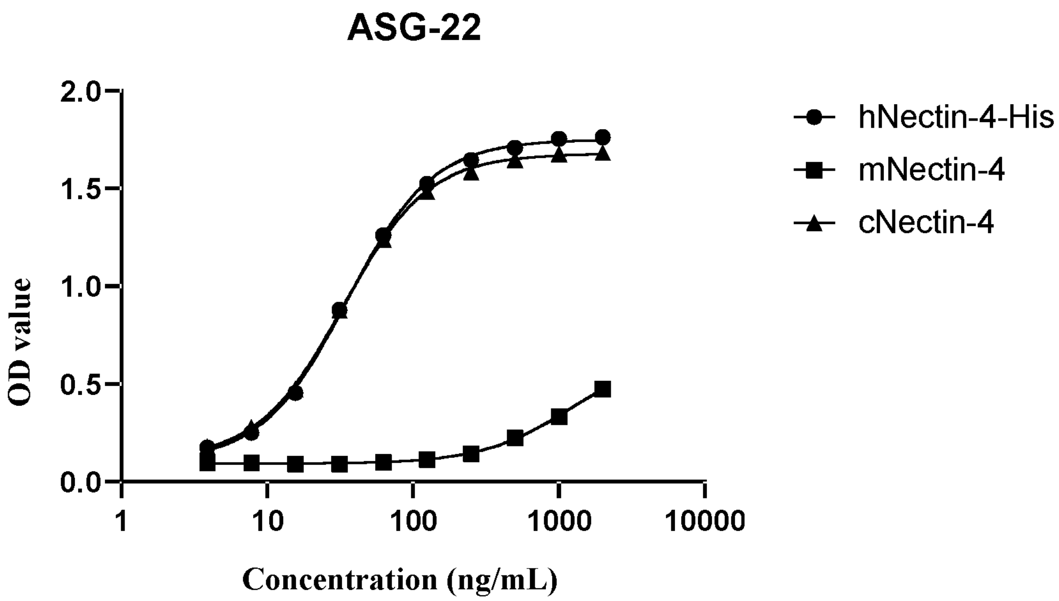
ASG-22
OD value
Concentration (ng/mL)
hNectin-4-His
mNectin-4
cNectin-4
2.0
1.5
1.0
0.5
0.0
1
10
100
1000
10000

FIG. 4A

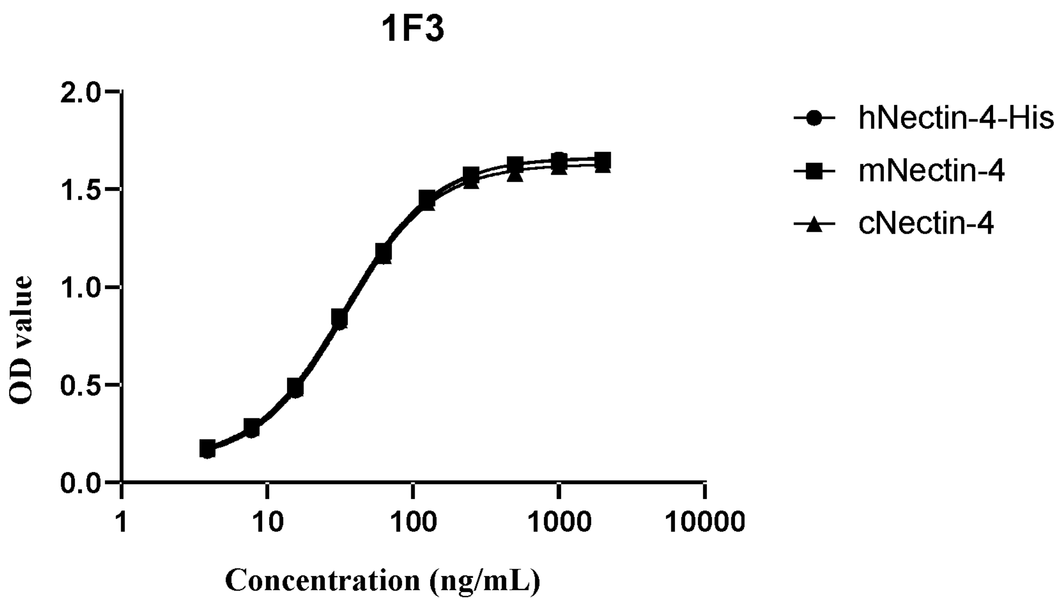
1F3
OD value
Concentration (ng/mL)
hNectin-4-His
mNectin-4
cNectin-4
2.0
1.5
1.0
0.5
0.0
1
10
100
1000
10000

FIG. 4B

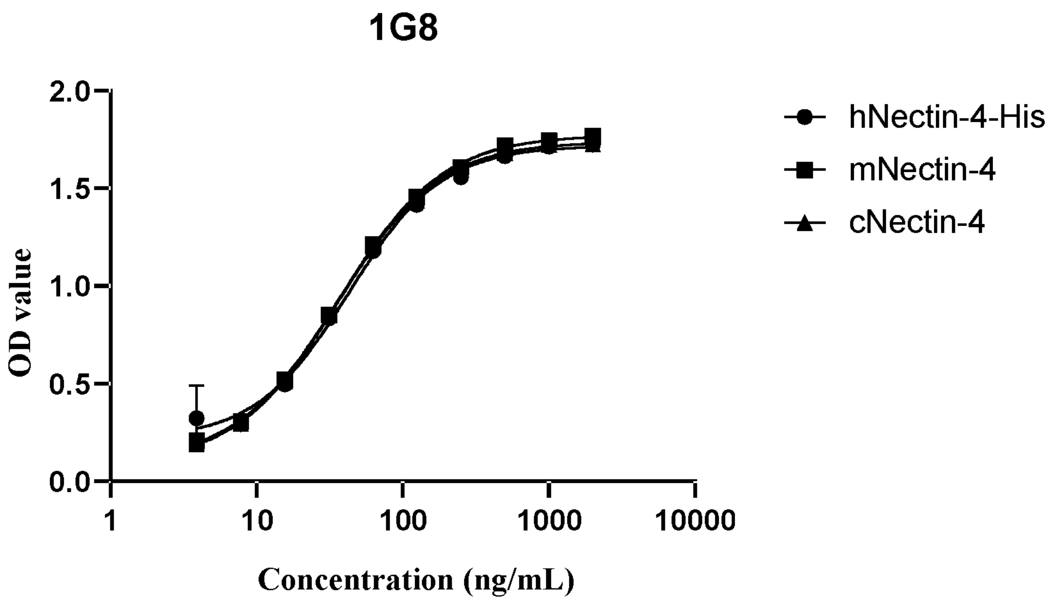
1G8
2.0
1.5
1.0
0.5
0.0
OD value
1
10
100
1000
10000
Concentration (ng/mL)
hNectin-4-His
mNectin-4
cNectin-4

FIG. 4C

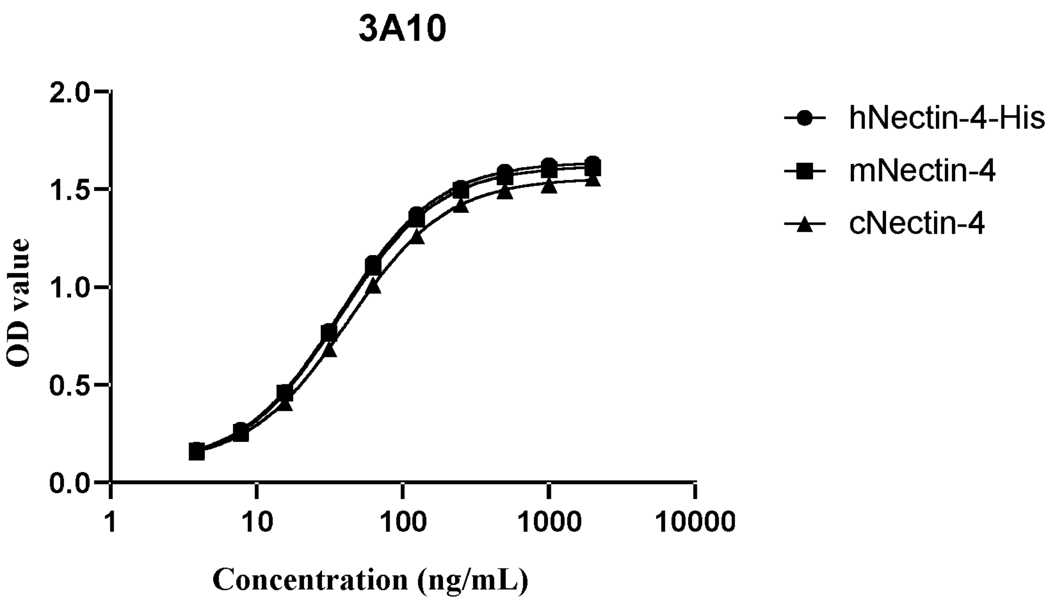
3A10
2.0
1.5
1.0
0.5
0.0
OD value
1
10
100
1000
10000
Concentration (ng/mL)
hNectin-4-His
mNectin-4
cNectin-4

FIG. 4D

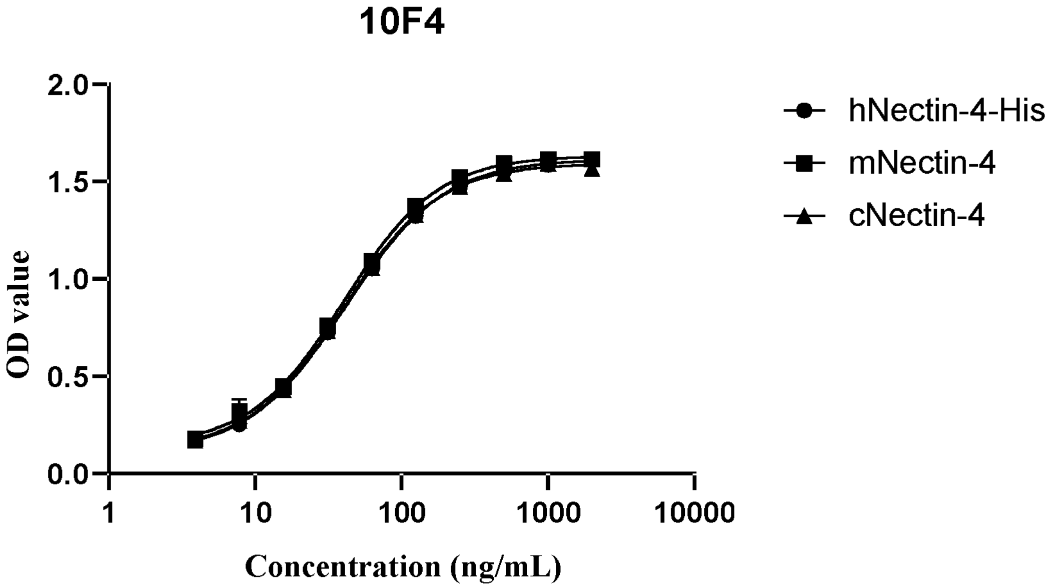
10F4
2.0
1.5
1.0
0.5
0.0
OD value
1
10
100
1000
10000
Concentration (ng/mL)
hNectin-4-His
mNectin-4
cNectin-4

FIG. 4E

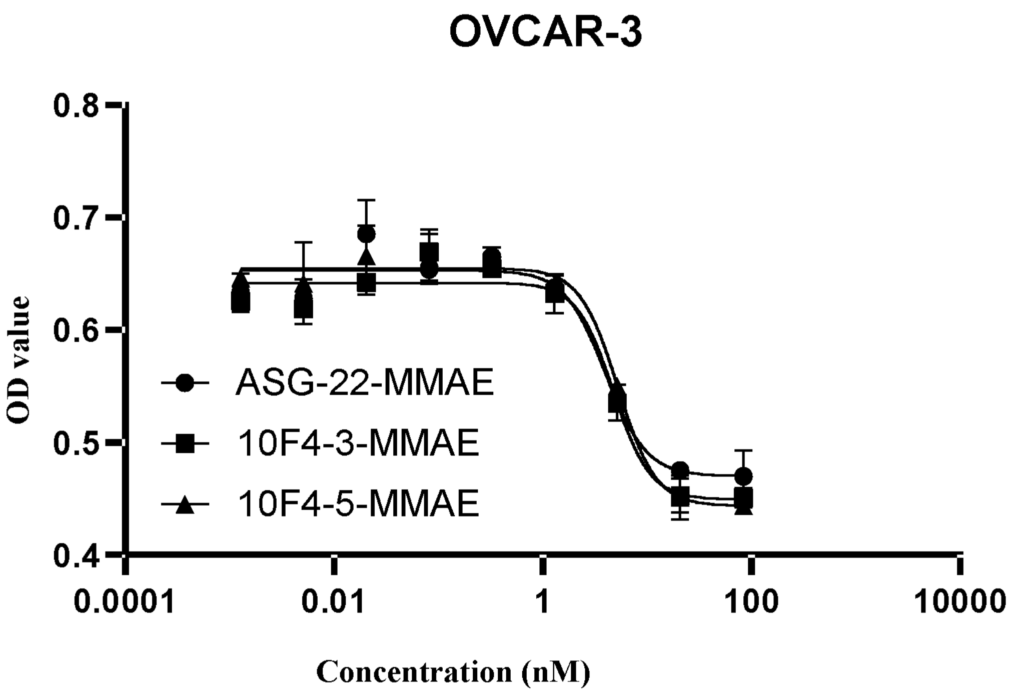
OVCAR-3
0.8
0.7
0.6
0.5
0.4
OD value
ASG-22-MMAE
10F4-3-MMAE
10F4-5-MMAE
0.0001
0.01
1
100
10000
Concentration (nM)

FIG. 5A

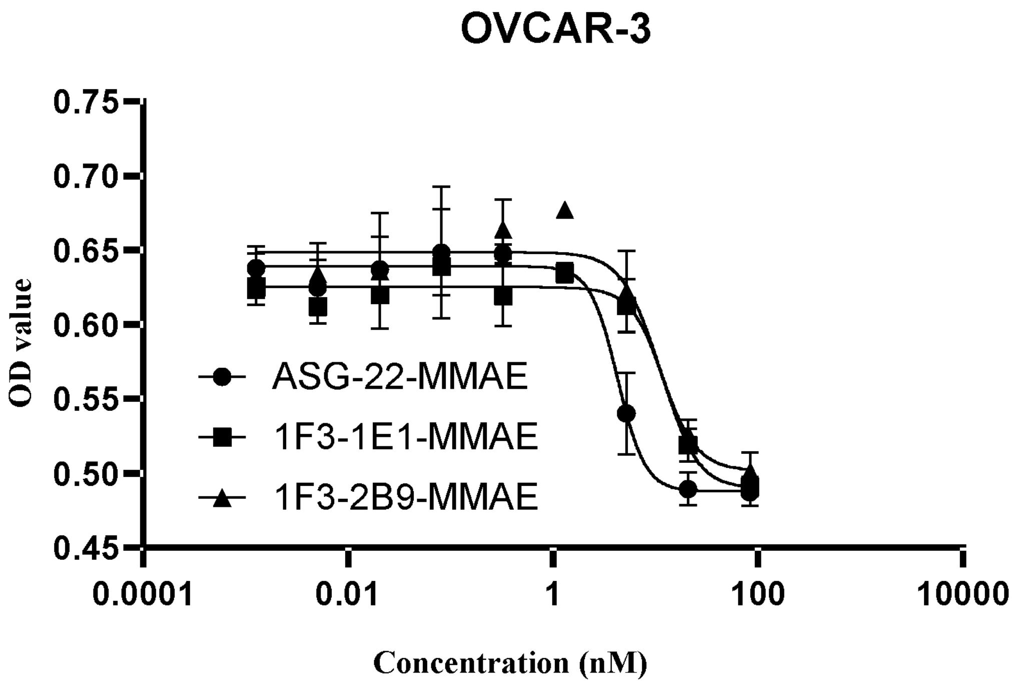
OVCAR-3
0.75
0.70
0.65
0.60
0.55
0.50
0.45
OD value
ASG-22-MMAE
1F3-1E1-MMAE
1F3-2B9-MMAE
0.0001
0.01
1
100
10000
Concentration (nM)

FIG. 5B

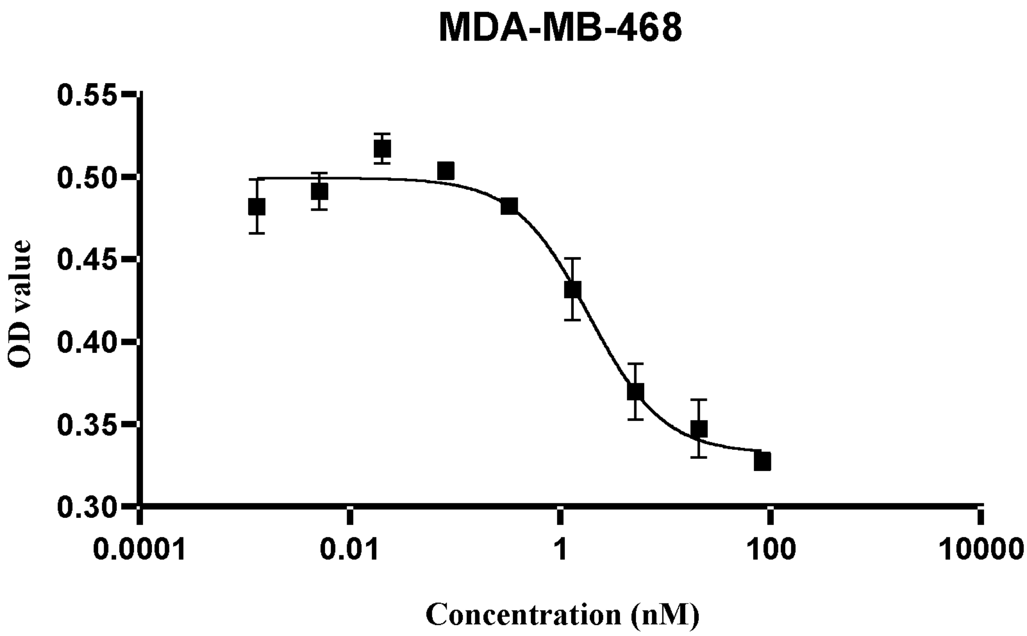
MDA-MB-468
0.55
0.50
0.45
0.40
0.35
0.30
OD value
0.0001
0.01
1
100
10000
Concentration (nM)

FIG. 5C

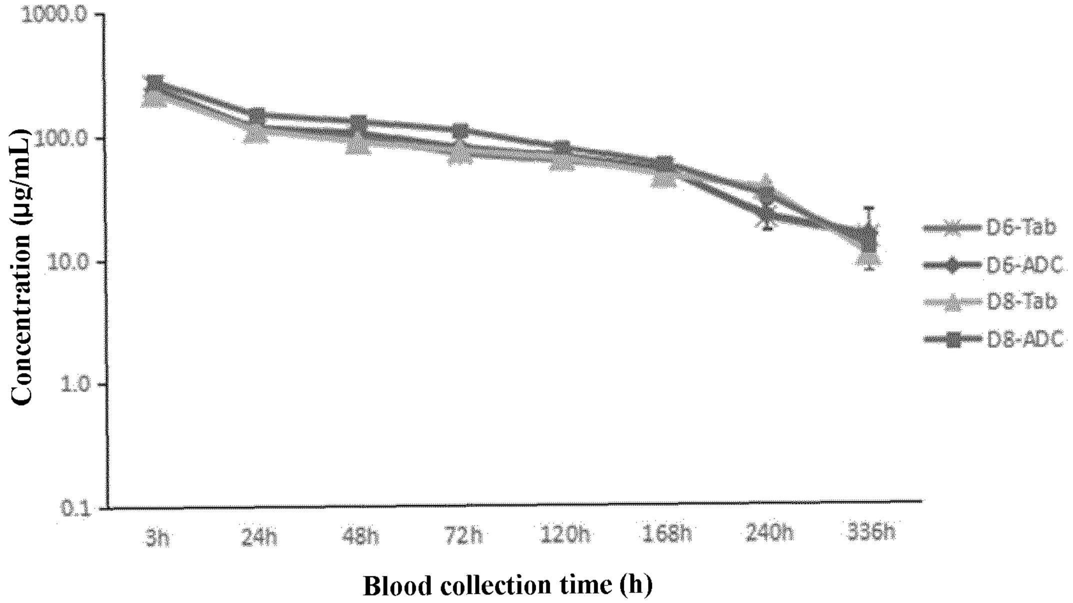

1000.0
100.0
10.0
1.0
0.1
Concentration (μg/mL)
3h
24h
48h
72h
120h
168h
240h
336h
Blood collection time (h)
D6-Tab
D6-ADC
D8-Tab
D8-ADC


FIG. 6

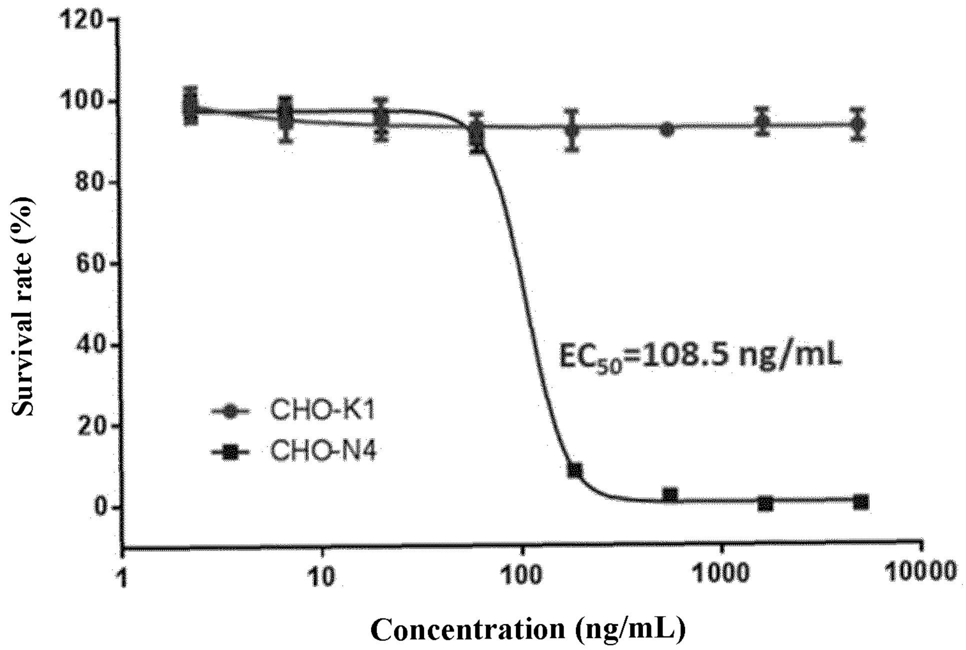

120
100
80
60
40
20
0
Survival rate (%)
EC50=108.5 ng/mL
CHO-K1
CHO-N4
1
10
100
1000
10000
Concentration (ng/mL)


FIG. 7A

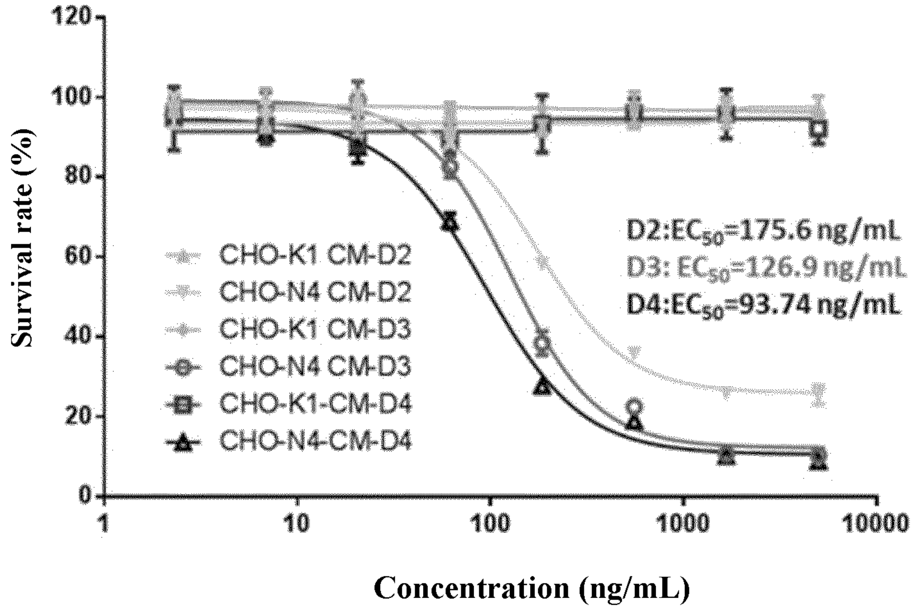
Survival rate (%)
Concentration (ng/mL)
CHO-K1 CM-D2
CHO-N4 CM-D2
CHO-K1 CM-D3
CHO-N4 CM-D3
CHO-K1-CM-D4
CHO-N4-CM-D4
D2:EC50=175.6 ng/mL
D3: EC50=126.9 ng/mL
D4:EC50=93.74 ng/mL

FIG. 7B

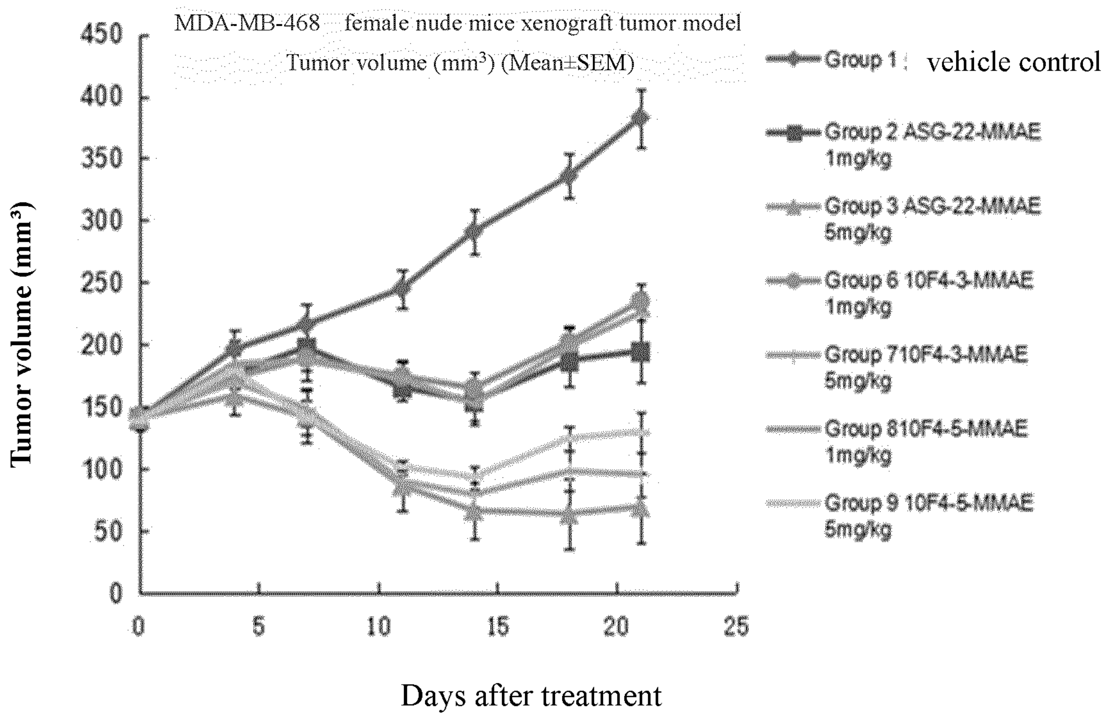
MDA-MB-468 female nude mice xenograft tumor model
Tumor volume (mm3) (Mean±SEM)
Tumor volume (mm3)
Days after treatment
Group 1 vehicle control
Group 2 ASG-22-MMAE 1mg/kg
Group 3 ASG-22-MMAE 5mg/kg
Group 6 10F4-3-MMAE 1mg/kg
Group 710F4-3-MMAE 5mg/kg
Group 810F4-5-MMAE 1mg/kg
Group 9 10F4-5-MMAE 5mg/kg

FIG. 8

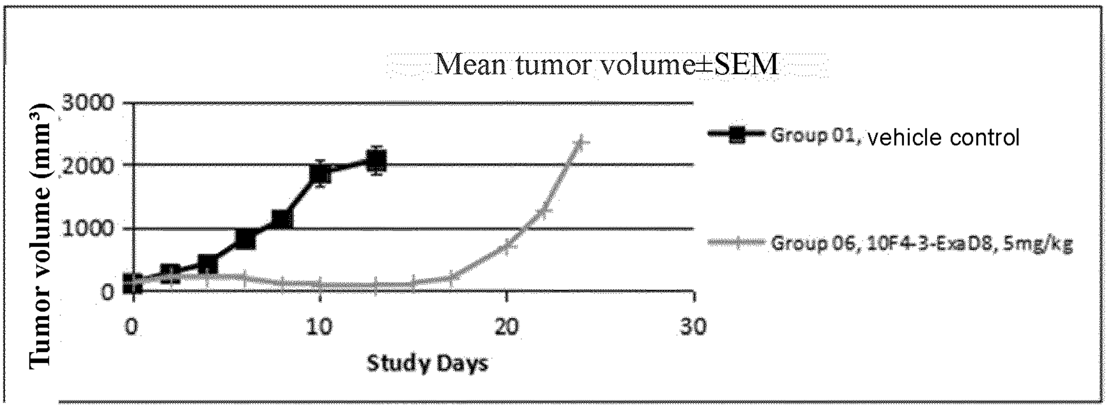
Mean tumor volume±SEM
Tumor volume (mm³)
3000
2000
1000
0
0
10
20
30
Study Days
Group 01, vehicle control
Group 06, 10F4-3-ExaD8, 5mg/kg

FIG. 9

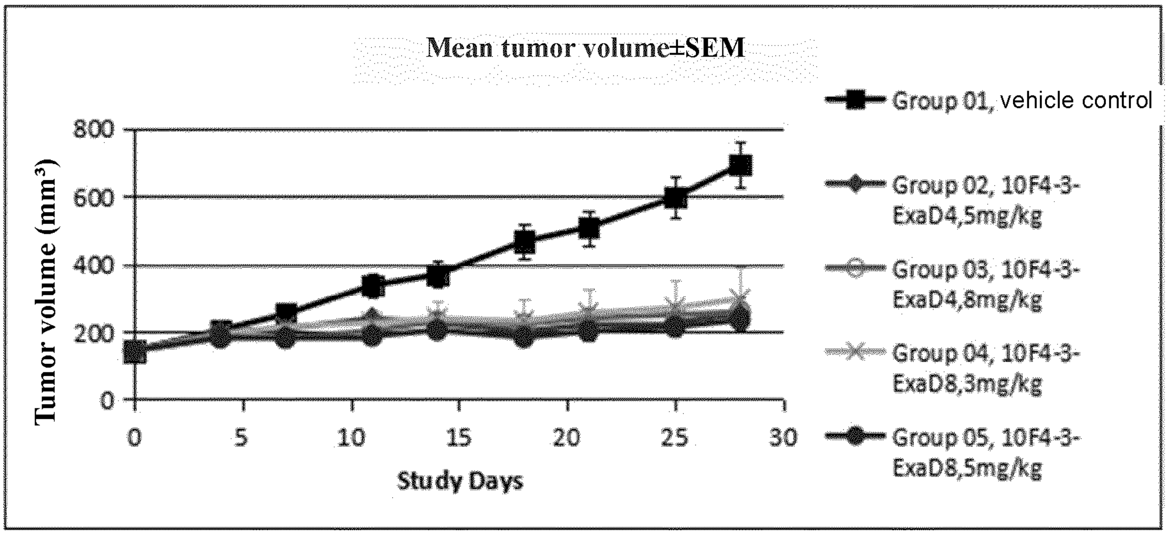
Mean tumor volume±SEM
Tumor volume (mm³)
800
600
400
200
0
0
5
10
15
20
25
30
Study Days
Group 01, vehicle control
Group 02, 10F4-3-ExaD4,5mg/kg
Group 03, 10F4-3-ExaD4,8mg/kg
Group 04, 10F4-3-ExaD8,3mg/kg
Group 05, 10F4-3-ExaD8,5mg/kg

FIG. 10

**INTERNATIONAL SEARCH REPORT**

International application No. **PCT/CN2023/107301**

**A. CLASSIFICATION OF SUBJECT MATTER**

A61K47/68(2017.01)i; A61K47/65(2017.01)i; C07K16/28(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science, baidu, Pubmed, NCBI, STN on Web, genbank, blast, 中文专利序列检索系统, China Patent Biological Sequence Search System, 百奥泰生物制药股份有限公司, 梅星星, Nectin-4, 偶联, 缀合, conjugates, 树碱, 依喜替康, exatecan, 框架, Fr1-4, 聚乙二醇, 连接子, linker, PEG, SEQ ID NOs: 7-38, 41-358

**C. DOCUMENTS CONSIDERED TO BE RELEVANT**

| Category* | Citation of document, with indication, where appropriate, of the relevant passages | Relevant to claim No. |
|---|---|---|
| PX | WO 2022253284 A1 (BIO-THERA SOLUTIONS, LTD.) 08 December 2022 (2022-12-08) claims 1-35 | 1-19, 28-30 |
| PY | WO 2022253284 A1 (BIO-THERA SOLUTIONS, LTD.) 08 December 2022 (2022-12-08) claims 1-35 | 20-30 |
| PX | WO 2022228406 A1 (JIANGSU HENGRUI PHARMACEUTICALS CO., LTD. et al.) 03 November 2022 (2022-11-03) claims 13-24 | 1, 28-30 |
| PY | WO 2022228406 A1 (JIANGSU HENGRUI PHARMACEUTICALS CO., LTD. et al.) 03 November 2022 (2022-11-03) claims 1-8 | 20-30 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 October 2023** | **19 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No. **PCT/CN2023/107301**

C. DOCUMENTS CONSIDERED TO BE RELEVANT

| Category* | Citation of document, with indication, where appropriate, of the relevant passages | Relevant to claim No. |
|---|---|---|
| X | WO 2021151984 A1 (INNATE PHARMA) 05 August 2021 (2021-08-05) page 11, lines 10-26, page 35, line 1 to page 38, line 13, page 46, line 5 to page 58, line 8, page 53, line 15, page 64, paragraph 2, page 66, line 5, page 70, line 15, page 73, line 30 to page 74, line 35, page 86, paragraph 2 from the bottom, page 93, line 20, and page 93, paragraph 1 | 1-30 |
| X | WO 2022057651 A1 (SICHUAN KELUN-BIOTECH BIOPHARMACEUTICAL CO., LTD.) 24 March 2022 (2022-03-24) claims 1-29 | 1, 28-30 |
| Y | WO 2022057651 A1 (SICHUAN KELUN-BIOTECH BIOPHARMACEUTICAL CO., LTD.) 24 March 2022 (2022-03-24) claims 1-29 | 2-30 |
| Y | US 2021093733 A1 (OBI PHARMA, INC.) 01 April 2021 (2021-04-01) description, page 58 | 2-30 |
| Y | WO 2022076767 A1 (AGENSYS, INC.) 14 April 2022 (2022-04-14) abstract, and claims 30-34 | 20-30 |
| A | WO 2021257525 A1 (BIOATLA, INC.) 23 December 2021 (2021-12-23) entire document | 1-30 |

Form PCT/ISA/210 (second sheet) (July 2022)

**INTERNATIONAL SEARCH REPORT**

International application No.
**PCT/CN2023/107301**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/107301**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29, 30**
because they relate to subject matter not required to be searched by this Authority, namely:

Claims 29 (in part) and 30 relate to a method for treatment of a disease (PCT Rule 39.1(iv)); however, the search opinion is still provided on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/107301**

| Patent document cited in search report | | | Publication date (day/month/year) | Patent family member(s) | | | Publication date (day/month/year) |
|---|---|---|---|---|---|---|---|
| WO | 2022253284 | A1 | 08 December 2022 | None | | | |
| WO | 2022228406 | A1 | 03 November 2022 | TW | 202302649 | A | 16 January 2023 |
| WO | 2021151984 | A1 | 05 August 2021 | EP | 4096717 | A1 | 07 December 2022 |
| | | | | US | 2023099149 | A1 | 30 March 2023 |
| | | | | CA | 3160557 | A1 | 05 August 2021 |
| | | | | AU | 2021213421 | A1 | 23 June 2022 |
| | | | | JP | 2023512036 | A | 23 March 2023 |
| WO | 2022057651 | A1 | 24 March 2022 | PE | 20230844 | A1 | 23 May 2023 |
| | | | | ECSP | 23019525 | A | 31 May 2023 |
| | | | | DOP | 2023000054 | A | 15 June 2023 |
| | | | | AU | 2021345721 | A1 | 20 April 2023 |
| | | | | IL | 301387 | A | 01 May 2023 |
| | | | | CO | 2023003210 | A2 | 17 April 2023 |
| | | | | EP | 4215546 | A1 | 26 July 2023 |
| | | | | BR | 112022026611 | A2 | 11 April 2023 |
| | | | | KR | 20230069111 | A | 18 May 2023 |
| | | | | CA | 3194765 | A1 | 24 March 2022 |
| US | 2021093733 | A1 | 01 April 2021 | None | | | |
| WO | 2022076767 | A1 | 14 April 2022 | KR | 20230106607 | A | 13 July 2023 |
| | | | | IL | 302006 | A | 01 June 2023 |
| | | | | TW | 202228788 | A | 01 August 2022 |
| | | | | CA | 3198359 | A1 | 14 April 2022 |
| | | | | EP | 4225379 | A1 | 16 August 2023 |
| | | | | AU | 2021356542 | A1 | 25 May 2023 |
| WO | 2021257525 | A1 | 23 December 2021 | US | 2023235054 | A1 | 27 July 2023 |
| | | | | TW | 202204425 | A | 01 February 2022 |
| | | | | CA | 3182395 | A1 | 23 December 2021 |
| | | | | KR | 20230038711 | A | 21 March 2023 |
| | | | | AU | 2021293183 | A1 | 16 February 2023 |
| | | | | JP | 2023531185 | A | 21 July 2023 |
| | | | | IL | 298903 | A | 01 February 2023 |
| | | | | EP | 4168453 | A1 | 26 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5892019 A
- US 5168062 A
- US 4510245 A
- US 4968615 A
- US 4399216 A
- US 4634665 A
- US 5179017 A
- US 4816397 A


### Non-patent literature cited in the description


- **KABAT, E. et al.** Sequences of Proteins of Immunological Interest. U.S. Department of Health and Human Services, 1983
- **CHOTHIA** ; **LESK**. *J. Mol. Biol.*, 1987, vol. 196, 901-917
- **WINNAKER**. From Genes to Clones. Verlagsgesellschaft, 1987
- **KABAT et al.** Sequences of Proteins of Immunological Interest. U.S. Dept. of Health and Human Services, 1983
- **CHOTHIA et al.** *J. Mol. Biol.*, 1987, vol. 196, 901-917
- **KABAT et al.** Sequence of Proteins of Immunological Interest. U.S. Dept. of Health and Human Services, 1983
- **MALMQVIST, M.** *Nature*, 1993, vol. 361, 186-87
- **DAVIES et al.** *Annual Rev Biochem*, 1990, vol. 59, 439-473
- Immunology-A Synthesis. Sinauer Associates, 1991
- Proteins: Structures and Molecular Principles. W. H. Freeman and Company, 1984
- Introduction to Protein Structure. Garland Publishing, 1991
- **THORNTON et al.** *Nature*, 1991, vol. 354, 105
- **OUYANG**. *J. Methods Mol Biol*, 2013, vol. 1045, 275-83
- **WU** ; **WU**. *J. Biol. Chem.*, 1987, vol. 262, 4429-4432
- **CHOTHIA et al.** *J. Mol. Biol.*, 1998, vol. 278, 457-479
- Recombinant DNA Technology for Production of Protein Therapeutics in Cultured Mammalian Cells. **D. L. HACKER** ; **F. M. WURM**. Reference Module in Life Sciences. 2017
- **GOEDDEL**. Gene Expression Technology: Methods in Enzymology. Academic Press, 1990, vol. 185
- **TIHOMIR S. DODEV et al.** A tool kit for rapid cloning and expression of recombinant antibodies. *Scientific Reports*, 2014, vol. 4 (5885)
- Solid Phase Peptide Synthesis. The Pierce Chemical Co., 1984
- **CHU et al.** *Roche Molecular Biologicals*, 2001
- **MURRAY et al.** *Current Opinion in Chemical Biology*, 2013, vol. 17, 420-426
- **D. WILKINSON**. The Scientist. Scientist, Inc., 17 April 2000, vol. 14, 25-28